(19) Europäisches Patentamt
European Patent Office
Office européen des brevets

(11) EP 4 755 887 A1

(12) # EUROPEAN PATENT APPLICATION
published in accordance with Art. 153(4) EPC

(43) Date of publication:
**10.06.2026 Bulletin 2026/24**

(21) Application number: **24848408.1**

(22) Date of filing: **05.08.2024**

(51) International Patent Classification (IPC):
***C07D 471/04*** (2006.01) ***A61K 31/437*** (2006.01)
***A61K 31/4375*** (2006.01) ***A61P 35/00*** (2006.01)
***A61P 37/06*** (2006.01)

(52) Cooperative Patent Classification (CPC):
**A61K 31/437; A61K 31/4375; A61P 35/00; A61P 37/06; C07D 471/04**

(86) International application number:
**PCT/CN2024/109822**

(87) International publication number:
**WO 2025/026455 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.08.2023 CN 202310971445**
**15.09.2023 CN 202311192377**
**08.11.2023 CN 202311478765**
**29.12.2023 CN 202311854055**
**22.03.2024 CN 202410335466**

(71) Applicant: **Haisco Pharmaceutical Group Co., Ltd.**
**Shannan, Tibet 856000 (CN)**

(72) Inventors:
- **ZHANG, Chen**
**Chengdu, Sichuan 611130 (CN)**
- **LIAO, Yuting**
**Chengdu, Sichuan 611130 (CN)**
- **XU, Jinxiong**
**Chengdu, Sichuan 611130 (CN)**
- **LU, Xianlin**
**Chengdu, Sichuan 611130 (CN)**
- **CHEN, Xiaogang**
**Chengdu, Sichuan 611130 (CN)**
- **LI, Sicheng**
**Chengdu, Sichuan 611130 (CN)**
- **TANG, Pingming**
**Chengdu, Sichuan 611130 (CN)**
- **TANG, Dachao**
**Chengdu, Sichuan 611130 (CN)**
- **CHENG, Xinfan**
**Chengdu, Sichuan 611130 (CN)**
- **LI, Yao**
**Chengdu, Sichuan 611130 (CN)**
- **YAN, Pangke**
**Chengdu, Sichuan 611130 (CN)**

(74) Representative: **AWA Denmark A/S**
**Strandgade 56**
**1401 Copenhagen K (DK)**

(54) # POLYCYCLIC FUSED-RING BTK DEGRADATION AGENT AND USE THEREOF IN MEDICINE

(57) A polycyclic fused-ring BTK degradation agent and a use thereof in medicine. Specifically, the present invention relates to a compound of general formula (I) or a stereoisomer, tautomer, solvate, prodrug, metabolite, pharmaceutically acceptable salt, or cocrystal thereof, an intermediate thereof and a preparation method therefor, and a use in BTK-related diseases, such as tumours or autoimmune system diseases, preferably relapsed/refractory B-cell malignancies.

B-L-K (I):

B selected from

EP 4 755 887 A1

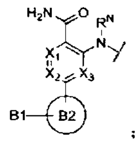

L selected from

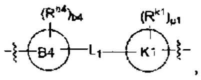

Left side connected to B , Right side connected to K: K selected from

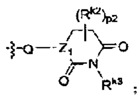

## Description

### Technical Field

The present invention belongs to the field of medicinal chemistry, and provides a polycyclic fused-ring BTK degradation agent and a use thereof in medicine. Specifically, the present invention relates to a compound of general formula (I) or a stereoisomer, tautomer, solvate, prodrug, metabolite, pharmaceutically acceptable salt, or co-crystal thereof, an intermediate thereof and a preparation method therefor, and a use in BTK-related diseases, such as tumours or autoimmune system diseases, preferably relapsed/refractory B-cell malignancies.

### Background Art

Bruton's tyrosine kinase (BTK), a member of the Tec family of non-receptor protein tyrosine kinases, is a key regulator in the B cell antigen receptor (BCR) signalling pathway, and is distributed in the lymphatic system, hematopoietic system and blood system. BTK mutations may activate downstream signalling pathways in tumour cell proliferation, differentiation, angiogenesis, etc., which may lead to X-linked agammaglobulinemia, non-Hodgkin's lymphoma (NHL) and many B-cell malignancies, including chronic lymphocytic leukaemia (CLL), mantle cell lymphoma, and diffuse large B-cell lymphoma. As mainly expressed in B cells and myeloid cells, BTK is a target with relatively high targeting ability and safety.

PROTAC (proteolysis targeting chimera) molecules are a class of bifunctional compounds that can simultaneously bind targeting proteins and E3 ubiquitin ligases. Such compounds can be recognised by proteasomes of cells, causing the degradation of targeting proteins, and can effectively reduce the content of targeting proteins in the cells. By introducing a ligand capable of binding to various targeting proteins into the PROTAC molecules, it is possible to apply the PROTAC technology to the treatment of various diseases, and this technology has attracted extensive attention in recent years.

Therefore, it is necessary to develop novel BTK-targeting PROTAC drugs for the treatment of BTK-related tumour diseases.

### Summary of the Invention

An objective of the present invention is to provide a compound with a novel structure, good efficacy, high bioavailability and higher safety that can inhibit or degrade BTK, for use in the treatment of BTK-related diseases such as tumours or autoimmune diseases, preferably B-cell malignancies, more preferably relapsed/refractory B-cell malignancies.

The compound of the present invention has good pharmacokinetic performance and bioavailability, oral performance and good safety.

The present invention provides a compound or a stereoisomer, tautomer, deuterated compound, solvate, prodrug, metabolite, pharmaceutically acceptable salt or co-crystal thereof, wherein the compound is a compound represented by general formula (I),

B-L-K (I);

in certain embodiments, the compound represented by general formula (I) is a compound represented by general formula (II),

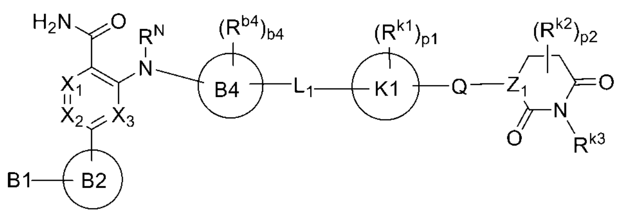

(II);

in certain embodiments, the compound represented by general formula (I) or (II) is a compound represented by general formula (III),

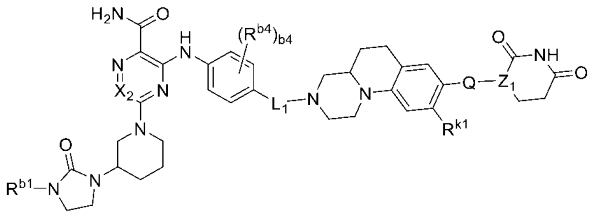

(III);

in certain embodiments, B is

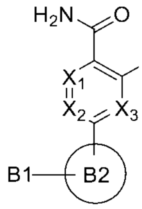

;

in certain embodiments, L is

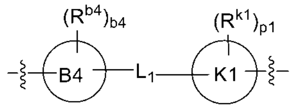

,

which is connected to B on the left side, and is connected to K on the right side;
in certain embodiments, K is

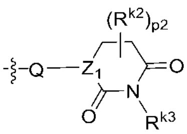

;

in certain embodiments, $X_1$, $X_2$, and $X_3$ are each independently selected from N or $CR^{b3}$;
in certain embodiments, B1 is absent or is selected from $C_{3-12}$ carbocyclyl or 4- to 12-membered heterocyclyl, wherein the carbocyclyl or heterocyclyl is optionally substituted with 1 to 4 $R^{b1}$;
in certain embodiments, B1 is absent or is selected from $C_{3-7}$ monocyclic carbocyclyl, $C_{5-12}$ fused carbocyclyl, $C_{5-12}$ spirocarbocyclyl, $C_{5-12}$ bridged carbocyclyl, 4- to 7-membered monocyclic heterocyclyl, 4- to 12-membered fused heterocyclyl, 5- to 12-membered spiroheterocyclyl, and 5- to 12-membered bridged heterocyclyl, wherein the carbocyclyl or heterocyclyl is optionally substituted with 1 to 4 $R^{b1}$;
in certain embodiments, B1 is absent or is selected from 4- to 6-membered heterocyclyl, cyclopropyl-spiro-5-membered heterocyclyl, cyclobutyl-spiro-5-membered heterocyclyl, 5,5-fused heterocyclyl, and 6,5-fused heterocyclyl, wherein the heterocyclyl is optionally substituted with 1 to 4 $R^{b1}$;
in certain embodiments, B1 is absent or is selected from the following groups optionally substituted with 1 to 2 $R^{b1}$:

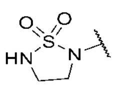 , 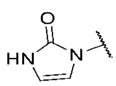 , 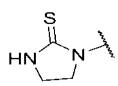 , 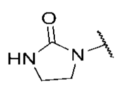 ,

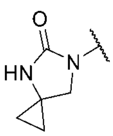 , 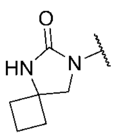 , 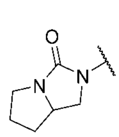 , 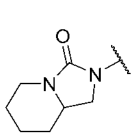 , 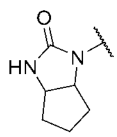 , 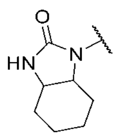 , 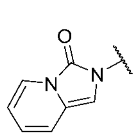 , 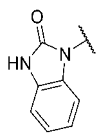 ,

and

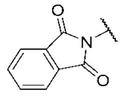;

in certain embodiments, B2 is selected from $C_{3-12}$ carbocyclyl or 4- to 12-membered heterocyclyl, wherein the carbocyclyl or heterocyclyl is optionally substituted with 1 to 4 $R^{b2}$;
in certain embodiments, B2 is selected from 4- to 7-membered monocyclic heterocyclyl, 4- to 12-membered fused heterocyclyl, 5- to 12-membered spiroheterocyclyl, and 5- to 12-membered bridged heterocyclyl, wherein the heterocyclyl is optionally substituted with 1 to 4 $R^{b2}$;
in certain embodiments, B2 is selected from 4- to 6-membered monocyclic heterocyclyl, 8- to 9-membered fused heterocyclyl, 7- to 11-membered spiroheterocyclyl, and 5- to 8-membered bridged heterocyclyl, wherein the heterocyclyl is optionally substituted with 1 to 4 $R^{b2}$;
in certain embodiments, B2 is selected from the following groups optionally substituted with 1 to 3 $R^{b2}$:

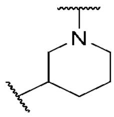, 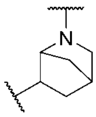, and 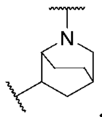,

which is connected to B on the right side;
in certain embodiments, B4 is selected from phenyl, 5- to 6-membered heteroaryl, a 9- to 12-membered fused bicyclic group,

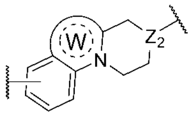,

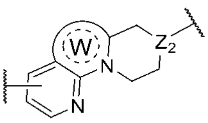, 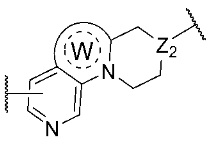, 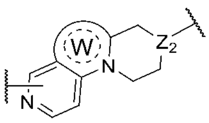, and 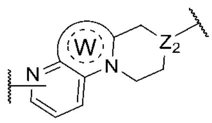; 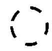

is selected from an aromatic or non-aromatic ring;
in certain embodiments, B4 is selected from phenyl, 5- to 6-membered heteroaryl,

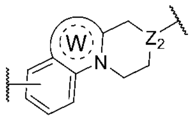, 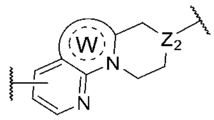, 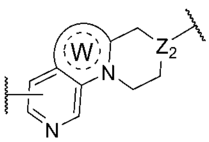, 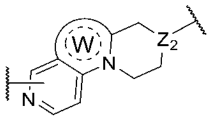,

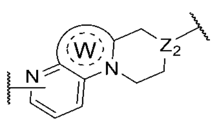,

benzofused 5-membered heterocyclyl, benzofused 6-membered heterocyclyl, benzofused 5-membered carbocyclyl, benzofused 6-membered carbocyclyl, benzofused 7-membered carbocyclyl, benzofused 8-membered carbocyclyl, pyridofused 5-membered heterocyclyl, and pyridofused 6-membered heterocyclyl;
in certain embodiments, B4 is selected from phenyl, pyrrolyl, pyrazolyl, imidazolyl, triazolyl, oxadiazolyl, furyl, thienyl, thiazolyl, isothiazolyl, thiadiazolyl, pyridyl, pyrimidyl, pyrazinyl, pyridazinyl, triazinyl,

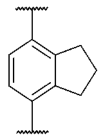, 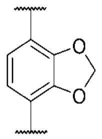, 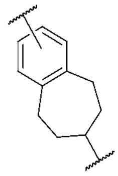,

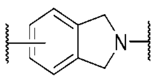, 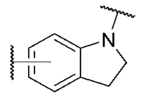, 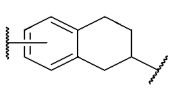, 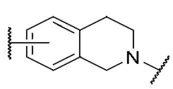, 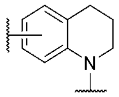, 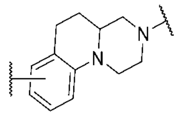,

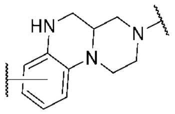, 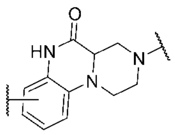, 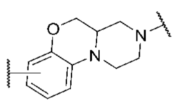, 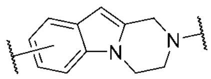, 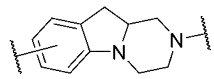,

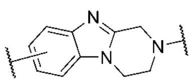, 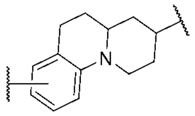, 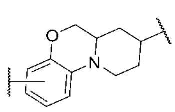, 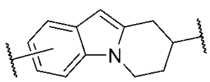, 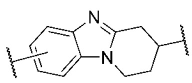,

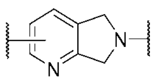, and 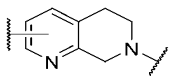;

in certain embodiments, K1 is selected from phenyl, 5- to 6-membered heteroaryl, a 9- to 12-membered fused bicyclic group,

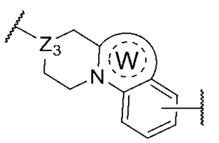,

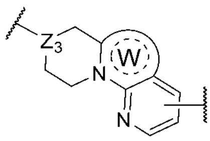, 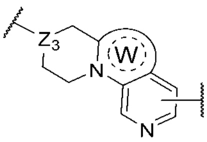, 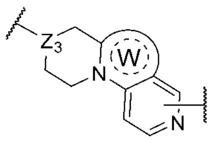, and 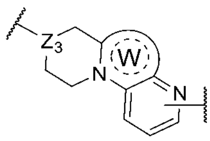; 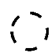

is selected from an aromatic or non-aromatic ring;

in certain embodiments, K1 is selected from phenyl, 5- to 6-membered heteroaryl,

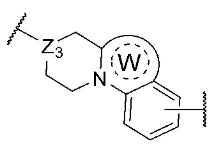, 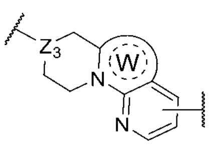, 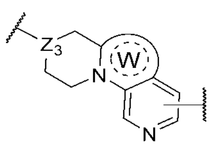, 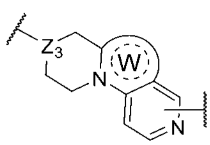,

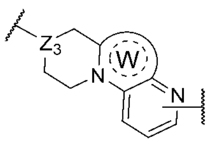,

benzofused 5-membered heterocyclyl, and benzofused 6-membered heterocyclyl;
in certain embodiments, K1 is selected from phenyl, pyrrolyl, pyrazolyl, imidazolyl, triazolyl, oxadiazolyl, furyl, thienyl, thiazolyl, isothiazolyl, thiadiazolyl, pyridyl, pyrimidyl, pyrazinyl, pyridazinyl, triazinyl,

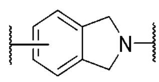, 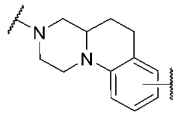,

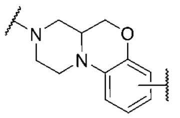, 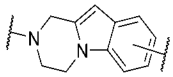, 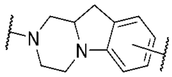, 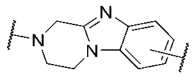, 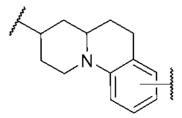,

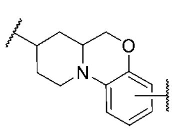, 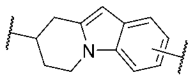, 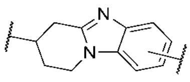, 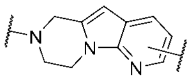, 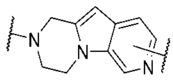,

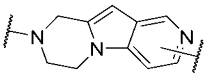, 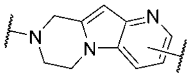, 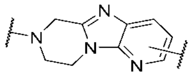, 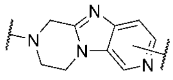, 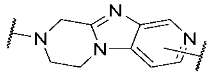,

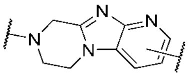, 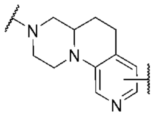, 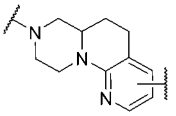, 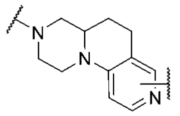, 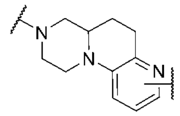,

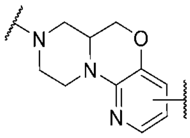, 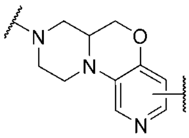, 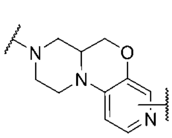, 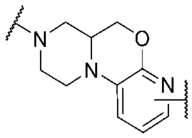, 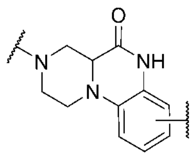,

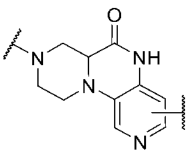, 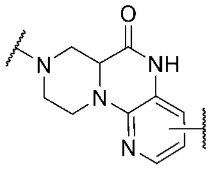, 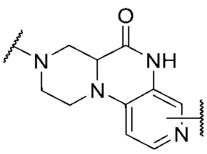, 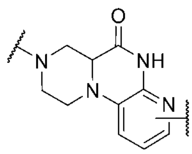, 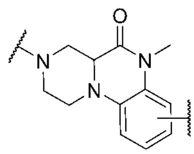,

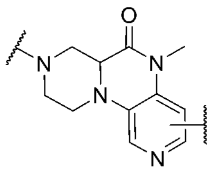, 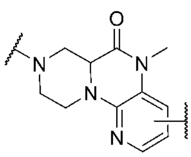, 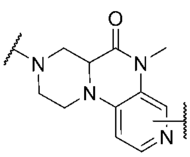, and 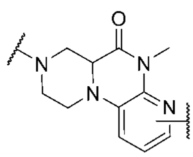;

in certain embodiments,

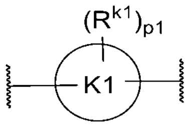

is selected from

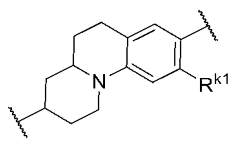,

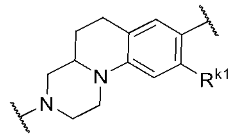, 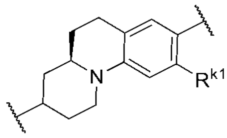, 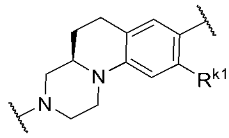, 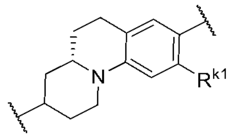,

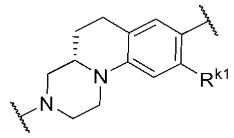, 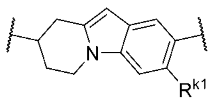, 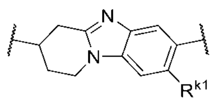, 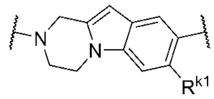,

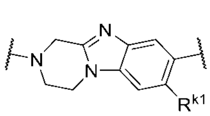, 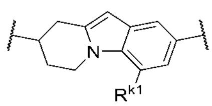, 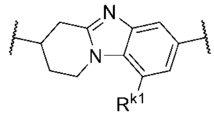, 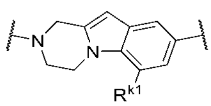,

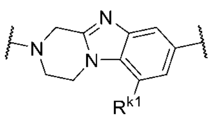, 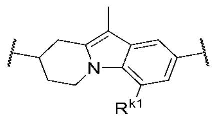, 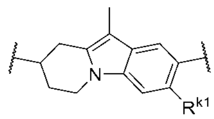, 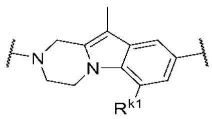,

and

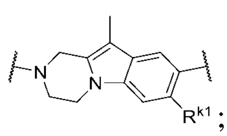;

in certain embodiments, W is 5- to 6-membered heterocyclyl;
in certain embodiments, $Z_2$ is selected from CH or N;
in certain embodiments, $Z_3$ is selected from CH or N;
in certain embodiments, $L_1$ is selected from -Ak1-, -Akl-Ak2-, -Cyl-, -Cy1-Cy2-, -Cy1-Ak1-, -Ak1-Cy1-, -Cy1-Ak1-Cy2-, -Cy1-Cy2-Ak1-, -Ak1-Cy1-Cy2-, - Cy1-Ak1-Cy2-Ak2-, and -Ak1-Cy1-Ak2-Cy2-;
in certain embodiments, Ak1 and Ak2 are each independently selected from a bond, $C_{1-8}$ alkylene, $C_{2-8}$ alkenylene, $C_{2-8}$ alkynylene, O, $NR^L$, $NR^LCO$, $CONR^L$, CO, and C≡C, wherein the alkylene, alkenylene, and alkynylene are optionally substituted with 1 to 4 substituents selected from deuterium, F, Cl, Br, I, OH, COOH, CN, $NH_2$, =O, $C_{1-6}$ alkyl, halogen-substituted $C_{1-6}$ alkyl, hydroxyl-substituted $C_{1-6}$ alkyl, $C_{1-6}$ alkoxy, $C_{3-7}$ carbocyclyl, and 4- to 6-membered heterocyclyl;
in certain embodiments, Ak1 and Ak2 are each independently selected from $C_{1-6}$ alkylene, $C_{2-6}$ alkenylene, $C_{2-6}$ alkynylene, O, $NR^L$, $NR^LCO$, $CONR^L$, and CO, wherein the alkylene, alkenylene, and alkynylene are optionally substituted with 1 to 4 substituents selected from deuterium, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl, $C_{1-4}$ alkoxy, $C_{3-6}$ carbocyclyl, and 4- to 6-membered heterocyclyl;
in certain embodiments, Ak1 and Ak2 are each independently selected from $C_{1-4}$ alkylene, $C_{2-4}$ alkenylene, $C_{2-4}$ alkynylene, O, $NR^L$, $NR^LCO$, and CO, wherein the alkylene, alkenylene, and alkynylene are optionally substituted with 1 to 4 substituents selected from deuterium, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl, $C_{1-4}$ alkoxy, $C_{3-6}$ carbocyclyl, and 4- to 6-membered heterocyclyl;
in certain embodiments, Ak1 and Ak2 are each independently selected from methylene, ethylene, vinylene, and ethynylene, wherein the methylene, ethylene, vinylene, and ethynylene are optionally substituted with 1 to 4 substituents selected from deuterium, F, Cl, Br, I, OH, $NH_2$, COOH, CN, =O, $C_{1-4}$ alkyl, halogen-substituted $C_{1-4}$

alkyl, hydroxyl-substituted $C_{1-4}$ alkyl, $C_{1-4}$ alkoxy, $C_{3-6}$ carbocyclyl, and 4- to 6-membered heterocyclyl;

in certain embodiments, Cyl and Cy2 are each independently selected from a bond, 3- to 7-membered monocyclic heterocyclyl, 4- to 12-membered fused heterocyclyl, 5- to 12-membered spiroheterocyclyl, 5- to 12-membered bridged heterocyclyl, $C_{3-7}$ monocyclic carbocyclyl, 4- to 12-membered fused carbocyclyl, 5- to 12-membered spirocarbocyclyl, 5- to 12-membered bridged carbocyclyl, 5- to 12-membered heteroaryl or 6- to 10-membered aryl, wherein the aryl, heteroaryl, carbocyclyl, and heterocyclyl are optionally substituted with 1 to 4 substituents selected from deuterium, F, Cl, Br, I, OH, COOH, $CONH_2$, CN, $NH_2$, =O, $C_{1-6}$ alkyl, halogen-substituted $C_{1-6}$ alkyl, hydroxyl-substituted $C_{1-6}$ alkyl, $C_{1-6}$ alkoxy, $C_{3-7}$ carbocyclyl, and 4- to 6-membered heterocyclyl;

in certain embodiments, Cy1 and Cy2 are each independently selected from 3-to 6-membered monocyclic heterocyclyl, 4- to 10-membered fused heterocyclyl, 5-to 11-membered spiroheterocyclyl, 5- to 10-membered bridged heterocyclyl, $C_{3-6}$ monocyclic carbocyclyl, 4- to 10-membered fused carbocyclyl, 5- to 11-membered spirocarbocyclyl, 5- to 10-membered bridged carbocyclyl, 5- to 10-membered heteroaryl or 6- to 10-membered aryl, wherein the aryl, heteroaryl, carbocyclyl, and heterocyclyl are optionally substituted with 1 to 4 substituents selected from deuterium, F, Cl, Br, I, OH, COOH, $CONH_2$, CN, $NH_2$, =O, $C_{1-4}$ alkyl, halogen-substituted $C_{1-4}$ alkyl, hydroxyl-substituted $C_{1-4}$ alkyl, $C_{1-4}$ alkoxy, $C_{3-6}$ carbocyclyl, and 4- to 6-membered heterocyclyl;

in certain embodiments, Cy1 and Cy2 are each independently selected from one of the following substituted or unsubstituted groups: cyclopropyl, cyclobutyl, cyclopentyl, cyclohexyl, azetidinyl, pyrrolidyl, piperidyl, morpholinyl, piperazinyl, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-pyrrolidyl, cyclopropyl-fused-piperidyl, cyclobutyl-fused-azetidinyl, cyclobutyl-fused-pyrrolidyl, cyclobutyl-fused-piperidyl, cyclopentyl-fused-azetidinyl, cyclopentyl-fused-pyrrolidyl, cyclopentyl-fused-piperidyl, cyclohexyl-fused-azetidinyl, cyclohexyl-fused-pyrrolidyl, cyclohexyl-fused-piperidyl, azetidinyl-fused-azetidinyl, azetidinyl-fused-pyrrolidyl, azetidinyl-fused-piperidyl, pyrrolidyl-fused-azetidinyl, pyrrolidyl-fused-pyrrolidyl, pyrrolidyl-fused-piperidyl, piperidyl-fused-azetidinyl, piperidyl-fused-pyrrolidyl, piperidyl-fused-piperidyl, cyclobutyl-spiro-azetidinyl, cyclobutyl-spiro-pyrrolidyl, cyclobutyl-spiro-piperidyl, cyclopentyl-spiro-azetidinyl, cyclopentyl-spiro-pyrrolidyl, cyclopentyl-spiro-piperidyl, cyclohexyl-spiro-azetidinyl, cyclohexyl-spiro-pyrrolidyl, cyclohexyl-spiro-piperidyl, azetidinyl-spiro-azetidinyl, azetidinyl-spiro-pyrrolidyl, azetidinyl-spiro-piperidyl, pyrrolidyl-spiro-azetidinyl, pyrrolidyl-spiro-pyrrolidyl, pyrrolidyl-spiro-piperidyl, piperidyl-spiro-azetidinyl, piperidyl-spiro-pyrrolidyl, piperidyl-spiro-piperidyl,

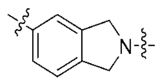 , 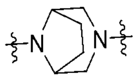 , 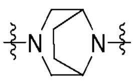 , 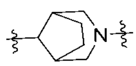 , 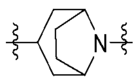 ,

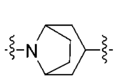 , 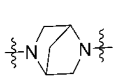 , 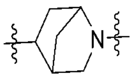 , 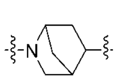 , 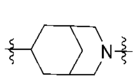 , 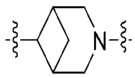 , 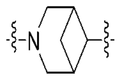 ,

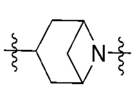 , 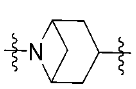 , 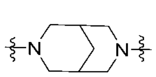 , 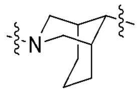 , 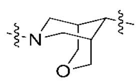 , 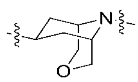

or

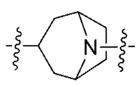 ,

which, when substituted, are optionally substituted with 1 to 4 substituents selected from deuterium, F, Cl, Br, I, OH, $NH_2$, COOH, $CONH_2$, CN, =O, $C_{1-4}$ alkyl, halogen-substituted $C_{1-4}$ alkyl, hydroxyl-substituted $C_{1-4}$ alkyl, $C_{1-4}$ alkoxy, $C_{3-7}$ carbocyclyl, and 4- to 6-membered heterocyclyl;

in certain embodiments, Cy1 and Cy2 are each independently selected from one of the following substituted or unsubstituted groups:

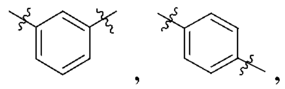,

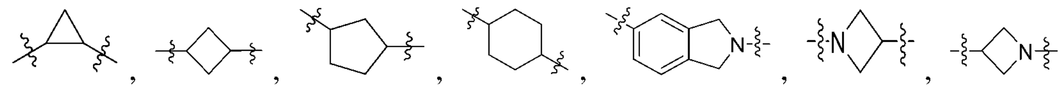,

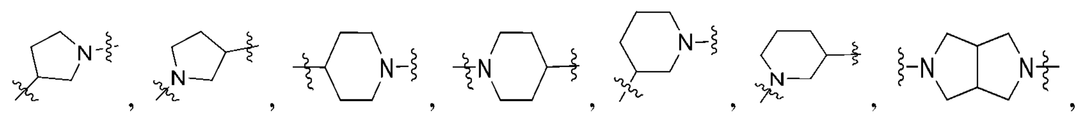,

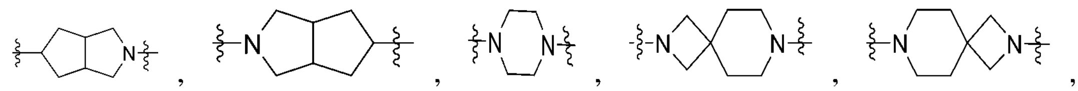,

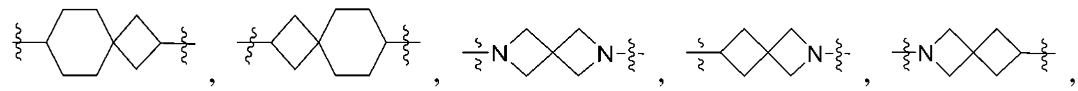,

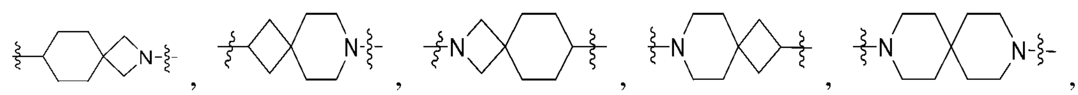,

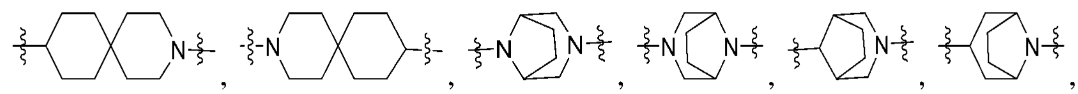,

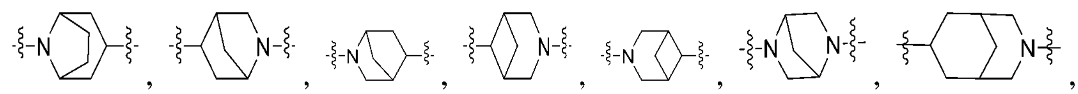,

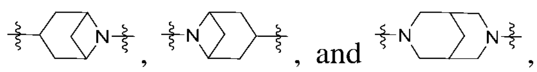,

which, when substituted, are optionally substituted with 1 to 4 substituents selected from deuterium, F, Cl, Br, I, OH, =O, $NH_2$, CN, $CONH_2$, COOH, $C_{1-4}$ alkyl, halogen-substituted $C_{1-4}$ alkyl, hydroxyl-substituted $C_{1-4}$ alkyl, $C_{1-4}$ alkoxy, $C_{3-7}$ carbocyclyl, and 4- to 6-membered heterocyclyl;

in certain embodiments, each $R^L$ or $R^N$ is independently selected from H or $C_{1-6}$ alkyl;

in certain embodiments, each $R^L$ or $R^N$ is independently H or $C_{1-4}$ alkyl;

in certain embodiments, each $R^L$ or $R^N$ is independently selected from H, methyl or ethyl;

in certain embodiments, Q is selected from $NR^qCO$, $CONR^q$, CO, $NR^q$ or a bond;

in certain embodiments, Q is selected from $CONR^q$, $NR^qCO$, $NR^q$ or a bond;

in certain embodiments, Zi is selected from N or $CR^{k2}$;

in certain embodiments, $R^{b1}$, $R^{b2}$, $R^{b3}$, $R^{b4}$, $R^{k1}$, $R^{k2}$, $R^{k3}$, and $R^q$ are each independently selected from H, deuterium, F, Cl, Br, I, OH, =O, =S, $NH_2$, CN, COOH, $CONH_2$, $C_{1-6}$ alkyl, $C_{1-6}$ alkoxy, $C_{3-7}$ carbocyclyl, 4- to 6-membered heterocyclyl, -O-$C_{3-7}$ carbocyclyl, and -O-4- to 6-membered heterocyclyl, wherein the alkyl, alkoxy, heterocyclyl, and carbocyclyl are optionally substituted with 1 to 6 substituents selected from deuterium, F, Cl, Br, I, OH, =O, $NH_2$, CN, $CONH_2$, COOH, $C_{1-6}$ alkyl, $C_{1-6}$ alkoxy, $C_{3-7}$ carbocyclyl, 4- to 6-membered heterocyclyl, - O-$C_{3-7}$ carbocyclyl, -O-4- to 6-membered heterocyclyl, fluoro-substituted $C_{3-7}$ carbocyclyl, $C_{2-6}$ alkynyl, and fluoro-substituted phenyl;

in certain embodiments, $R^{b1}$, $R^{b2}$, $R^{b3}$, $R^{b4}$, $R^{k1}$, $R^{k2}$, $R^{k3}$, and $R^q$ are each independently selected from H, deuterium, F, Cl, Br, I, OH, =O, =S, $NH_2$, CN, COOH, $CONH_2$, $C_{1-4}$ alkyl, $C_{1-4}$ alkoxy, $C_{3-6}$ carbocyclyl, 4- to 6-membered heterocyclyl, -O-$C_{3-6}$ carbocyclyl, and -O-4- to 6-membered heterocyclyl, wherein the alkyl, alkoxy, heterocyclyl, and carbocyclyl are optionally substituted with 1 to 6 substituents selected from deuterium, F, Cl, Br, I, OH, =O, $NH_2$, CN, $CONH_2$, COOH, $C_{1-4}$ alkyl, $C_{1-4}$ alkoxy, $C_{3-7}$ carbocyclyl, 4- to 6-membered heterocyclyl, - O-$C_{3-7}$ carbocyclyl, -O-4- to 6-membered heterocyclyl, fluoro-substituted $C_{3-7}$ carbocyclyl, $C_{2-6}$ alkynyl, and fluoro-substituted phenyl;

in certain embodiments, $R^{b1}$, $R^{b2}$, $R^{b3}$, $R^{b4}$, $R^{k1}$, $R^{k2}$, $R^{k3}$, and $R^q$ are each independently selected from H, deuterium, F, Cl, Br, I, OH, =O, =S, $NH_2$, CN, COOH, $CONH_2$, methyl, ethyl, propyl, isopropyl, butyl, tert-butyl, methoxy, ethoxy, propoxy, isopropyloxy, cyclopropyl, cyclobutyl, cyclopentyl, cyclohexyl, phenyl, azetidinyl, oxetanyl, tetrahydrofuryl, oxanyl, pyrrolidyl, pyrrolyl, pyrazolyl, pyridyl, -O-cyclopropyl, -O-cyclobutyl, -O-cyclopentyl, -O-cyclohexyl, and -O-phenyl, wherein the methyl, ethyl, propyl, isopropyl, butyl, tert-butyl, methoxy, ethoxy, propoxy, isopropyloxy, cyclopropyl, cyclobutyl, cyclopropyl, cyclobutyl, cyclopentyl, cyclohexyl, phenyl, azetidinyl, oxetanyl, tetrahydrofuryl, oxanyl, pyrrolidyl, pyrrolyl, pyrazolyl, and pyridyl are optionally substituted with 1 to 6 substituents selected from deuterium, F, Cl, Br, I, OH, =O, $NH_2$, CN, $CONH_2$, COOH, $C_{1-4}$ alkyl, $C_{1-4}$ alkoxy, $C_{3-7}$ carbocyclyl, 4- to 6-membered heterocyclyl, - O-$C_{3-7}$ carbocyclyl, -O-4- to 6-membered heterocyclyl, fluoro-substituted $C_{3-7}$ carbocyclyl, $C_{2-6}$ alkynyl, and fluoro-substituted phenyl;

in certain embodiments, each $R^{b1}$ is independently selected from H, deuterium, F, Cl, Br, I, OH, =O, =S, $NH_2$, CN, COOH, $CONH_2$, $CF_3$, $CD_3$, $CHF_2$, $CH_2F$, methyl, ethyl, propyl, isopropyl, butyl, tert-butyl, methoxy, ethoxy, propoxy, isopropyloxy, cyclopropyl, cyclobutyl, cyclopentyl, cyclohexyl, phenyl, pyridyl, oxetanyl, tetrahydrofuryl, oxanyl, -$CH_2$-cyclopropyl, -$CH_2CF_3$, fluoro-substituted phenyl,

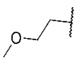, 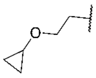, 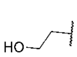, 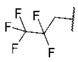, 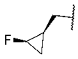, 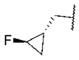, 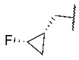, 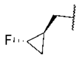, 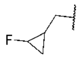, 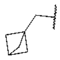

or

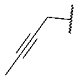;

in certain embodiments, $R^{b1}$ is independently selected from $CF_3$, $CD_3$, $CHF_2$, $CH_2F$, methyl, ethyl, propyl, isopropyl, butyl, tert-butyl, cyclopropyl, cyclobutyl, cyclopentyl, cyclohexyl, phenyl, pyridyl, oxetanyl, tetrahydrofuryl, oxanyl, -$CH_2$-cyclopropyl, -$CH_2CF_3$, fluoro-substituted phenyl,

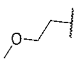, 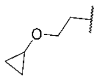, 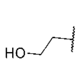, 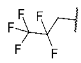,

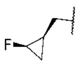, 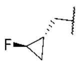, 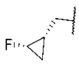, 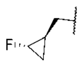, 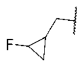, 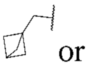 or 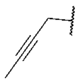;

in certain embodiments, $R^{b2}$, $R^{b3}$, $R^{b4}$, $R^{k1}$, and $R^{k2}$ are each independently selected from H, deuterium, F, Cl, Br, I, OH, =O, $NH_2$, CN, COOH, $CONH_2$, $CF_3$, $CHF_2$, $CH_2F$, methyl, ethyl, propyl, isopropyl, butyl, tert-butyl, methoxy, ethoxy, propoxy, isopropyloxy, cyclopropyl, and $CH_2OH$;

in certain embodiments, $R^{b4}$ and $R^{k1}$ are each independently selected from H, deuterium, F, Cl, Br, I, OH, $NH_2$, CN, COOH, $CONH_2$, $CF_3$, $CHF_2$, $CH_2F$, methyl, ethyl, propyl, isopropyl, butyl, tert-butyl, methoxy, ethoxy, propoxy, isopropyloxy, cyclopropyl, and $CH_2OH$;

in certain embodiments, $R^{b4}$ and $R^{k1}$ are each independently selected from H, deuterium, and F;

in certain embodiments, each $R^{k3}$ is independently selected from H, deuterium, and methyl;

in certain embodiments, each $R^q$ is independently selected from H, deuterium, and methyl;

in certain embodiments, each b4 is independently selected from 0, 1, 2, 3, 4, 5 or 6;

in certain embodiments, b4 is selected from 0 or 1;

in certain embodiments, each p1 or p2 is independently selected from 0, 1, 2, 3 or 4;

in certain embodiments, $L_1$ is selected from one of the fragments shown in Table L1-1;

Table L1-1

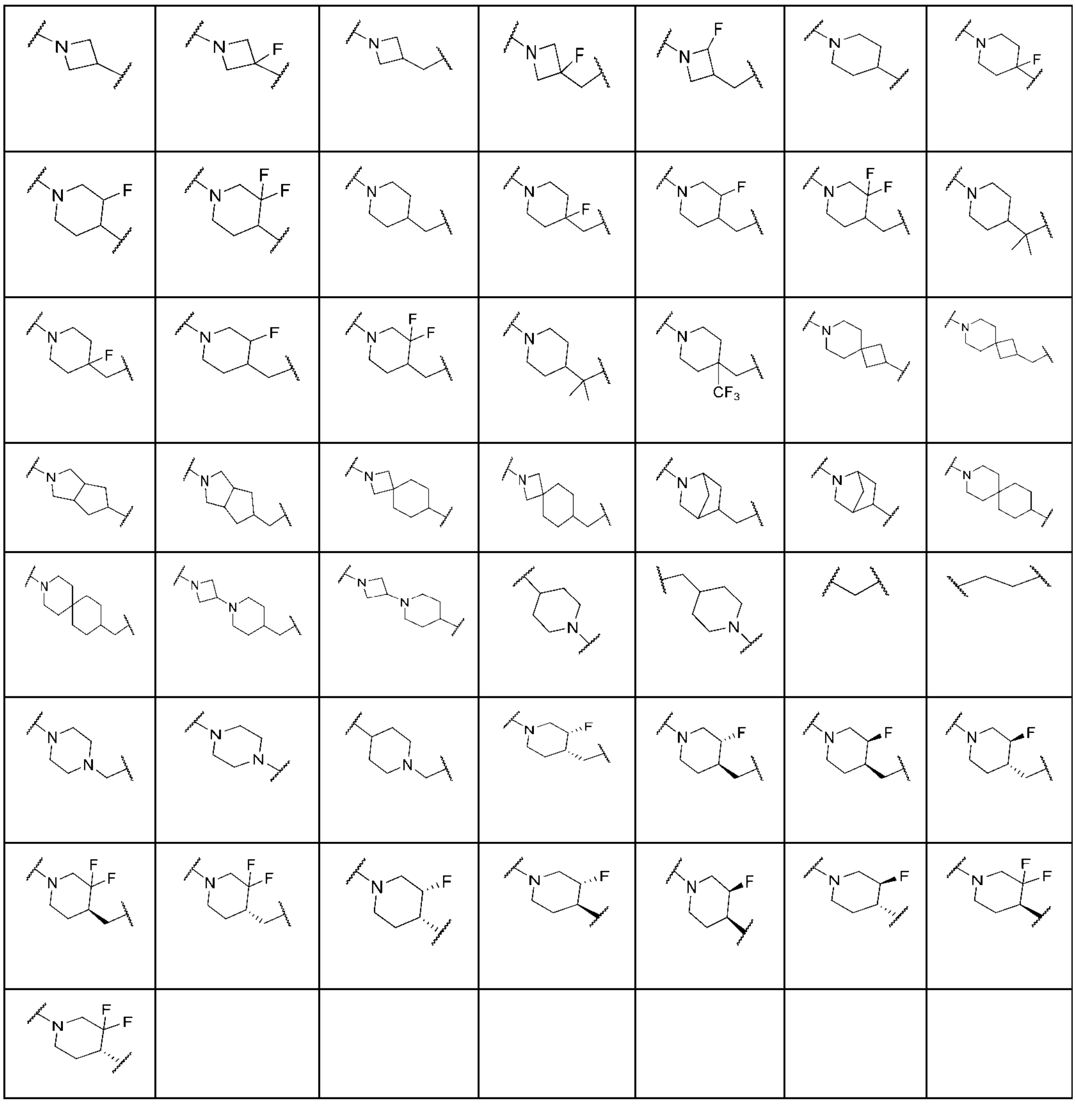

;

in certain embodiments, L is selected from one of the fragments shown in Table A;

Table A

| | | | |
|---|---|---|---|
| 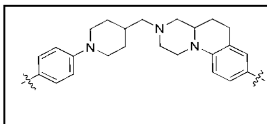 | 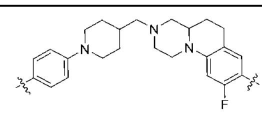 | 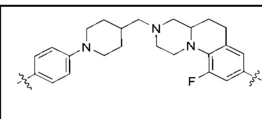 | 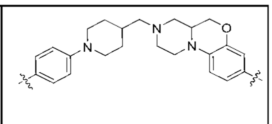 |
| 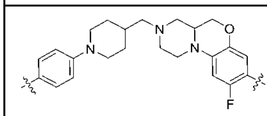 | 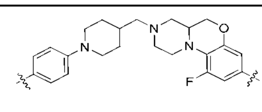 | 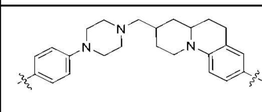 | 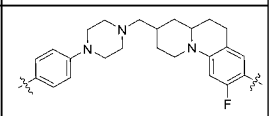 |
| 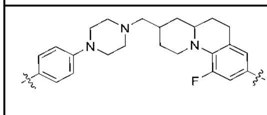 | 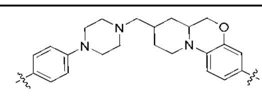 | 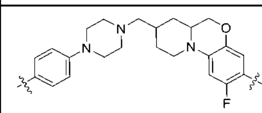 | 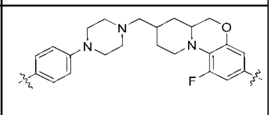 |

| | | | |
|---|---|---|---|
| 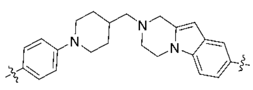 | 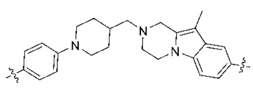 | 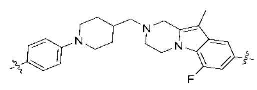 | 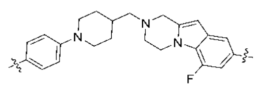 |
| 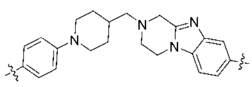 | 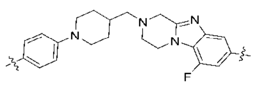 | 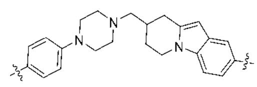 | 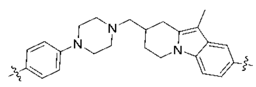 |
| 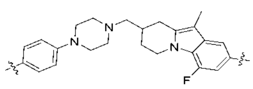 | 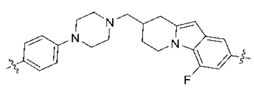 | 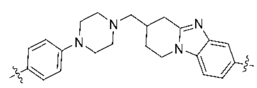 | 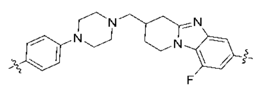 |
| 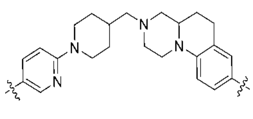 | 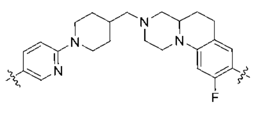 | 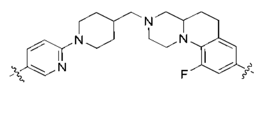 | 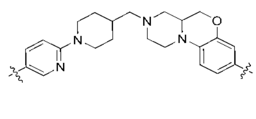 |
| 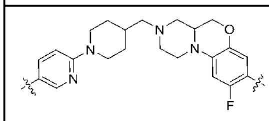 | 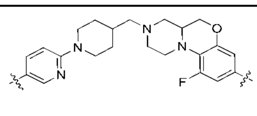 | 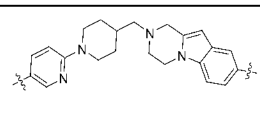 | 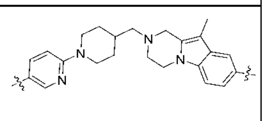 |
| 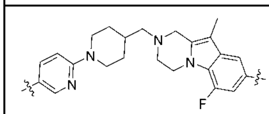 | 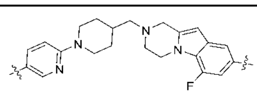 | 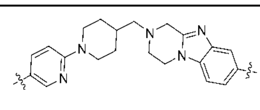 | 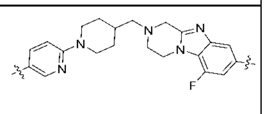 |
| 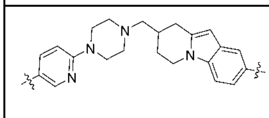 | 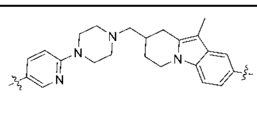 | 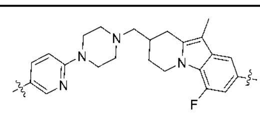 | 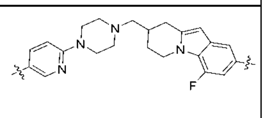 |
| 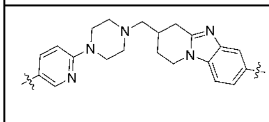 | 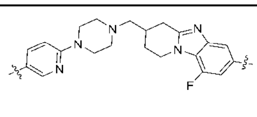 | 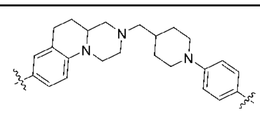 | 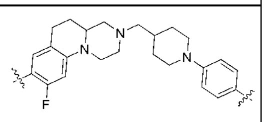 |
| 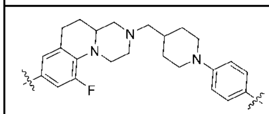 | 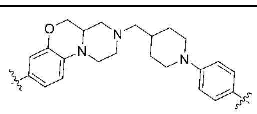 | 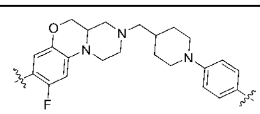 | 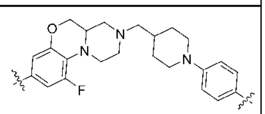 |
| 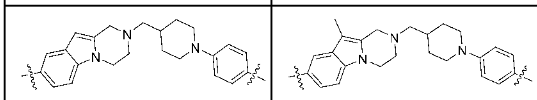 | | 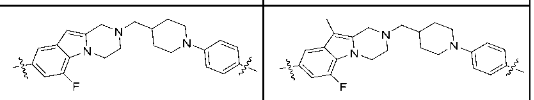 | |
| 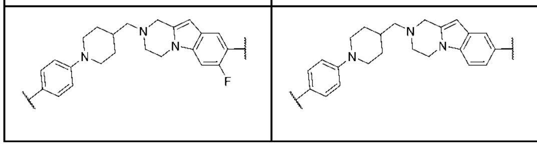 | | 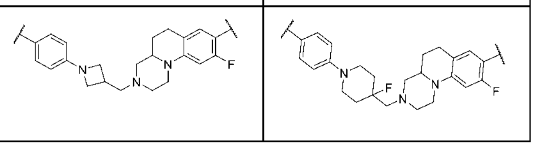 | |

| | | | |
|---|---|---|---|
| 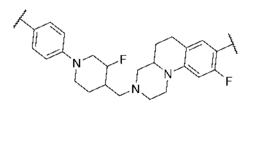 | 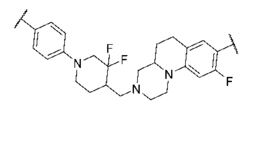 | 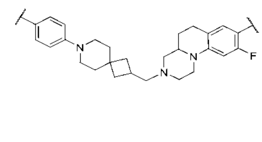 | 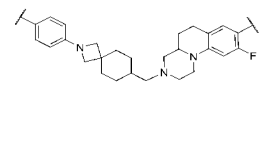 |
| 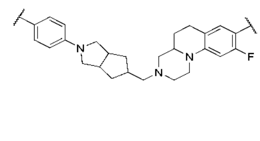 | 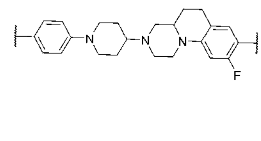 | 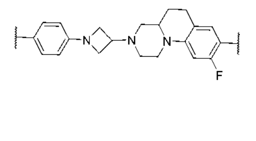 | 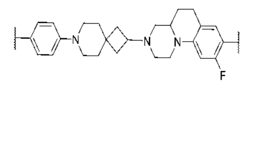 |
| 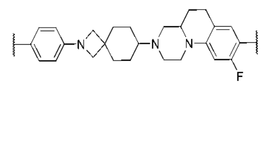 | 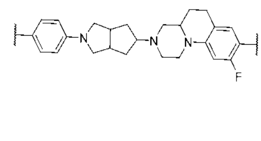 | 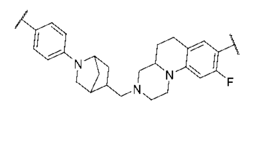 | 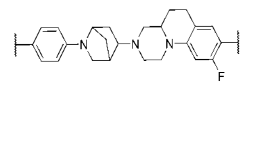 |
| 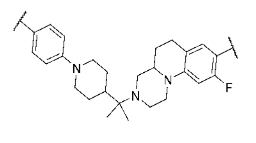 | 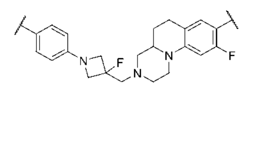 | 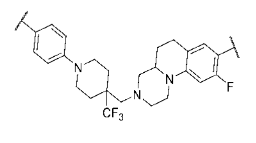 | 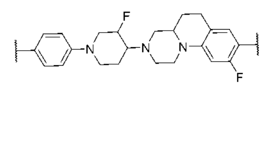 |
| 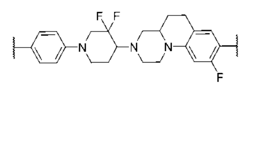 | 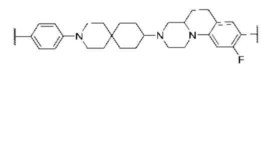 | 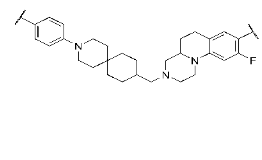 | 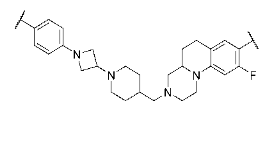 |
| 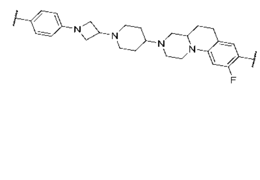 | 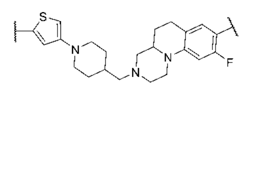 | 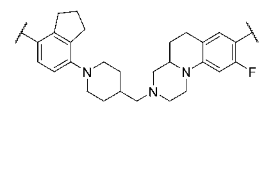 | 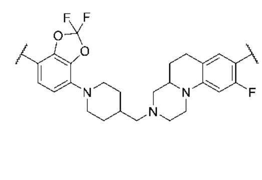 |
| 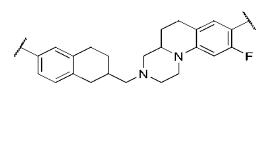 | 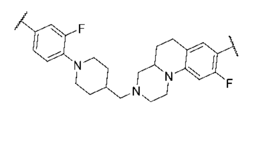 | 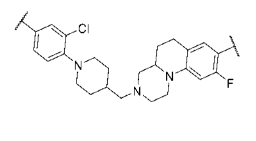 | 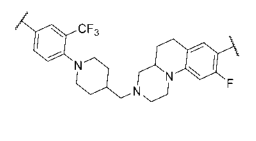 |
| 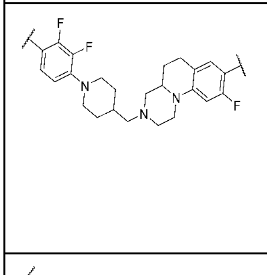 | 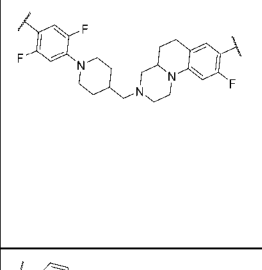 | 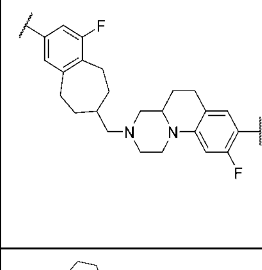 | 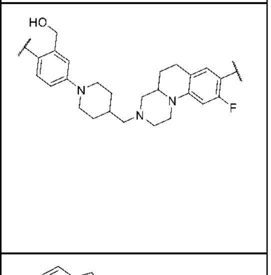 |
| 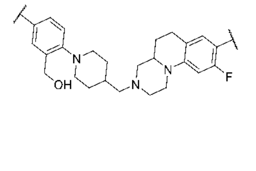 | 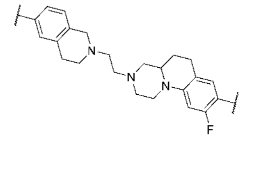 | 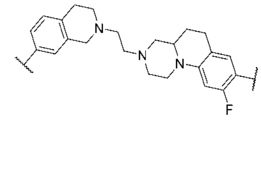 | 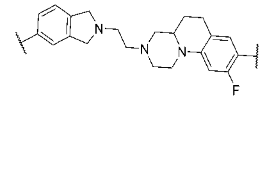 |
| 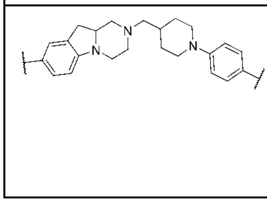 | 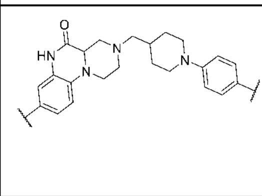 | 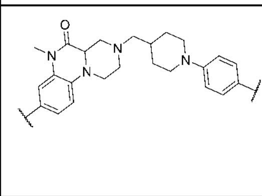 | 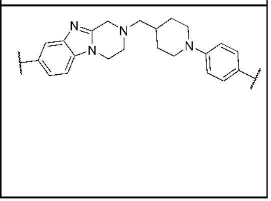 |

| | | | |
|---|---|---|---|
| 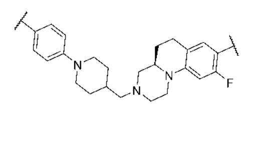 | 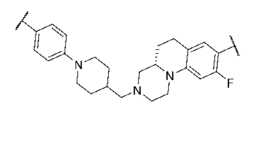 | 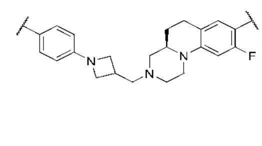 | 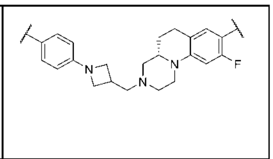 |
| 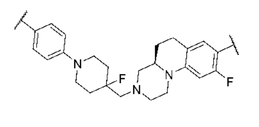 | 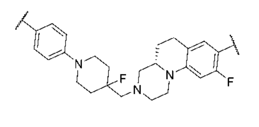 | 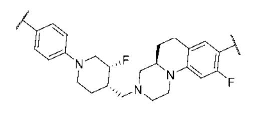 | 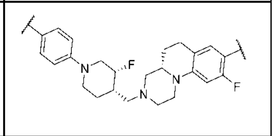 |
| 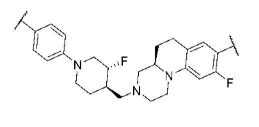 | 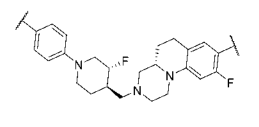 | 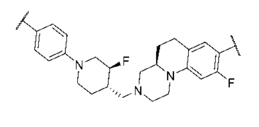 | 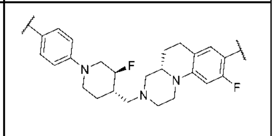 |
| 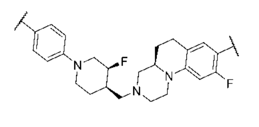 | 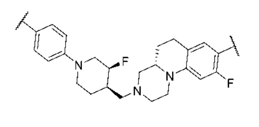 | 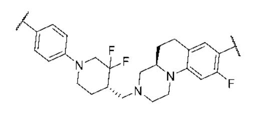 | 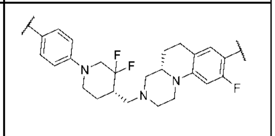 |
| 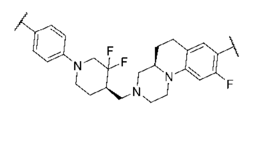 | 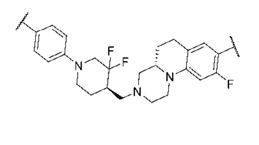 | 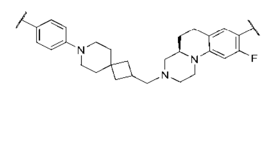 | 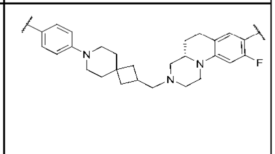 |
| 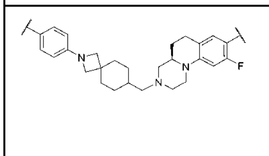 | 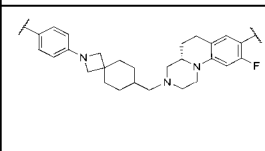 | 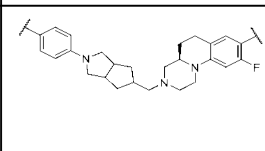 | 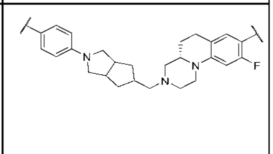 |
| 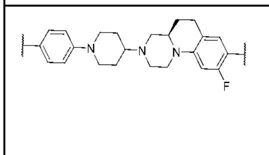 | 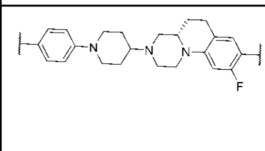 | 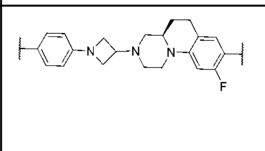 | 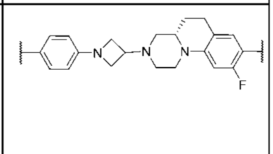 |
| 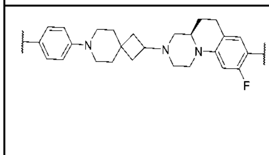 | 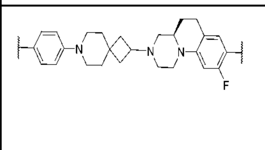 | 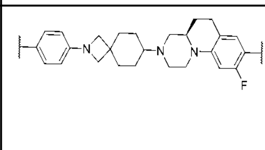 | 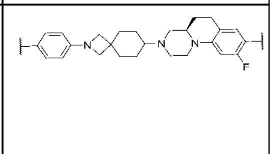 |
| 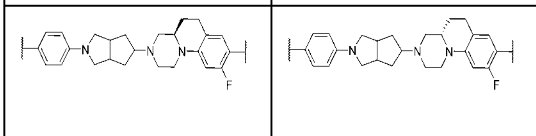 | | 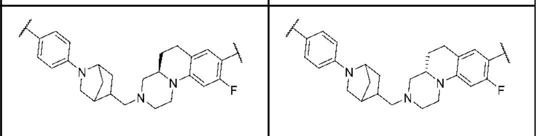 | |
| 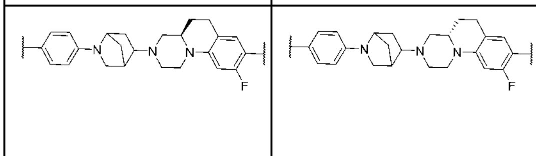 | | 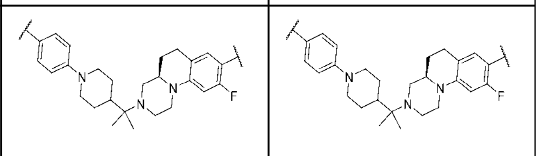 | |
| 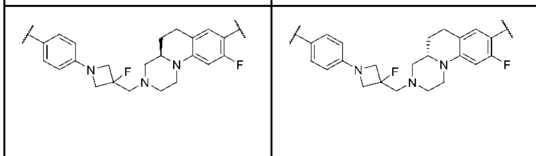 | | 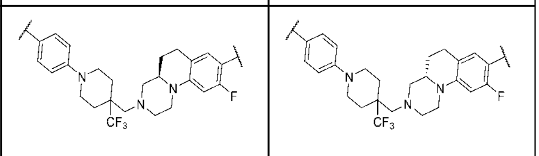 | |
| 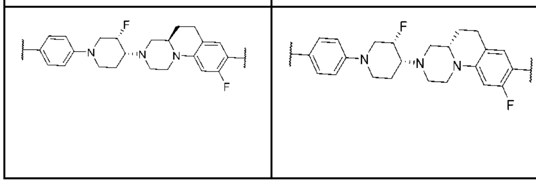 | | 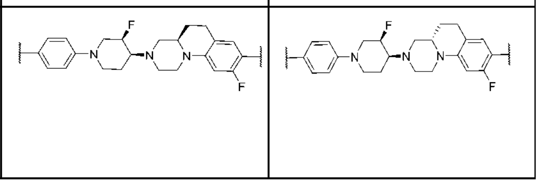 | |

| | | | |
|---|---|---|---|
| 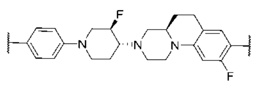 | 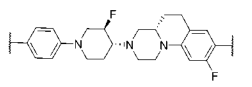 | 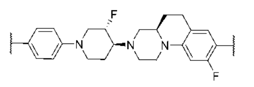 | 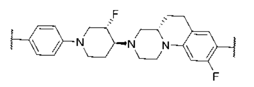 |
| 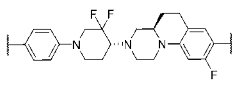 | 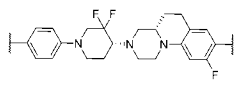 | 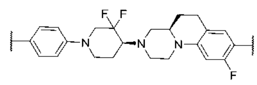 | 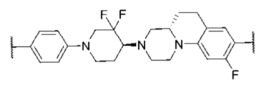 |
| 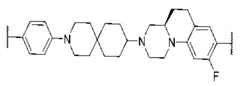 | 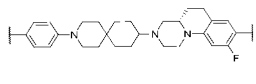 | 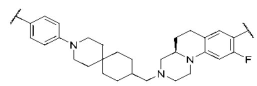 | 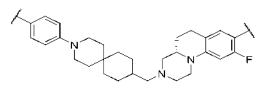 |
| 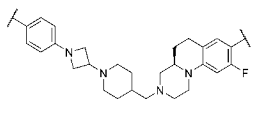 | 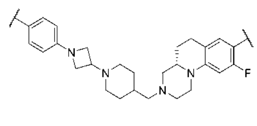 | 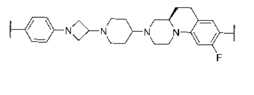 | 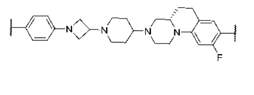 |
| 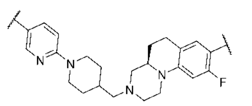 | 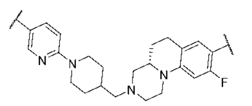 | 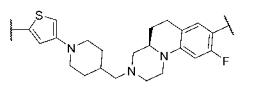 | 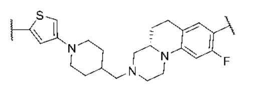 |
| 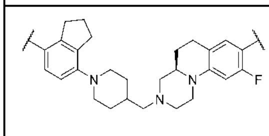 | 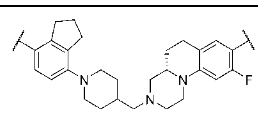 | 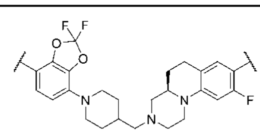 | 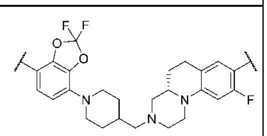 |
| 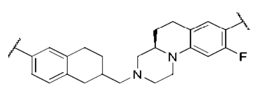 | 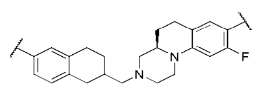 | 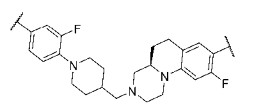 | 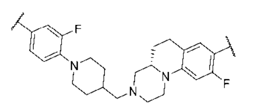 |
| 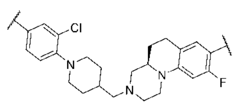 | 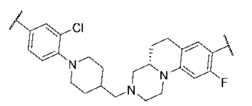 | 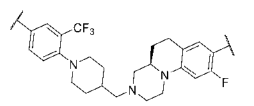 | 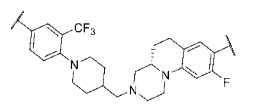 |
| 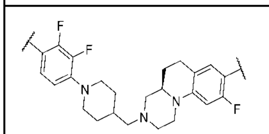 | 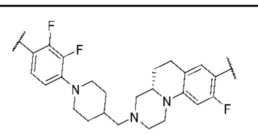 | 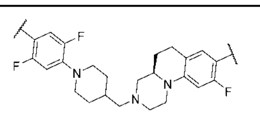 | 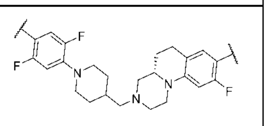 |
| 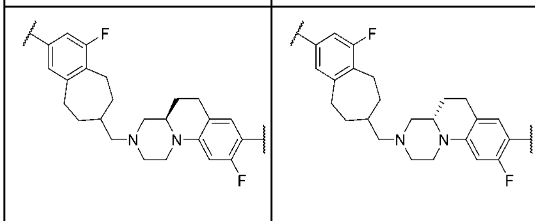 | | 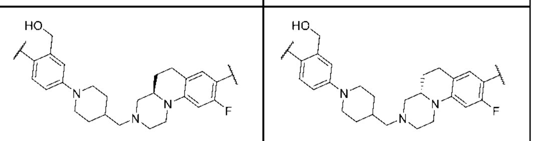 | |
| 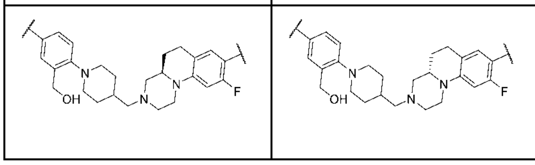 | | 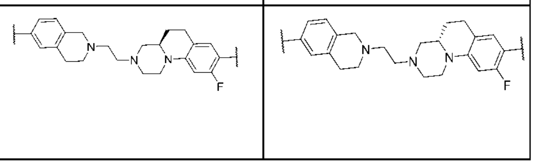 | |

| | | | |
|---|---|---|---|
| 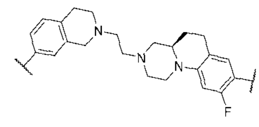 | 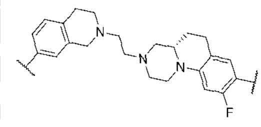 | 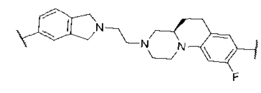 | 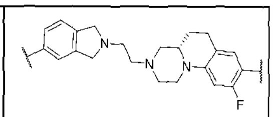 |
| 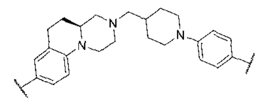 | 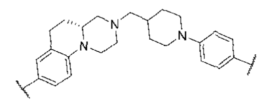 | 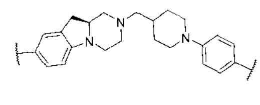 | 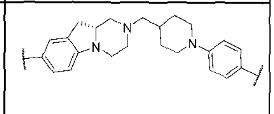 |
| 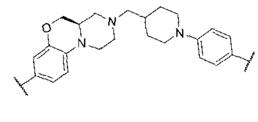 | 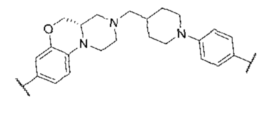 | 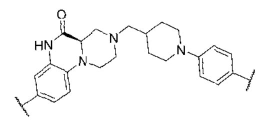 | 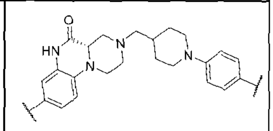 |
| 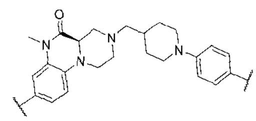 | 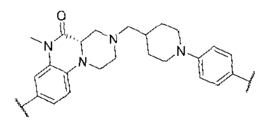 | 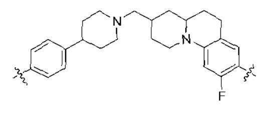 | 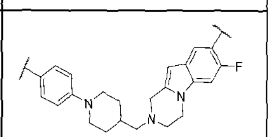 |
| 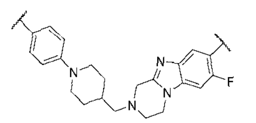 | 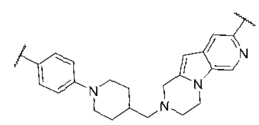 | 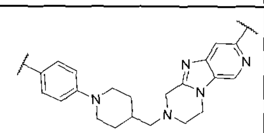 | 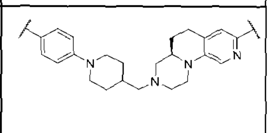 |
| 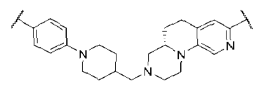 | 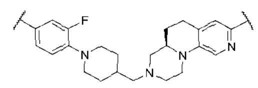 | 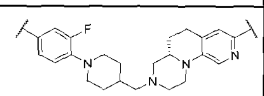 | 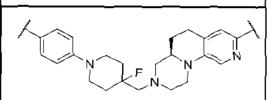 |
| 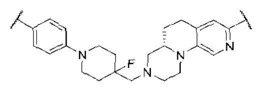 | 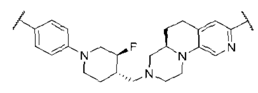 | 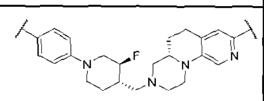 | 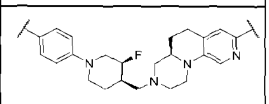 |
| 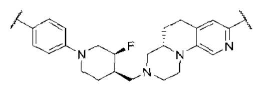 | 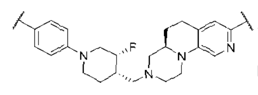 | 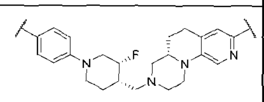 | 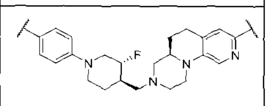 |
| 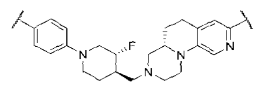 | 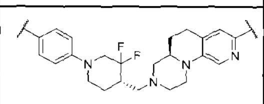 | 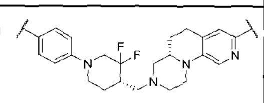 | 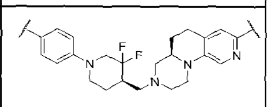 |
| 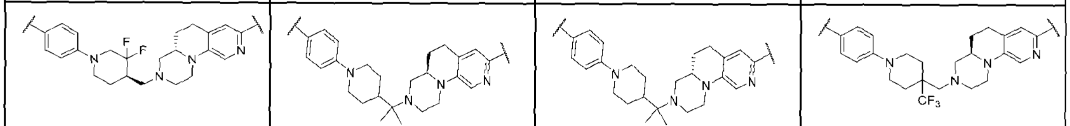 | | | |
| 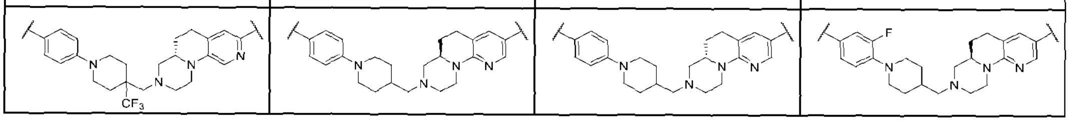 | | | |

| | | | |
|---|---|---|---|
| 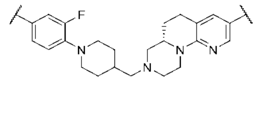 | 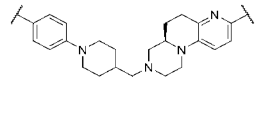 | 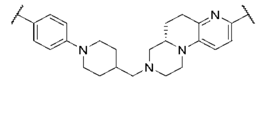 | 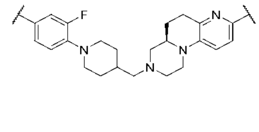 |
| 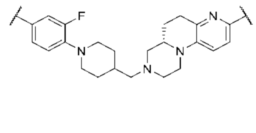 | 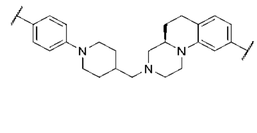 | 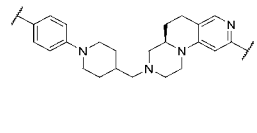 | 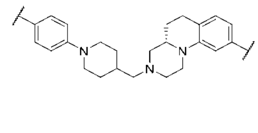 |
| 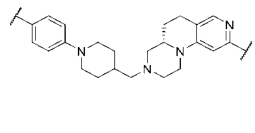 | 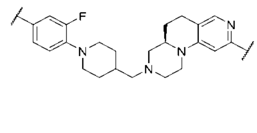 | 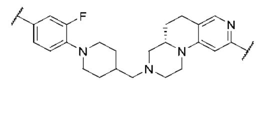 | 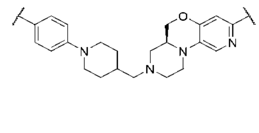 |
| 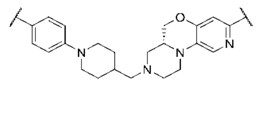 | 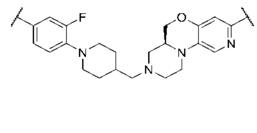 | 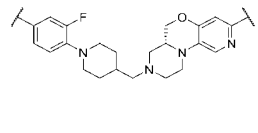 | 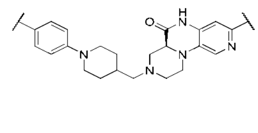 |
| 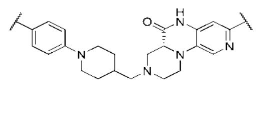 | 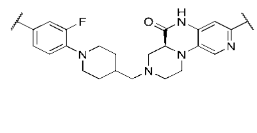 | 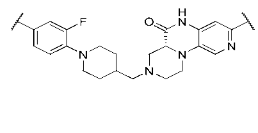 | 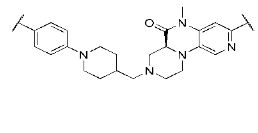 |
| 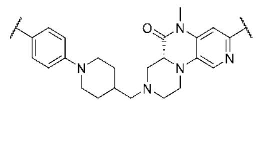 | 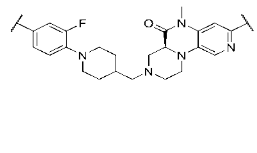 | 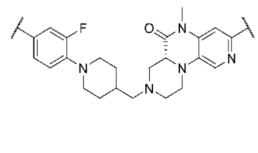 | 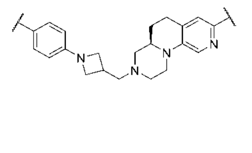 |
| 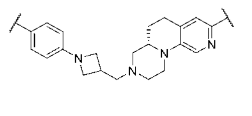 | 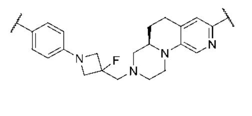 | 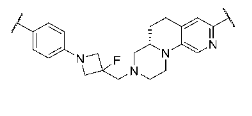 | 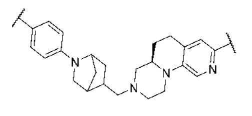 |
| 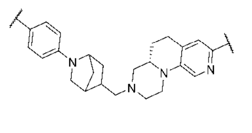 | 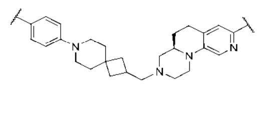 | 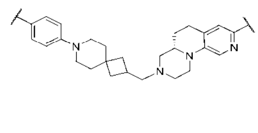 | 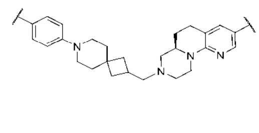 |
| 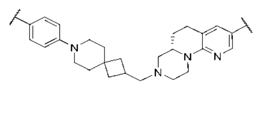 | 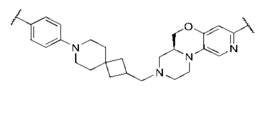 | 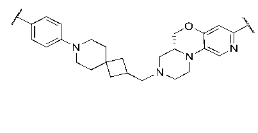 | 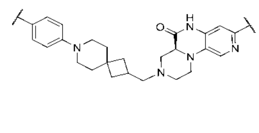 |
| 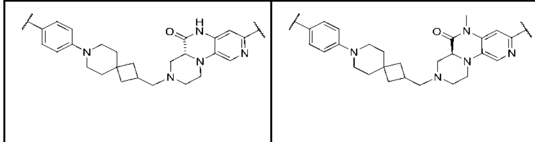 | | 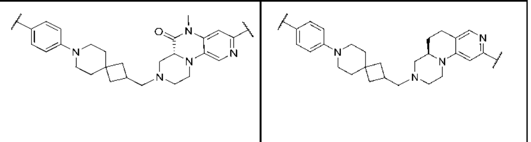 | |
| 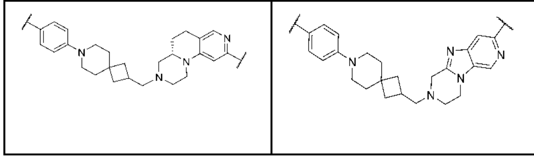 | | 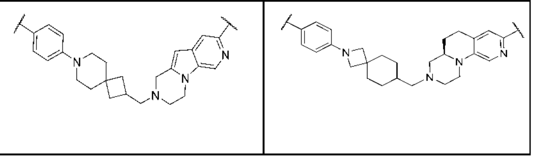 | |

| | | | |
|---|---|---|---|
| 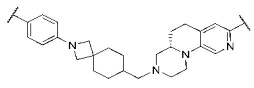 | 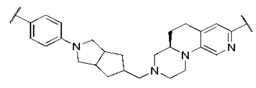 | 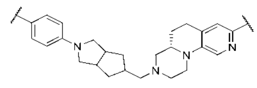 | 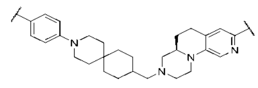 |
| 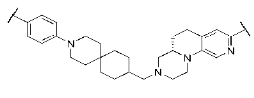 | 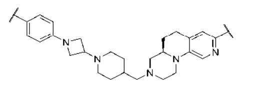 | 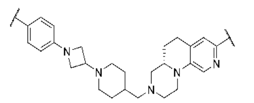 | 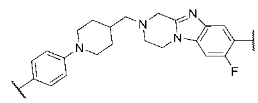 |
| 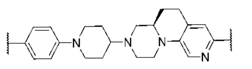 | 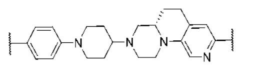 | 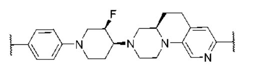 | 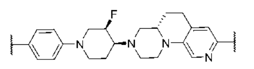 |
| 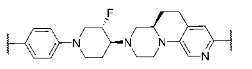 | 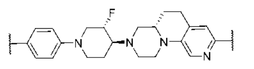 | 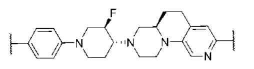 | 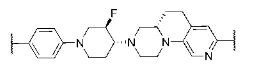 |
| 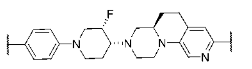 | 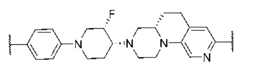 | 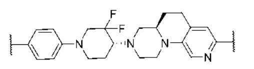 | 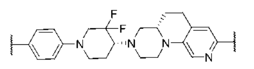 |
| 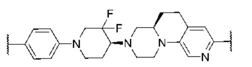 | 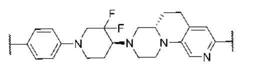 | 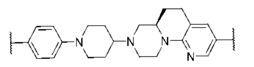 | 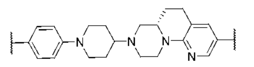 |
| 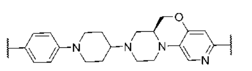 | 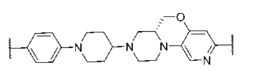 | 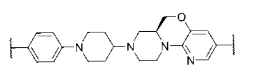 | 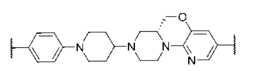 |
| 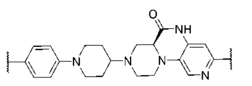 | 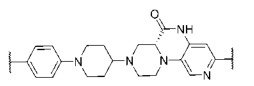 | 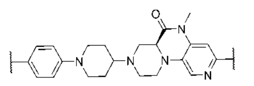 | 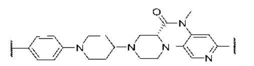 |
| 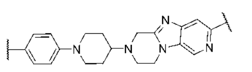 | 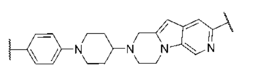 | 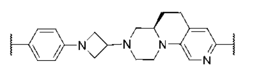 | 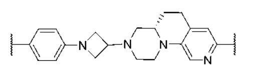 |
| 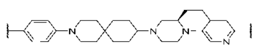 | 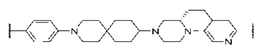 | 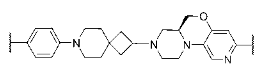 | 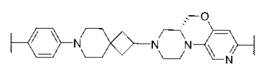 |
| | | | |
| | | | |
| | | | |
| | | | |
| | | | |

;

in certain embodiments, B is selected from one of the structural fragments shown in Table B-1;

Table B-1

| | | | | | | |
|---|---|---|---|---|---|---|
| 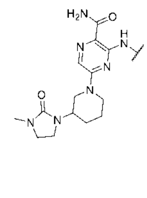 | 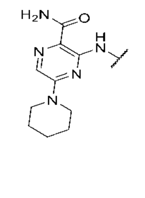 | 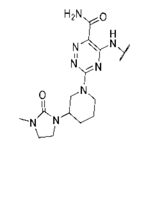 | 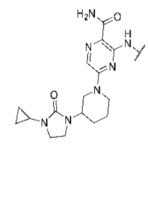 | 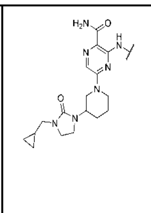 | 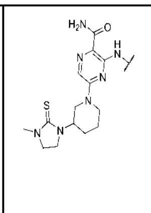 | 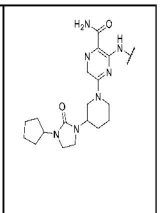 |
| 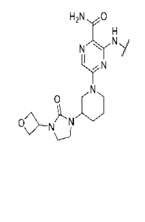 | 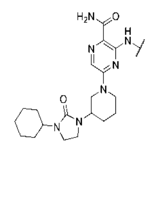 | 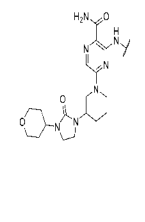 | 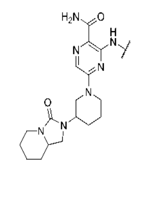 | 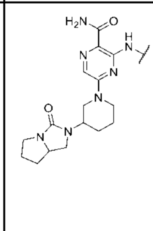 | 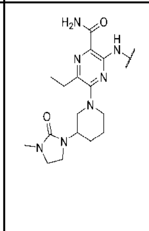 | 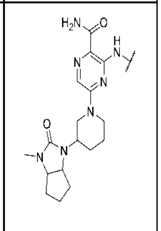 |
| 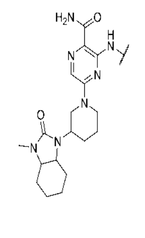 | 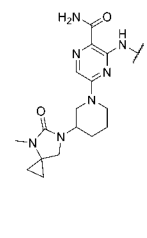 | 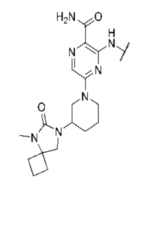 | 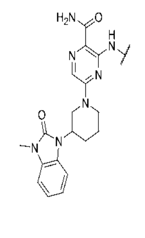 | 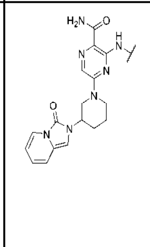 | 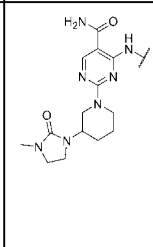 | 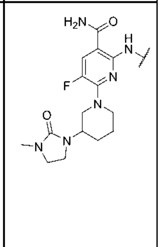 |
| 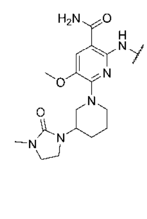 | 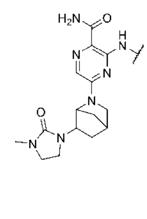 | 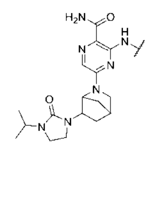 | 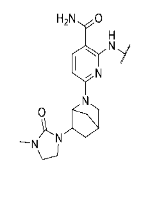 | 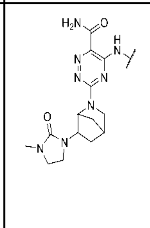 | 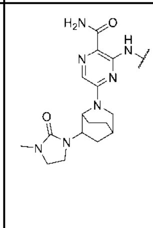 | 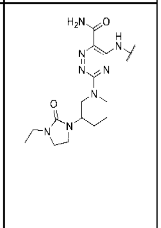 |
| 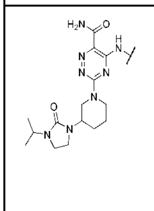 | 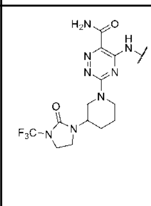 | 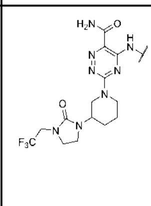 | 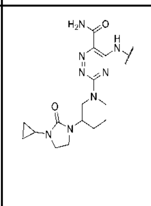 | 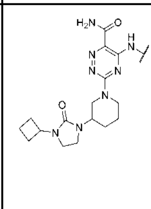 | 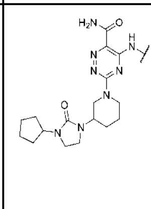 | 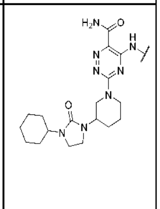 |
| 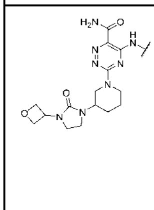 | 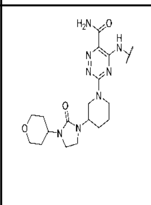 | 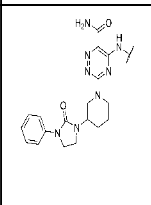 | 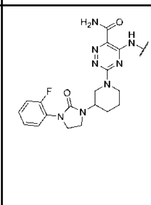 | 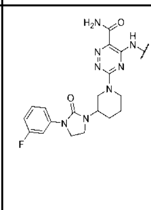 | | |
| | | | | | | |

| | | | | | | |
|---|---|---|---|---|---|---|
| 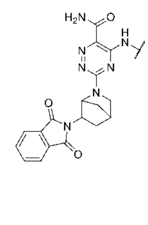 | 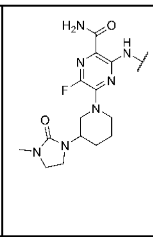 | 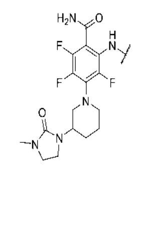 | 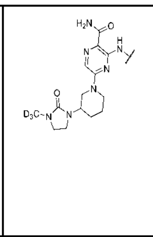 | 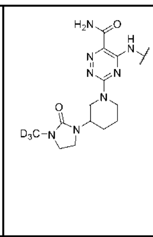 | 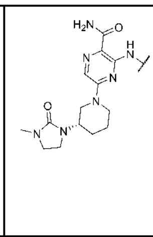 | 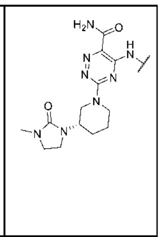 |
| 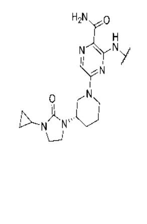 | 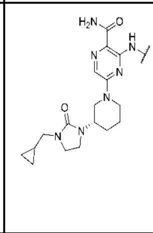 | 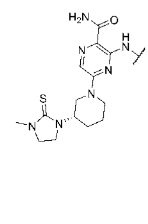 | 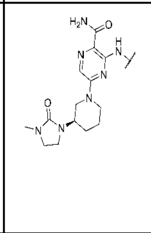 | 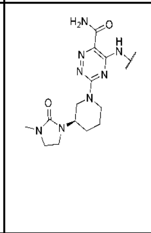 | 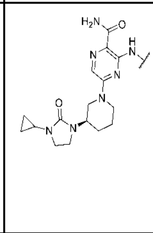 | 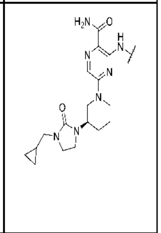 |
| 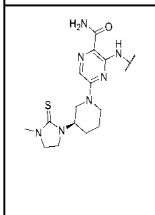 | 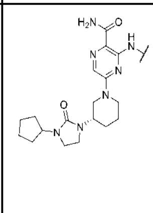 | 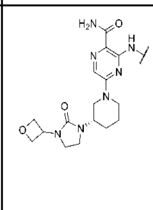 | 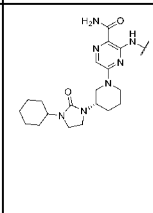 | 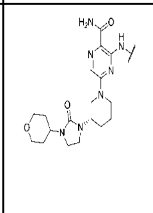 | 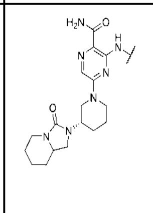 | 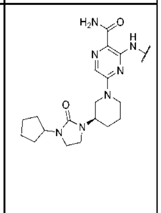 |
| 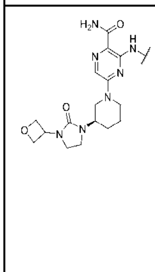 | 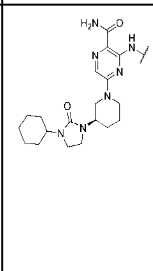 | 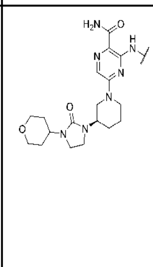 | 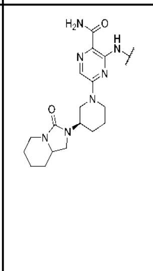 | 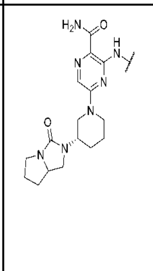 | 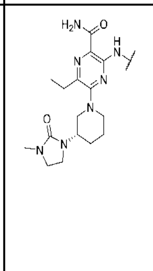 | 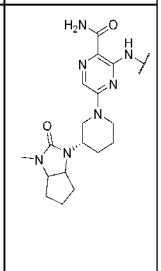 |
| 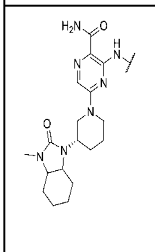 | 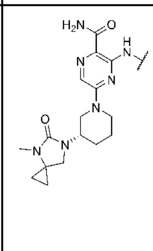 | 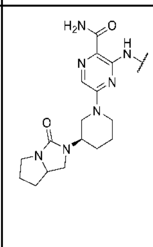 | 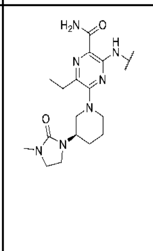 | 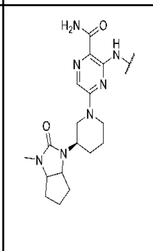 | 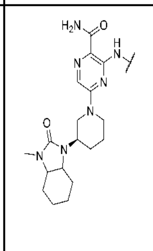 | 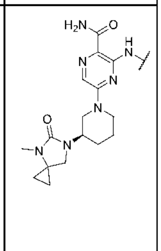 |
| 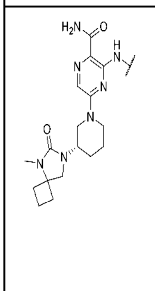 | 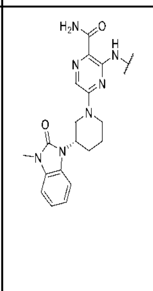 | 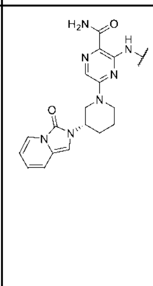 | 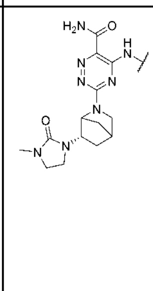 | 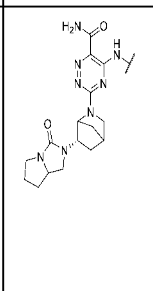 | | |
| | | | | | | |

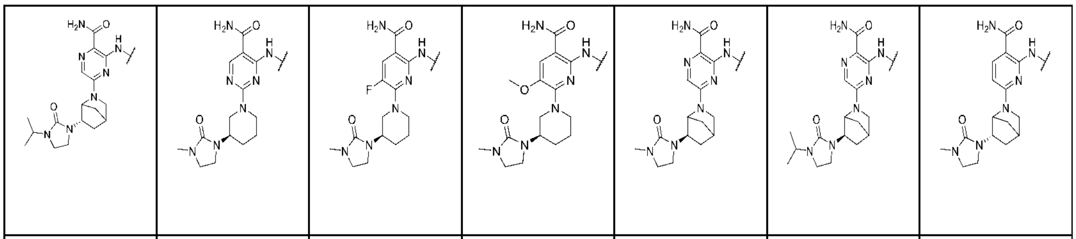

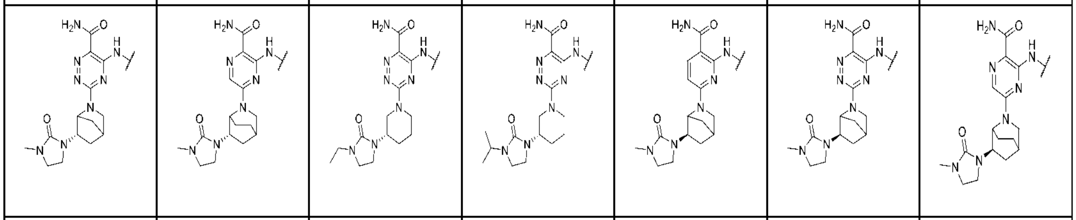

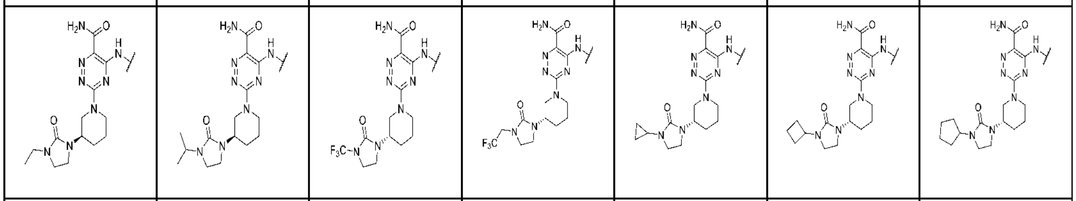

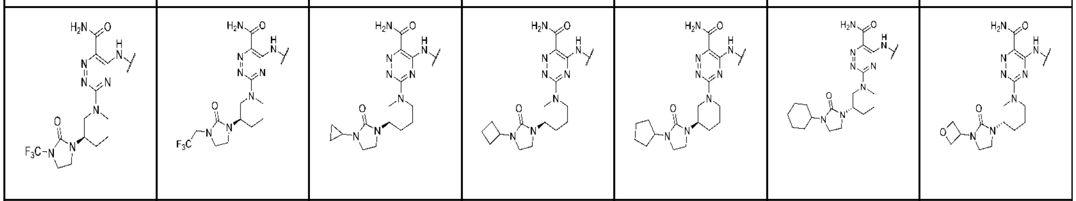

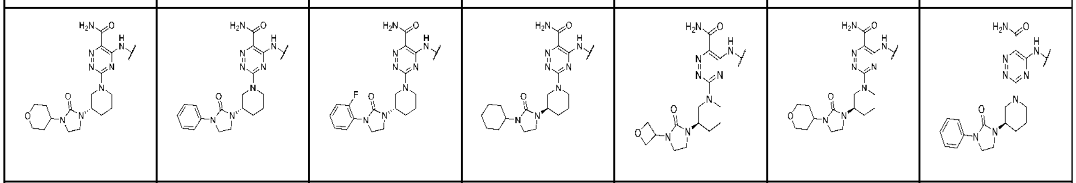

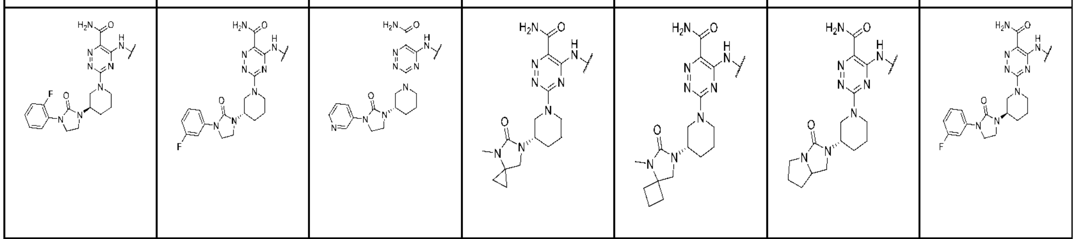

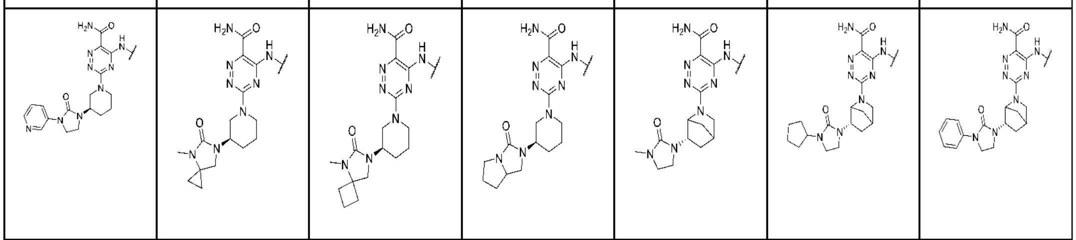

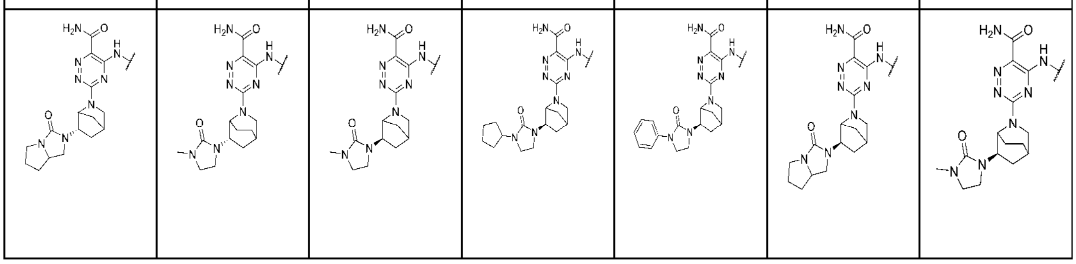

| | | | | | | |
|---|---|---|---|---|---|---|
| 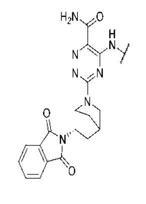 | 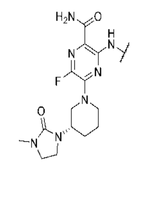 | 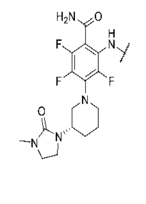 | 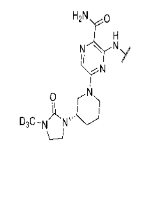 | 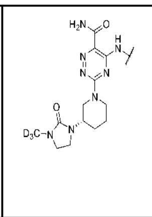 | 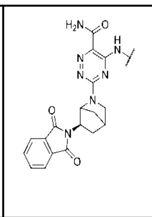 | 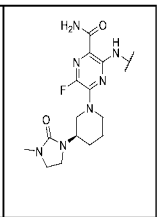 |
| 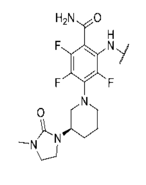 | 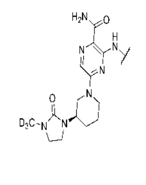 | 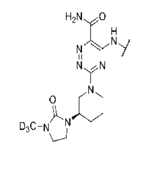 | 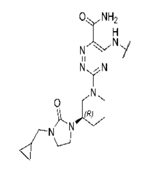 | 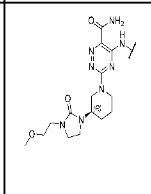 | 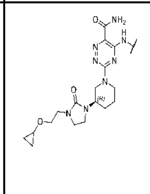 | 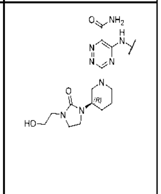 |
| 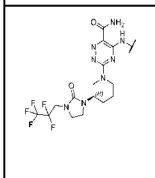 | 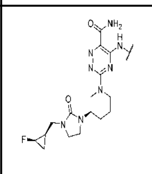 | 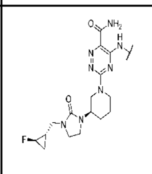 | 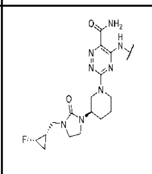 | 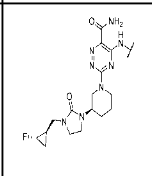 | 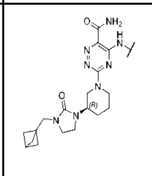 | 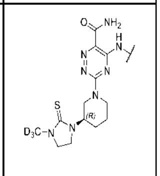 |
| 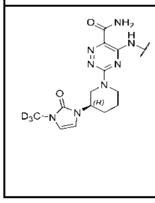 | 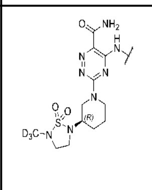 | 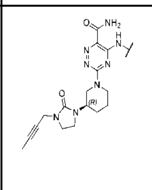 | | | | |

.

in certain embodiments, K is selected from

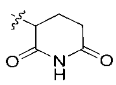, 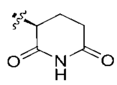, 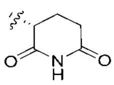,

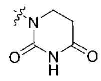, 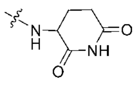, and 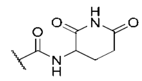;

in some embodiments, K is selected from

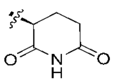, 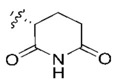, 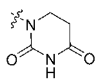,

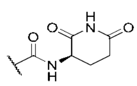, 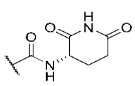, 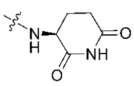 or 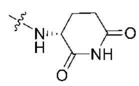;

optionally,

K1 is selected from

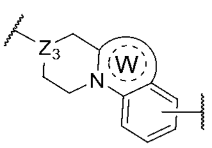, 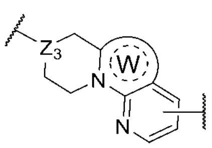, 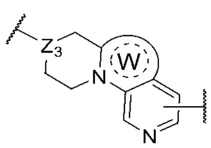,

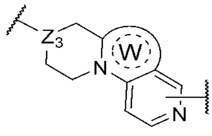, and 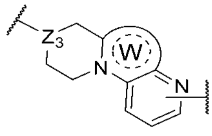,

or B4 is selected from

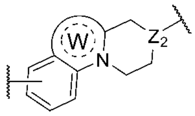,

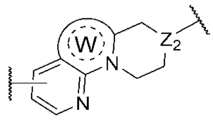, 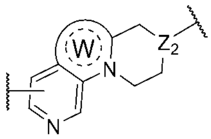, 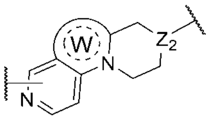, and 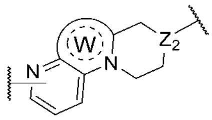.

Optionally, the compound satisfies the following conditions:

K1 is selected from

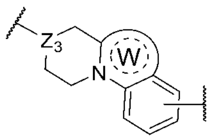, 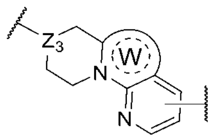, 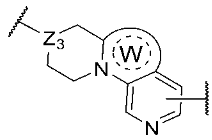,

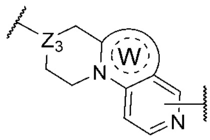, and 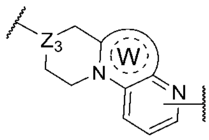,

and the remaining definitions in general formula (I) or general formula (II) are as described above;

or B4 is selected from

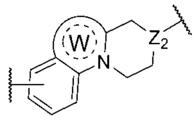, 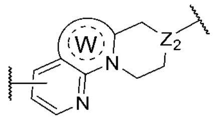, 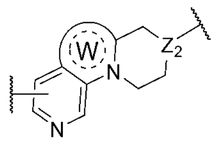,

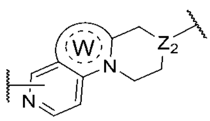, and 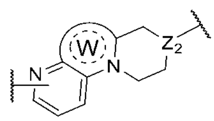,

and the remaining definitions in general formula (I) or general formula (II) are as described above.

A first embodiment of the present invention provides the above-mentioned compound represented by general formula (I) or the stereoisomer, tautomer, deuterated compound, solvate, prodrug, metabolite, pharmaceutically acceptable salt or co-crystal thereof, wherein

B is

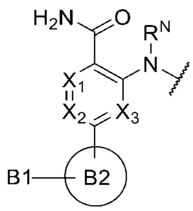

;

L is

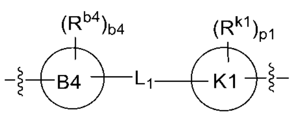

,

which is connected to B on the left side, and is connected to K on the right side;
K is

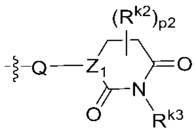

;

B1 is absent or is selected from $C_{3-12}$ carbocyclyl or 4- to 12-membered heterocyclyl, wherein the carbocyclyl or heterocyclyl is optionally substituted with 1 to 4 $R^{b1}$;
B2 is selected from $C_{3-12}$ carbocyclyl or 4- to 12-membered heterocyclyl, wherein the carbocyclyl or heterocyclyl is optionally substituted with 1 to 4 $R^{b2}$;
B4 is selected from phenyl, 5- to 6-membered heteroaryl, a 9- to 12-membered fused bicyclic group,

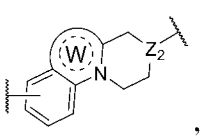

,

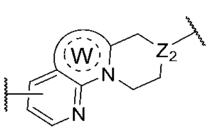

,

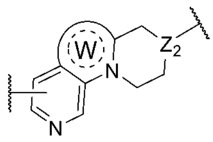

,

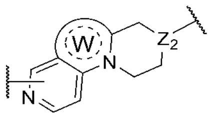

, and

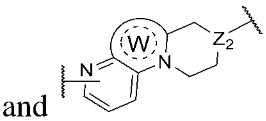

;

K1 is selected from phenyl, 5- to 6-membered heteroaryl, a 9- to 12-membered fused bicyclic group,

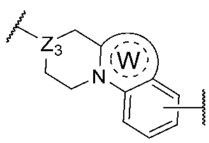

,

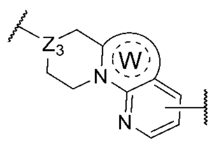

,

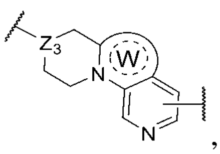

,

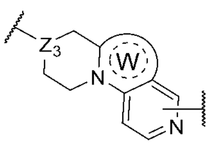

, and

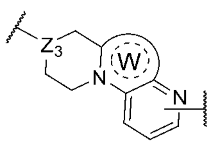

;

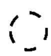

is selected from an aromatic or non-aromatic ring;
W is 5- to 6-membered heterocyclyl;
$Z_2$ is selected from CH or N;
$Z_3$ is selected from CH or N;
Li is selected from -Ak1-, -Ak1-Ak2-, -Cy1-, -Cy1-Cy2-, -Cy1-Ak1-, -Ak1-Cy1-, -Cy1-Ak1-Cy2-, -Cy1-Cy2-Ak1-, -Ak1-Cy1-Cy2-, -Cy1-Ak1-Cy2-Ak2-, and -Ak1-Cy1-Ak2-Cy2-;
Q is selected from $NR^qCO$, $CONR^q$, CO, $NR^q$ or a bond;
$X_1$, $X_2$, and $X_3$ are each independently selected from N or $CR^{b3}$;
$Z_1$ is selected from N or $CR^{k2}$;
Ak1 and Ak2 are each independently selected from a bond, $C_{1-8}$ alkylene, $C_{2-8}$ alkenylene, $C_{2-8}$ alkynylene, O, $NR^L$, $NR^LCO$, $CONR^L$, CO, and C≡C, wherein the alkylene, alkenylene, and alkynylene are optionally substituted with 1 to 4 substituents selected from deuterium, F, Cl, Br, I, OH, COOH, CN, $NH_2$, =O, $C_{1-6}$ alkyl, halogen-substituted $C_{1-6}$ alkyl, hydroxyl-substituted $C_{1-6}$ alkyl, $C_{1-6}$ alkoxy, $C_{3-7}$ carbocyclyl, and 4- to 6-membered heterocyclyl;
each $R^L$ or $R^N$ is independently selected from H or $C_{1-6}$ alkyl;
Cy1 and Cy2 are each independently selected from a bond, 3- to 7-membered monocyclic heterocyclyl, 4- to 12-membered fused heterocyclyl, 5- to 12-membered spiroheterocyclyl, 5- to 12-membered bridged heterocyclyl, $C_{3-7}$ monocyclic carbocyclyl, 4- to 12-membered fused carbocyclyl, 5- to 12-membered spirocarbocyclyl, 5- to 12-membered bridged carbocyclyl, 5- to 12-membered heteroaryl or 6- to 10-membered aryl, wherein the aryl, heteroaryl, carbocyclyl, and heterocyclyl are optionally substituted with 1 to 4 substituents selected from deuterium, F, Cl, Br, I, OH, COOH, $CONH_2$, CN, $NH_2$, =O, $C_{1-6}$ alkyl, halogen-substituted $C_{1-6}$ alkyl, hydroxyl-substituted $C_{1-6}$ alkyl, $C_{1-6}$ alkoxy, $C_{3-7}$ carbocyclyl, and 4- to 6-membered heterocyclyl;
$R^{b1}$, $R^{b2}$, $R^{b3}$, $R^{b4}$, $R^{k1}$, $R^{k2}$, $R^{k3}$, and $R^q$ are each independently selected from H, deuterium, F, Cl, Br, I, OH, =O, =S, $NH_2$, CN, COOH, $CONH_2$, $C_{1-6}$ alkyl, $C_{1-6}$ alkoxy, $C_{3-7}$ carbocyclyl, 4- to 6-membered heterocyclyl, -O-$C_{3-7}$ carbocyclyl, and -O-4- to 6-membered heterocyclyl, wherein the alkyl, alkoxy, heterocyclyl, and carbocyclyl are optionally substituted with 1 to 4 substituents selected from deuterium, F, Cl, Br, I, OH, =O, $NH_2$, CN, $CONH_2$, COOH, $C_{1-6}$ alkyl, $C_{1-6}$ alkoxy, $C_{3-7}$ carbocyclyl, 4- to 6-membered heterocyclyl, -O-$C_{3-7}$ carbocyclyl, -O-4- to 6-membered heterocyclyl, fluoro-substituted $C_{3-7}$ carbocyclyl, $C_{2-6}$ alkynyl, and fluoro-substituted phenyl;
each b4 is independently selected from 0, 1, 2, 3, 4, 5 or 6;
each p1 or p2 is independently selected from 0, 1, 2, 3 or 4;
provided that
K1 is selected from

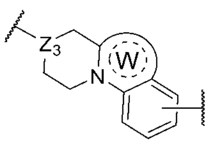, 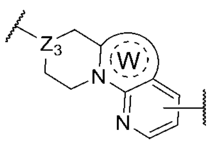, 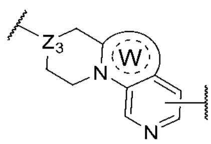,

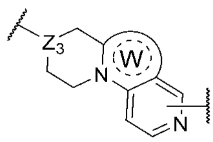, and 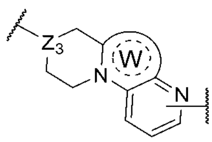,

or B4 is selected from

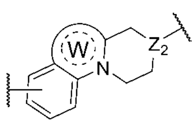,

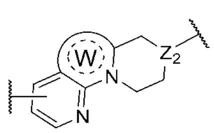, 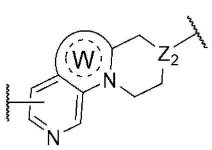, 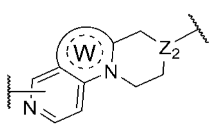, and 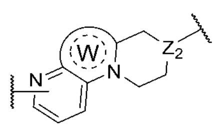.

A second embodiment of the present invention provides the above-mentioned compound represented by

general formula (I) or the stereoisomer, tautomer, deuterated compound, solvate, prodrug, metabolite, pharmaceutically acceptable salt or co-crystal thereof, wherein

B1 is absent or is selected from $C_{3-7}$ monocyclic carbocyclyl, $C_{5-12}$ fused carbocyclyl, $C_{5-12}$ spirocarbocyclyl, $C_{5-12}$ bridged carbocyclyl, 4- to 7-membered monocyclic heterocyclyl, 4- to 12-membered fused heterocyclyl, 5- to 12-membered spiroheterocyclyl, and 5- to 12-membered bridged heterocyclyl, wherein the carbocyclyl or heterocyclyl is optionally substituted with 1 to 4 $R^{b1}$;

B2 is selected from 4- to 7-membered monocyclic heterocyclyl, 4- to 12-membered fused heterocyclyl, 5- to 12-membered spiroheterocyclyl, and 5- to 12-membered bridged heterocyclyl, wherein the heterocyclyl is optionally substituted with 1 to 4 $R^{b2}$;

B4 is selected from phenyl, 5- to 6-membered heteroaryl,

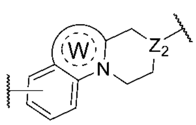,

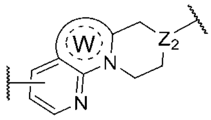, 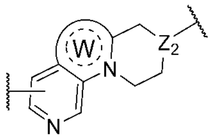, 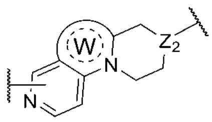, 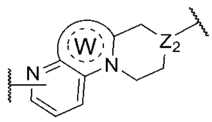,

benzofused 5-membered heterocyclyl, benzofused 6-membered heterocyclyl, benzofused 5-membered carbocyclyl, benzofused 6-membered carbocyclyl, benzofused 7-membered carbocyclyl, benzofused 8-membered carbocyclyl, pyridofused 5-membered heterocyclyl, and pyridofused 6-membered heterocyclyl;

K1 is selected from phenyl, 5- to 6-membered heteroaryl,

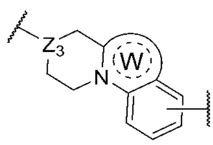,

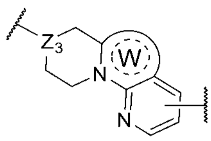, 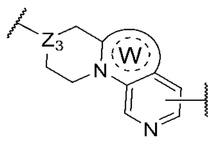, 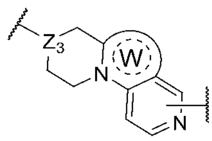, 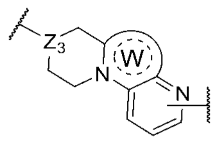,

benzofused 5-membered heterocyclyl, and benzofused 6-membered heterocyclyl;

Ak1 and Ak2 are each independently selected from $C_{1-6}$ alkylene, $C_{2-6}$ alkenylene, $C_{2-6}$ alkynylene, O, $NR^L$, $NR^LCO$, $CONR^L$, and CO, wherein the alkylene, alkenylene, and alkynylene are optionally substituted with 1 to 4 substituents selected from deuterium, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl, $C_{1-4}$ alkoxy, $C_{3-6}$ carbocyclyl, and 4- to 6-membered heterocyclyl;

each $R^L$ or $R^N$ is independently H or $C_{1-4}$ alkyl;

Cy1 and Cy2 are each independently selected from 3- to 6-membered monocyclic heterocyclyl, 4- to 10-membered fused heterocyclyl, 5- to 11-membered spiroheterocyclyl, 5- to 10-membered bridged heterocyclyl, $C_{3-6}$ monocyclic carbocyclyl, 4- to 10-membered fused carbocyclyl, 5- to 11-membered spirocarbocyclyl, 5- to 10-membered bridged carbocyclyl, 5- to 10-membered heteroaryl or 6- to 10-membered aryl, wherein the aryl, heteroaryl, carbocyclyl, and heterocyclyl are optionally substituted with 1 to 4 substituents selected from deuterium, F, Cl, Br, I, OH, COOH, $CONH_2$, CN, $NH_2$, =O, $C_{1-4}$ alkyl, halogen-substituted $C_{1-4}$ alkyl, hydroxyl-substituted $C_{1-4}$ alkyl, $C_{1-4}$ alkoxy, $C_{3-6}$ carbocyclyl, and 4- to 6-membered heterocyclyl;

$R^{b1}$, $R^{b2}$, $R^{b3}$, $R^{b4}$, $R^{k1}$, $R^{k2}$, $R^{k3}$, and $R^q$ are each independently selected from H, deuterium, F, Cl, Br, I, OH, =O, =S, $NH_2$, CN, COOH, $CONH_2$, $C_{1-4}$ alkyl, $C_{1-4}$ alkoxy, $C_{3-6}$ carbocyclyl, 4- to 6-membered heterocyclyl, -O-$C_{3-6}$ carbocyclyl, and -O-4- to 6-membered heterocyclyl, wherein the alkyl, alkoxy, heterocyclyl, and carbocyclyl are optionally substituted with 1 to 6 substituents selected from deuterium, F, Cl, Br, I, OH, =O, $NH_2$, CN, $CONH_2$, COOH, $C_{1-4}$ alkyl, $C_{1-4}$ alkoxy, $C_{3-7}$ carbocyclyl, 4- to 6-membered heterocyclyl, -O-$C_{3-7}$ carbocyclyl, -O-4- to 6-membered

heterocyclyl, fluoro-substituted $C_{3-7}$ carbocyclyl, $C_{2-6}$ alkynyl, and fluoro-substituted phenyl;
the remaining definitions are the same as those in the first embodiment of the present invention.

A third embodiment of the present invention provides the above-mentioned compound represented by general formula (I) or the stereoisomer, tautomer, deuterated compound, solvate, prodrug, metabolite, pharmaceutically acceptable salt or co-crystal thereof, wherein

B1 is absent or is selected from 4- to 6-membered heterocyclyl, cyclopropyl-spiro-5-membered heterocyclyl, cyclobutyl-spiro-5-membered heterocyclyl, 5,5-fused heterocyclyl, and 6,5-fused heterocyclyl, wherein the heterocyclyl is optionally substituted with 1 to 4 $R^{b1}$;
B2 is selected from 4- to 6-membered monocyclic heterocyclyl, 8- to 9-membered fused heterocyclyl, 7- to 11-membered spiroheterocyclyl, and 5- to 8-membered bridged heterocyclyl, wherein the heterocyclyl is optionally substituted with 1 to 4 $R^{b2}$;
Ak1 and Ak2 are each independently selected from $C_{1-4}$ alkylene, $C_{2-4}$ alkenylene, $C_{2-4}$ alkynylene, O, $NR^L$, $NR^LCO$, and CO, wherein the alkylene, alkenylene, and alkynylene are optionally substituted with 1 to 4 substituents selected from deuterium, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl, $C_{1-4}$ alkoxy, $C_{3-6}$ carbocyclyl, and 4- to 6-membered heterocyclyl;
each $R^L$ or $R^N$ is independently selected from H, methyl or ethyl;
Cy1 and Cy2 are each independently selected from one of the following substituted or unsubstituted groups: cyclopropyl, cyclobutyl, cyclopentyl, cyclohexyl, azetidinyl, pyrrolidyl, piperidyl, morpholinyl, piperazinyl, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-pyrrolidyl, cyclopropyl-fused-piperidyl, cyclobutyl-fused-azetidinyl, cyclobutyl-fused-pyrrolidyl, cyclobutyl-fused-piperidyl, cyclopentyl-fused-azetidinyl, cyclopentyl-fused-pyrrolidyl, cyclopentyl-fused-piperidyl, cyclohexyl-fused-azetidinyl, cyclohexyl-fused-pyrrolidyl, cyclohexyl-fused-piperidyl, azetidinyl-fused-azetidinyl, azetidinyl-fused-pyrrolidyl, azetidinyl-fused-piperidyl, pyrrolidyl-fused-azetidinyl, pyrrolidyl-fused-pyrrolidyl, pyrrolidyl-fused-piperidyl, piperidyl-fused-azetidinyl, piperidyl-fused-pyrrolidyl, piperidyl-fused-piperidyl, cyclobutyl-spiro-azetidinyl, cyclobutyl-spiro-pyrrolidyl, cyclobutyl-spiro-piperidyl, cyclopentyl-spiro-azetidinyl, cyclopentyl-spiro-pyrrolidyl, cyclopentyl-spiro-piperidyl, cyclohexyl-spiro-azetidinyl, cyclohexyl-spiro-pyrrolidyl, cyclohexyl-spiro-piperidyl, azetidinyl-spiro-azetidinyl, azetidinyl-spiro-pyrrolidyl, azetidinyl-spiro-piperidyl, pyrrolidyl-spiro-azetidinyl, pyrrolidyl-spiro-pyrrolidyl, pyrrolidyl-spiro-piperidyl, piperidyl-spiro-azetidinyl, piperidyl-spiro-pyrrolidyl, piperidyl-spiro-piperidyl,

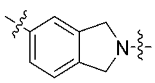, 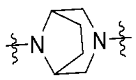, 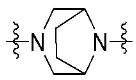, 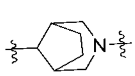, 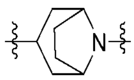, 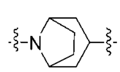, 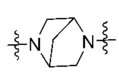,

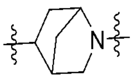, 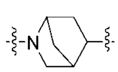, 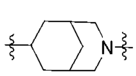, 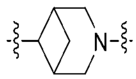, 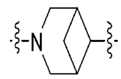, 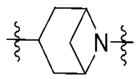, 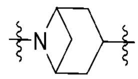,

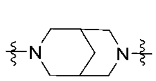, 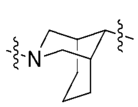, 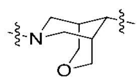, 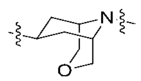 or 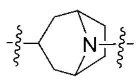,

which, when substituted, are optionally substituted with 1 to 4 substituents selected from deuterium, F, Cl, Br, I, OH, $NH_2$, COOH, $CONH_2$, CN, =O, $C_{1-4}$ alkyl, halogen-substituted $C_{1-4}$ alkyl, hydroxyl-substituted $C_{1-4}$ alkyl, $C_{1-4}$ alkoxy, $C_{3-7}$ carbocyclyl, and 4- to 6-membered heterocyclyl;
the remaining definitions are the same as those in the first or second embodiment of the present invention.

A fourth embodiment of the present invention provides the above-mentioned compound represented by general formula (I) or the stereoisomer, tautomer, deuterated compound, solvate, prodrug, metabolite, pharmaceutically acceptable salt or co-crystal thereof, wherein

B1 is absent or is selected from the following groups optionally substituted with 1 to 2 $R^{b1}$;

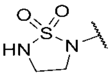, 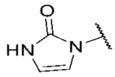, 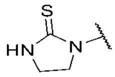, 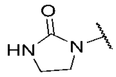, 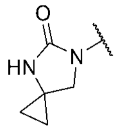, 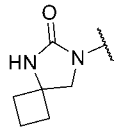,

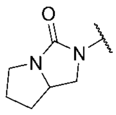, 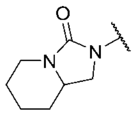, 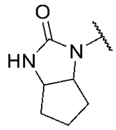, 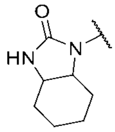, 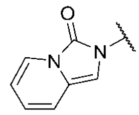, 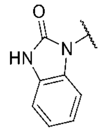, and 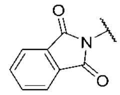;

B2 is selected from the following groups optionally substituted with 1 to 3 $R^{b2}$:

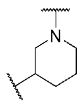, 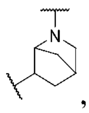, and 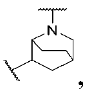,

which is connected to B on the right side;

B4 is selected from phenyl, pyrrolyl, pyrazolyl, imidazolyl, triazolyl, oxadiazolyl, furyl, thienyl, thiazolyl, isothiazolyl, thiadiazolyl, pyridyl, pyrimidyl, pyrazinyl, pyridazinyl, triazinyl,

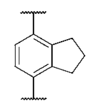, 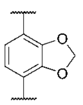, 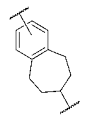, 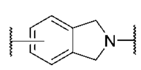, 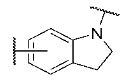,

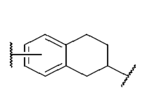, 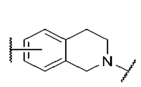, 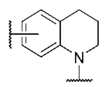, 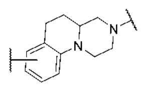, 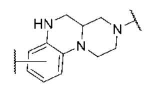, 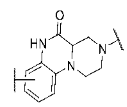, 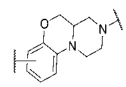,

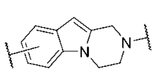, 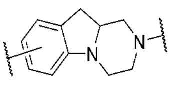, 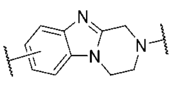, 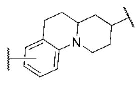, 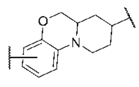, 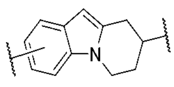,

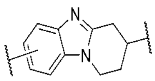, 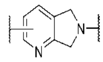, and 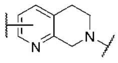;

b4 is selected from 0, 1, 2 or 3;

K1 is selected from phenyl, pyrrolyl, pyrazolyl, imidazolyl, triazolyl, oxadiazolyl, furyl, thienyl, thiazolyl, isothiazolyl, thiadiazolyl, pyridyl, pyrimidyl, pyrazinyl, pyridazinyl, triazinyl,

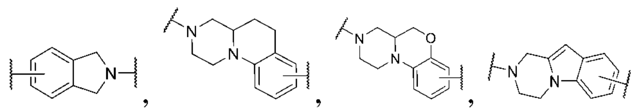,

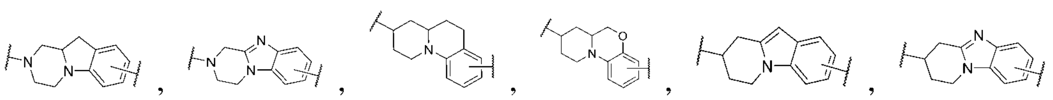,

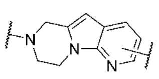, 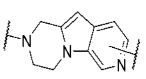, 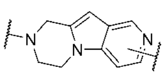, 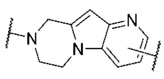, 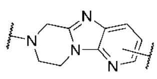, 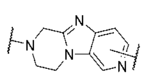,

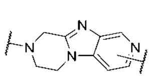, 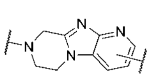, 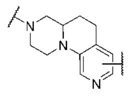, 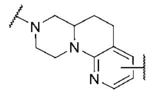, 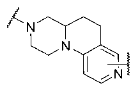, 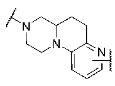, 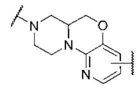,

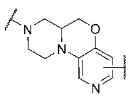, 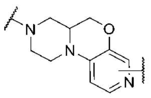, 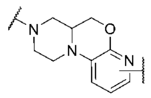, 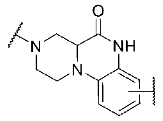, 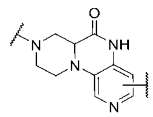, 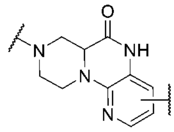,

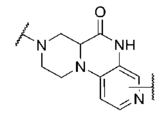, 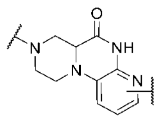, 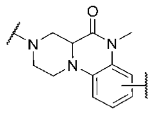, 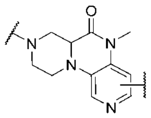, 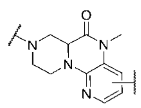, 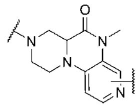,

and

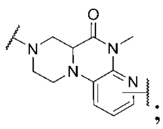;

Ak1 and Ak2 are each independently selected from methylene, ethylene, vinylene, and ethynylene, wherein the methylene, ethylene, vinylene, and ethynylene are optionally substituted with 1 to 4 substituents selected from deuterium, F, Cl, Br, I, OH, $NH_2$, COOH, CN, =O, $C_{1-4}$ alkyl, halogen-substituted $C_{1-4}$ alkyl, hydroxyl-substituted $C_{1-4}$ alkyl, $C_{1-4}$ alkoxy, $C_{3-6}$ carbocyclyl, and 4- to 6-membered heterocyclyl;

Cy1 and Cy2 are each independently selected from one of the following substituted or unsubstituted groups:

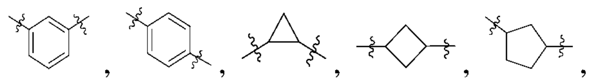, , , , ,

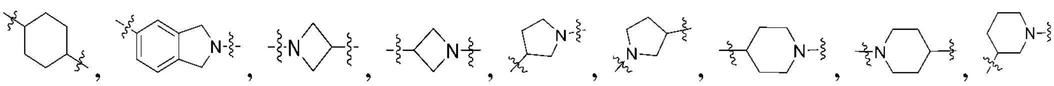, , , , , , , , ,

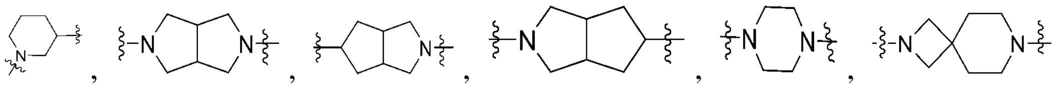, , , , , ,

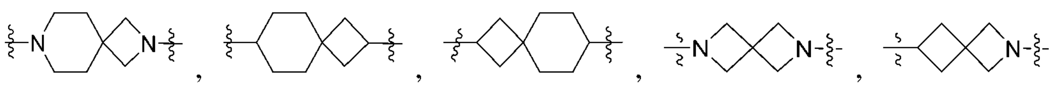, , , , ,

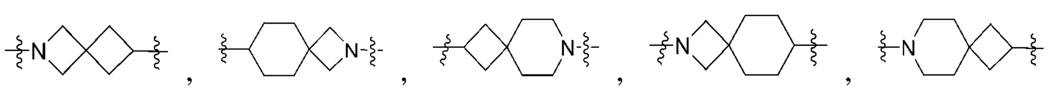, , , , ,

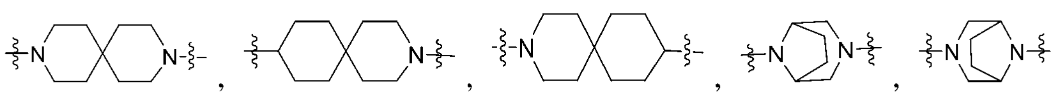, , , , ,

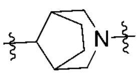, 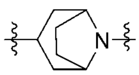, 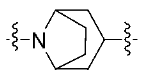, 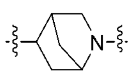, 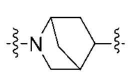, 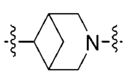, 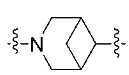,

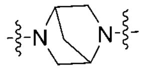, 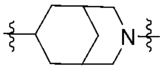, 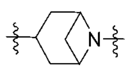, 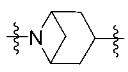, and 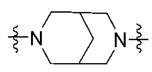,

which, when substituted, are optionally substituted with 1 to 4 substituents selected from deuterium, F, Cl, Br, I, OH, =O, $NH_2$, CN, $CONH_2$, COOH, $C_{1-4}$ alkyl, halogen-substituted $C_{1-4}$ alkyl, hydroxyl-substituted $C_{1-4}$ alkyl, $C_{1-4}$ alkoxy, $C_{3-7}$ carbocyclyl, and 4- to 6-membered heterocyclyl;

$R^{b1}$, $R^{b2}$, $R^{b3}$, $R^{b4}$, $R^{k1}$, $R^{k2}$, $R^{k3}$, and $R^q$ are each independently selected from H, deuterium, F, Cl, Br, I, OH, =O, =S, $NH_2$, CN, COOH, $CONH_2$, methyl, ethyl, propyl, isopropyl, butyl, tert-butyl, methoxy, ethoxy, propoxy, isopropyloxy, cyclopropyl, cyclobutyl, cyclopentyl, cyclohexyl, phenyl, azetidinyl, oxetanyl, tetrahydrofuryl, oxanyl, pyrrolidyl, pyrrolyl, pyrazolyl, pyridyl, -O-cyclopropyl, - O-cyclobutyl, -O-cyclopentyl, -O-cyclohexyl, and -O-phenyl, wherein the methyl, ethyl, propyl, isopropyl, butyl, tert-butyl, methoxy, ethoxy, propoxy, isopropyloxy, cyclopropyl, cyclobutyl, cyclopropyl, cyclobutyl, cyclopentyl, cyclohexyl, phenyl, azetidinyl, oxetanyl, tetrahydrofuryl, oxanyl, pyrrolidyl, pyrrolyl, pyrazolyl, and pyridyl are optionally substituted with 1 to 6 substituents selected from deuterium, F, Cl, Br, I, OH, =O, $NH_2$, CN, $CONH_2$, COOH, $C_{1-4}$ alkyl, $C_{1-4}$ alkoxy, $C_{3-7}$ carbocyclyl, 4- to 6-membered heterocyclyl, -O-$C_{3-7}$ carbocyclyl, -O-4- to 6-membered heterocyclyl, fluoro-substituted $C_{3-7}$ carbocyclyl, $C_{2-6}$ alkynyl, and fluoro-substituted phenyl;

the remaining definitions are the same as those in the first, second, or third embodiment of the present invention.

A fifth embodiment of the present invention provides the above-mentioned compound represented by general formula (I) or the stereoisomer, tautomer, deuterated compound, solvate, prodrug, metabolite, pharmaceutically acceptable salt or co-crystal thereof, wherein

Li is selected from one of the structural fragments in Table L1-1;

Q is selected from $CONR^q$, $NR^qCO$, $NR^q$ or a bond;

each $R^{b1}$ is independently selected from H, deuterium, F, Cl, Br, I, OH, =O, =S, $NH_2$, CN, COOH, $CONH_2$, $CF_3$, $CD_3$, $CHF_2$, $CH_2F$, methyl, ethyl, propyl, isopropyl, butyl, tert-butyl, methoxy, ethoxy, propoxy, isopropyloxy, cyclopropyl, cyclobutyl, cyclopentyl, cyclohexyl, phenyl, pyridyl, oxetanyl, tetrahydrofuryl, oxanyl, -$CH_2$-cyclopropyl, -$CH_2CF_3$, fluoro-substituted phenyl,

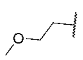, 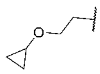,

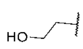, 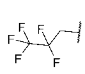, 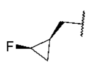, 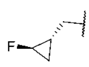, 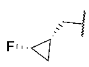, 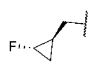, 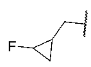, 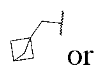 or 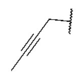;

$R^{b2}$, $R^{b3}$, $R^{b4}$, $R^{k1}$, and $R^{k2}$ are each independently selected from H, deuterium, F, Cl, Br, I, OH, =O, $NH_2$, CN, COOH, $CONH_2$, $CF_3$, $CHF_2$, $CH_2F$, methyl, ethyl, propyl, isopropyl, butyl, tert-butyl, methoxy, ethoxy, propoxy, isopropyloxy, cyclopropyl, and $CH_2OH$;

each $R^{k3}$ is independently selected from H, deuterium, and methyl;

each $R^q$ is independently selected from H, deuterium, and methyl;

the remaining definitions are the same as those in the first, second, third, or fourth embodiment of the present invention.

A sixth embodiment of the present invention provides the above-mentioned compound represented by general formula (I) or the stereoisomer, tautomer, deuterated compound, solvate, prodrug, metabolite, pharmaceutically acceptable salt or co-crystal thereof, wherein

B is selected from one of the fragments shown in Table B-1;

L is selected from one of the fragments shown in Table A;

K is selected from

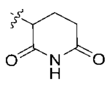, 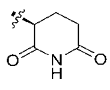, 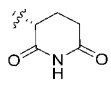, 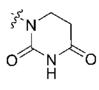, 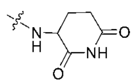,

and

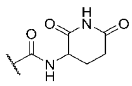.

A seventh embodiment of the present invention provides the above-mentioned compound represented by general formula (I) or the stereoisomer, tautomer, deuterated compound, solvate, prodrug, metabolite, pharmaceutically acceptable salt or co-crystal thereof, wherein the compound represented by general formula (I) is a compound represented by general formula (III), wherein

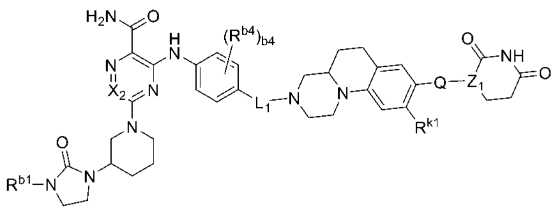

(III);

$X_2$ is selected from N or CH;

Q is selected from CONH, NHCO, NH or a bond;

$Z_1$ is selected from N or CH, provided that Q and $Z_1$ do not form an N-N bond;

$L_1$, $R^{b1}$, $R^{b4}$, $R^{k1}$ or b4 has the same meanings as defined in any one of the first, second, third and fourth embodiments of the present invention, preferably has the same meanings as defined in the fourth embodiment.

An eighth embodiment of the present invention provides the above-mentioned compound represented by general formula (III) or the stereoisomer, tautomer, deuterated compound, solvate, prodrug, metabolite, pharmaceutically acceptable salt or co-crystal thereof, wherein

Li is selected from

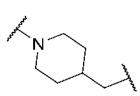, 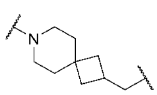, 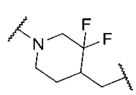, 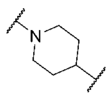,

and

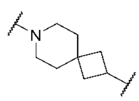;

$R^{b1}$ is independently selected from $CF_3$, $CD_3$, $CHF_2$, $CH_2F$, methyl, ethyl, propyl, isopropyl, butyl, tert-butyl, cyclopropyl, cyclobutyl, cyclopentyl, cyclohexyl, phenyl, pyridyl, oxetanyl, tetrahydrofuryl, oxanyl, $-CH_2$-cyclopropyl, - $CH_2CF_3$, fluoro-substituted phenyl,

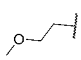, 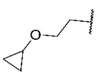, 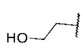, 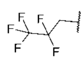, 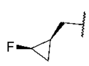, 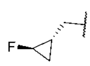,

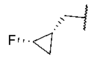, 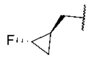, 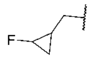, 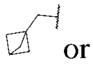 or 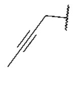;

$R^{b4}$ and $R^{k1}$ are each independently selected from H, deuterium, F, Cl, Br, I, OH, $NH_2$, CN, COOH, $CONH_2$, $CF_3$, $CHF_2$,

$CH_2F$, methyl, ethyl, propyl, isopropyl, butyl, tert-butyl, methoxy, ethoxy, propoxy, isopropyloxy, cyclopropyl, and $CH_2OH$;
b4 is selected from 0 or 1;
the remaining definitions are the same as those in the seventh embodiment of the present invention.

The present invention relates to a compound as described below or a stereoisomer, tautomer, deuterated compound, solvate, prodrug, metabolite, pharmaceutically acceptable salt or co-crystal thereof, wherein the compound has a structure selected from one of the following structures:

Table E-1

| | | |
|---|---|---|
| 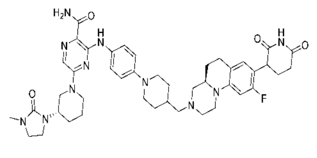 | 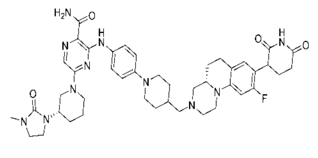 | 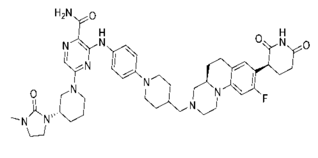 |
| 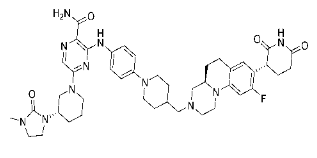 | 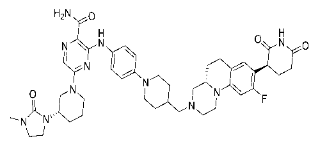 | 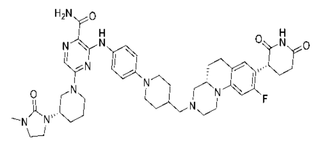 |
| 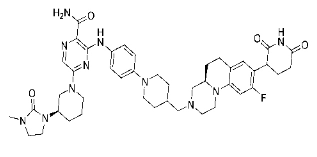 | 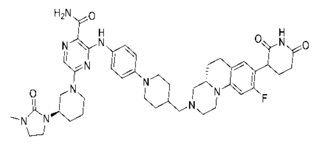 | 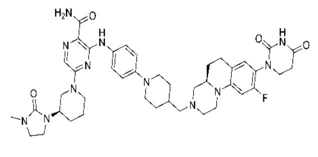 |
| 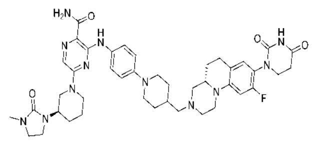 | 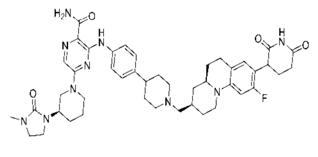 | 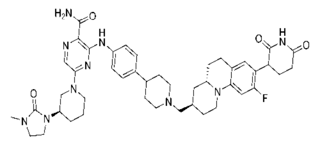 |
| 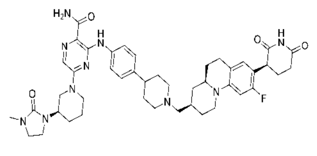 | 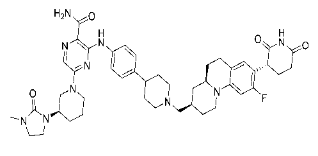 | 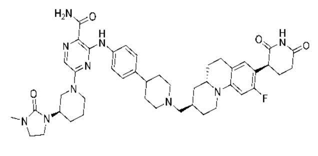 |
| 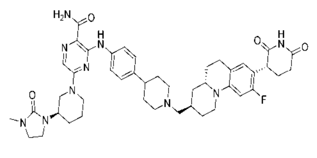 | 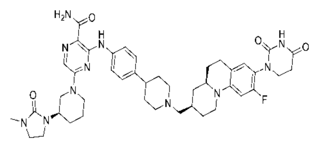 | 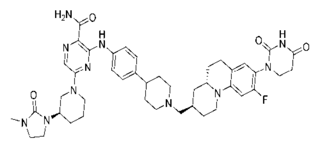 |
| 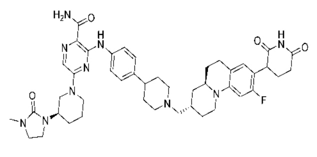 | 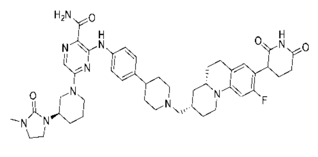 | 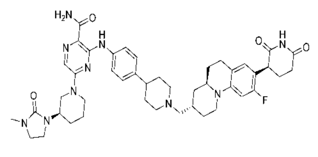 |
| 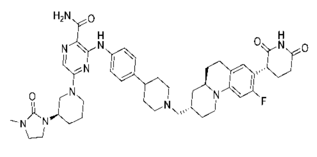 | 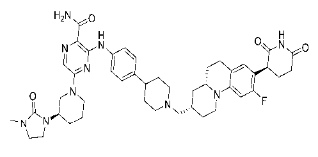 | 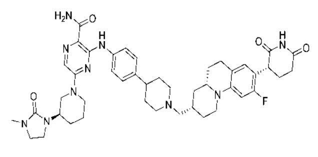 |

| | | |
|---|---|---|
| 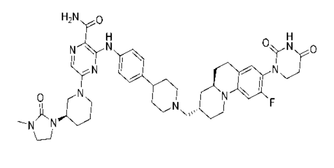 | 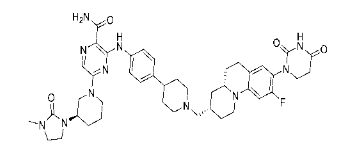 | 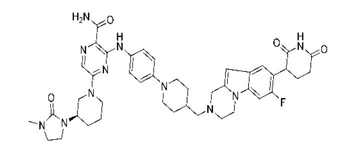 |
| 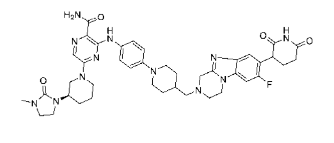 | 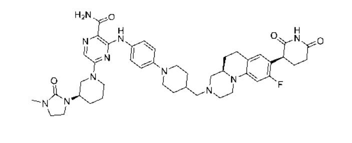 | 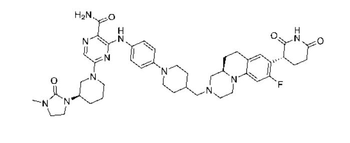 |
| 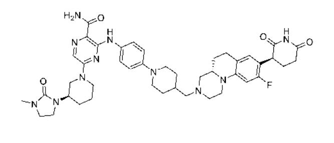 | 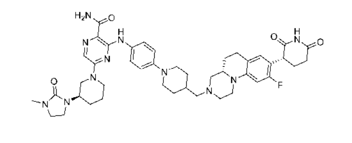 | 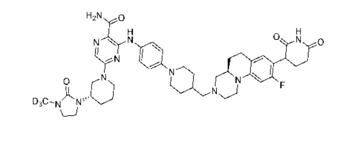 |
| 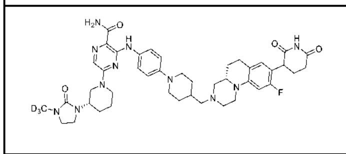 | 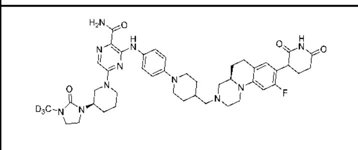 | 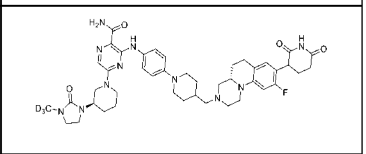 |
| 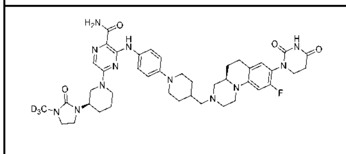 | 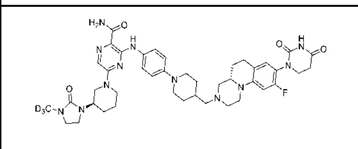 | 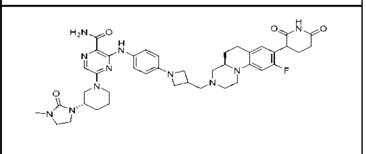 |
| 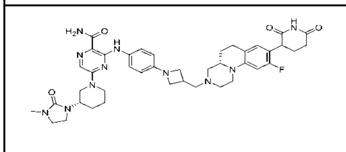 | 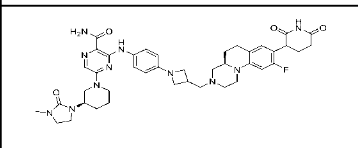 | 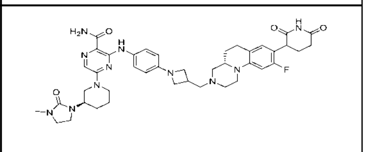 |
| 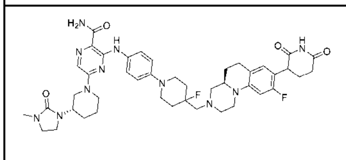 | 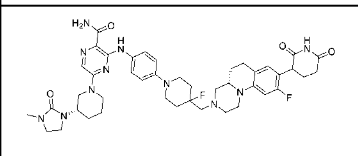 | 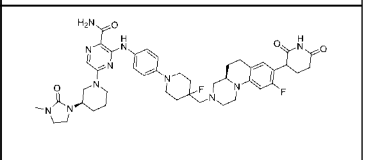 |
| 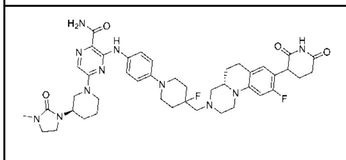 | 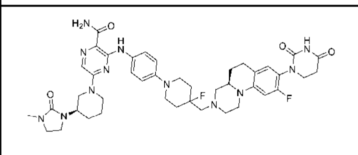 | 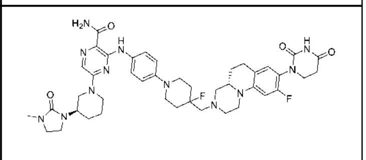 |
| 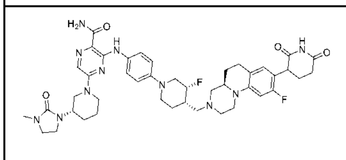 | 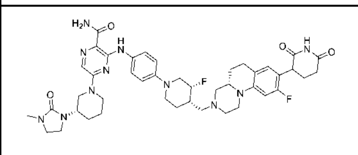 | 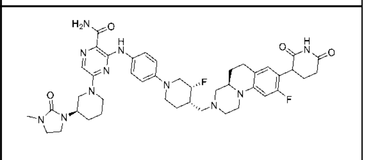 |
| 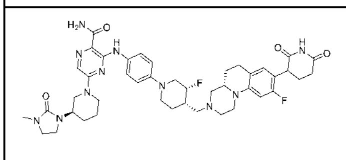 | 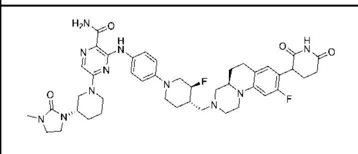 | 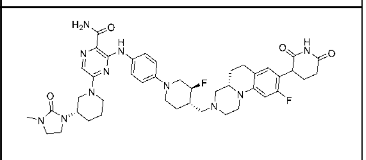 |
| 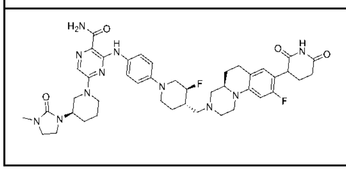 | 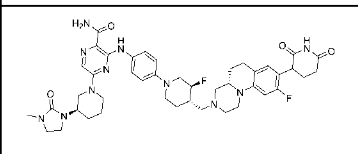 | 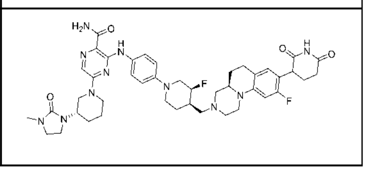 |

| | | |
|---|---|---|
| 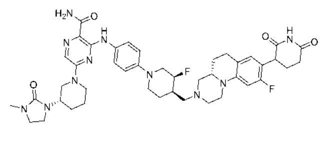 | 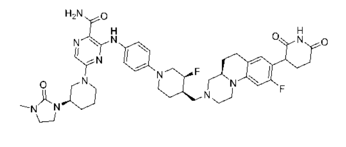 | 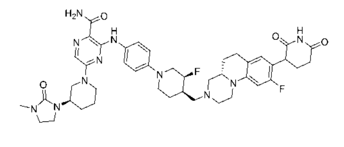 |
| 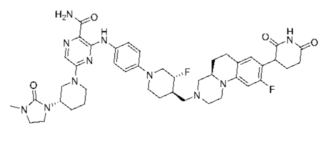 | 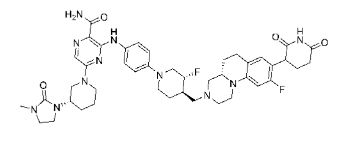 | 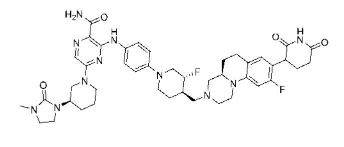 |
| 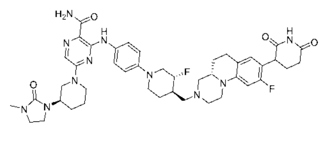 | 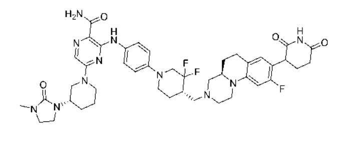 | 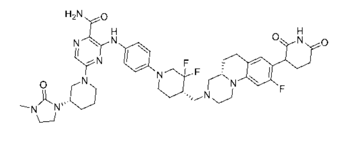 |
| 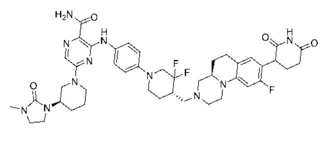 | 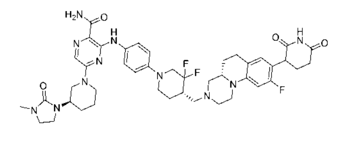 | 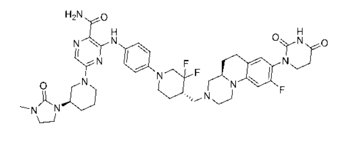 |
| 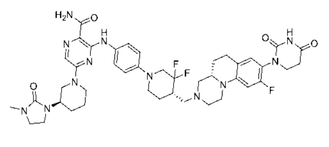 | 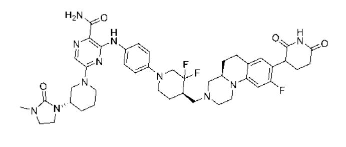 | 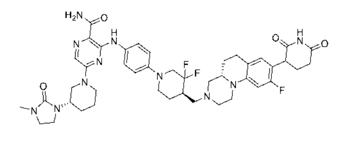 |
| 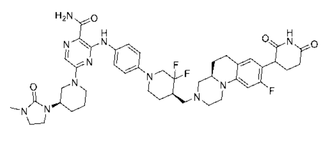 | 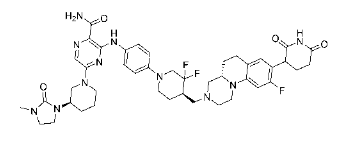 | 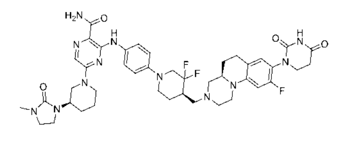 |
| 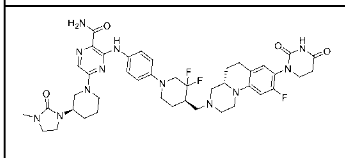 | 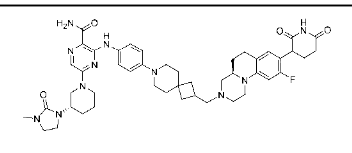 | 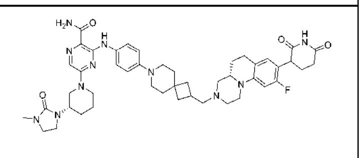 |
| 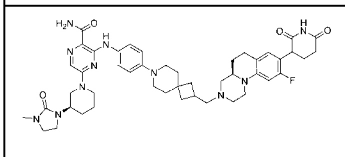 | 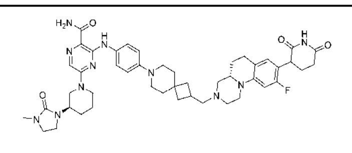 | 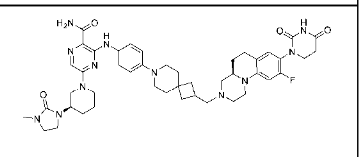 |
| 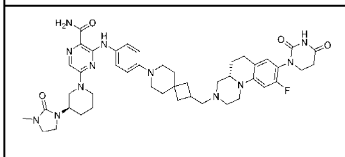 | 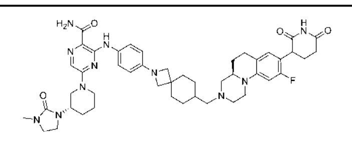 | 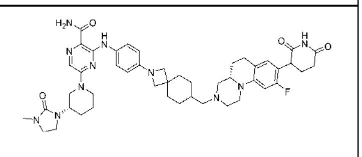 |
| 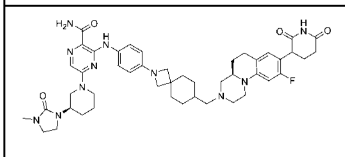 | 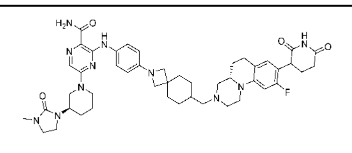 | 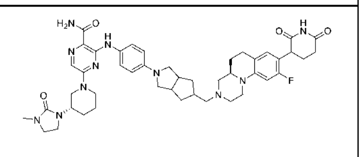 |
| 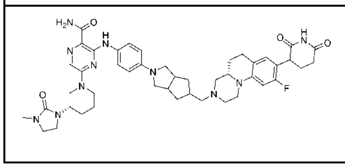 | 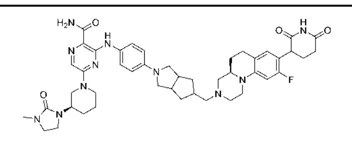 | 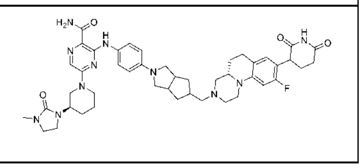 |

| | | |
|---|---|---|
| 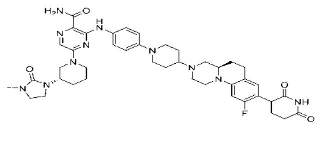 | 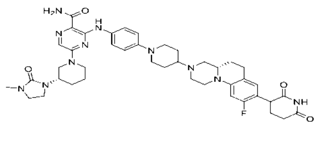 | 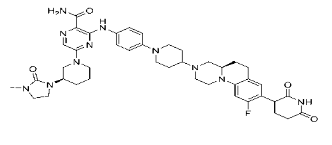 |
| 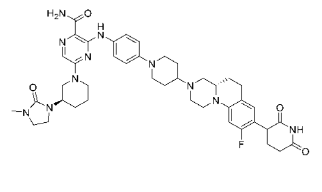 | 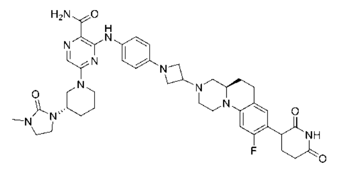 | 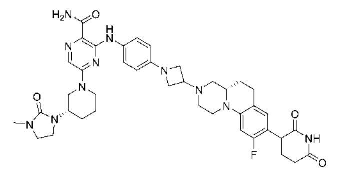 |
| 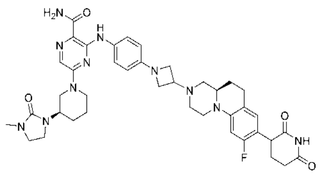 | 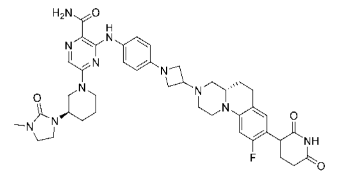 | 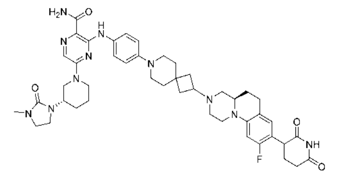 |
| 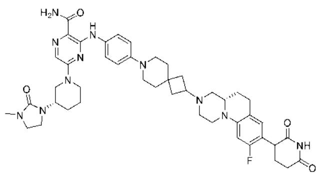 | 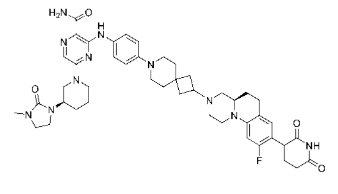 | 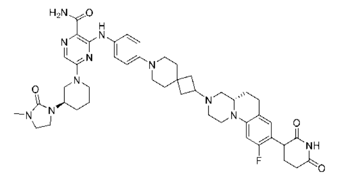 |
| 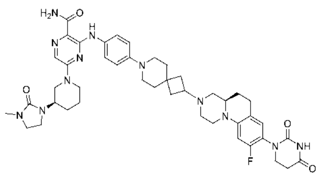 | 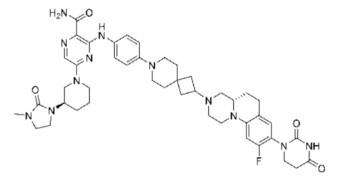 | 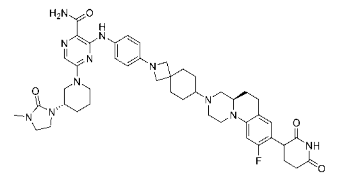 |
| 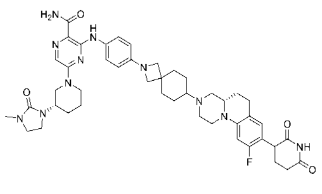 | 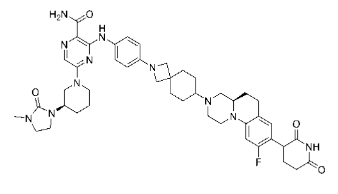 | 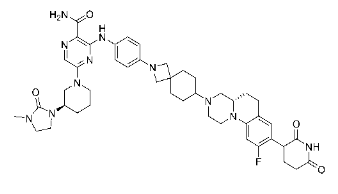 |
| 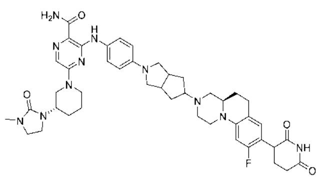 | 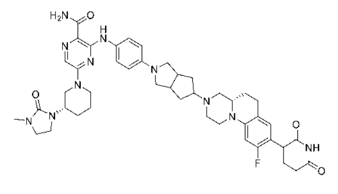 | 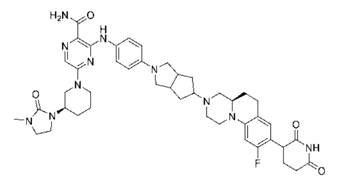 |
| 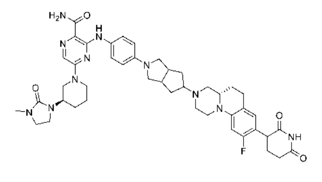 | 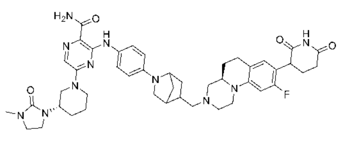 | 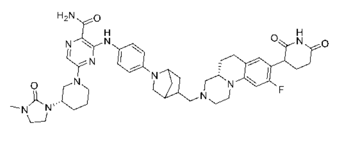 |
| 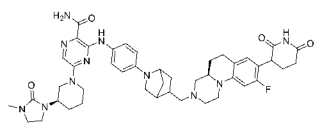 | 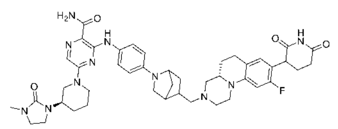 | 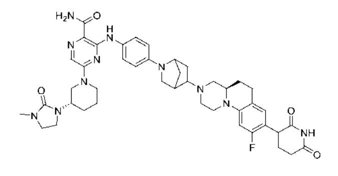 |

| | | |
|---|---|---|
| 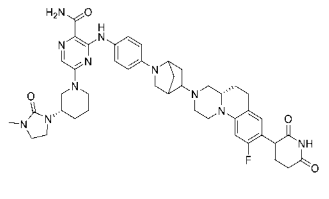 | 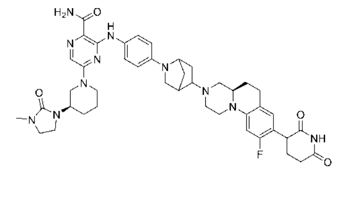 | 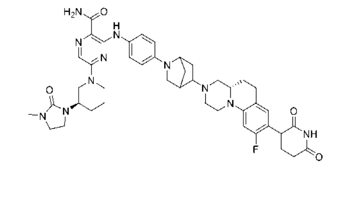 |
| 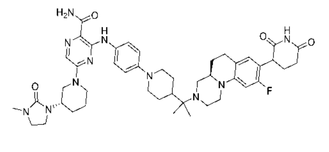 | 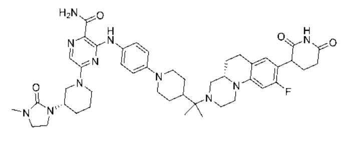 | 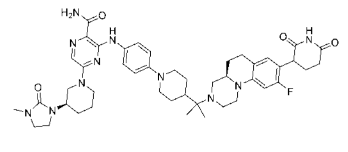 |
| 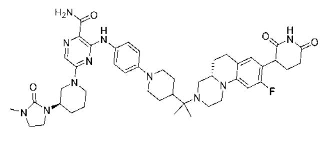 | 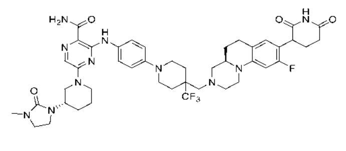 | 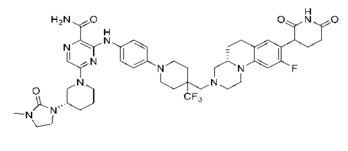 |
| 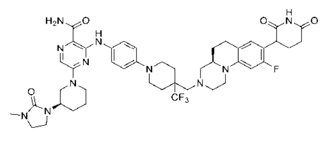 | 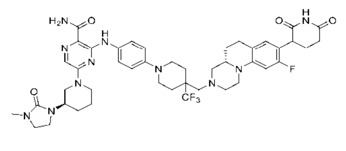 | 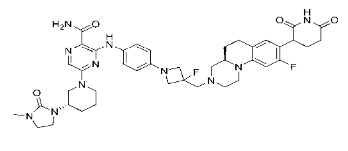 |
| 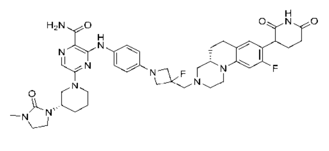 | 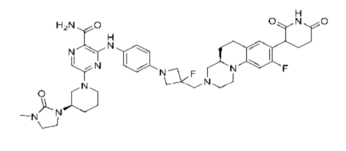 | 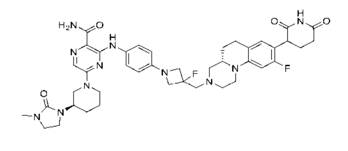 |
| 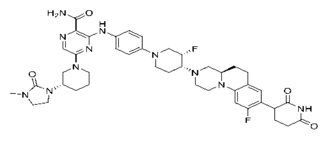 | 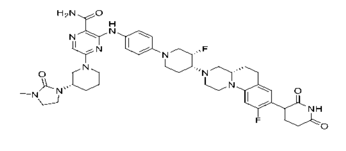 | 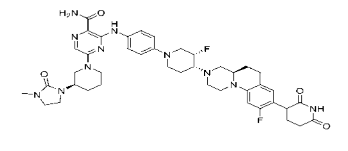 |
| 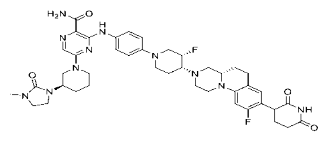 | 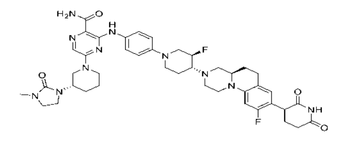 | 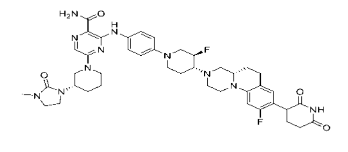 |
| 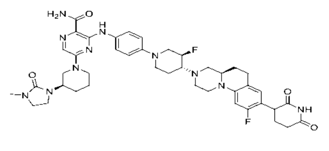 | 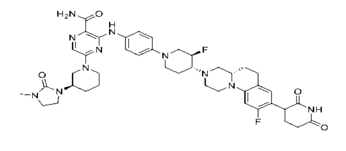 | 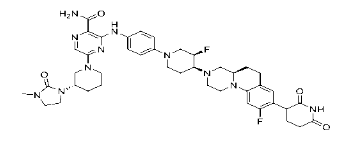 |
| 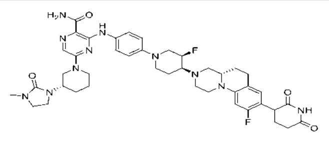 | 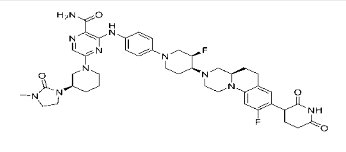 | 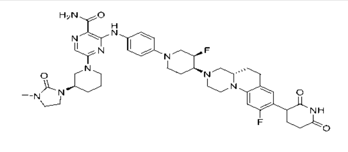 |
| 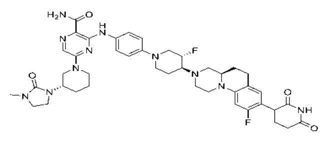 | 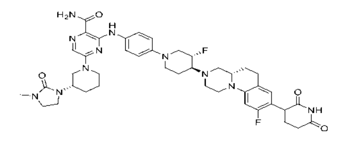 | 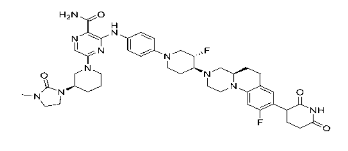 |

| | | |
|---|---|---|
| 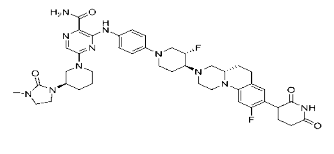 | 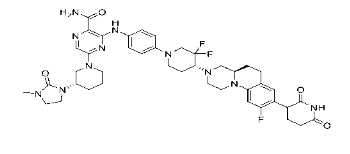 | 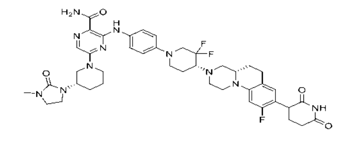 |
| 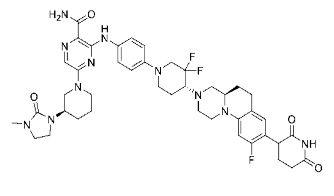 | 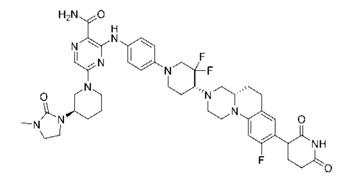 | 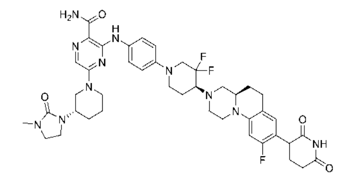 |
| 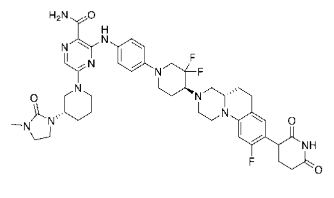 | 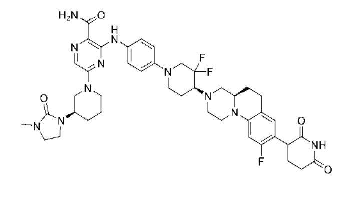 | 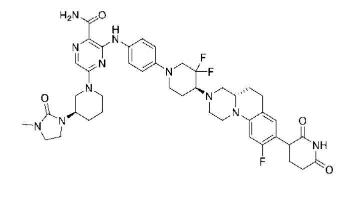 |
| 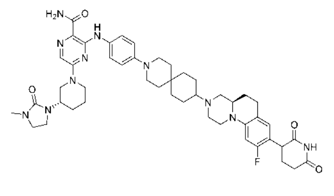 | 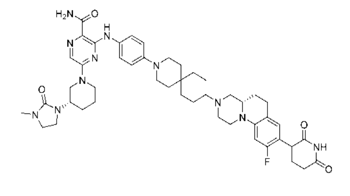 | 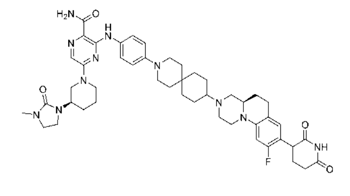 |
| 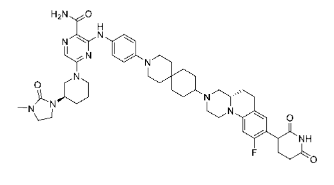 | 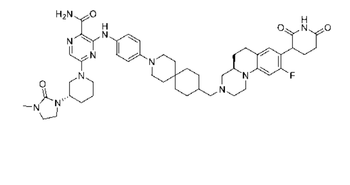 | 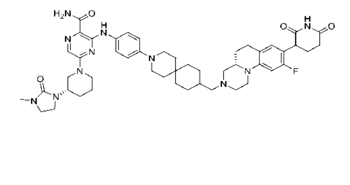 |
| 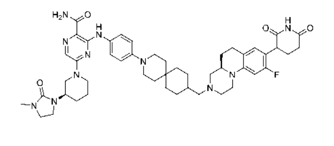 | 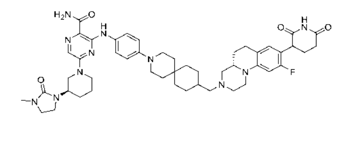 | 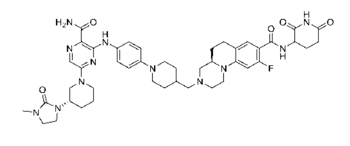 |
| 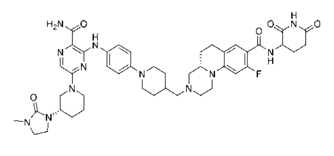 | 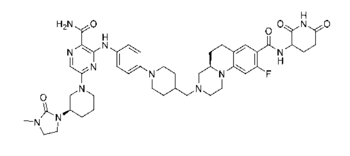 | 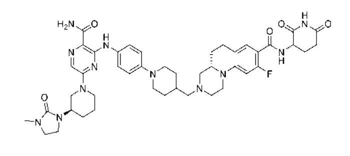 |
| 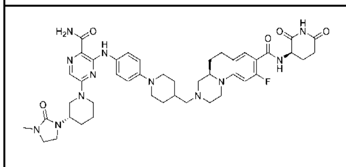 | 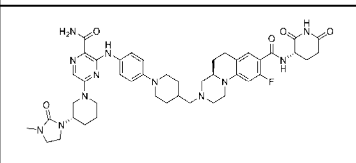 | 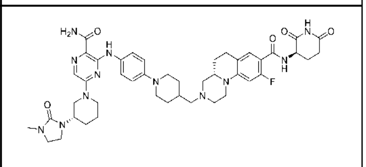 |
| 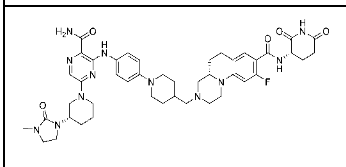 | 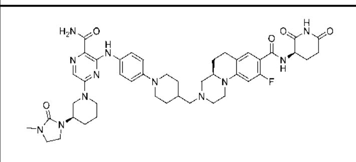 | 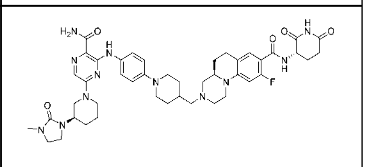 |
| 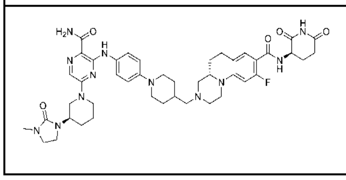 | 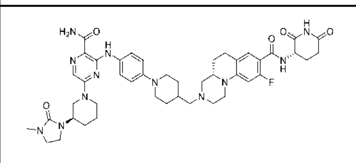 | 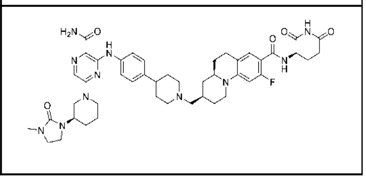 |

| | | |
|---|---|---|
| 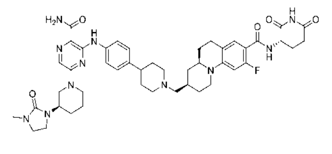 | 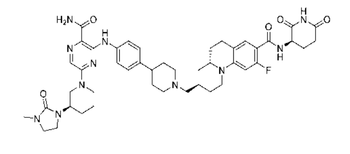 | 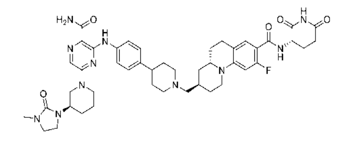 |
| 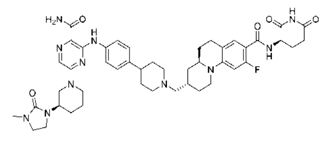 | 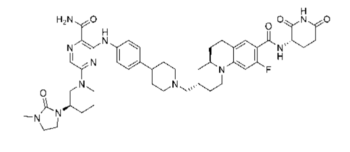 | 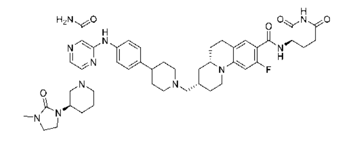 |
| 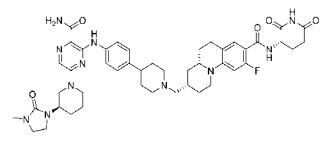 | 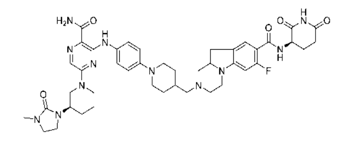 | 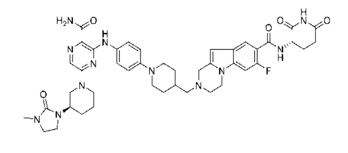 |
| 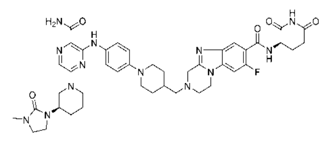 | 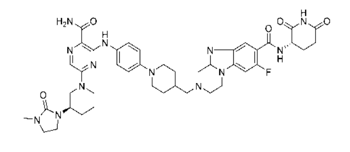 | 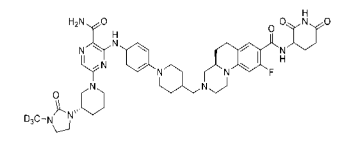 |
| 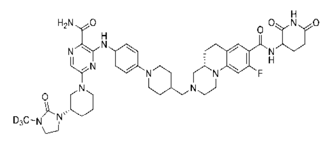 | 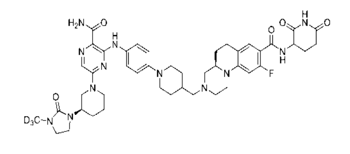 | 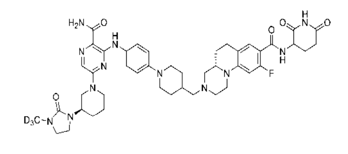 |
| 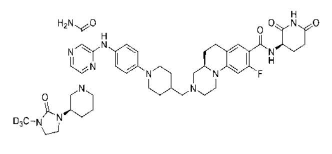 | 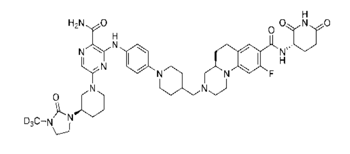 | 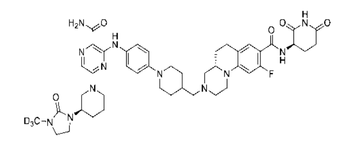 |
| 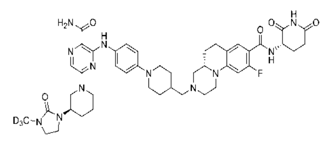 | 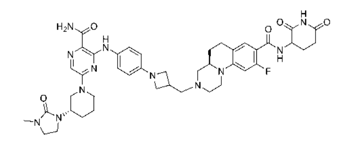 | 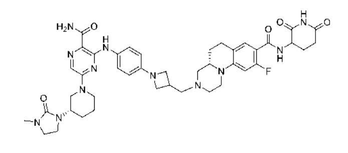 |
| 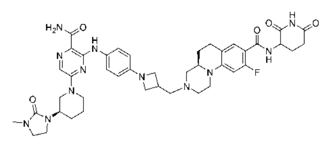 | 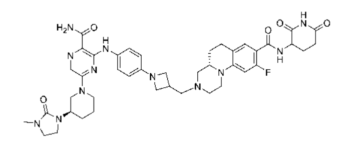 | 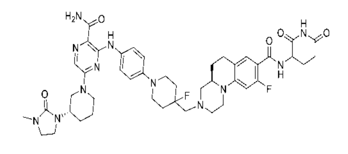 |
| 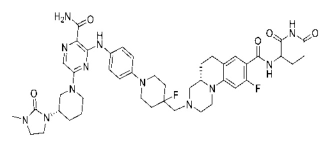 | 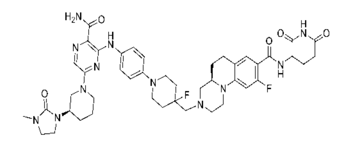 | 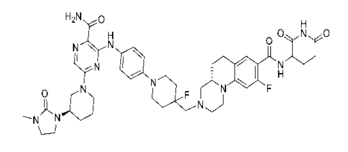 |
| 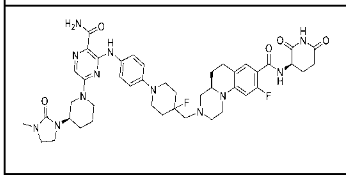 | 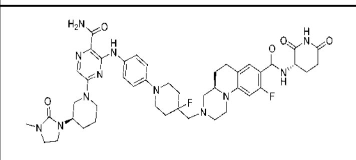 | 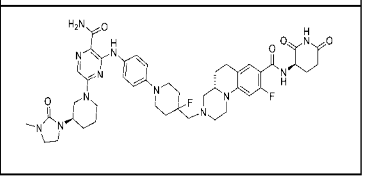 |

| | | |
|---|---|---|
| 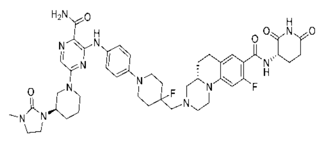 | 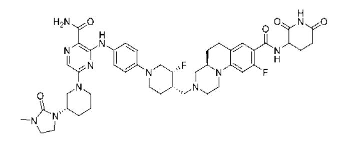 | 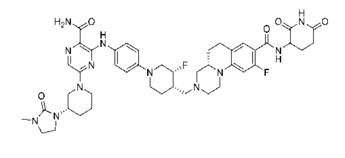 |
| 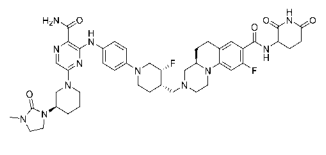 | 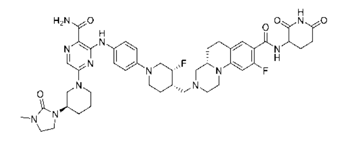 | 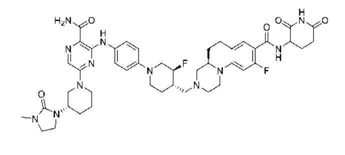 |
| 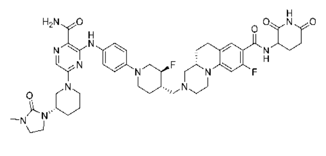 | 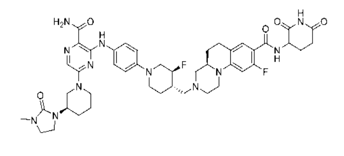 | 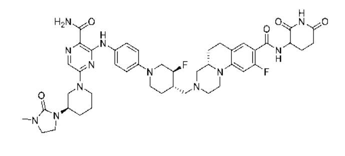 |
| 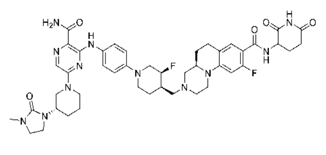 | 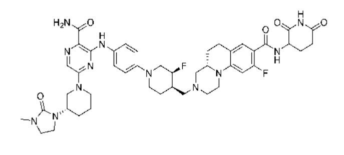 | 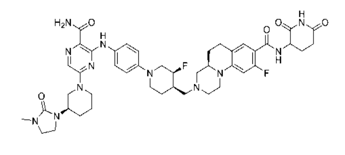 |
| 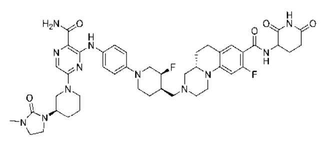 | 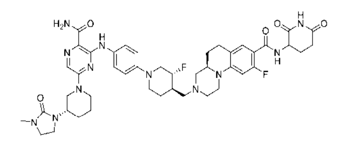 | 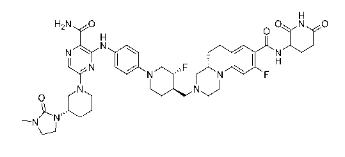 |
| 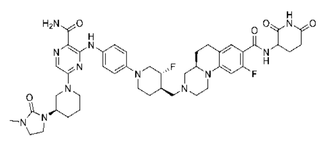 | 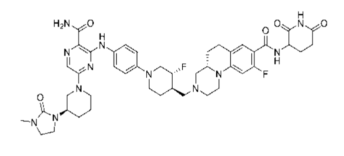 | 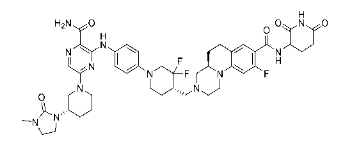 |
| 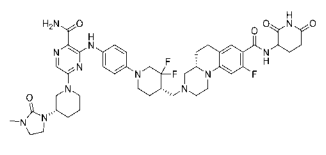 | 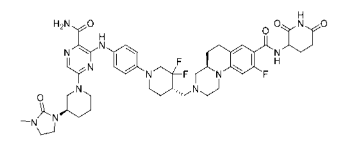 | 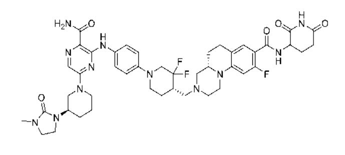 |
| 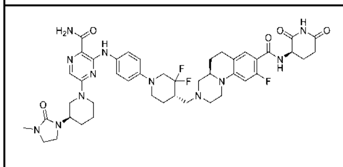 | 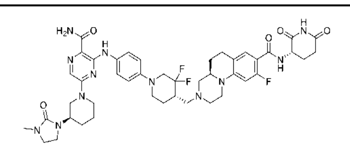 | 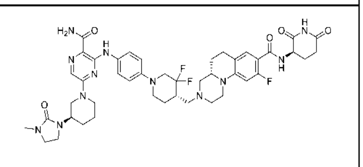 |
| 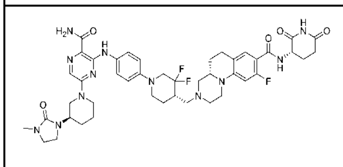 | 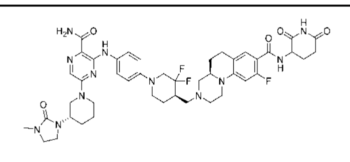 | 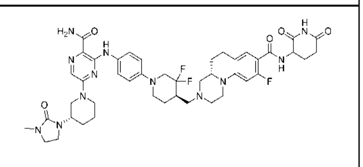 |
| 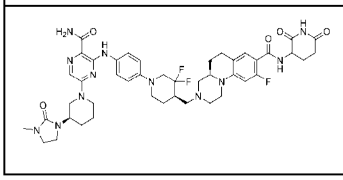 | 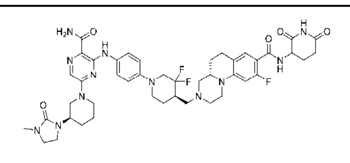 | 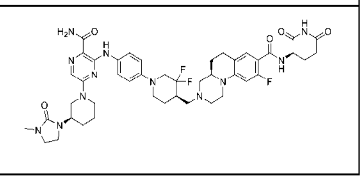 |

| | | |
|---|---|---|
| 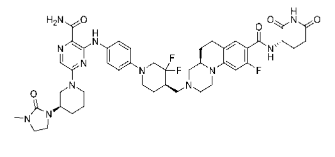 | 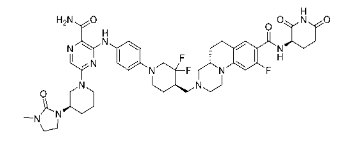 | 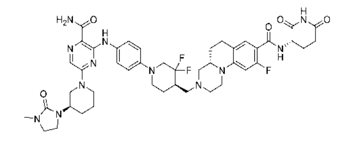 |
| 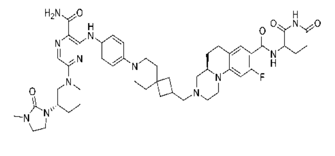 | 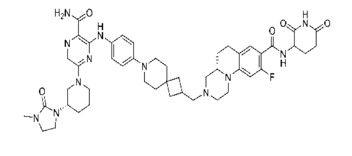 | 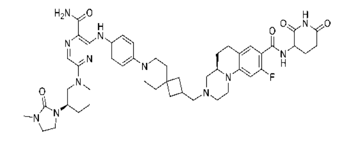 |
| 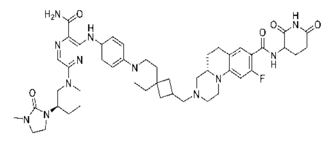 | 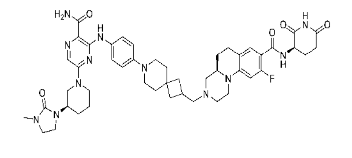 | 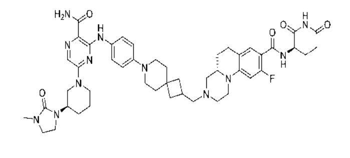 |
| 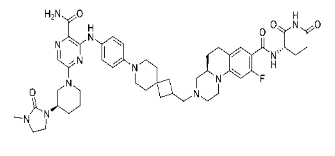 | 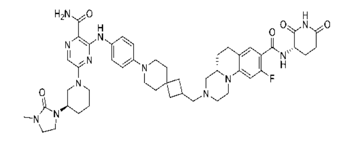 | 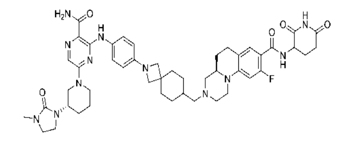 |
| 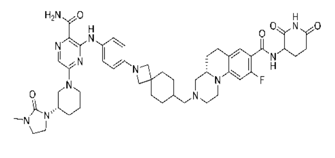 | 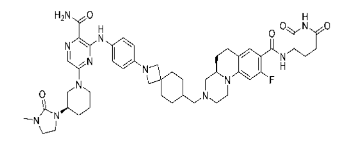 | 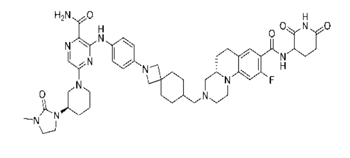 |
| 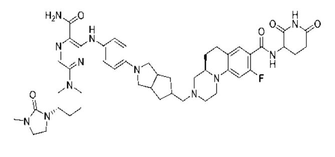 | 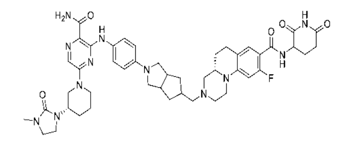 | 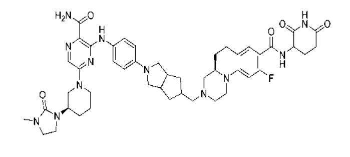 |
| 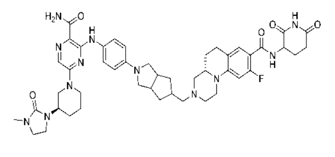 | 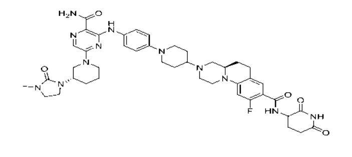 | 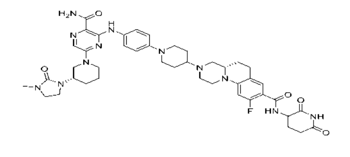 |
| 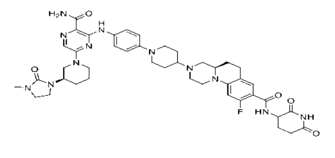 | 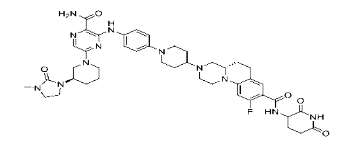 | 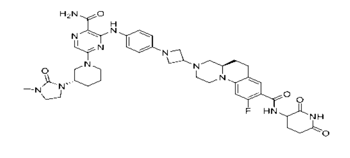 |
| 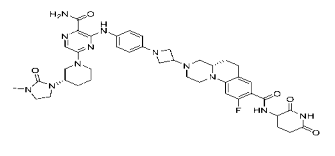 | 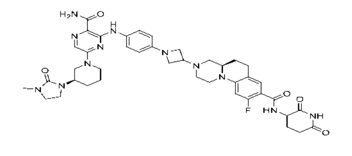 | 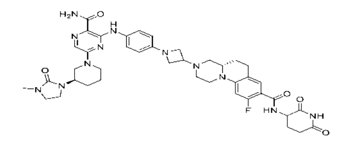 |
| 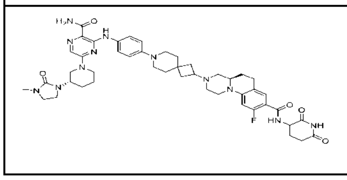 | 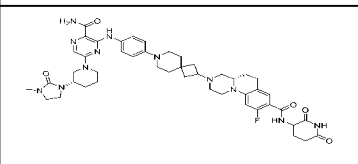 | 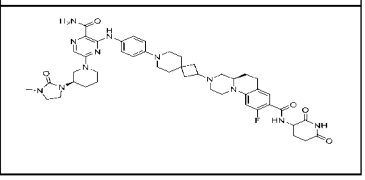 |

| | | |
|---|---|---|
| 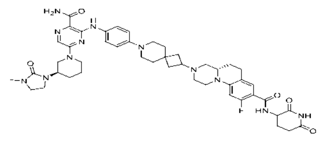 | 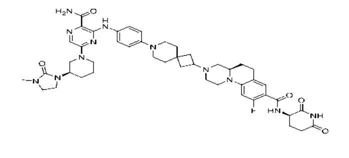 | 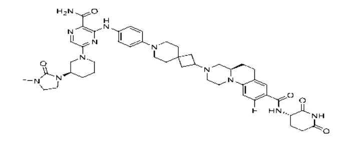 |
| 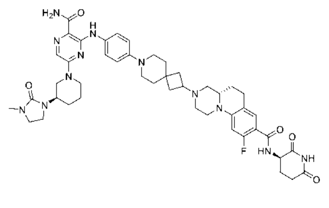 | 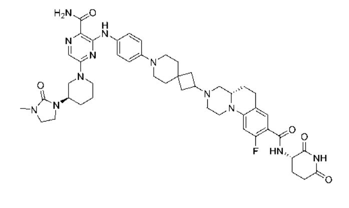 | 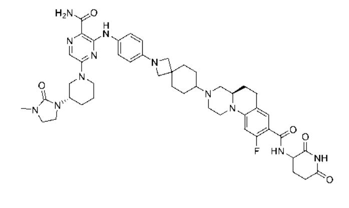 |
| 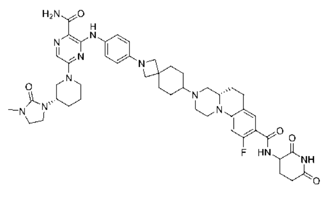 | 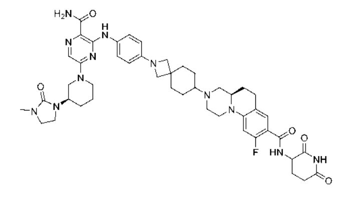 | 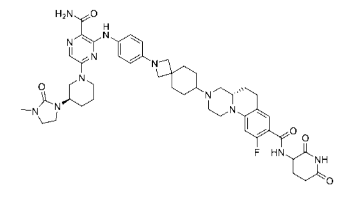 |
| 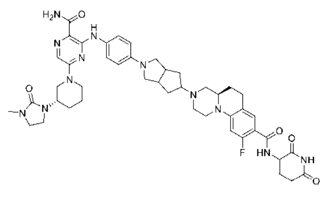 | 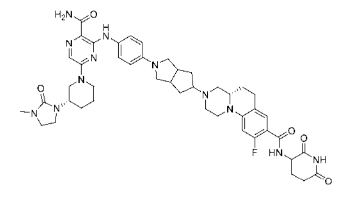 | 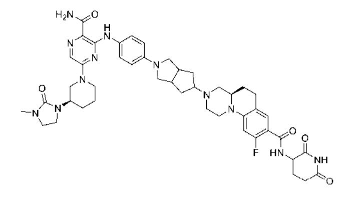 |
| 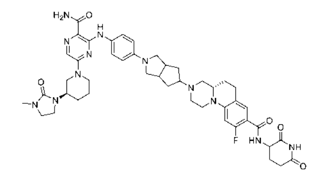 | 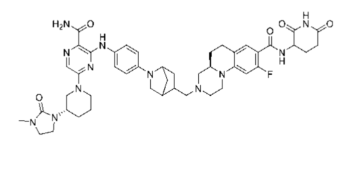 | 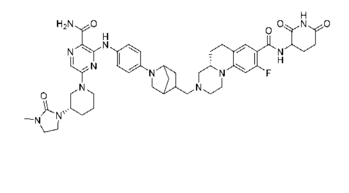 |
| 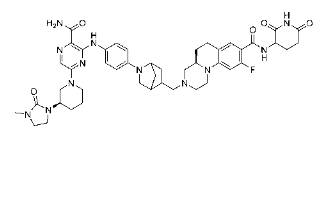 | 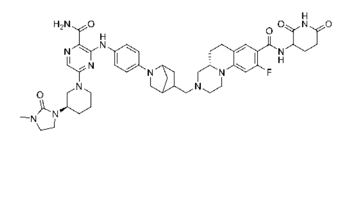 | 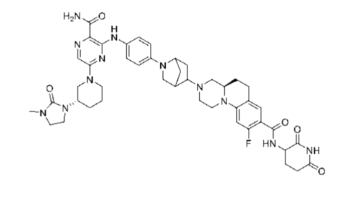 |
| 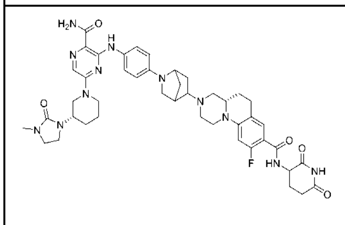 | 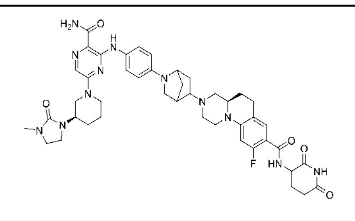 | 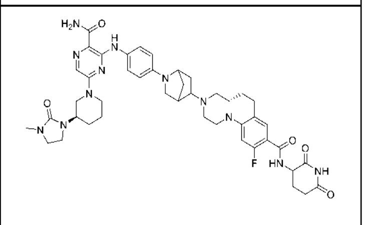 |
| 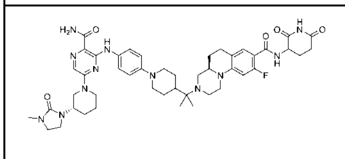 | 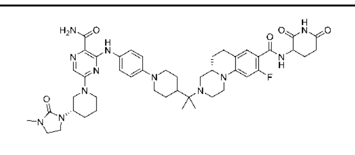 | 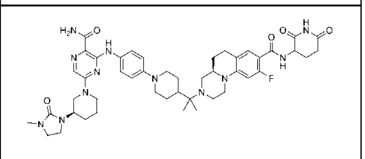 |
| 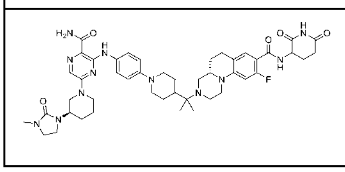 | 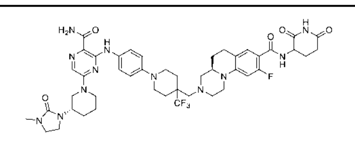 | 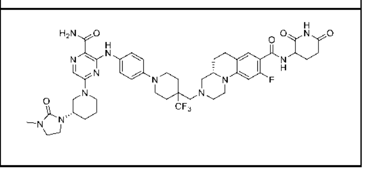 |

| | | |
|---|---|---|
| 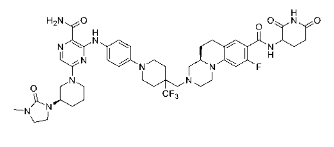 | 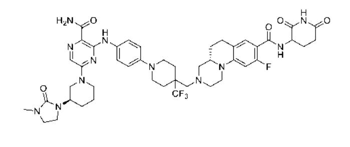 | 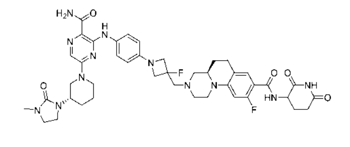 |
| 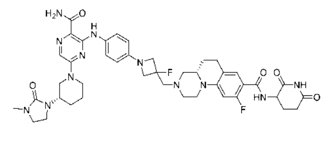 | 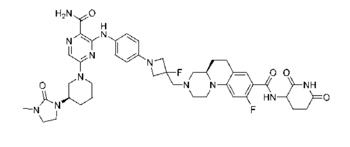 | 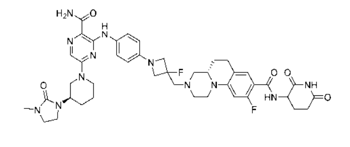 |
| 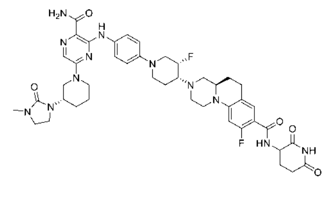 | 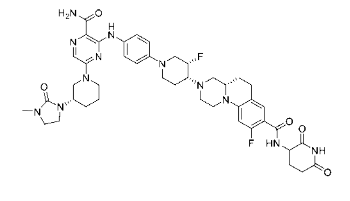 | 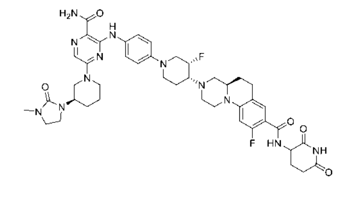 |
| 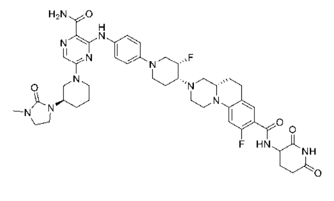 | 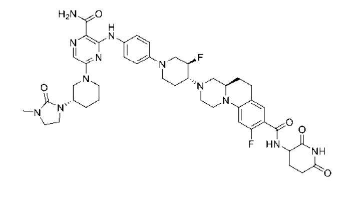 | 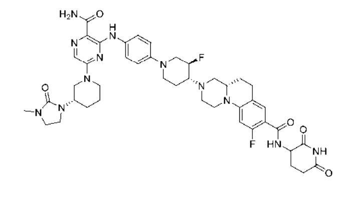 |
| 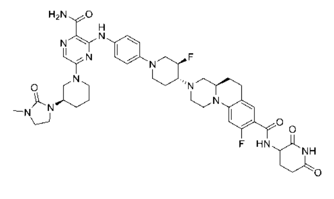 | 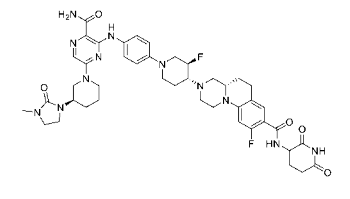 | 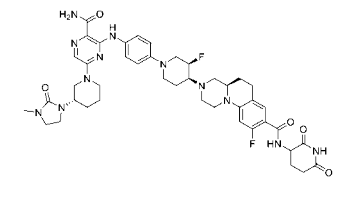 |
| 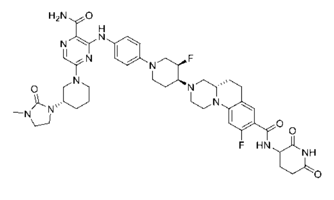 | 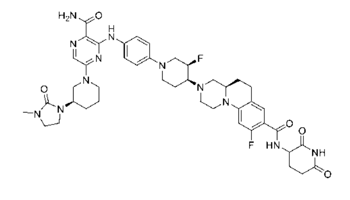 | 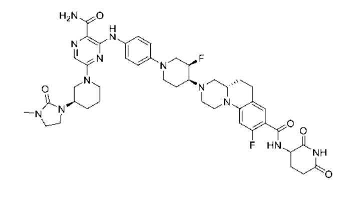 |
| 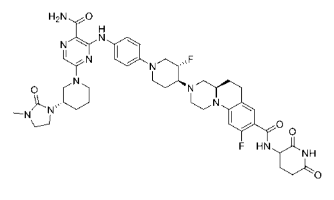 | 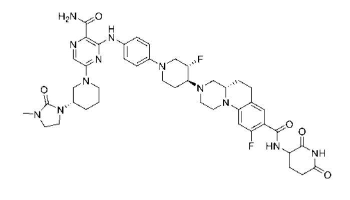 | 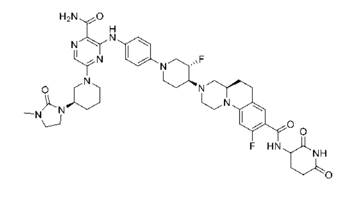 |
| 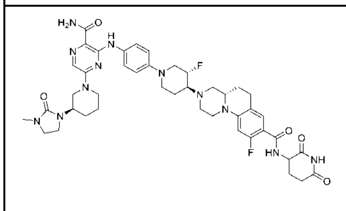 | 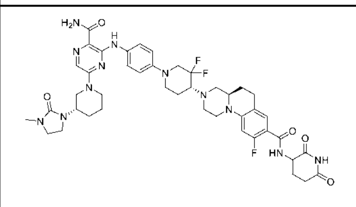 | 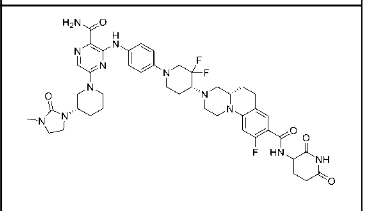 |
| 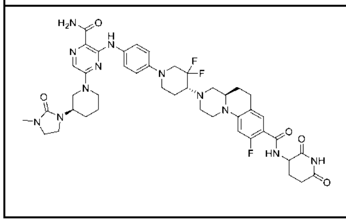 | 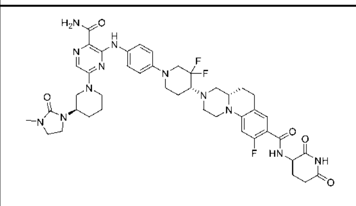 | 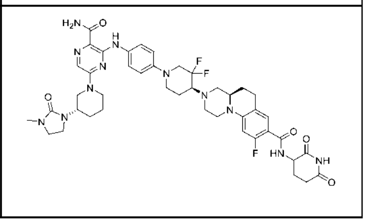 |

| | | |
|---|---|---|
| 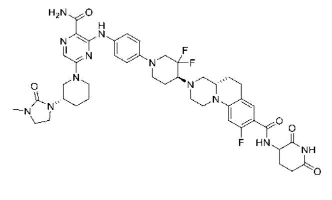 | 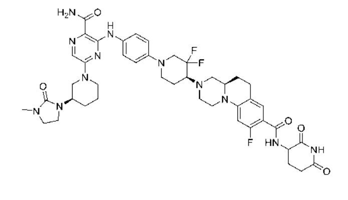 | 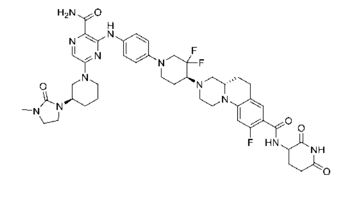 |
| 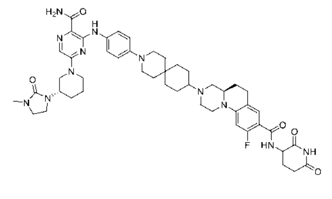 | 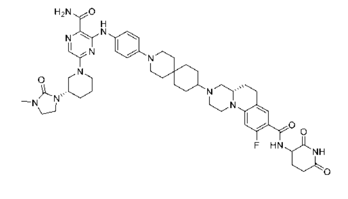 | 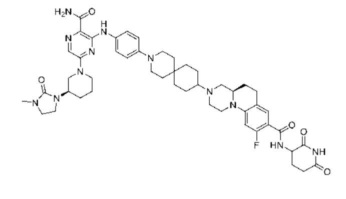 |
| 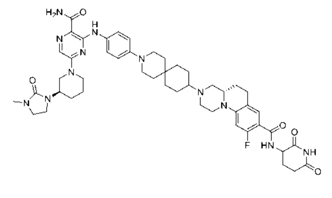 | 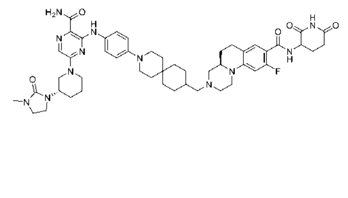 | 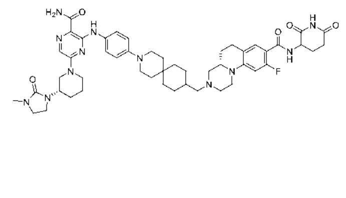 |
| 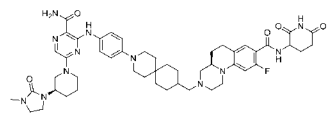 | 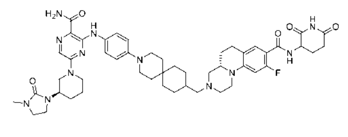 | 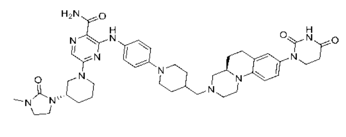 |
| 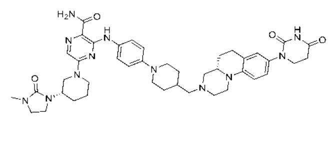 | 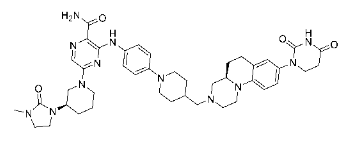 | 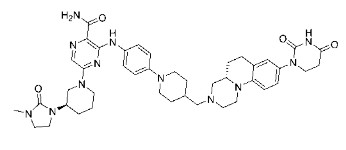 |
| 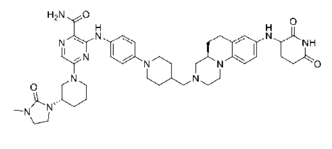 | 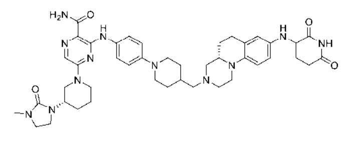 | 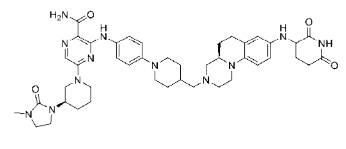 |
| 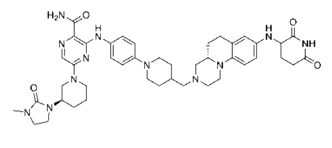 | 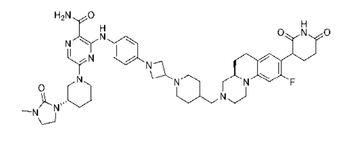 | 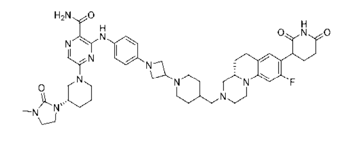 |
| 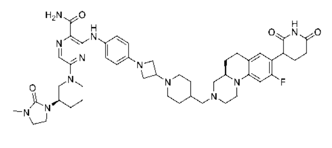 | 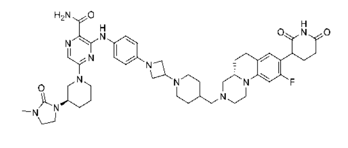 | 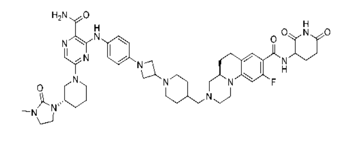 |
| 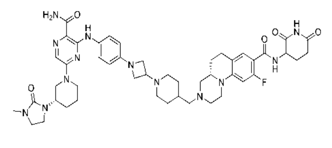 | 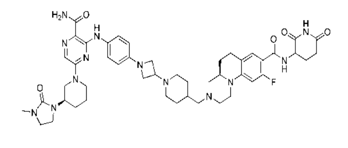 | 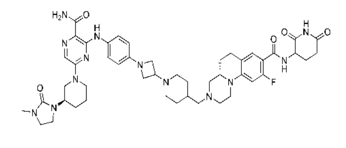 |
| 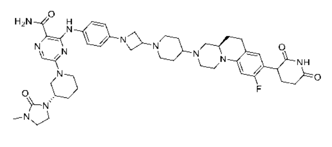 | 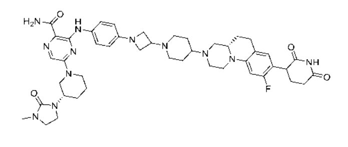 | 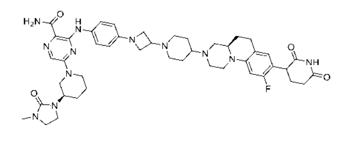 |

| | | |
|---|---|---|
| 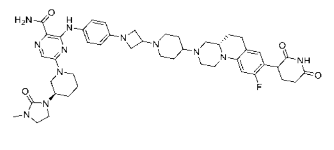 | 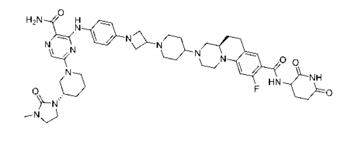 | 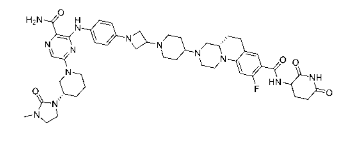 |
| 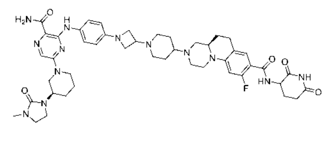 | 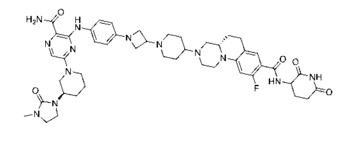 | 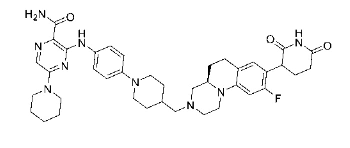 |
| 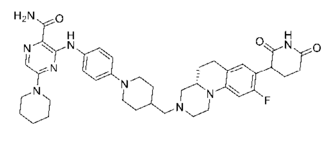 | 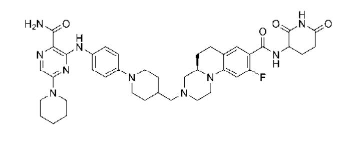 | 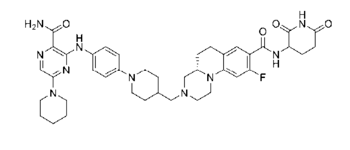 |
| 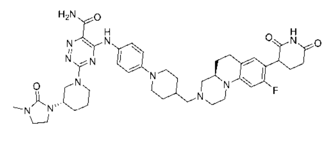 | 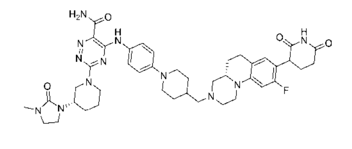 | 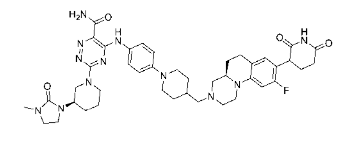 |
| 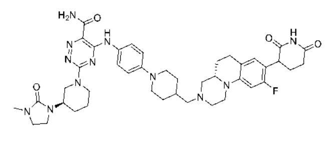 | 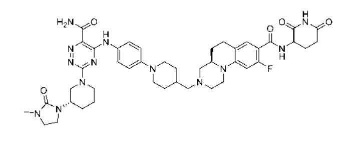 | 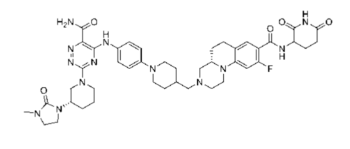 |
| 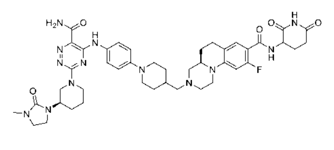 | 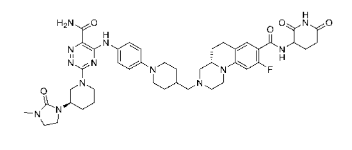 | 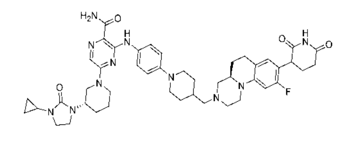 |
| 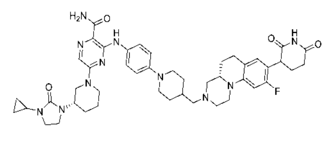 | 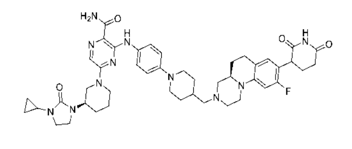 | 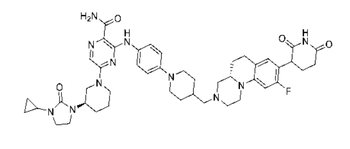 |
| 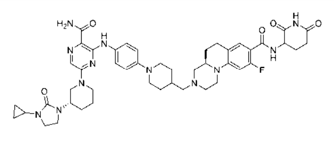 | 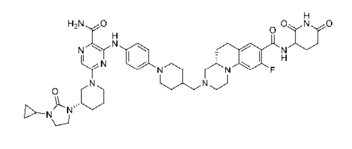 | 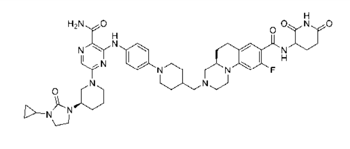 |
| 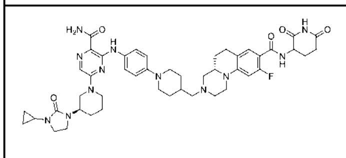 | 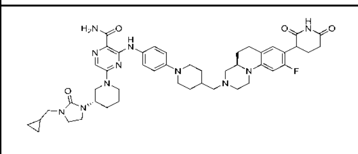 | 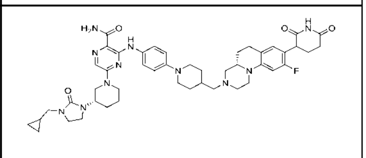 |
| 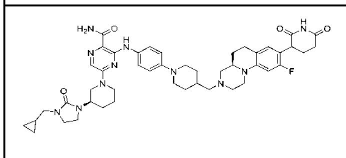 | 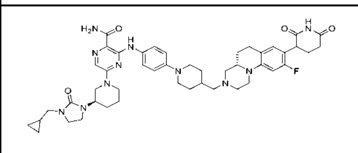 | 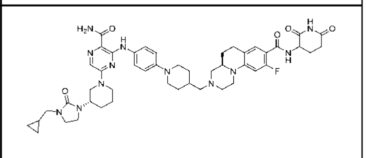 |
| 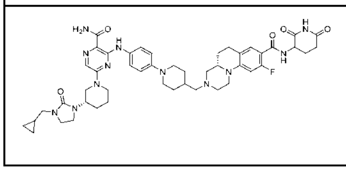 | 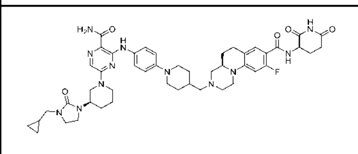 | 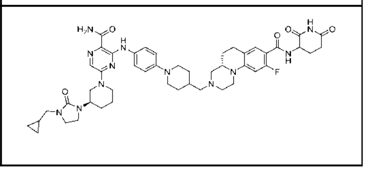 |

| | | |
|---|---|---|
| 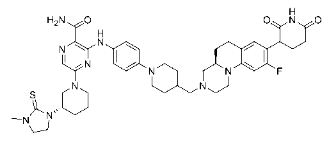 | 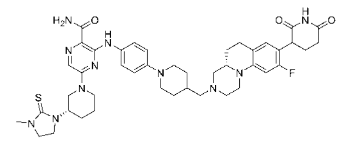 | 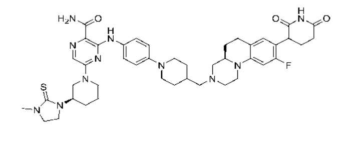 |
| 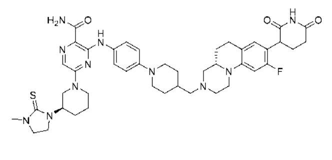 | 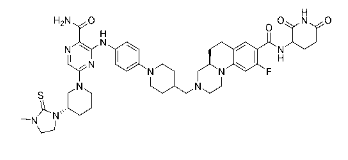 | 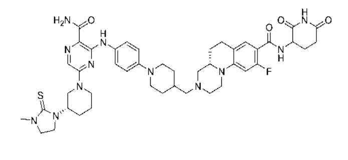 |
| 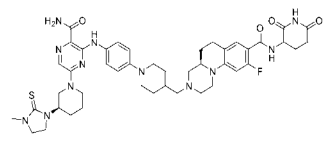 | 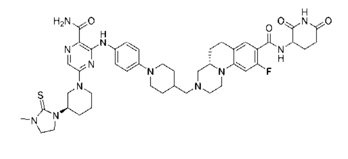 | 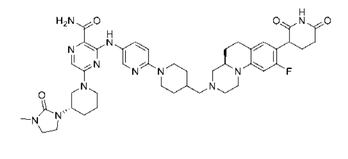 |
| 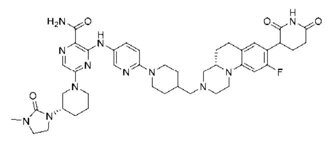 | 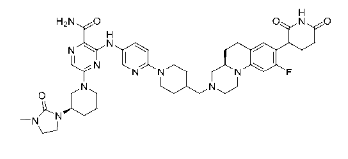 | 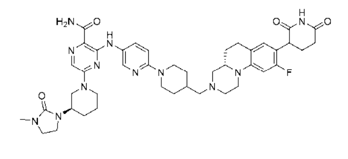 |
| 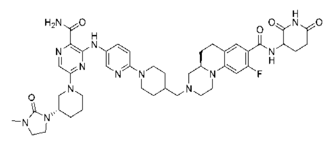 | 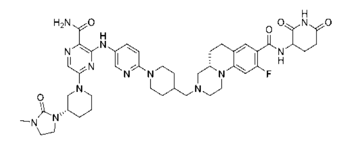 | 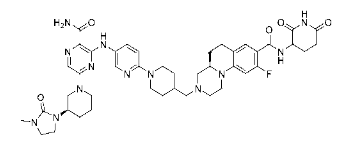 |
| 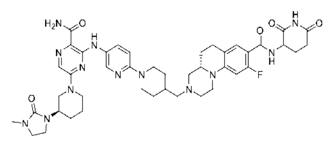 | 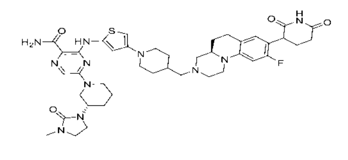 | 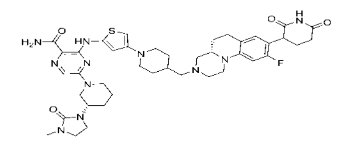 |
| 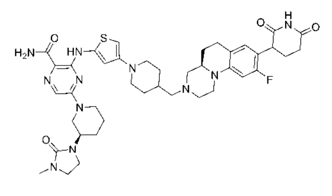 | 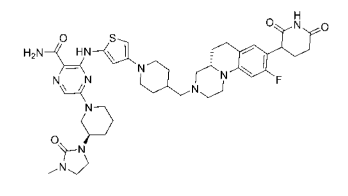 | 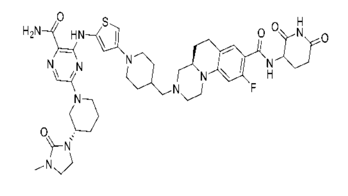 |
| 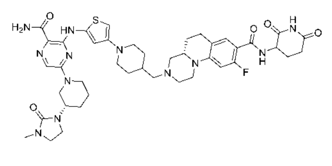 | 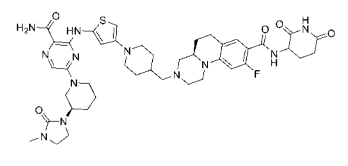 | 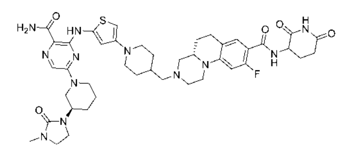 |
| 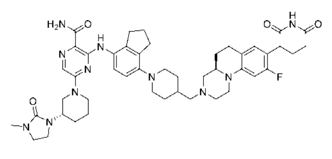 | 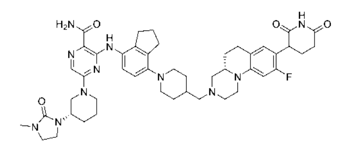 | 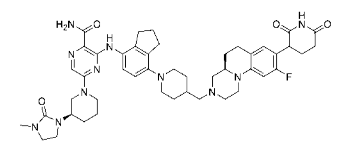 |
| 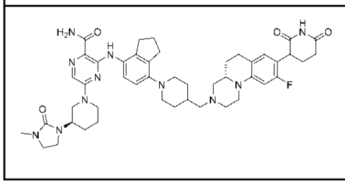 | 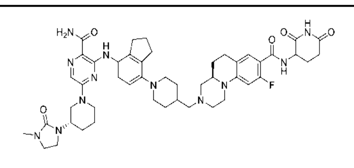 | 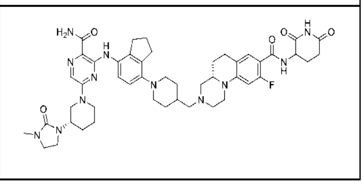 |

| | | |
|---|---|---|
| 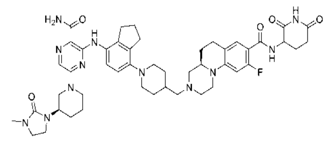 | 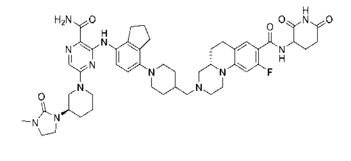 | 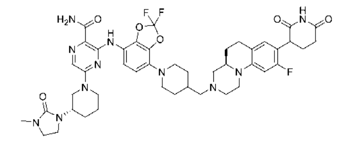 |
| 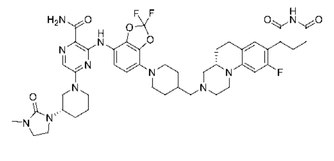 | 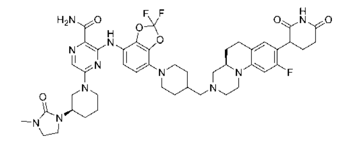 | 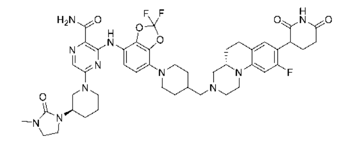 |
| 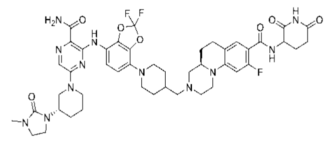 | 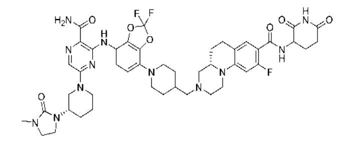 | 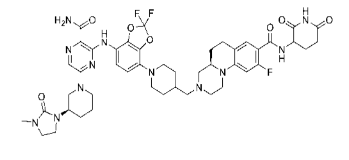 |
| 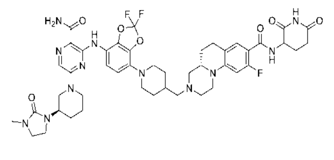 | 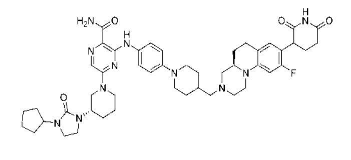 | 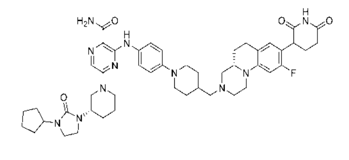 |
| 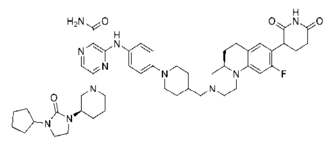 | 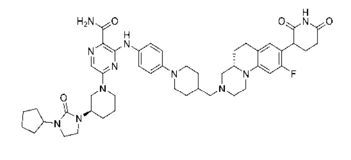 | 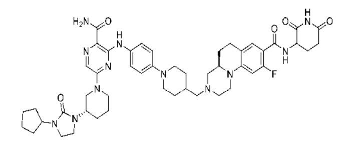 |
| 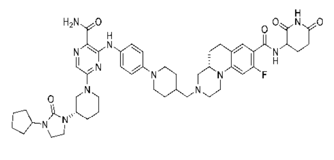 | 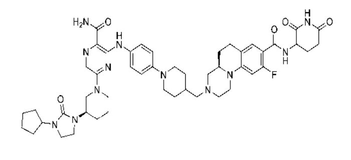 | 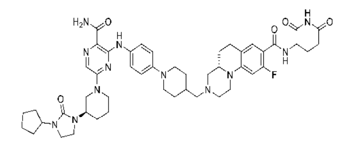 |
| 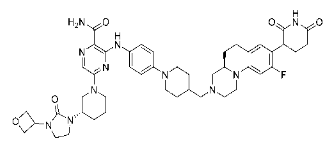 | 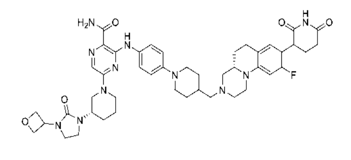 | 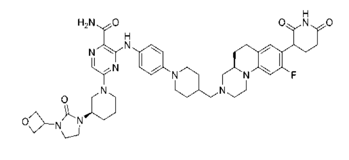 |
| 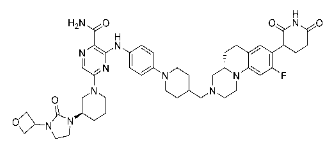 | 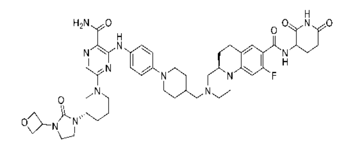 | 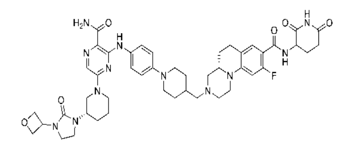 |
| 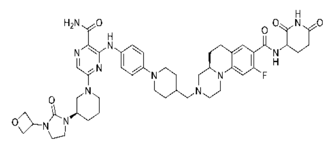 | 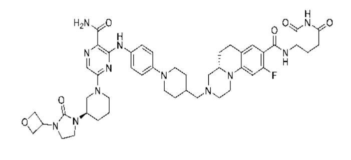 | 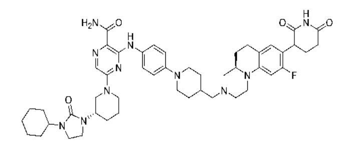 |
| 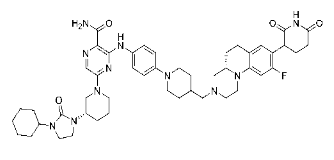 | 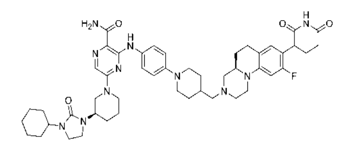 | 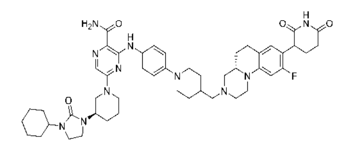 |

| | | |
|---|---|---|
| 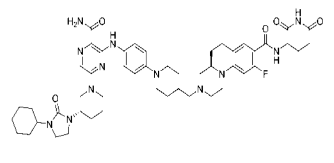 | 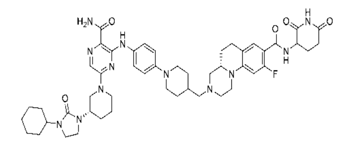 | 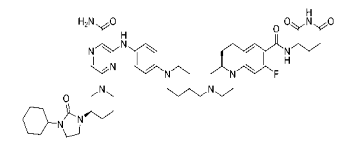 |
| 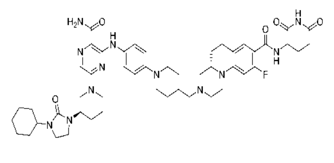 | 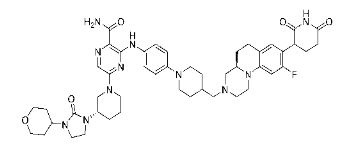 | 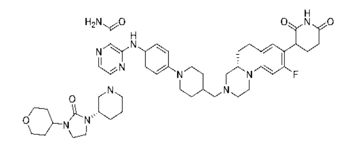 |
| 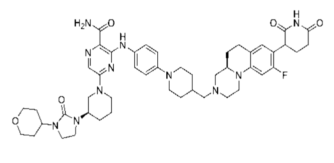 | 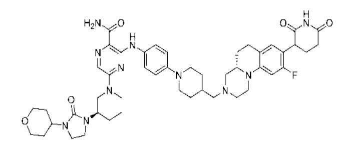 | 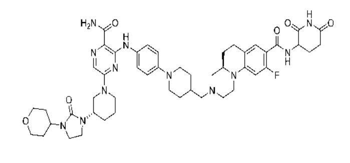 |
| 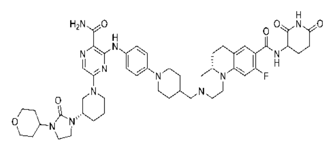 | 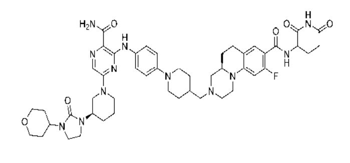 | 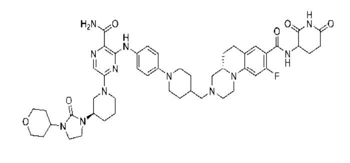 |
| 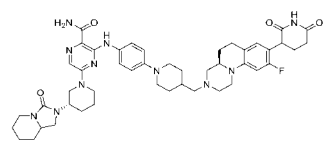 | 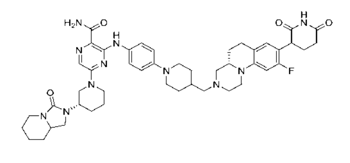 | 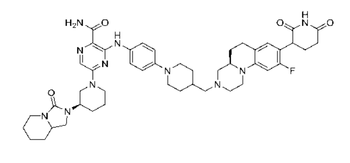 |
| 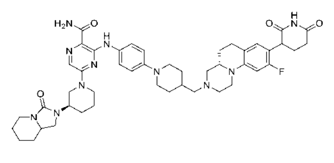 | 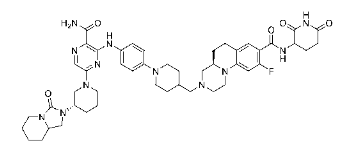 | 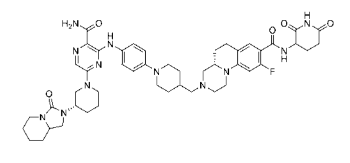 |
| 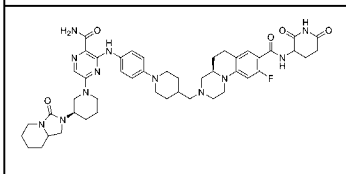 | 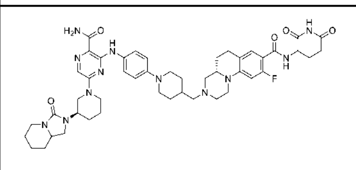 | 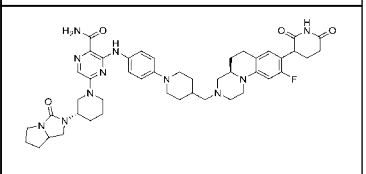 |
| 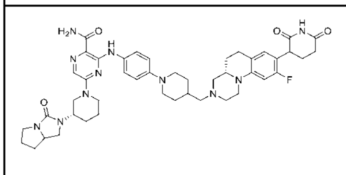 | 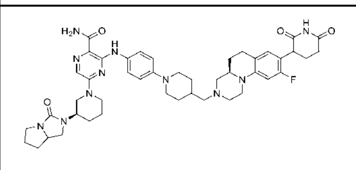 | 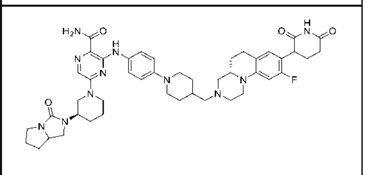 |
| 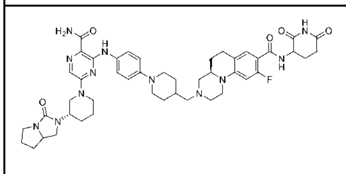 | 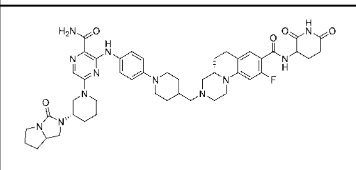 | 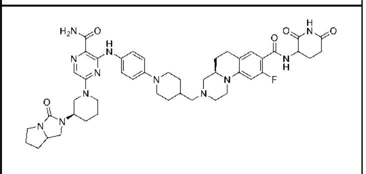 |
| 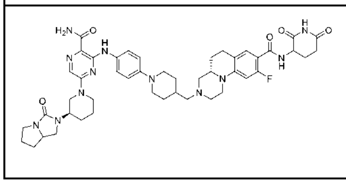 | 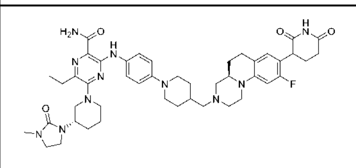 | 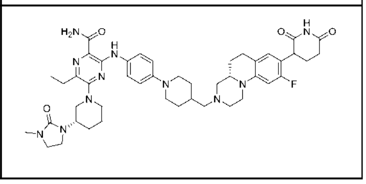 |

| | | |
|---|---|---|
| 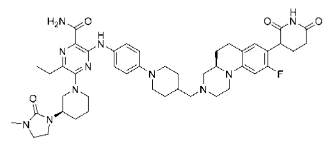 | 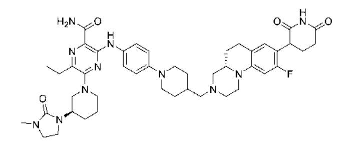 | 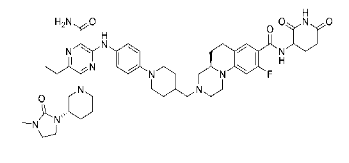 |
| 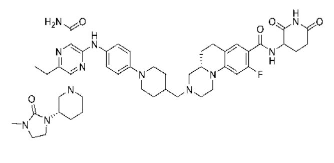 | 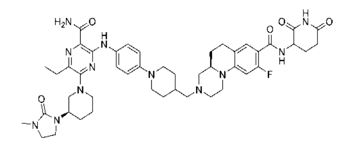 | 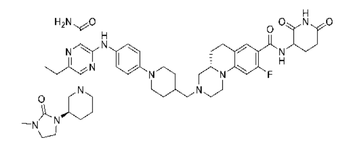 |
| 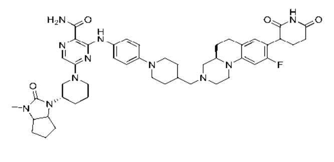 | 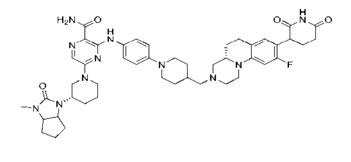 | 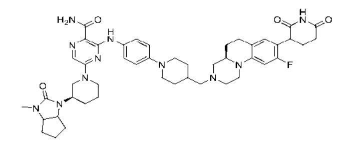 |
| 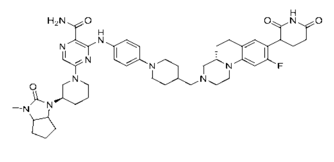 | 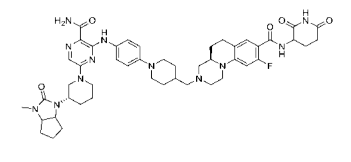 | 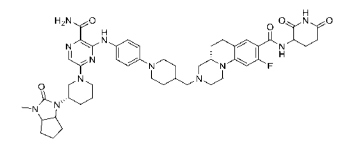 |
| 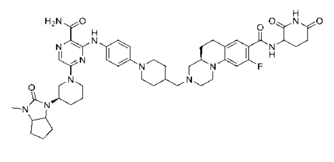 | 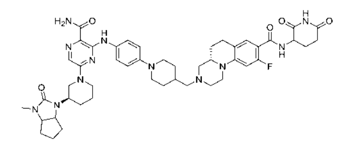 | 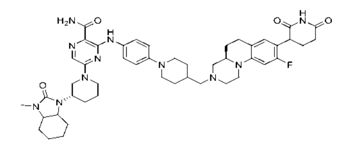 |
| 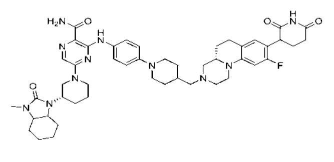 | 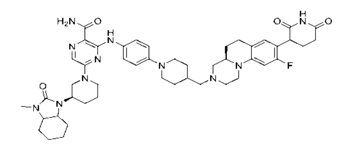 | 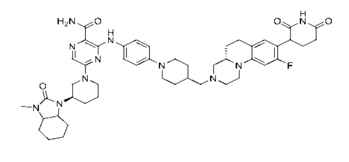 |
| 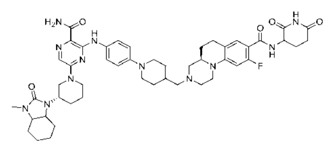 | 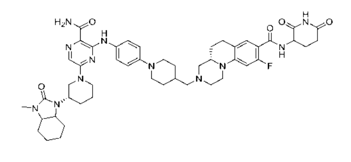 | 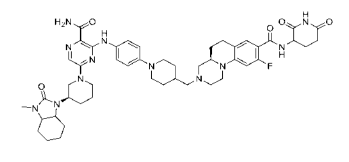 |
| 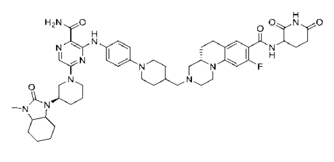 | 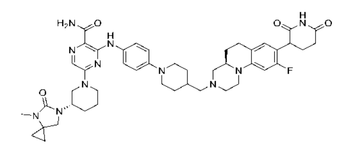 | 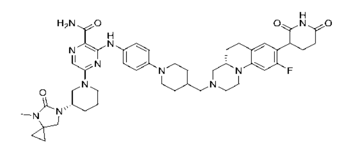 |
| 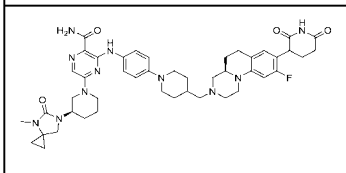 | 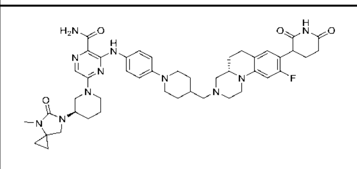 | 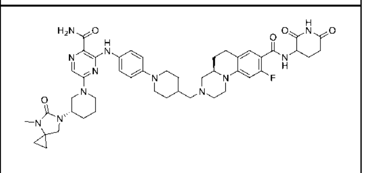 |
| 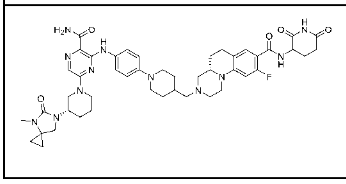 | 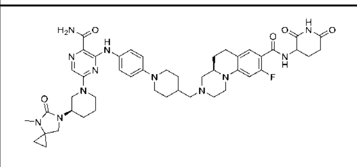 | 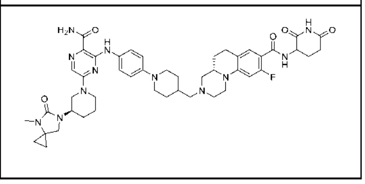 |

| | | |
|---|---|---|
| 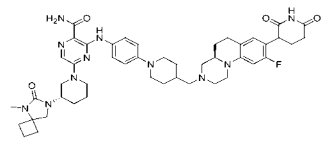 | 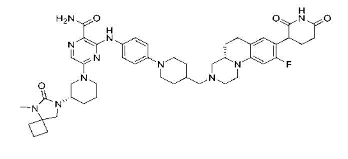 | 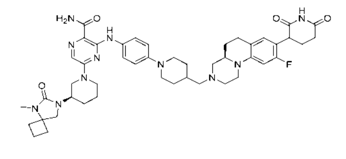 |
| 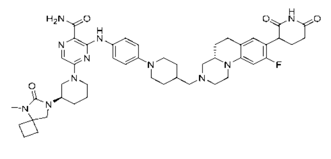 | 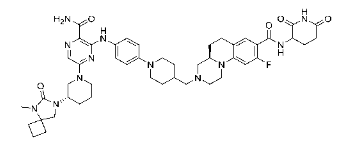 | 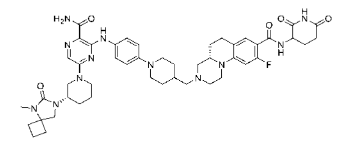 |
| 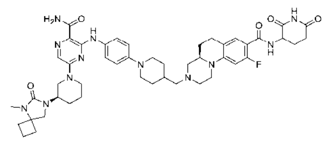 | 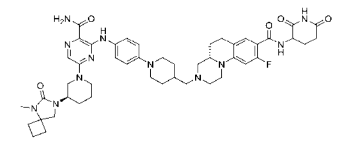 | 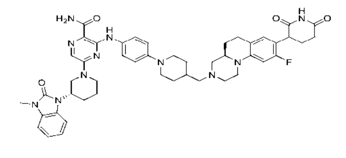 |
| 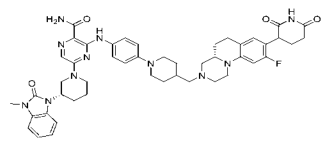 | 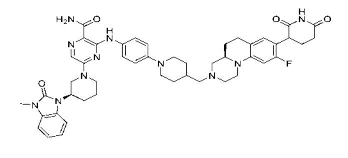 | 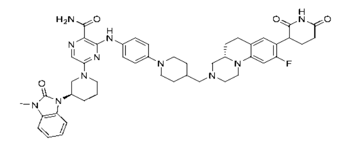 |
| 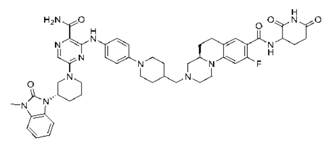 | 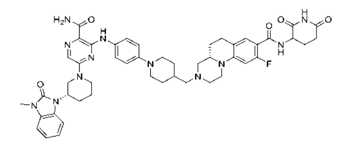 | 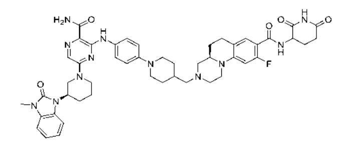 |
| 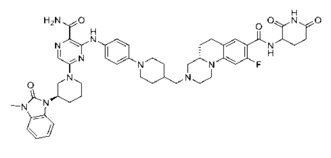 | 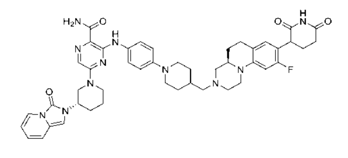 | 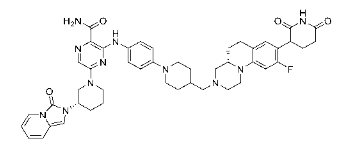 |
| 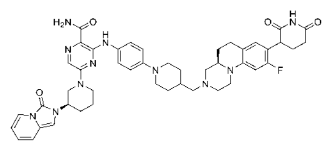 | 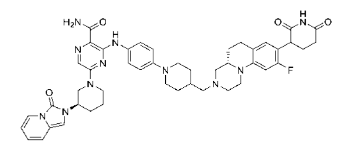 | 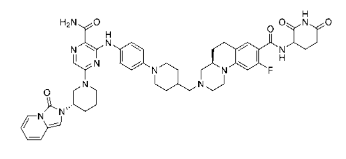 |
| 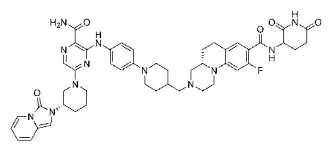 | 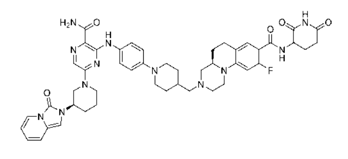 | 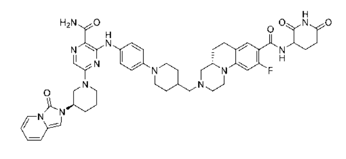 |
| 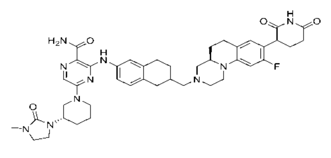 | 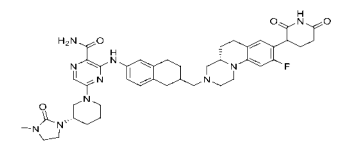 | 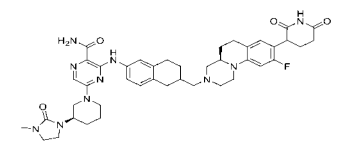 |
| 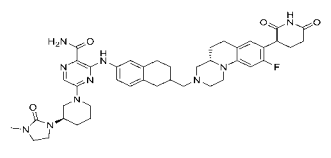 | 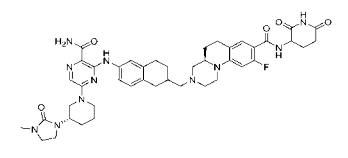 | 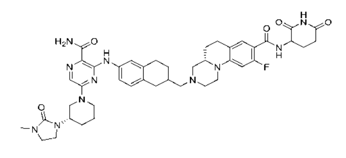 |

| | | |
|---|---|---|
| 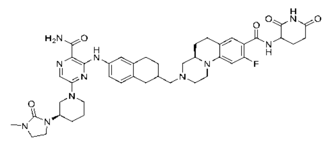 | 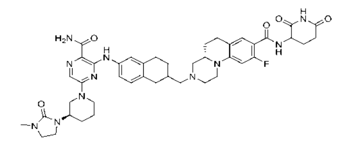 | 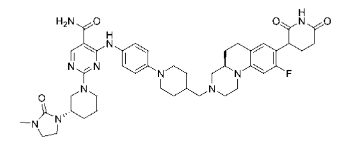 |
| 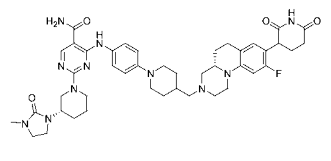 | 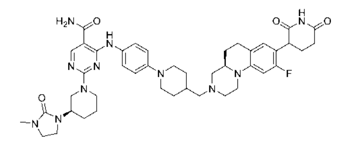 | 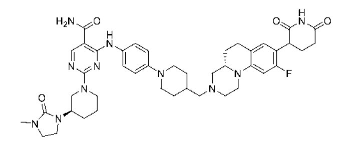 |
| 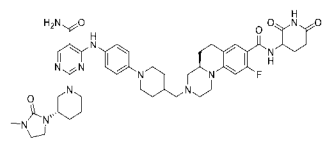 | 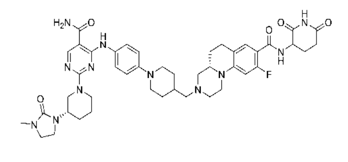 | 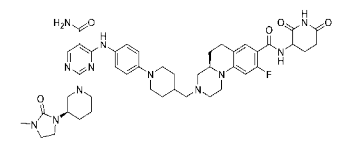 |
| 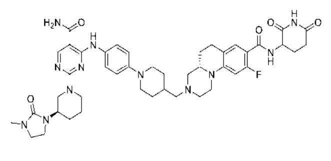 | 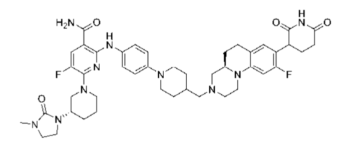 | 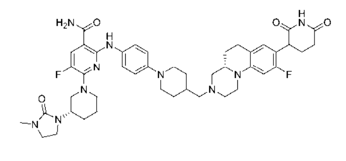 |
| 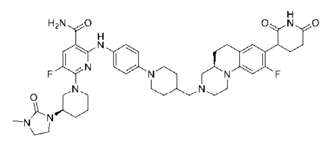 | 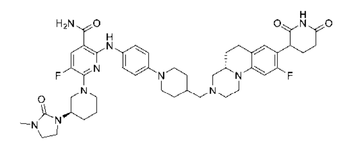 | 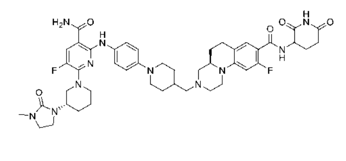 |
| 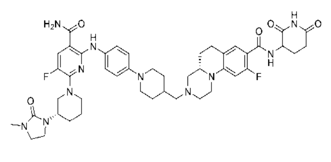 | 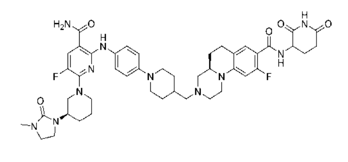 | 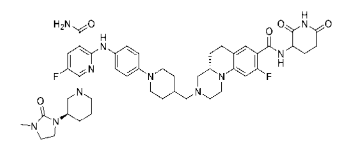 |
| 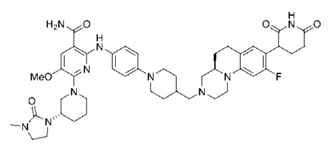 | 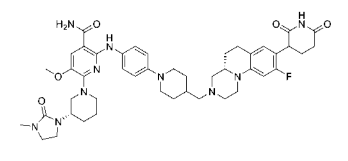 | 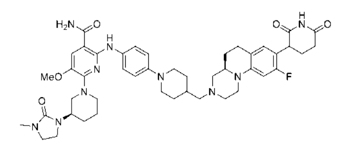 |
| 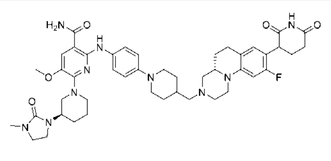 | 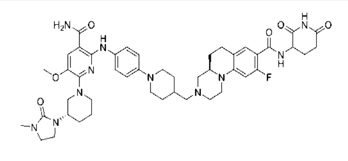 | 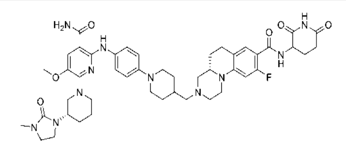 |
| 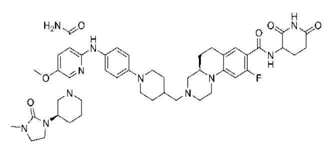 | 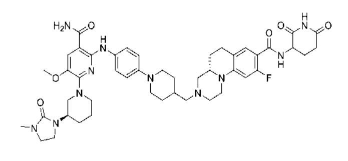 | 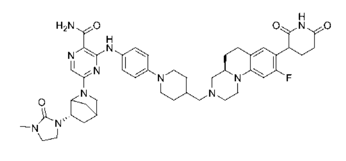 |
| 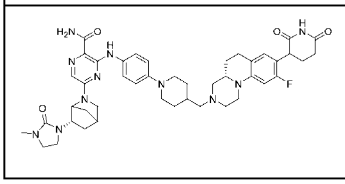 | 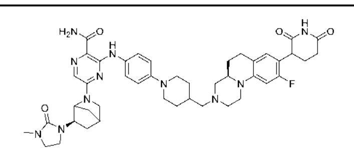 | 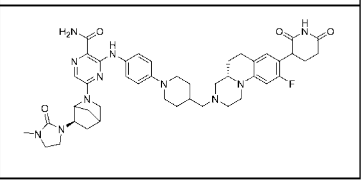 |

| | | |
|---|---|---|
| 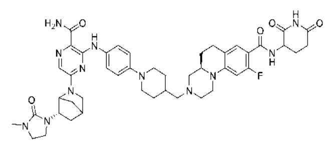 | 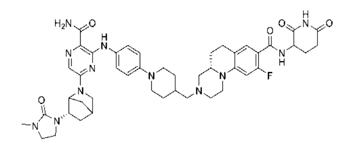 | 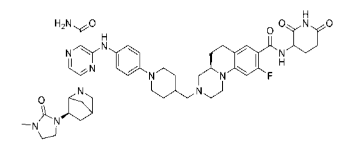 |
| 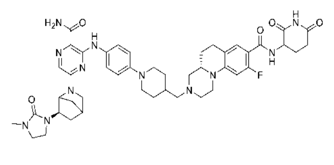 | 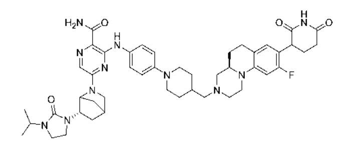 | 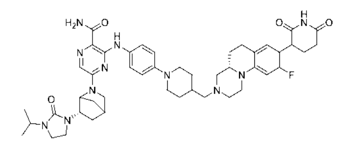 |
| 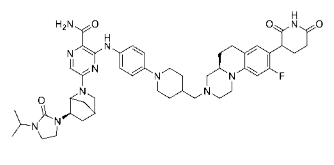 | 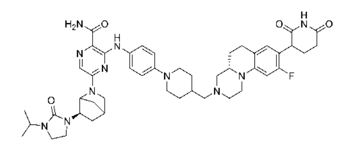 | 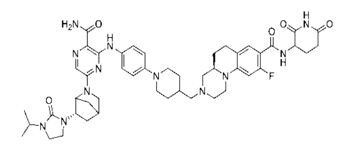 |
| 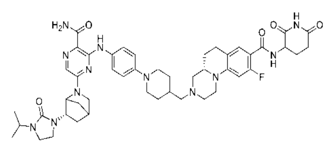 | 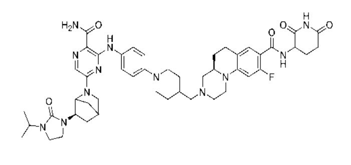 | 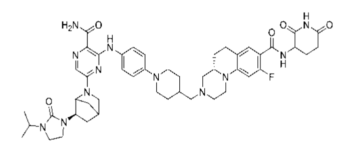 |
| 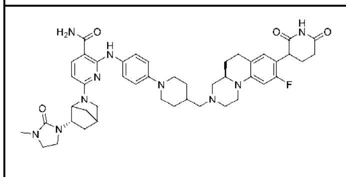 | 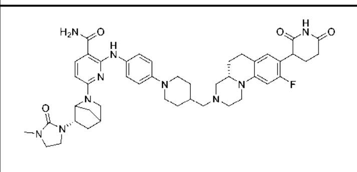 | 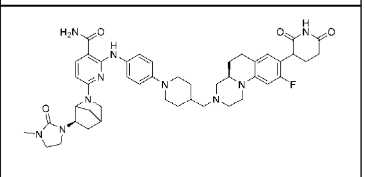 |
| 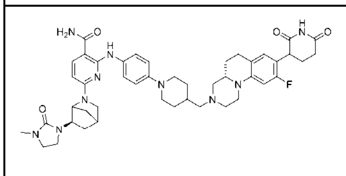 | 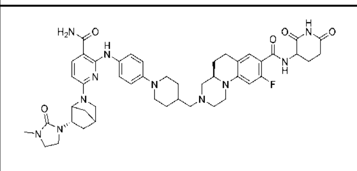 | 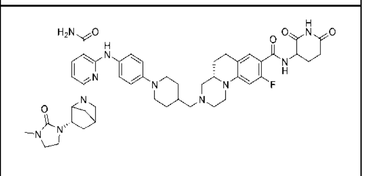 |
| 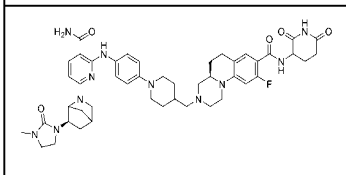 | 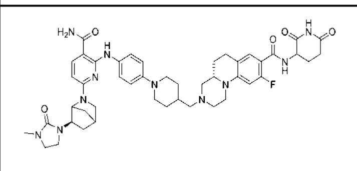 | 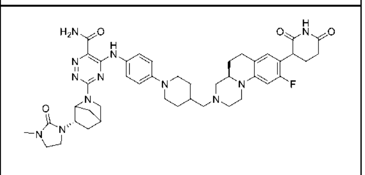 |
| 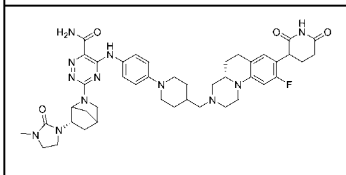 | 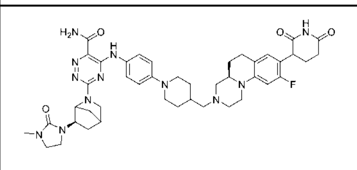 | 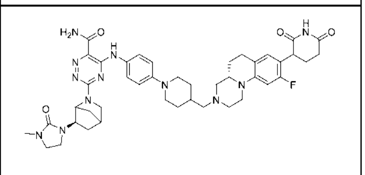 |
| 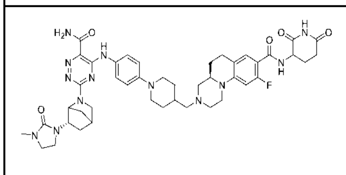 | 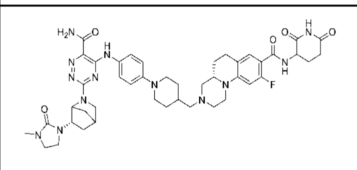 | 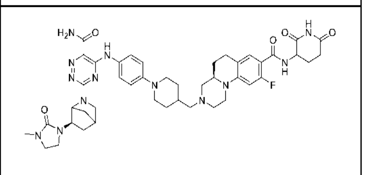 |
| 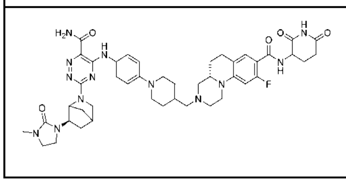 | 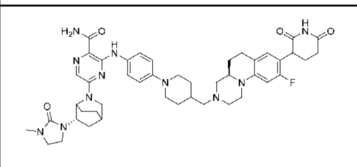 | 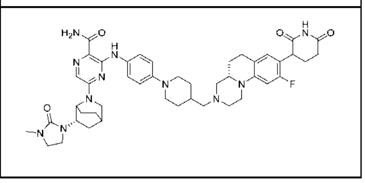 |

| | | |
|---|---|---|
| 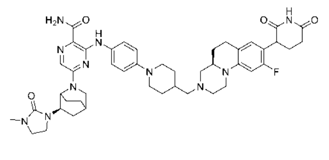 | 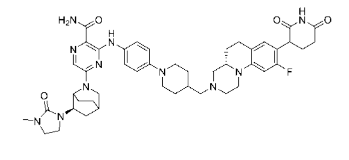 | 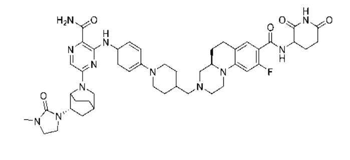 |
| 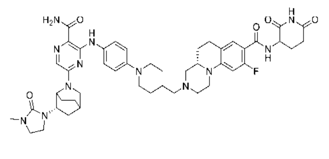 | 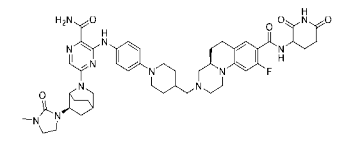 | 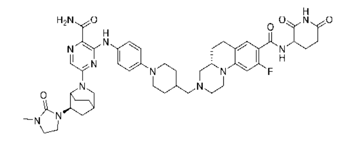 |
| 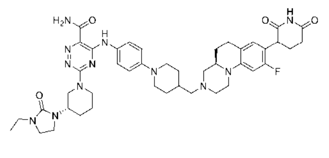 | 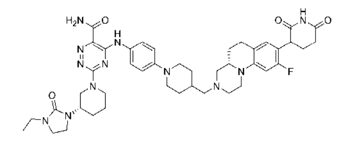 | 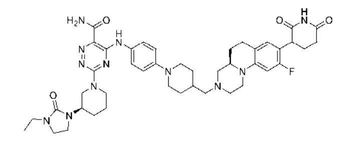 |
| 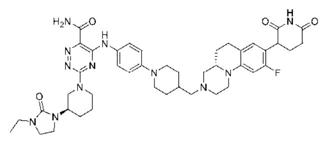 | 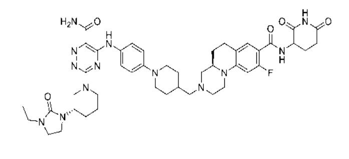 | 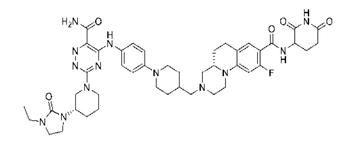 |
| 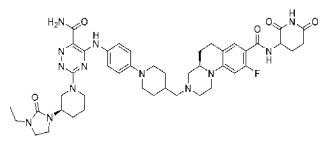 | 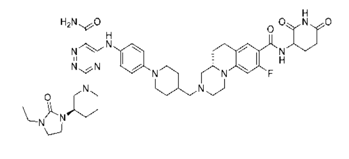 | 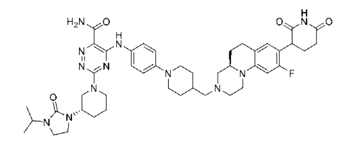 |
| 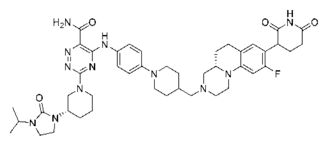 | 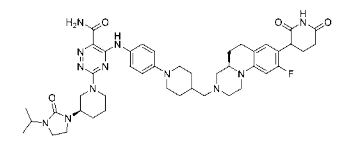 | 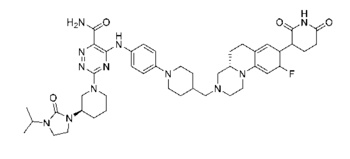 |
| 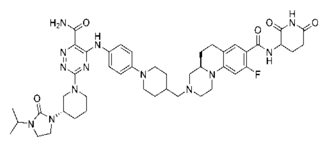 | 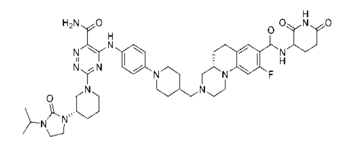 | 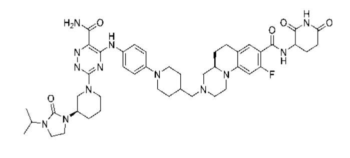 |
| 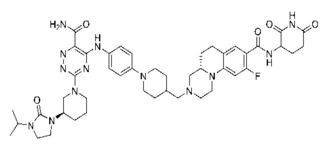 | 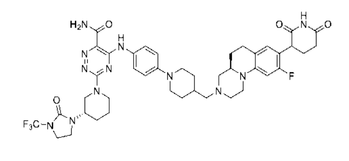 | 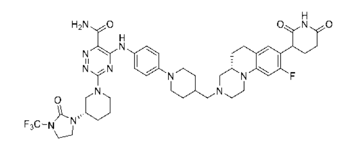 |
| 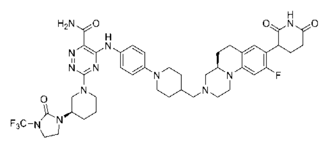 | 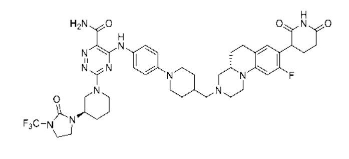 | 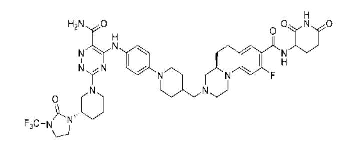 |
| 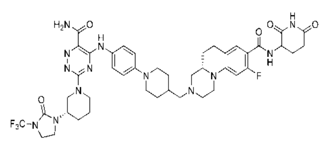 | 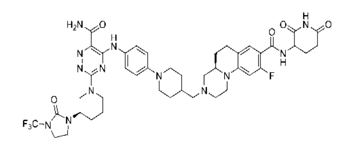 | 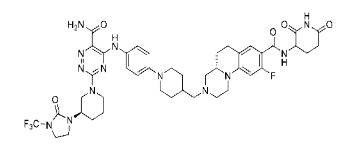 |

| | | |
|---|---|---|
| 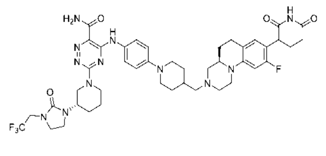 | 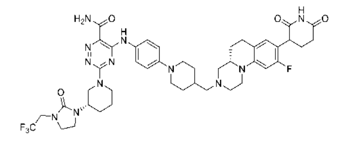 | 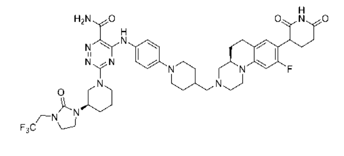 |
| 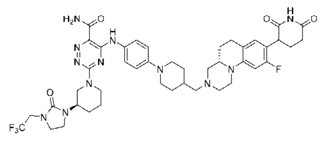 | 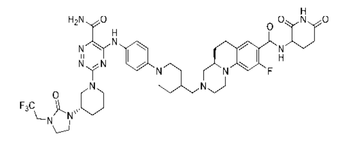 | 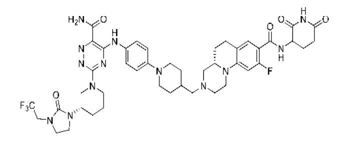 |
| 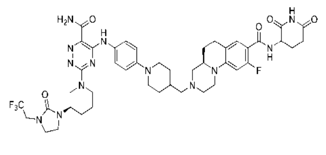 | 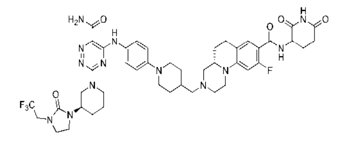 | 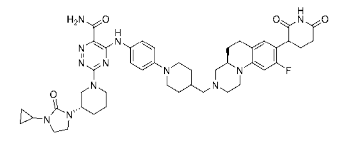 |
| 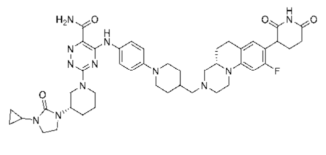 | 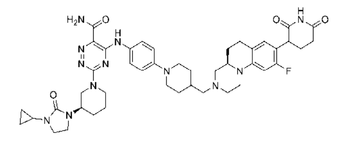 | 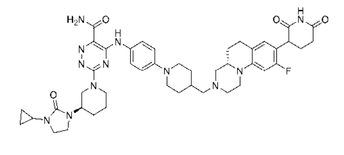 |
| 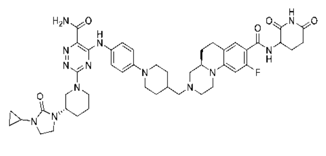 | 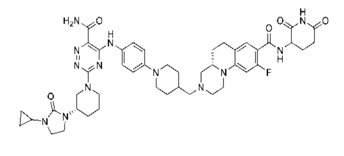 | 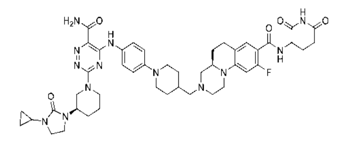 |
| 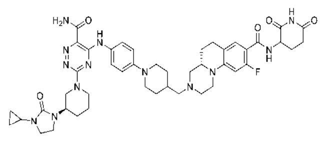 | 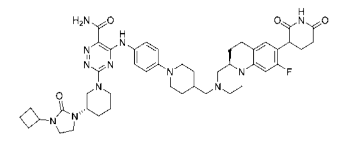 | 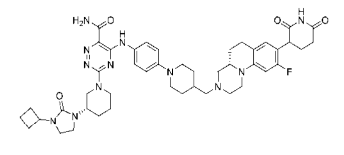 |
| 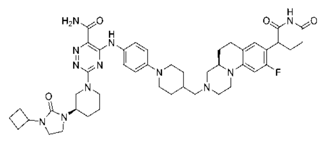 | 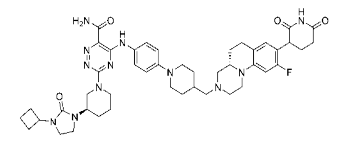 | 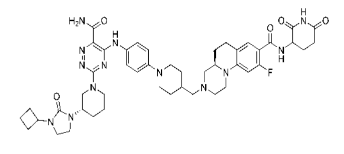 |
| 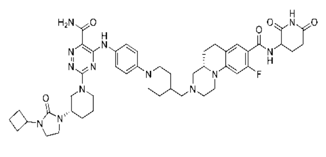 | 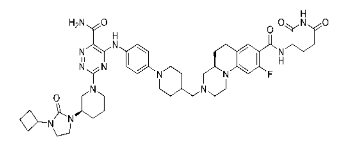 | 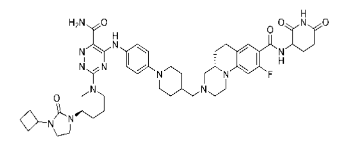 |
| 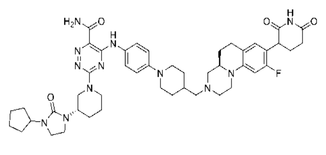 | 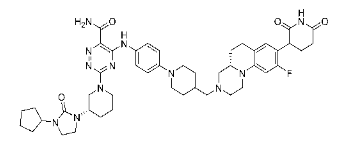 | 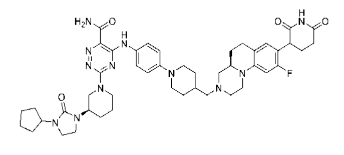 |
| 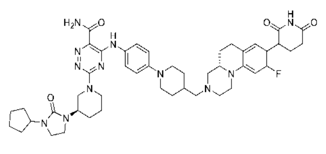 | 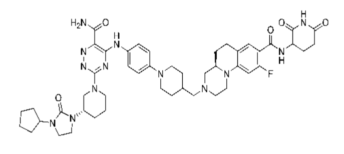 | 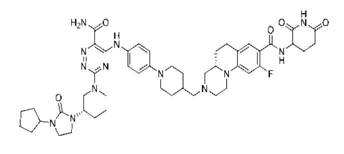 |

| | | |
|---|---|---|
| 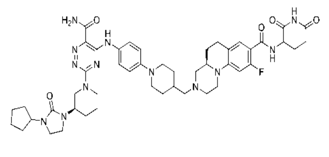 | 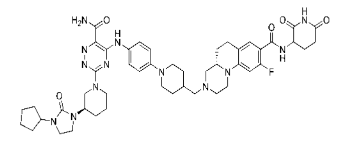 | 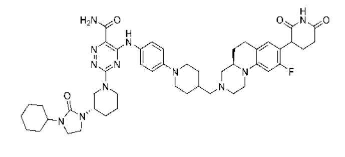 |
| 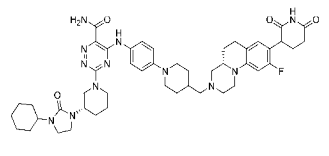 | 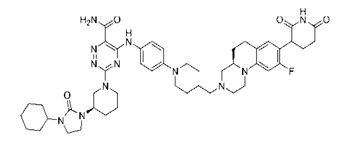 | 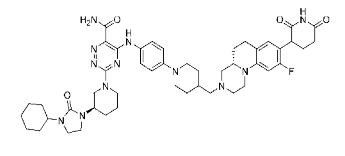 |
| 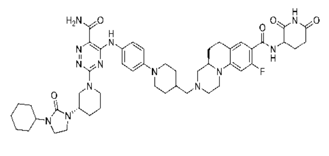 | 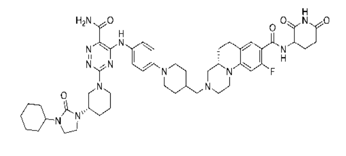 | 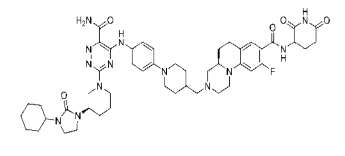 |
| 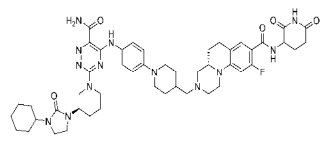 | 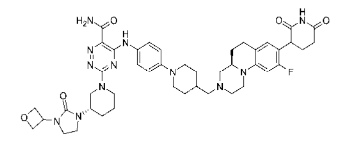 | 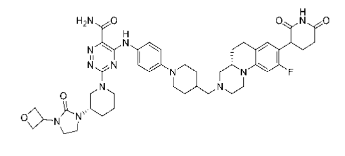 |
| 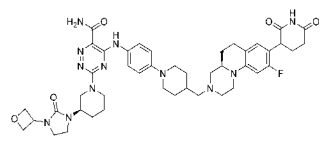 | 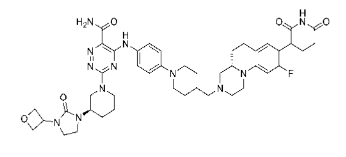 | 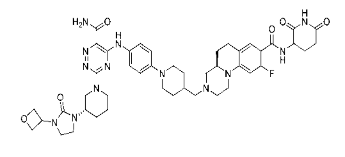 |
| 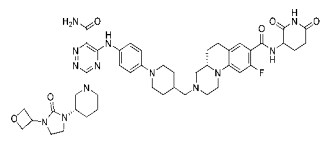 | 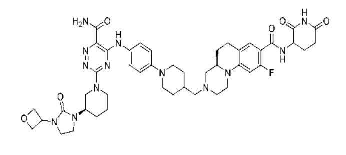 | 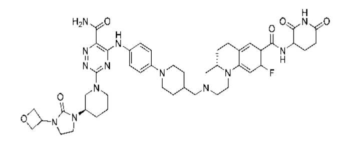 |
| 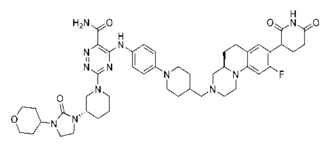 | 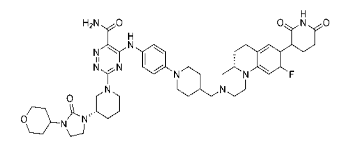 | 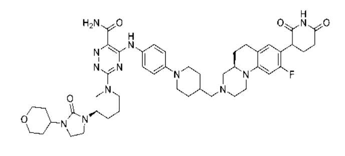 |
| 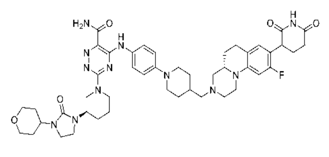 | 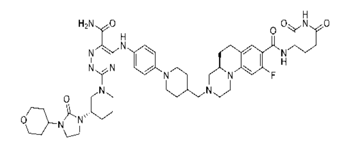 | 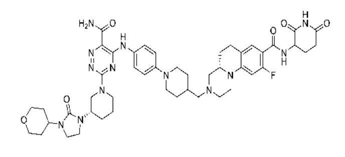 |
| 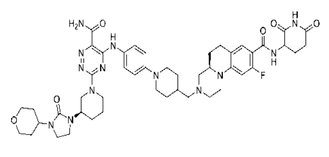 | 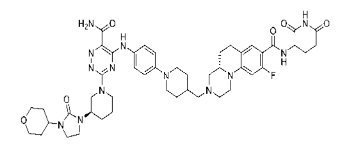 | 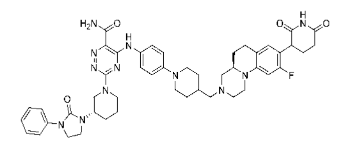 |
| 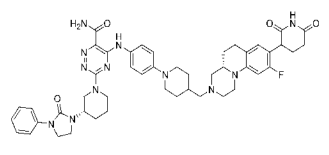 | 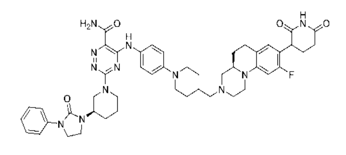 | 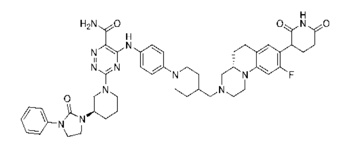 |

| | | |
|---|---|---|
| 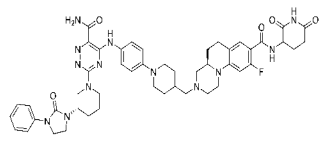 | 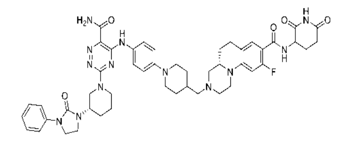 | 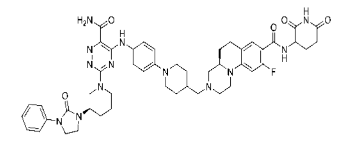 |
| 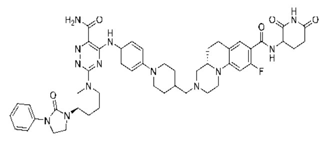 | 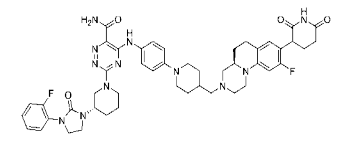 | 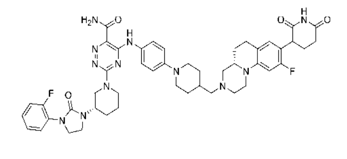 |
| 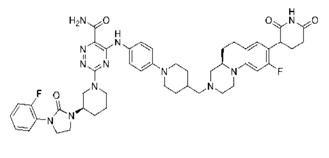 | 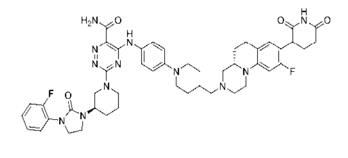 | 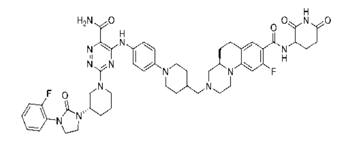 |
| 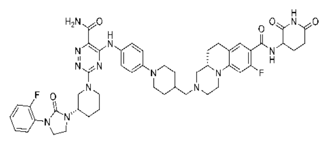 | 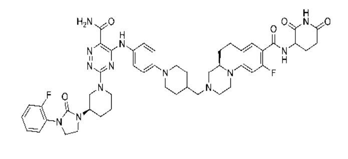 | 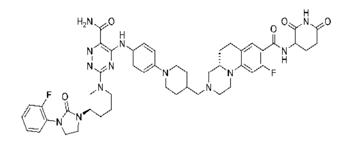 |
| 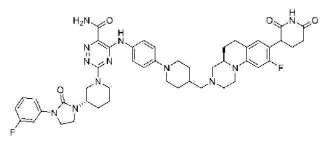 | 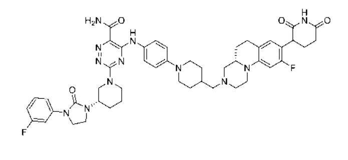 | 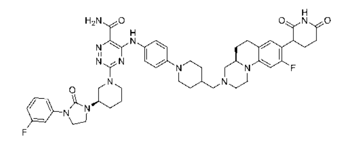 |
| 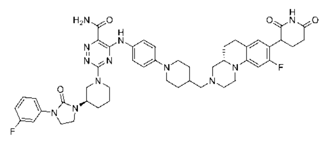 | 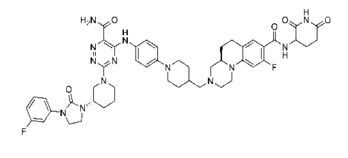 | 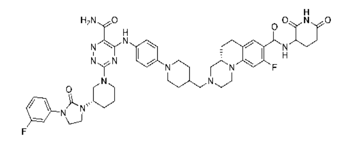 |
| 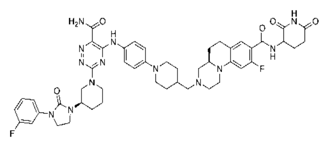 | 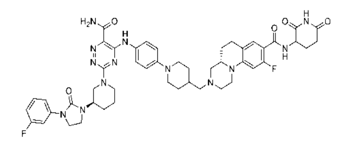 | 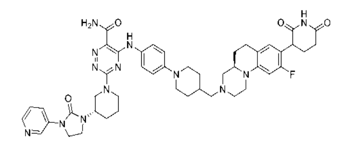 |
| 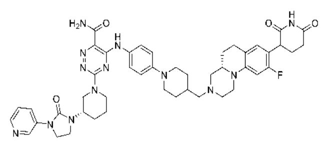 | 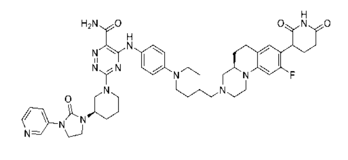 | 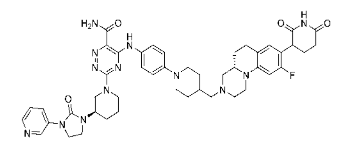 |
| 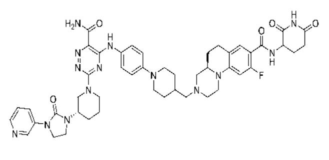 | 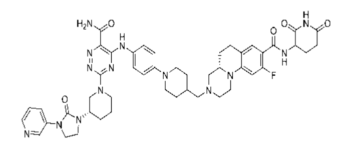 | 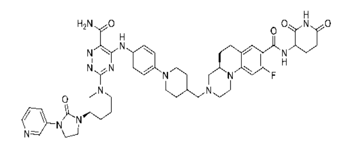 |
| 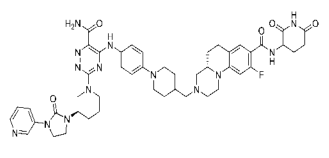 | 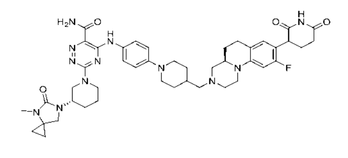 | 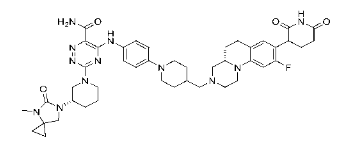 |

| | | |
|---|---|---|
| 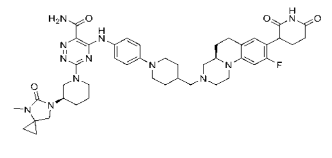 | 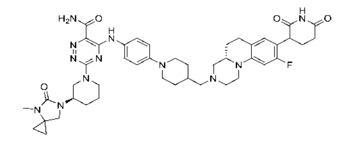 | 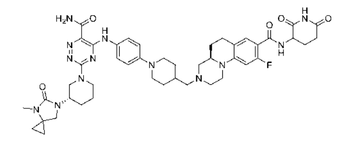 |
| 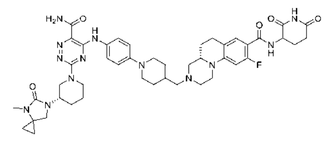 | 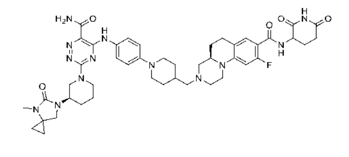 | 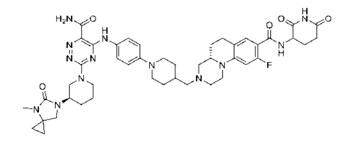 |
| 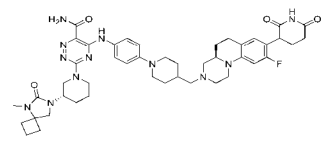 | 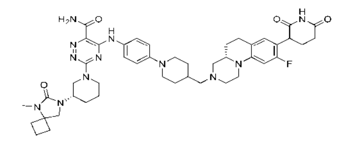 | 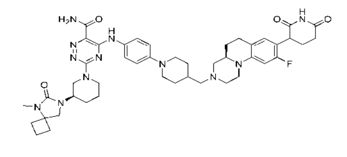 |
| 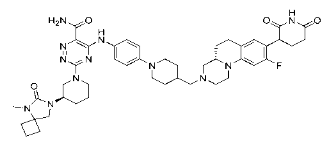 | 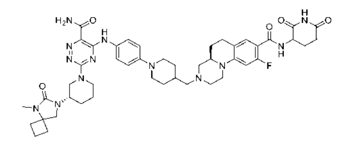 | 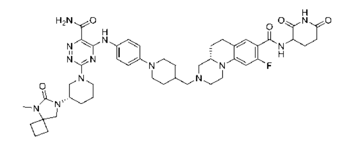 |
| 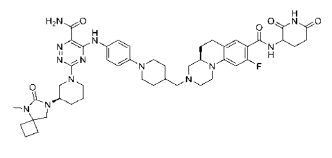 | 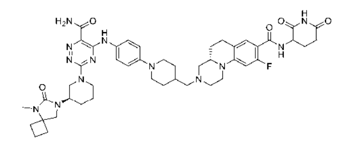 | 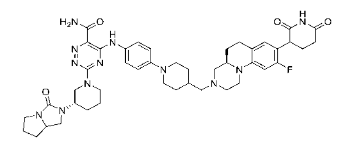 |
| 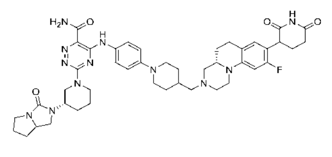 | 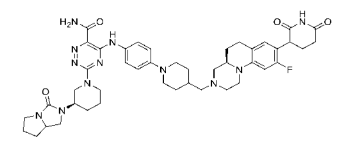 | 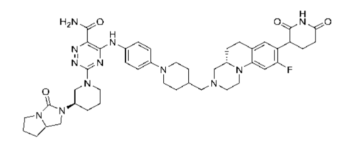 |
| 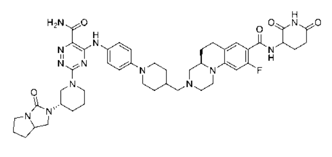 | 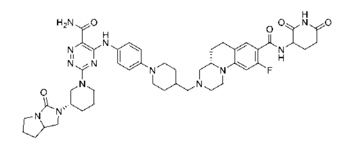 | 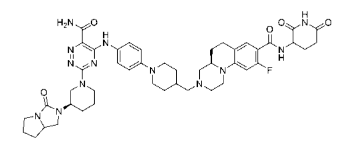 |
| 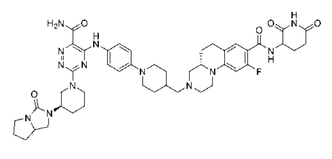 | 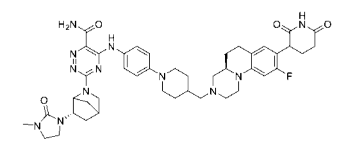 | 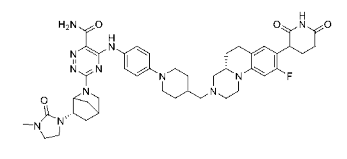 |
| 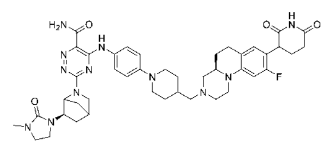 | 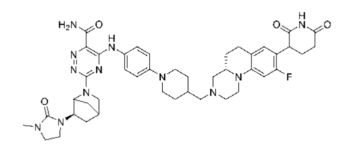 | 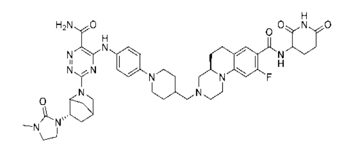 |
| 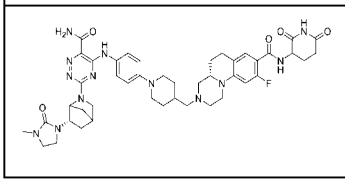 | 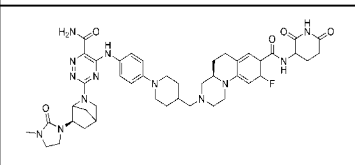 | 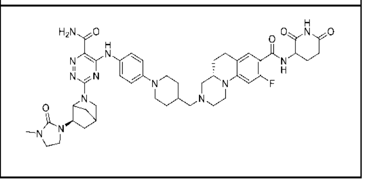 |

| | | |
|---|---|---|
| 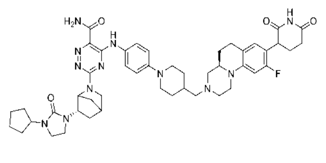 | 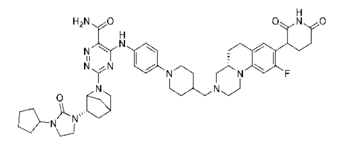 | 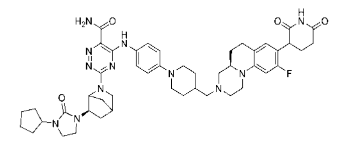 |
| 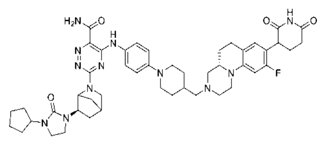 | 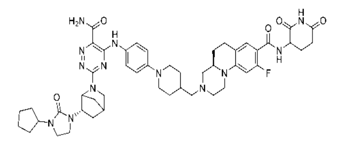 | 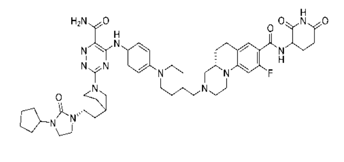 |
| 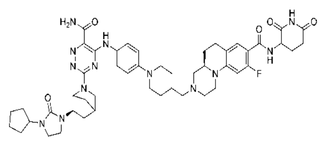 | 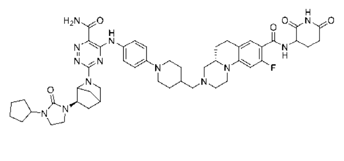 | 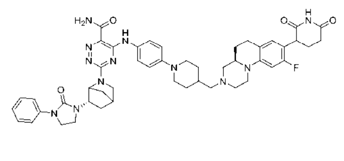 |
| 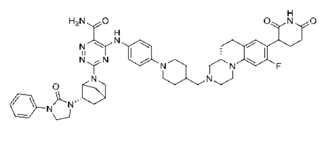 | 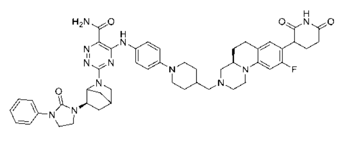 | 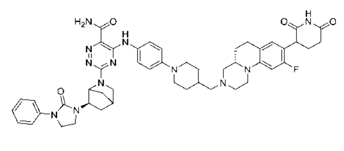 |
| 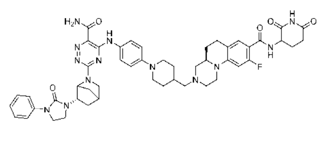 | 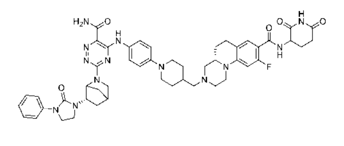 | 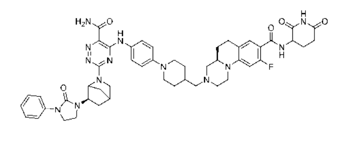 |
| 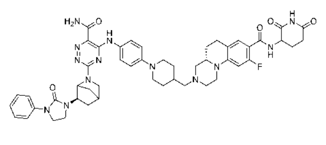 | 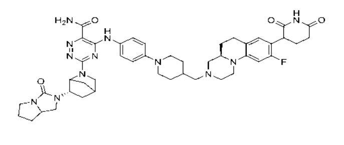 | 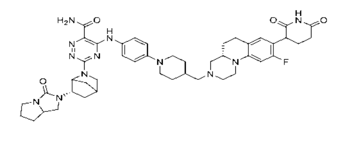 |
| 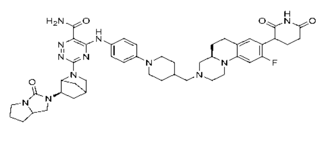 | 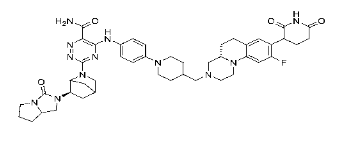 | 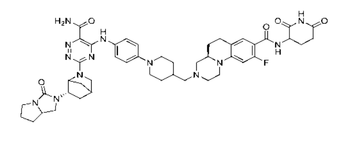 |
| 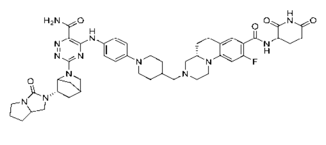 | 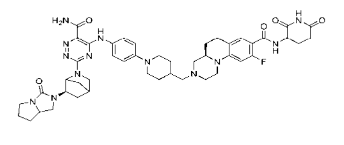 | 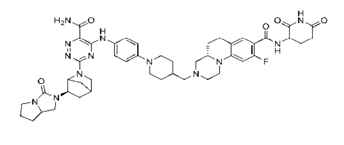 |
| 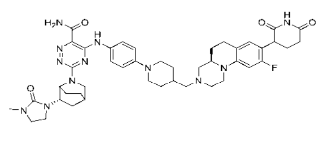 | 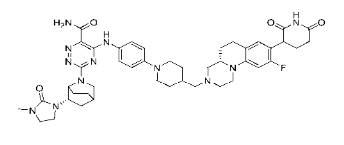 | 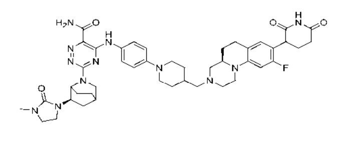 |
| 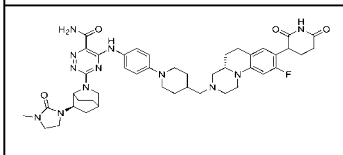 | 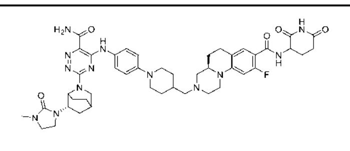 | 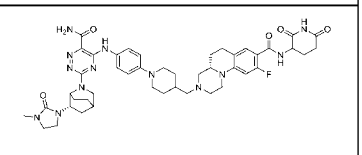 |
| 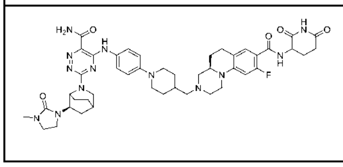 | 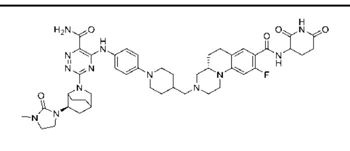 | 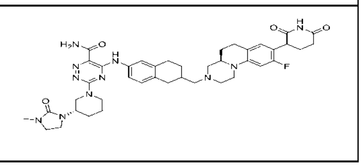 |

| | | |
|---|---|---|
| 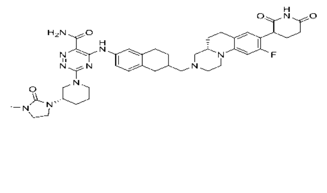 | 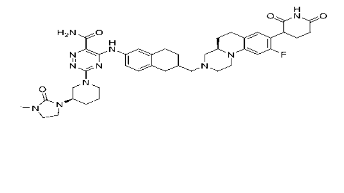 | 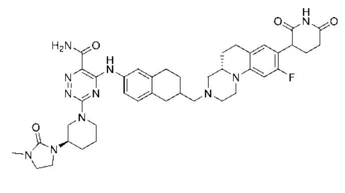 |
| 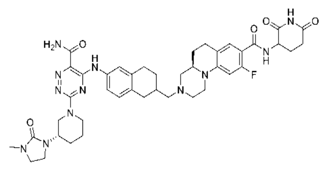 | 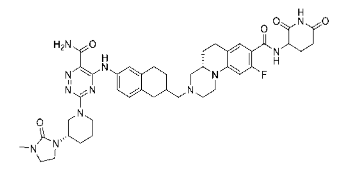 | 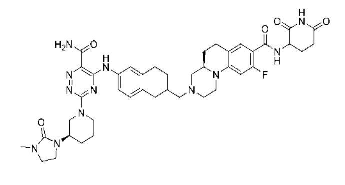 |
| 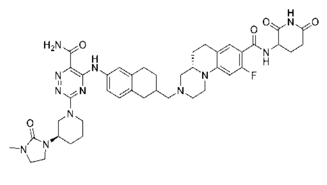 | 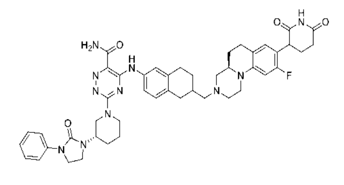 | 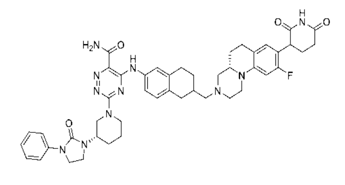 |
| 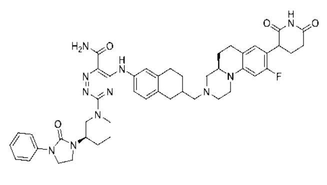 | 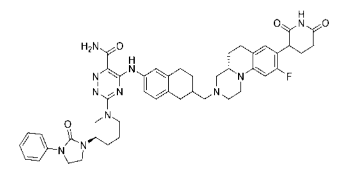 | 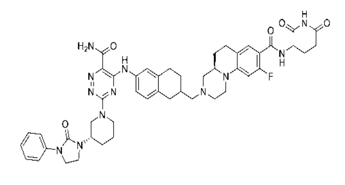 |
| 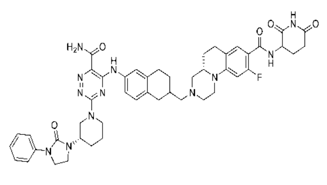 | 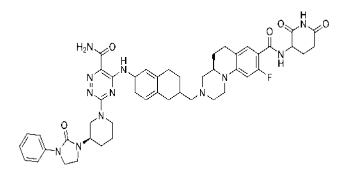 | 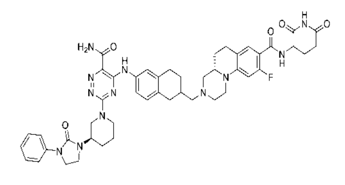 |
| 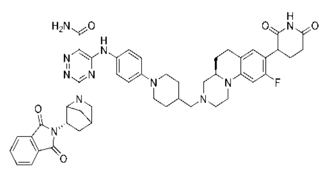 | 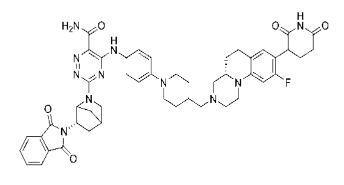 | 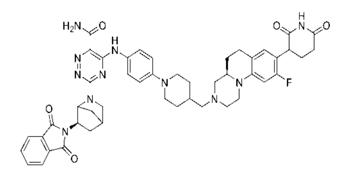 |
| 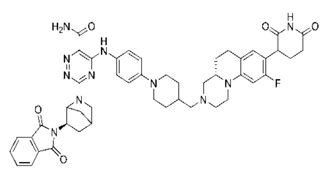 | 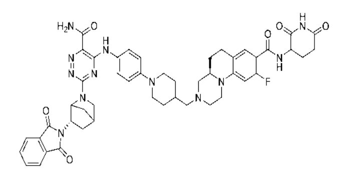 | 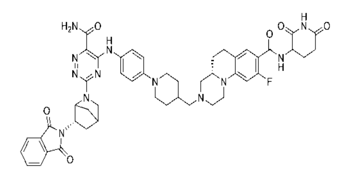 |
| 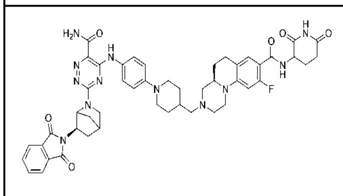 | 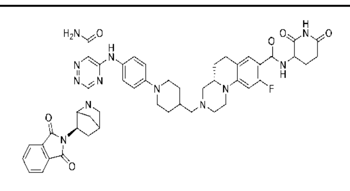 | 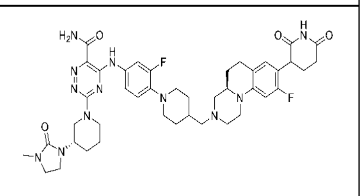 |
| 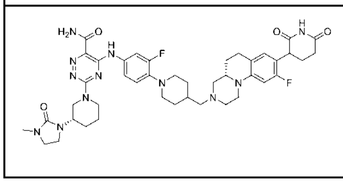 | 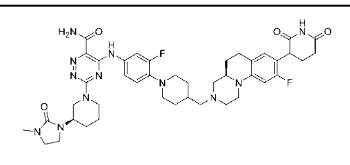 | 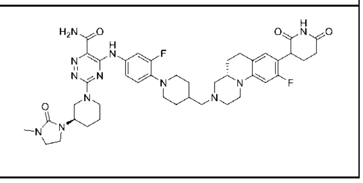 |

| | | |
|---|---|---|
| 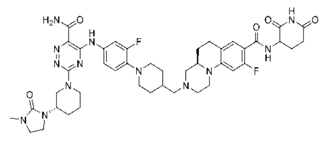 | 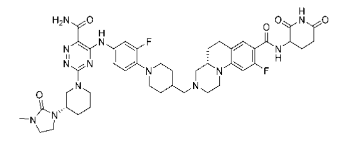 | 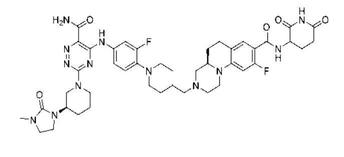 |
| 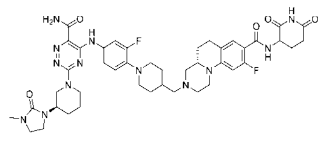 | 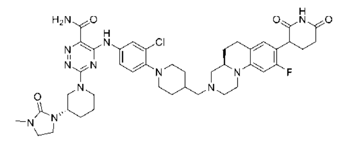 | 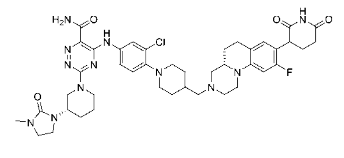 |
| 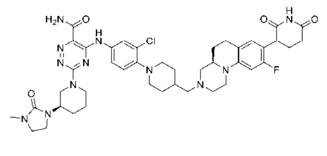 | 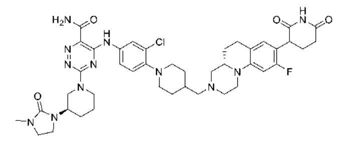 | 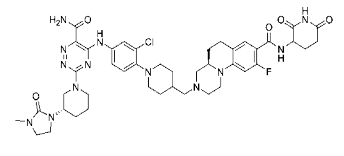 |
| 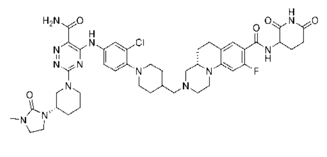 | 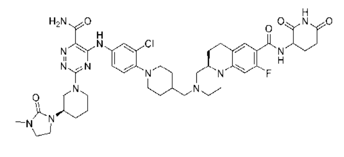 | 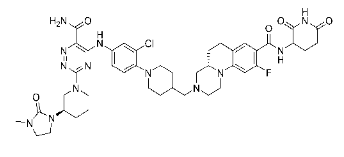 |
| 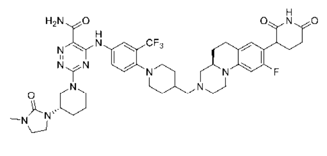 | 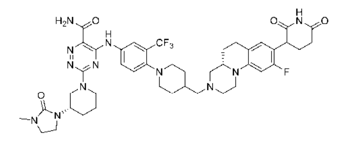 | 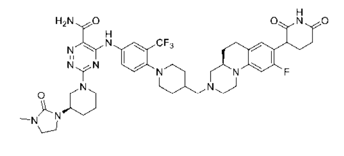 |
| 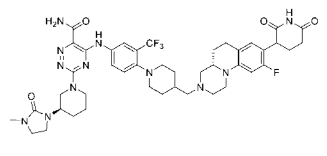 | 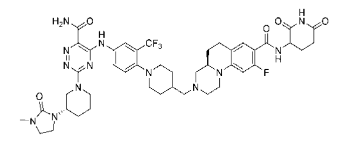 | 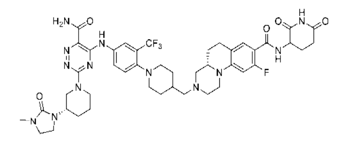 |
| 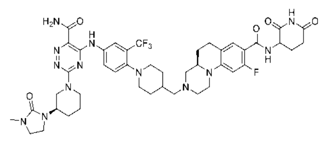 | 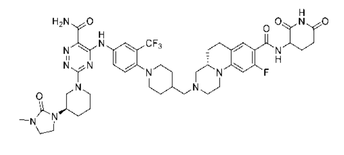 | 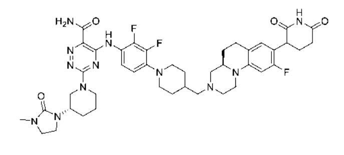 |
| 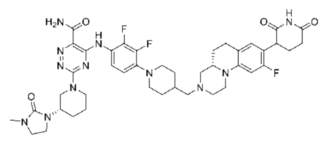 | 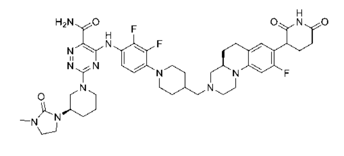 | 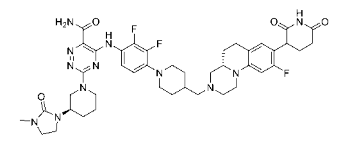 |
| 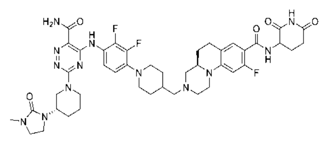 | 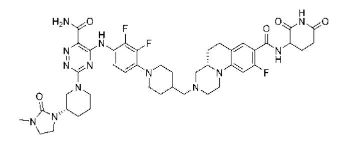 | 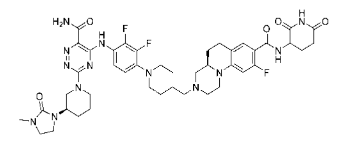 |
| 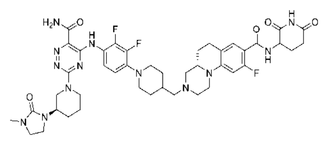 | 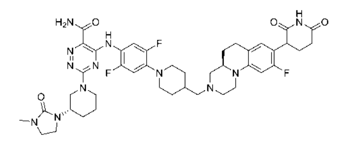 | 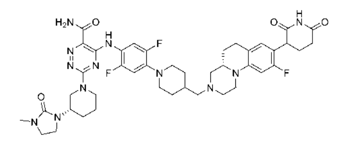 |

| | | |
|---|---|---|
| 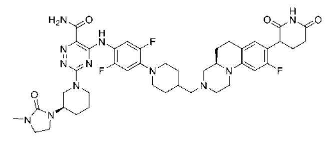 | 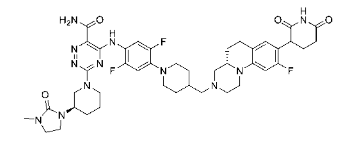 | 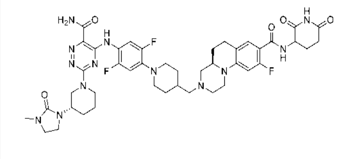 |
| 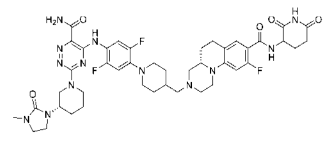 | 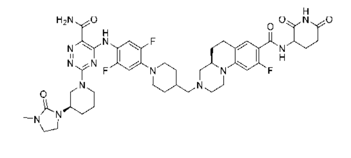 | 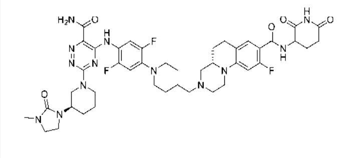 |
| 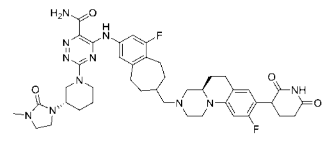 | 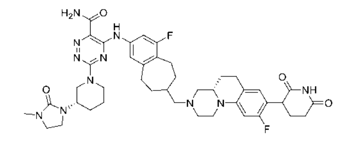 | 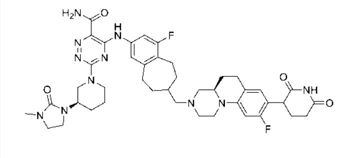 |
| 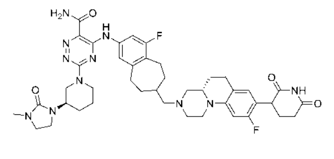 | 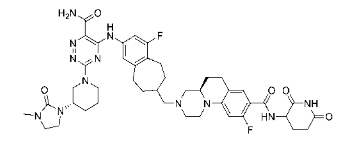 | 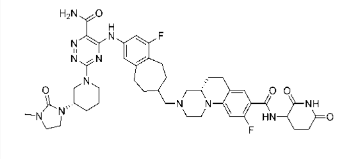 |
| 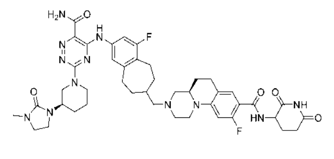 | 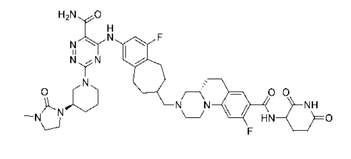 | 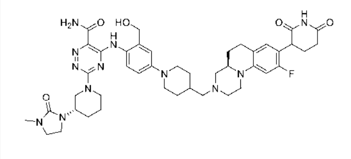 |
| 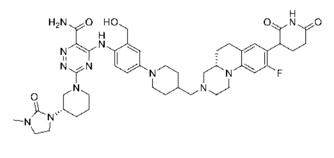 | 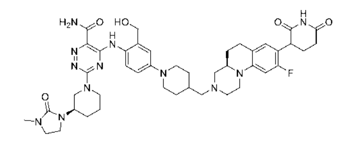 | 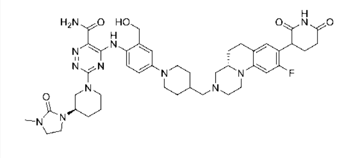 |
| 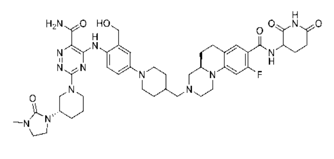 | 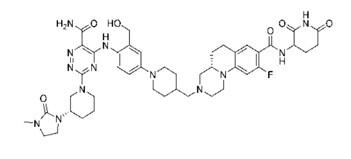 | 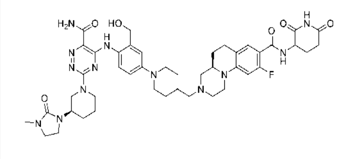 |
| 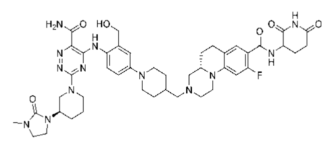 | 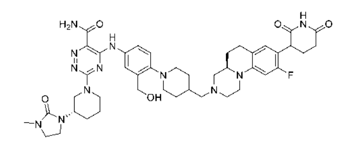 | 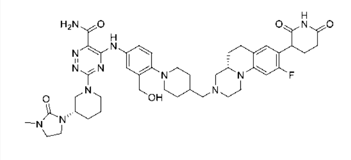 |
| 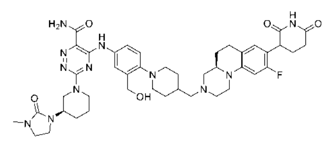 | 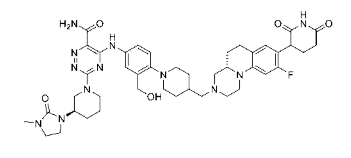 | 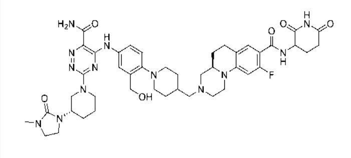 |
| 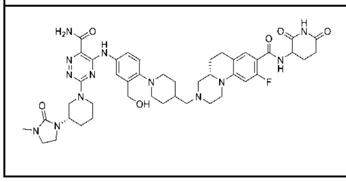 | 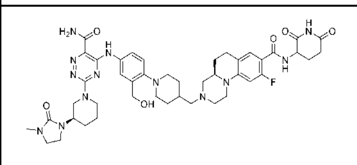 | 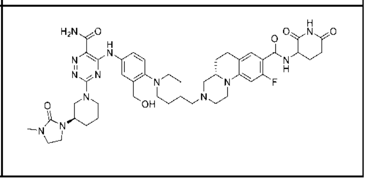 |

| | | |
|---|---|---|
| 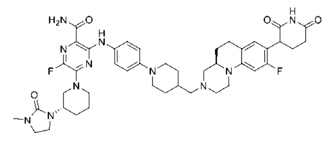 | 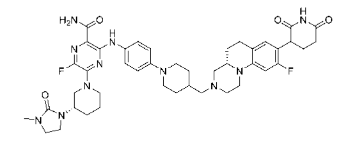 | 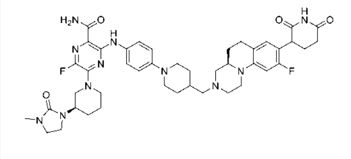 |
| 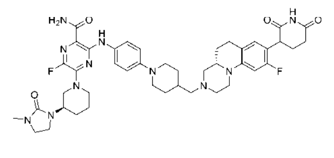 | 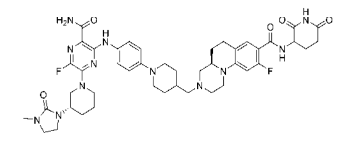 | 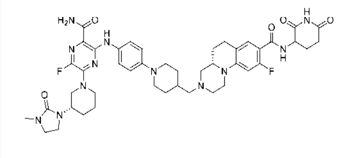 |
| 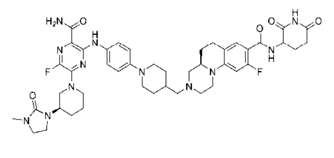 | 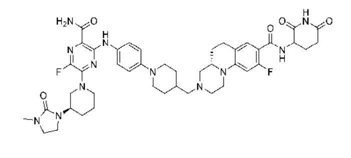 | 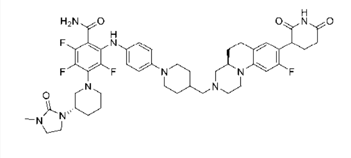 |
| 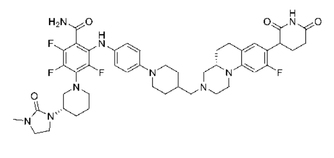 | 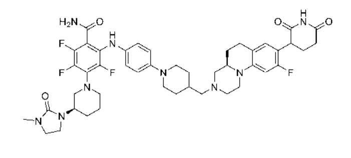 | 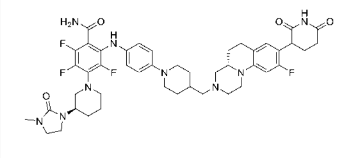 |
| 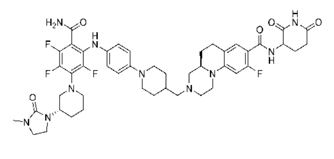 | 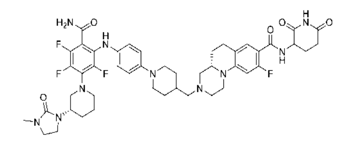 | 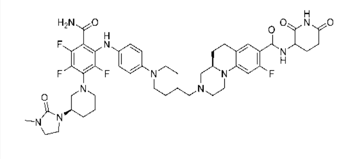 |
| 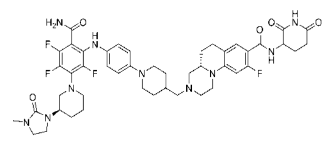 | 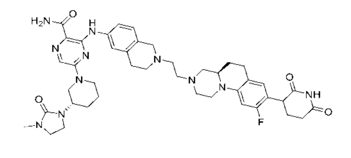 | 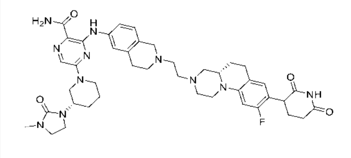 |
| 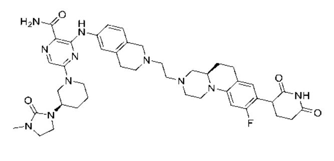 | 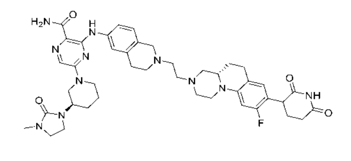 | 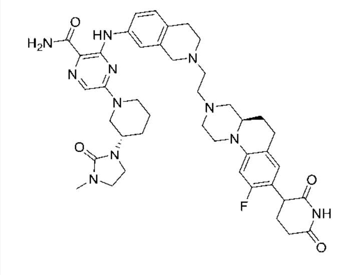 |
| 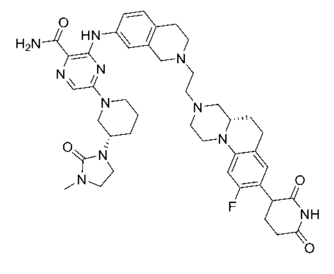 | 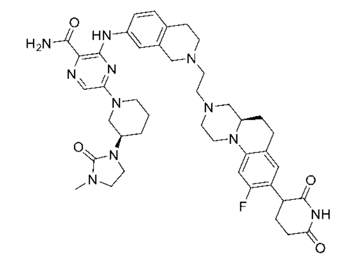 | 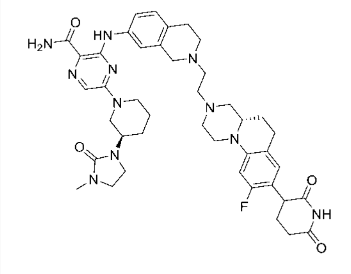 |
| 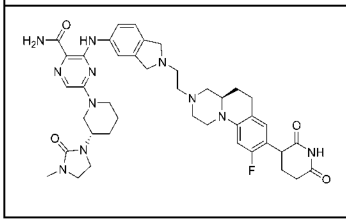 | 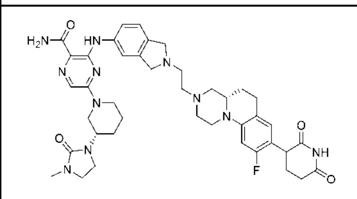 | 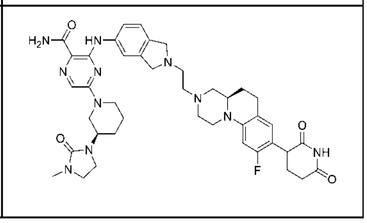 |

| | | |
|---|---|---|
| 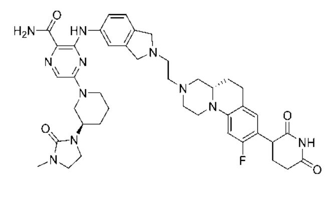 | 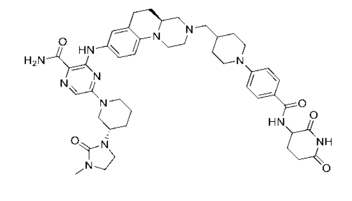 | 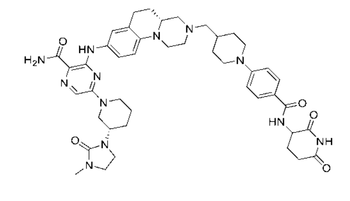 |
| 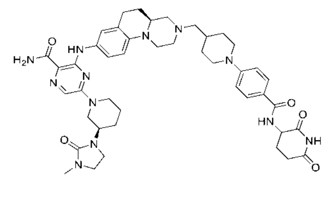 | 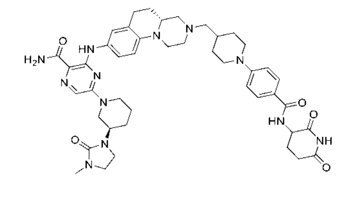 | 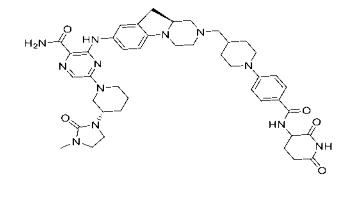 |
| 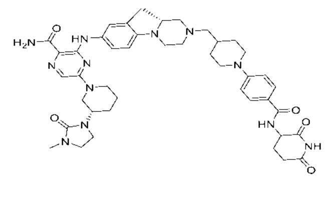 | 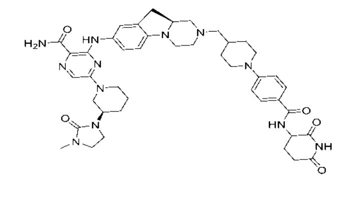 | 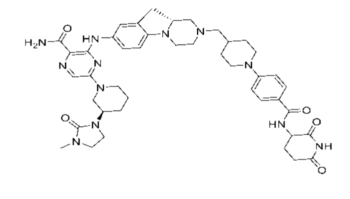 |
| 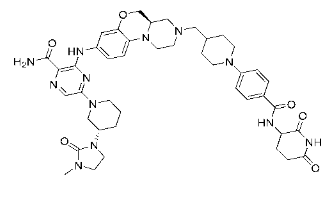 | 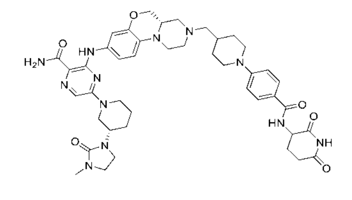 | 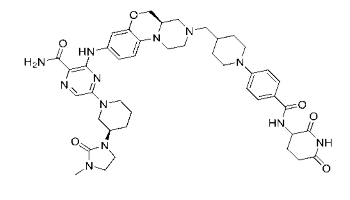 |
| 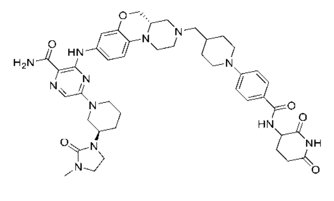 | 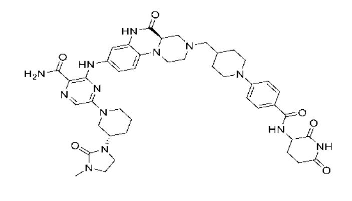 | 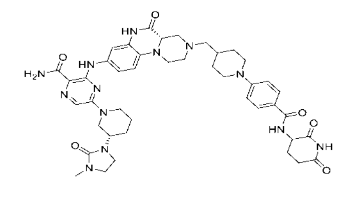 |
| 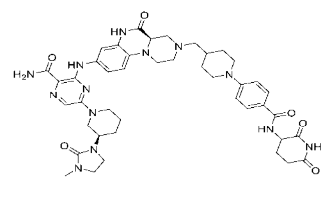 | 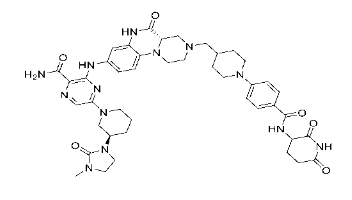 | 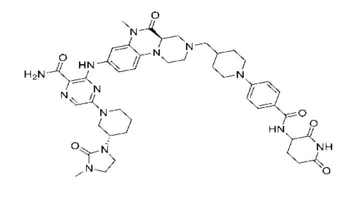 |
| 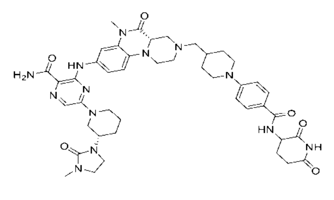 | 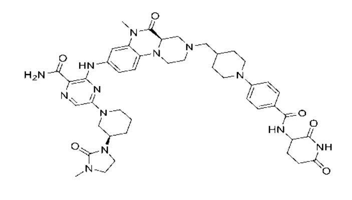 | 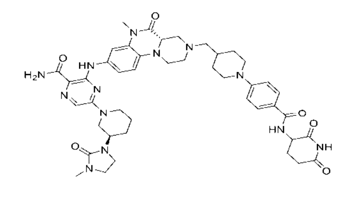 |
| 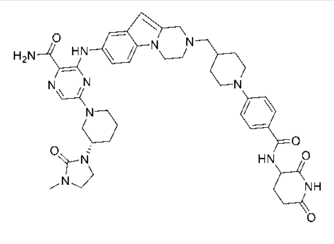 | 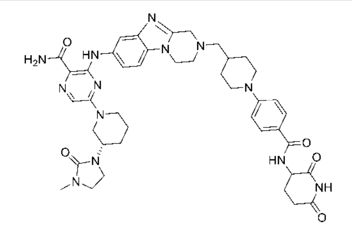 | 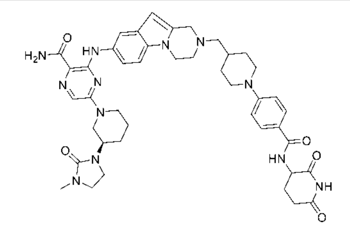 |

| | | |
|---|---|---|
| 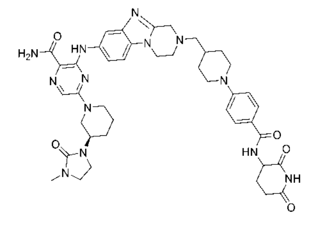 | 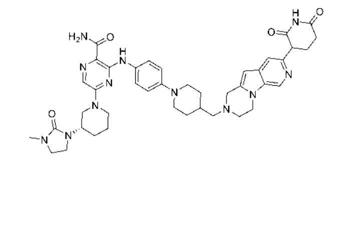 | 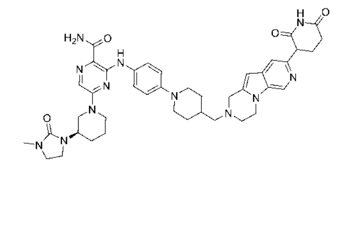 |
| 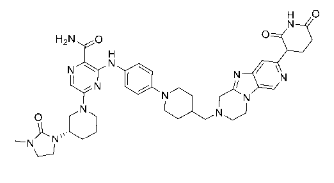 | 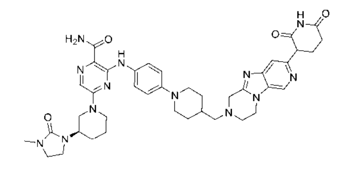 | 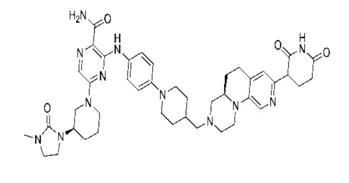 |
| 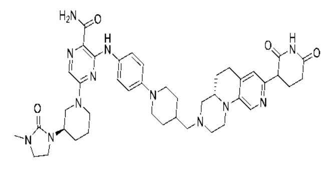 | 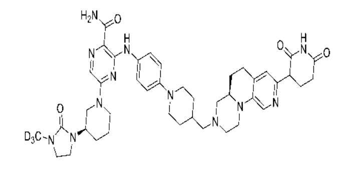 | 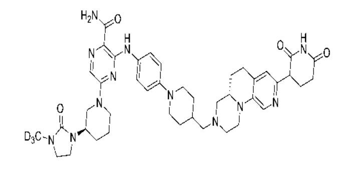 |
| 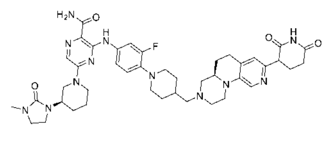 | 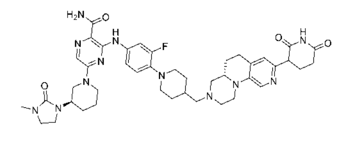 | 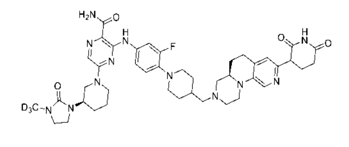 |
| 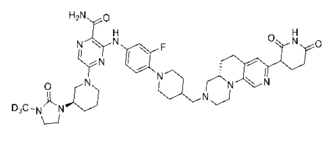 | 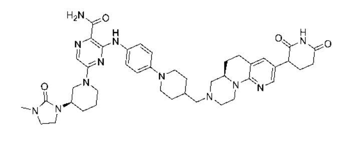 | 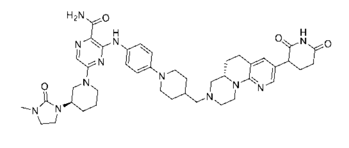 |
| 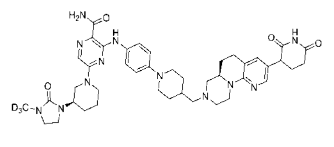 | 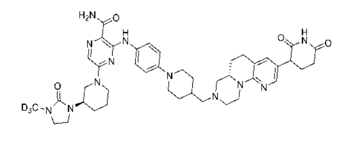 | 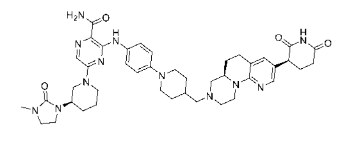 |
| 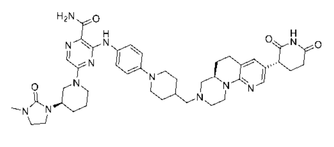 | 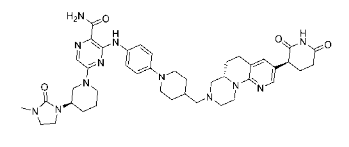 | 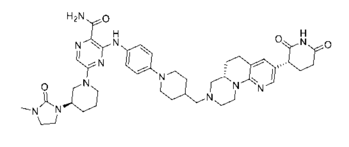 |
| 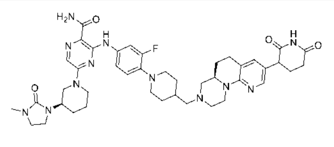 | 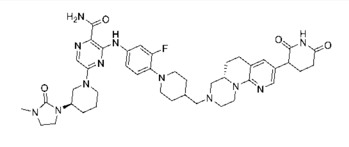 | 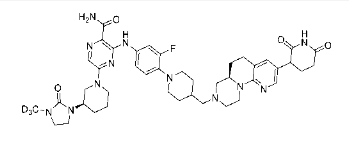 |
| 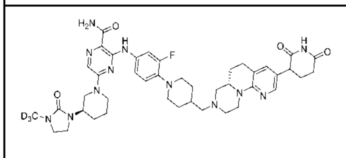 | 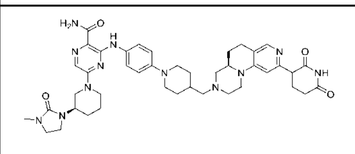 | 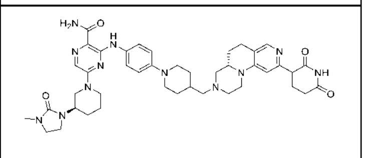 |
| 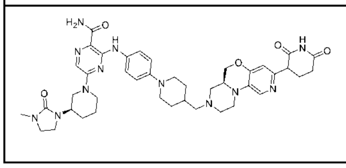 | 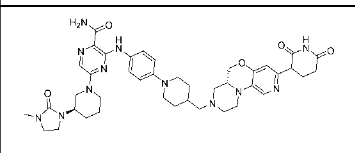 | 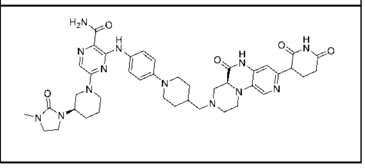 |

| | | |
|---|---|---|
| 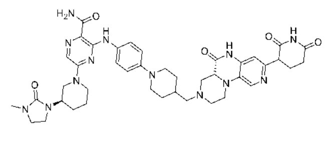 | 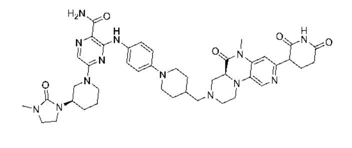 | 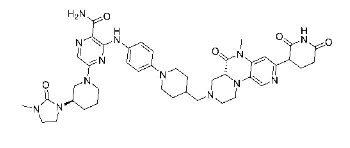 |
| 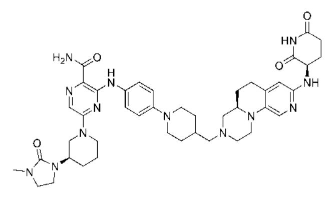 | 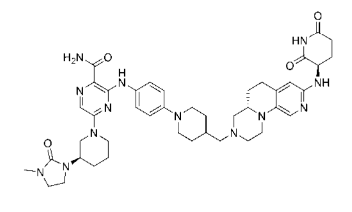 | 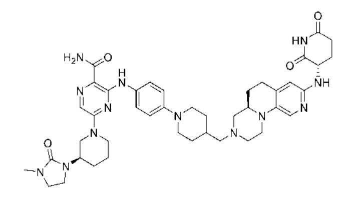 |
| 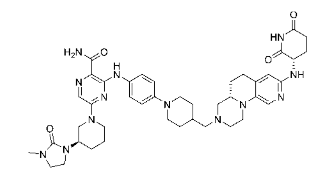 | 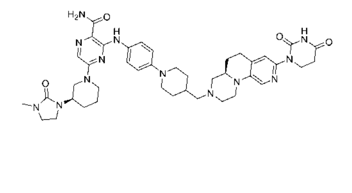 | 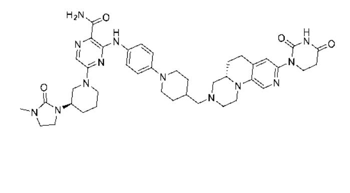 |
| 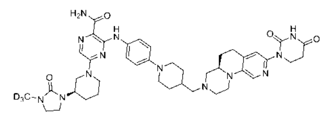 | 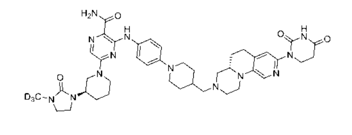 | 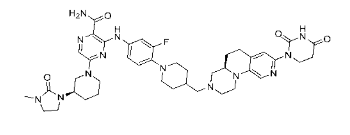 |
| 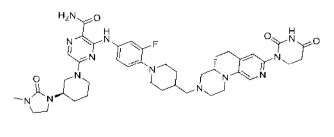 | 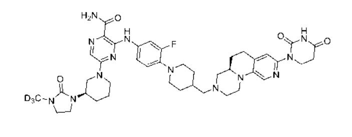 | 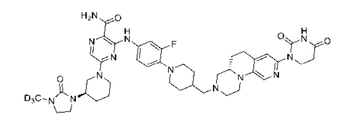 |
| 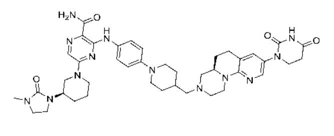 | 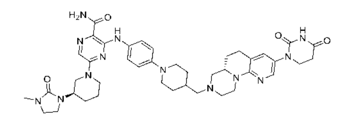 | 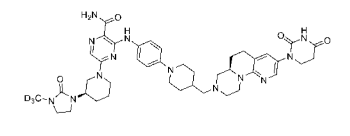 |
| 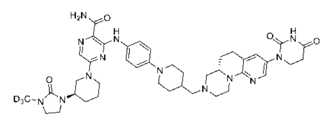 | 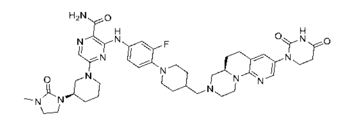 | 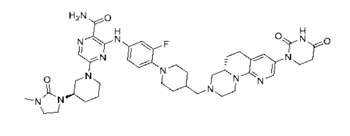 |
| 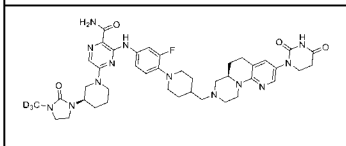 | 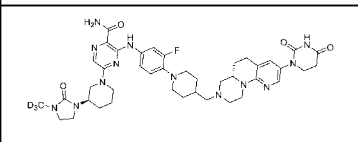 | 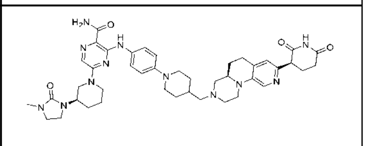 |
| 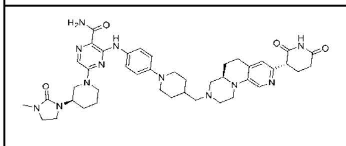 | 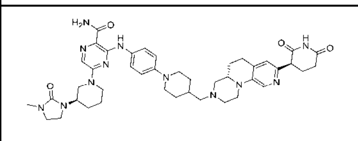 | 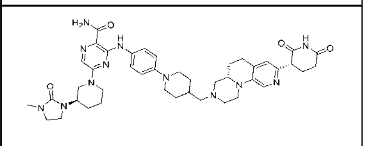 |
| 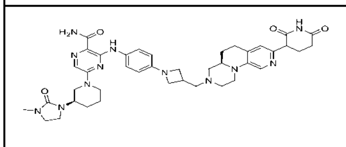 | 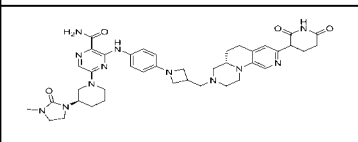 | 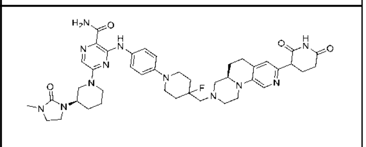 |
| 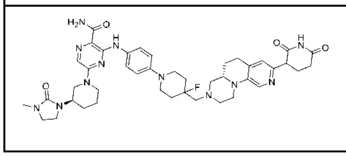 | 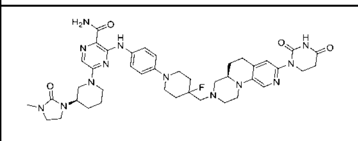 | 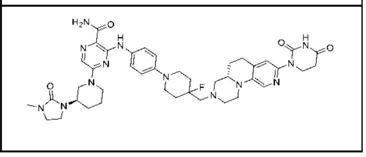 |

| | | |
|---|---|---|
| 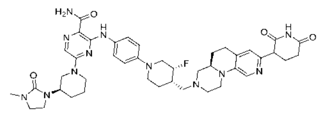 | 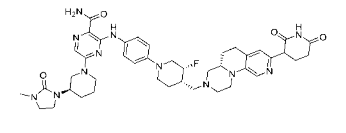 | 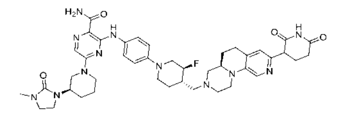 |
| 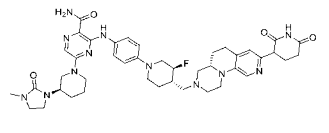 | 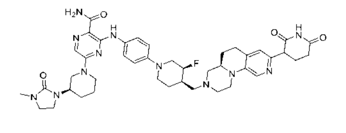 | 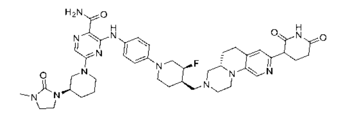 |
| 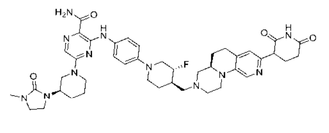 | 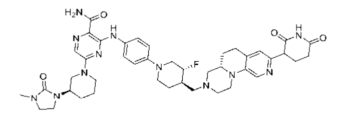 | 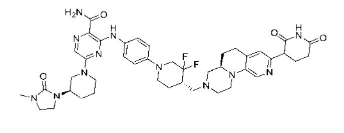 |
| 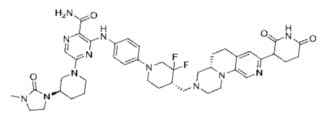 | 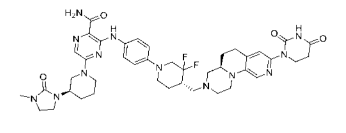 | 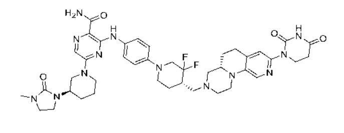 |
| 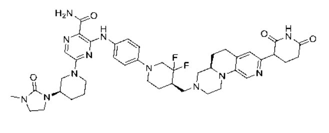 | 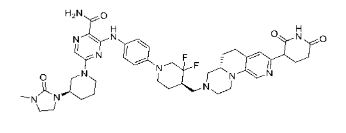 | 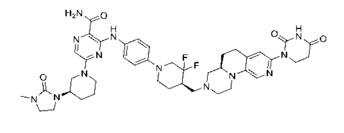 |
| 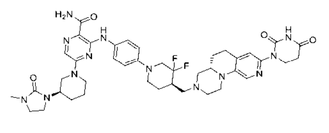 | 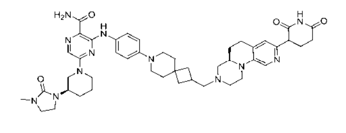 | 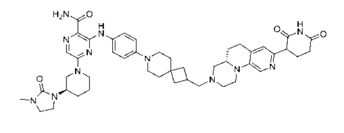 |
| 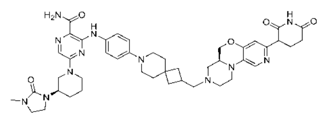 | 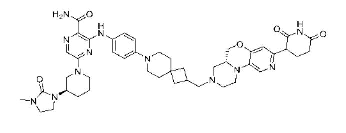 | 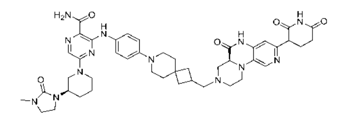 |
| 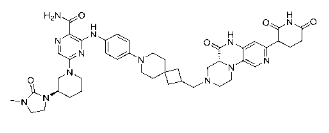 | 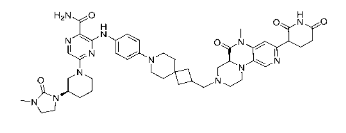 | 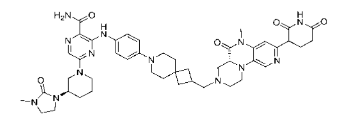 |
| 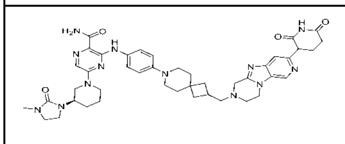 | 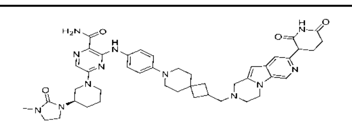 | 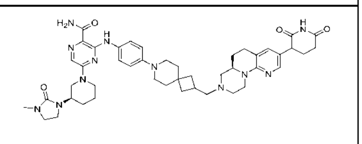 |
| 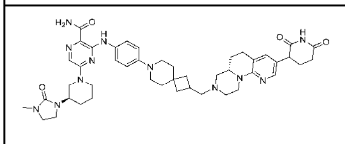 | 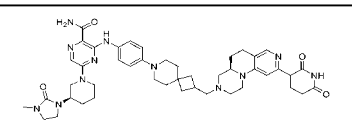 | 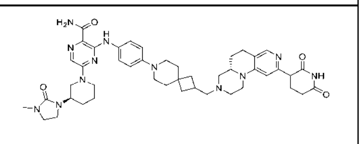 |
| 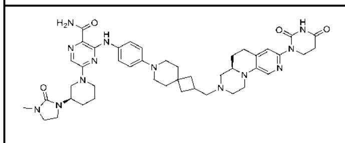 | 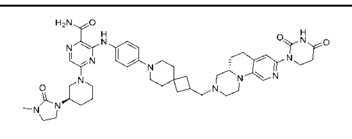 | 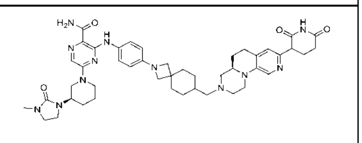 |
| 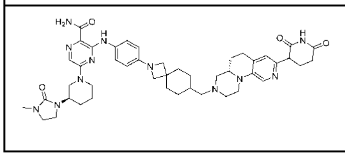 | 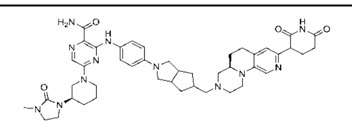 | 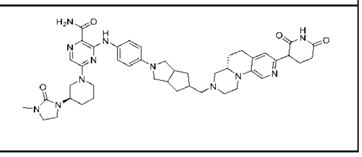 |

| | | |
|---|---|---|
| 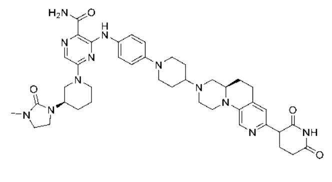 | 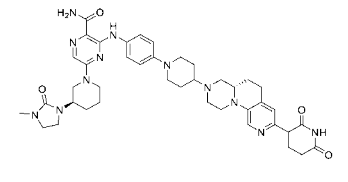 | 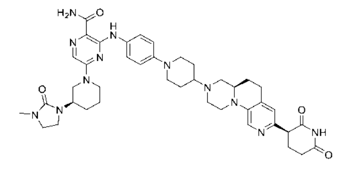 |
| 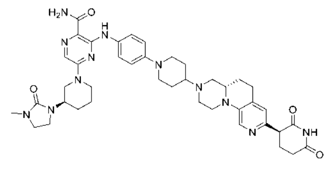 | 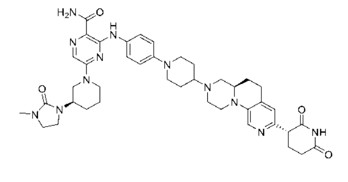 | 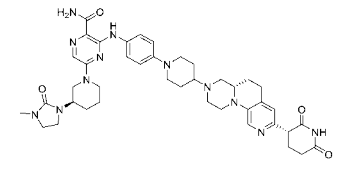 |
| 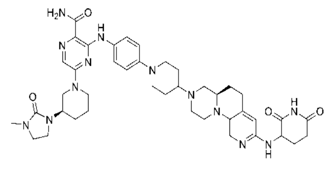 | 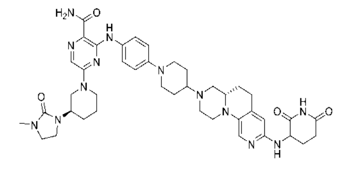 | 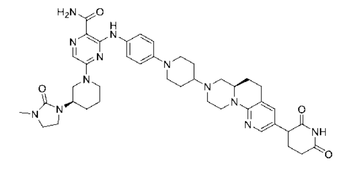 |
| 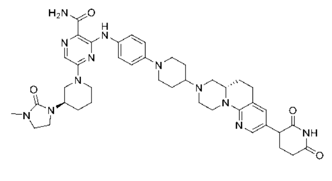 | 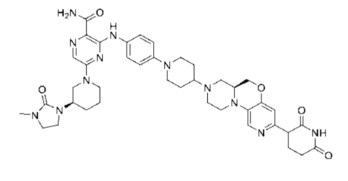 | 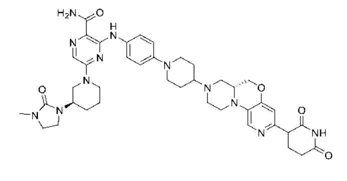 |
| 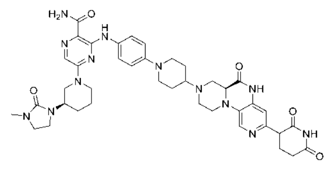 | 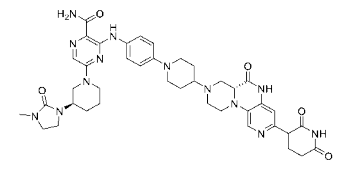 | 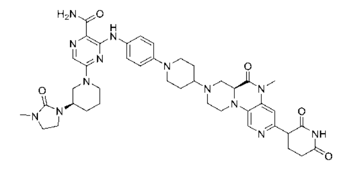 |
| 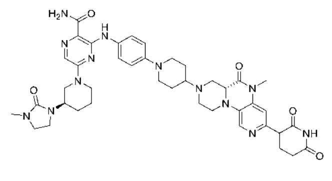 | 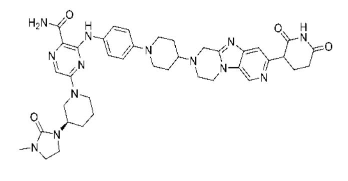 | 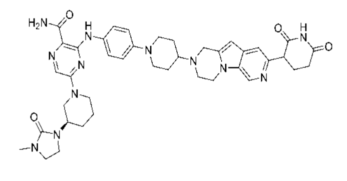 |
| 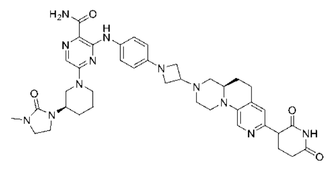 | 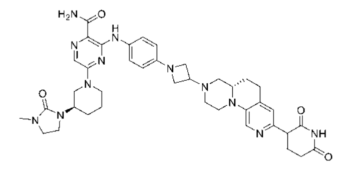 | 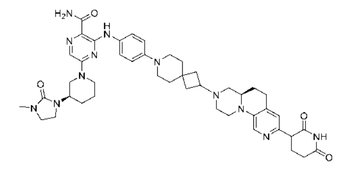 |
| 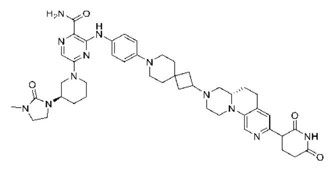 | 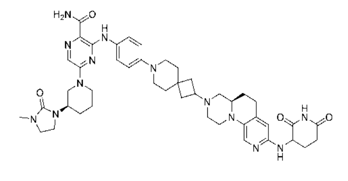 | 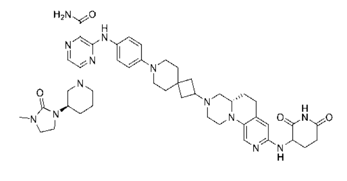 |
| 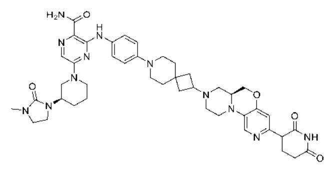 | 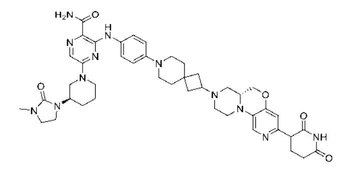 | 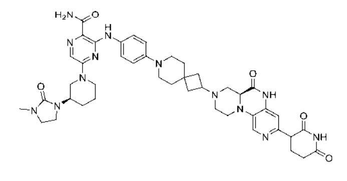 |

| | | |
|---|---|---|
| 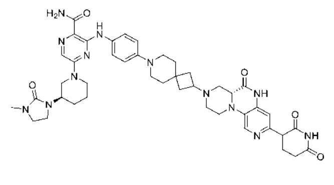 | 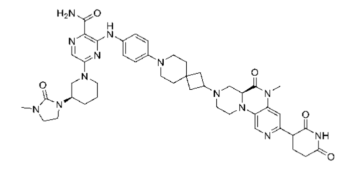 | 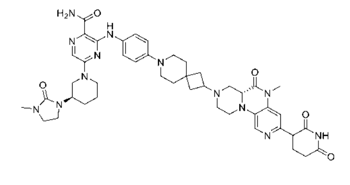 |
| 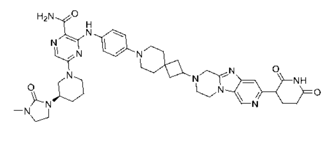 | 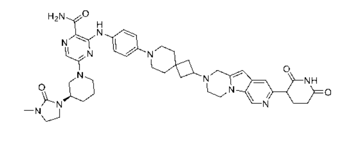 | 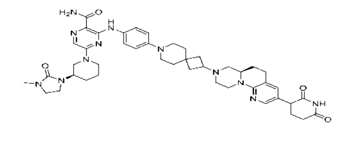 |
| 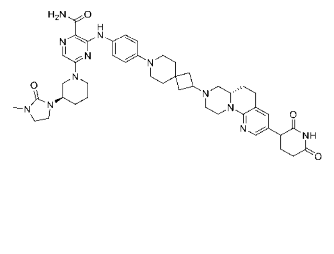 | 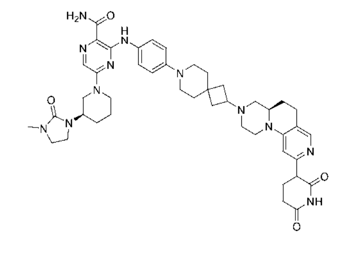 | 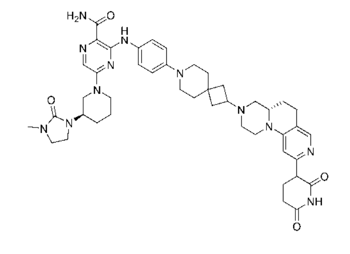 |
| 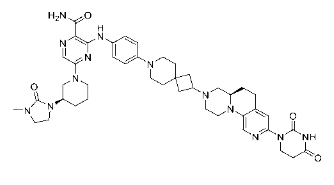 | 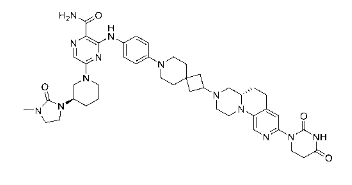 | 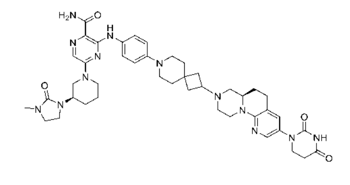 |
| 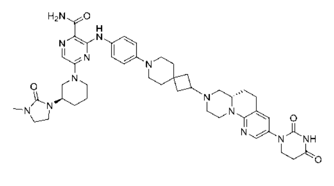 | 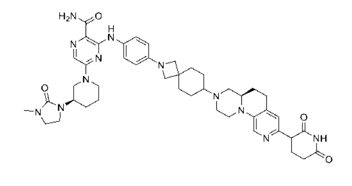 | 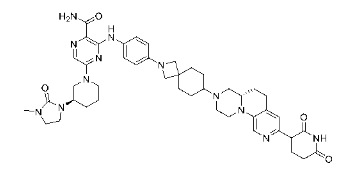 |
| 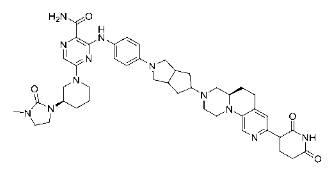 | 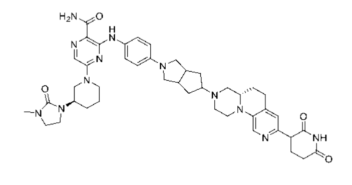 | 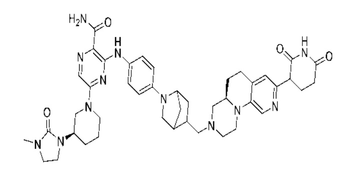 |
| 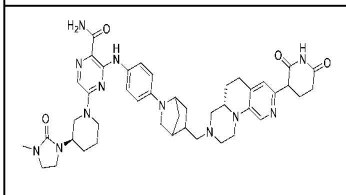 | 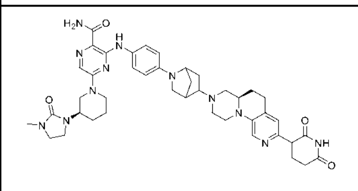 | 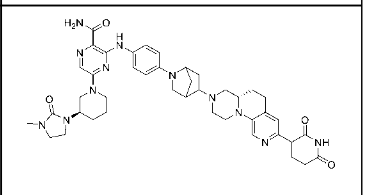 |
| 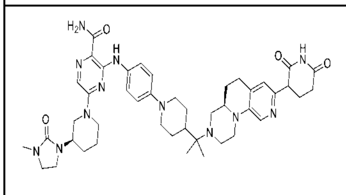 | 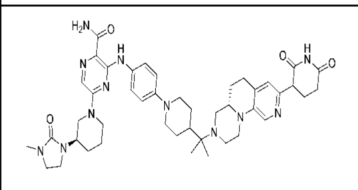 | 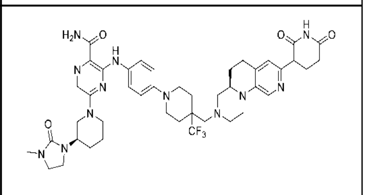 |
| 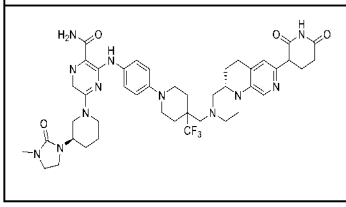 | 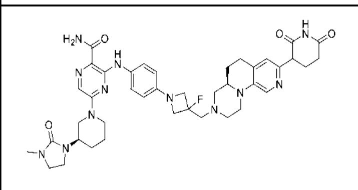 | 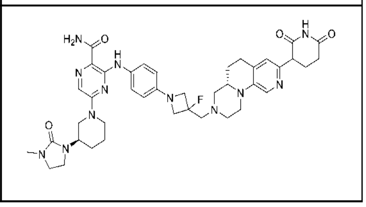 |

| | | |
|---|---|---|
| 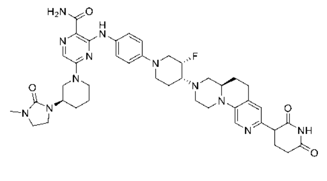 | 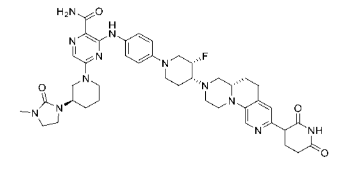 | 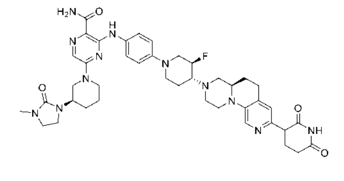 |
| 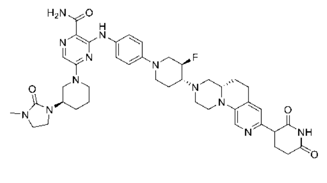 | 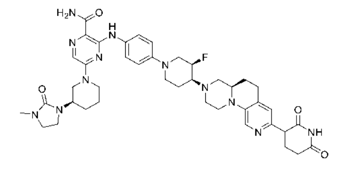 | 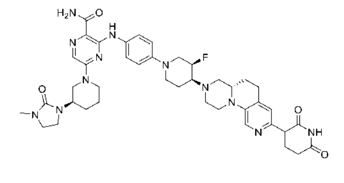 |
| 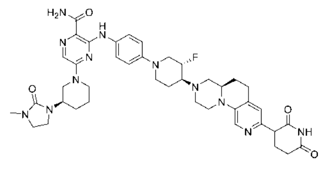 | 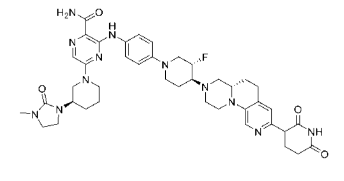 | 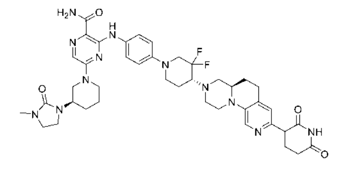 |
| 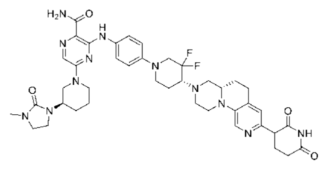 | 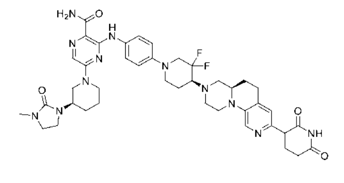 | 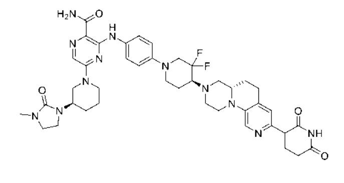 |
| 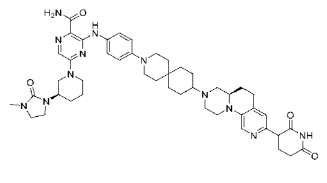 | 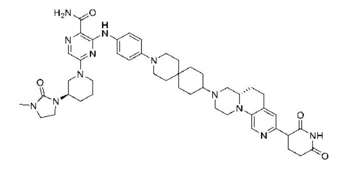 | 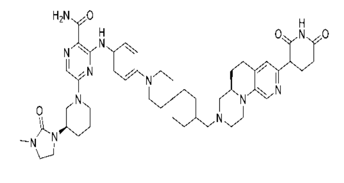 |
| 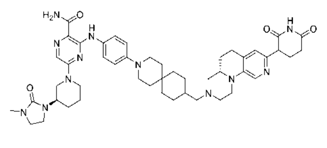 | 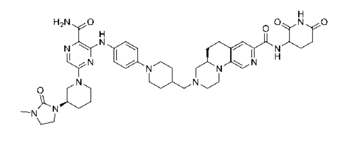 | 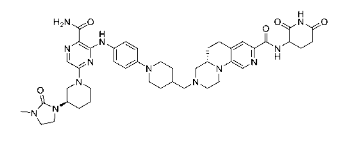 |
| 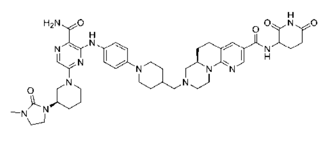 | 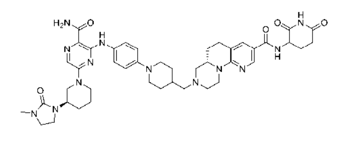 | 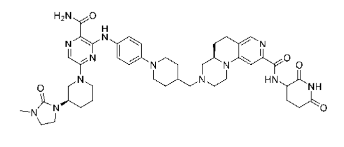 |
| 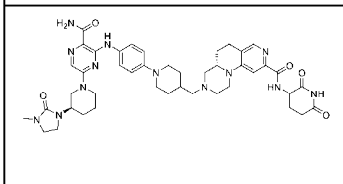 | 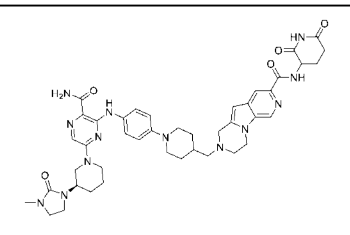 | 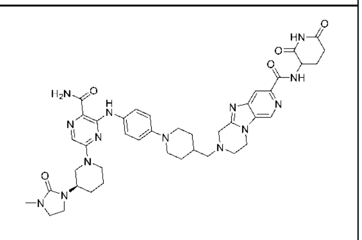 |
| 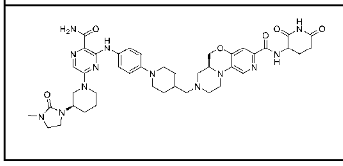 | 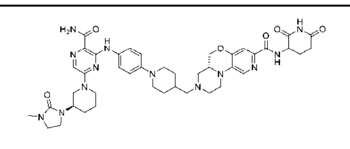 | 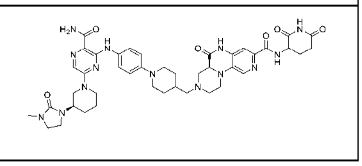 |

| | | |
|---|---|---|
| 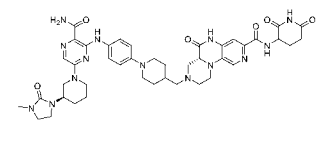 | 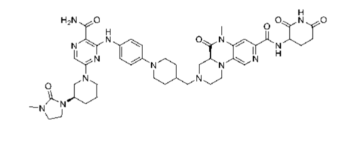 | 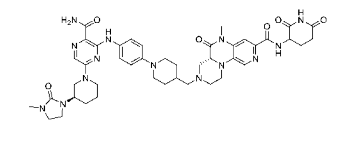 |
| 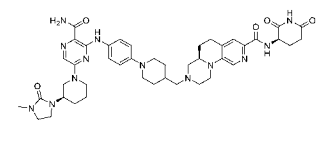 | 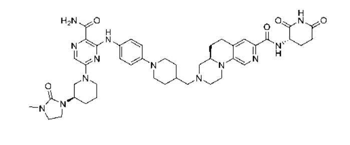 | 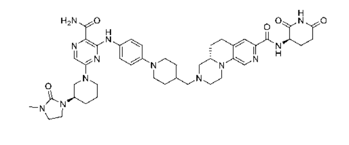 |
| 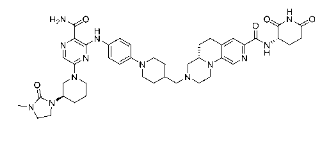 | 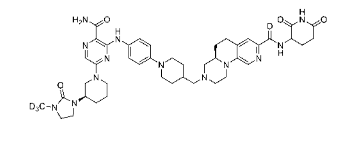 | 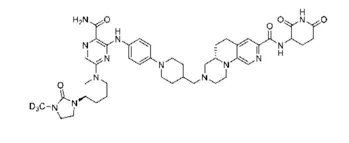 |
| 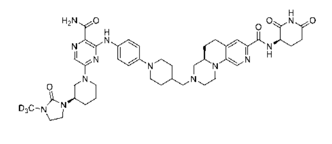 | 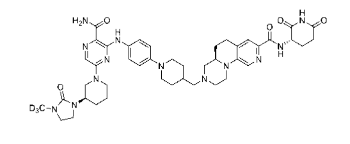 | 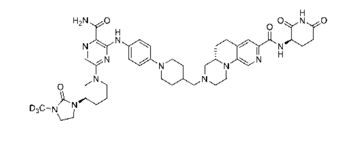 |
| 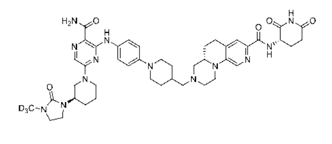 | 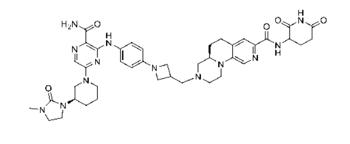 | 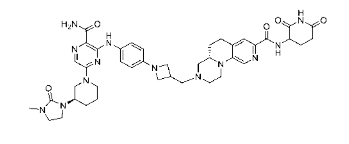 |
| 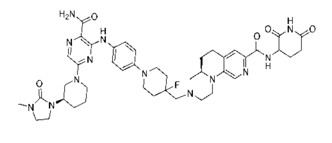 | 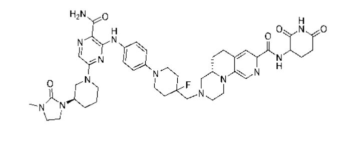 | 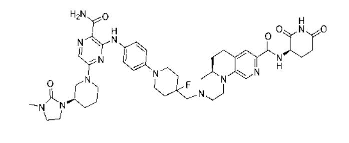 |
| 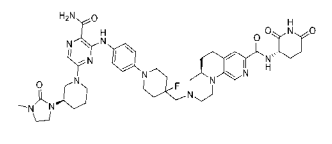 | 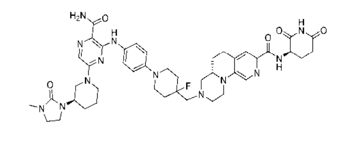 | 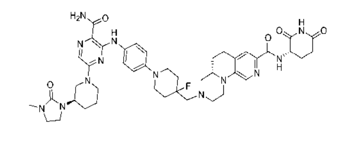 |
| 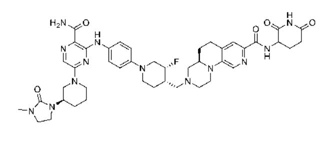 | 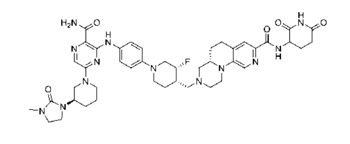 | 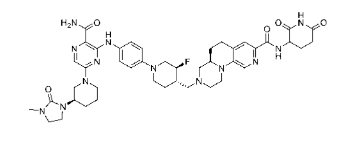 |
| 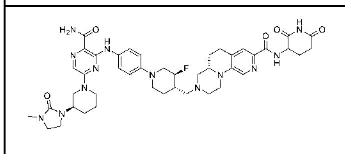 | 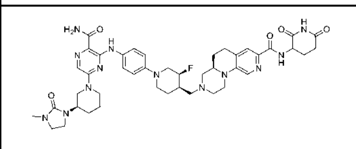 | 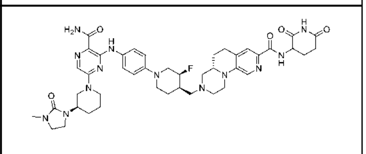 |
| 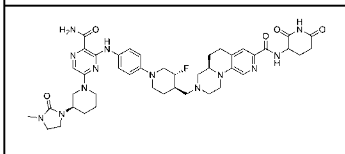 | 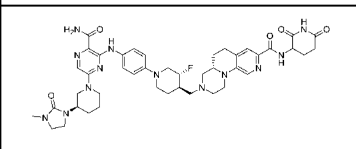 | 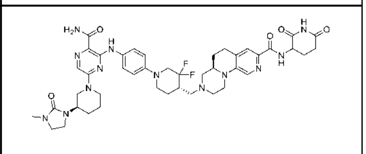 |
| 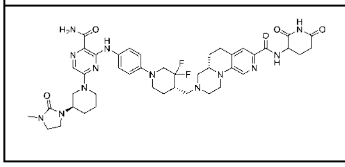 | 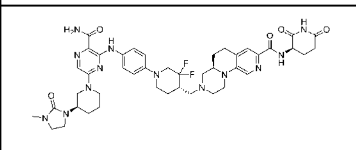 | 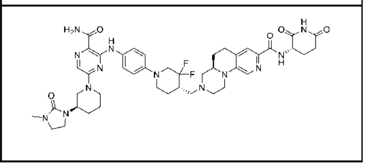 |

| | | |
|---|---|---|
| 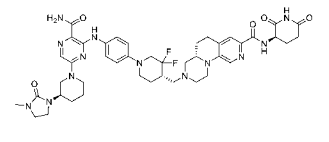 | 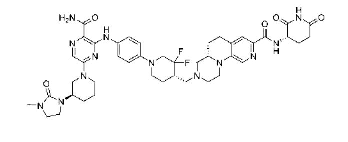 | 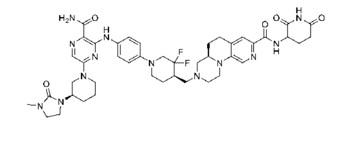 |
| 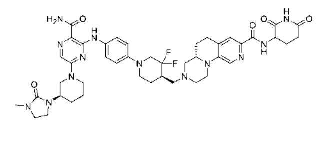 | 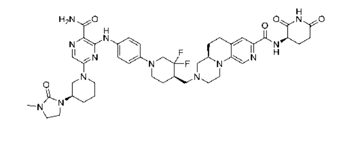 | 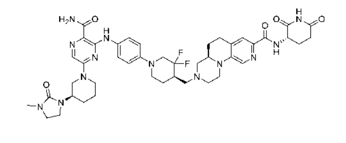 |
| 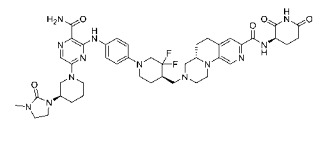 | 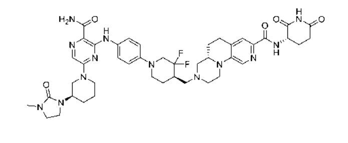 | 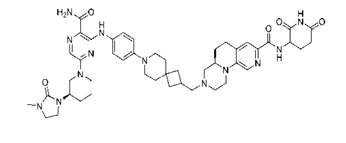 |
| 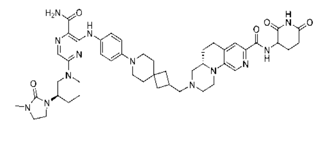 | 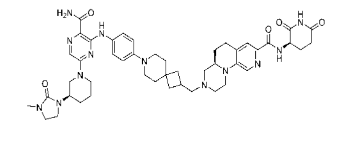 | 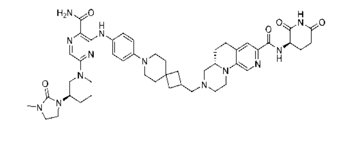 |
| 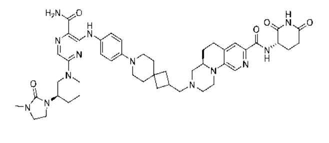 | 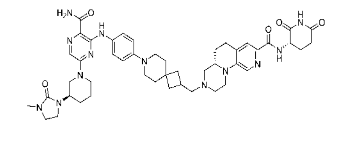 | 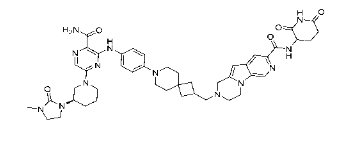 |
| 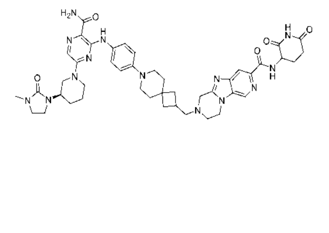 | 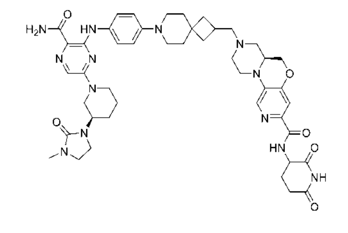 | 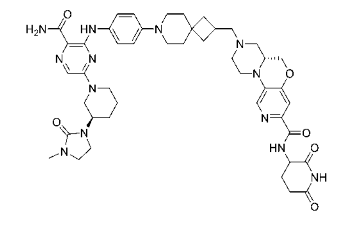 |
| 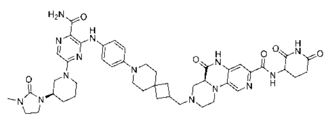 | 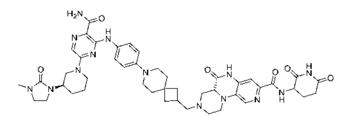 | 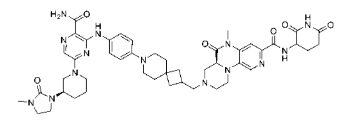 |
| 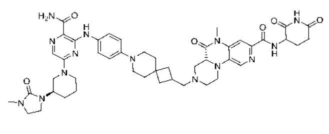 | 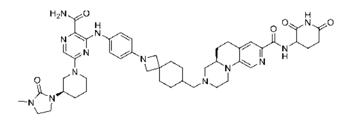 | 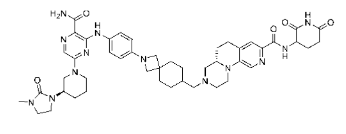 |
| 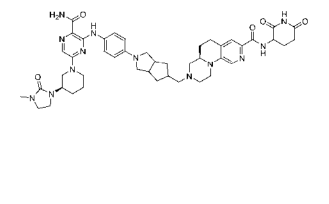 | 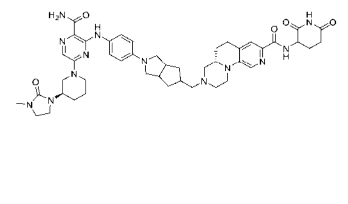 | 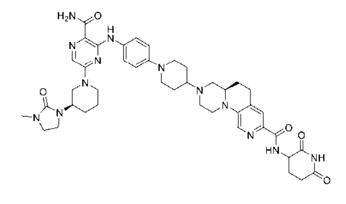 |
| 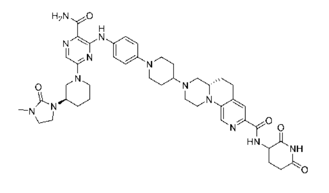 | 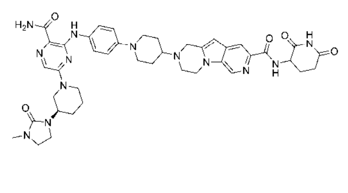 | 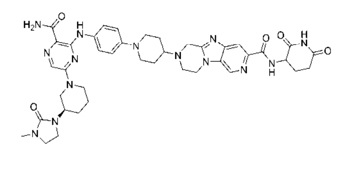 |

| | | |
|---|---|---|
| 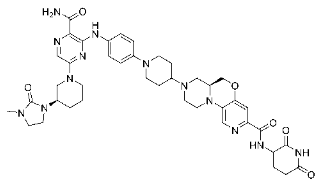 | 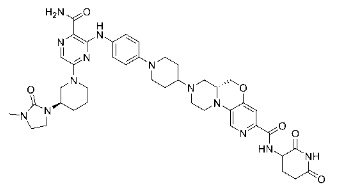 | 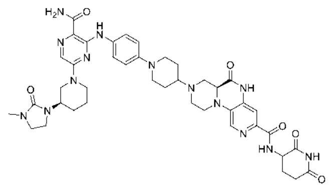 |
| 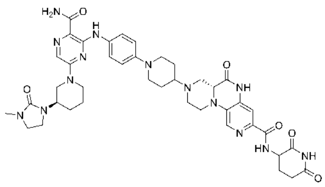 | 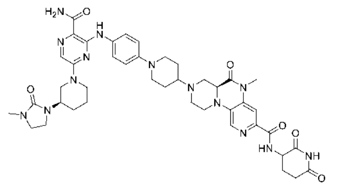 | 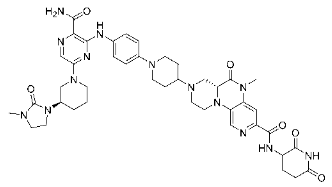 |
| 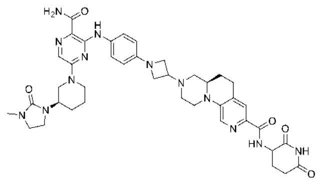 | 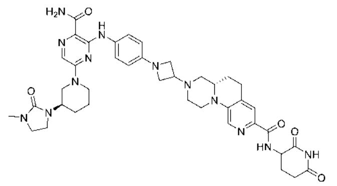 | 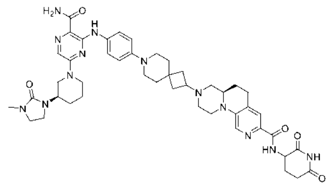 |
| 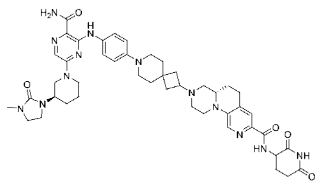 | 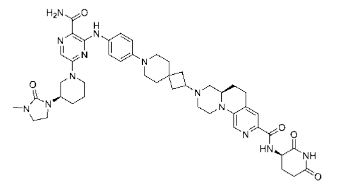 | 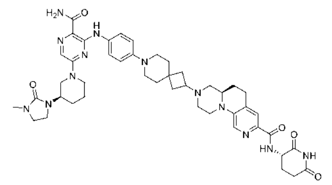 |
| 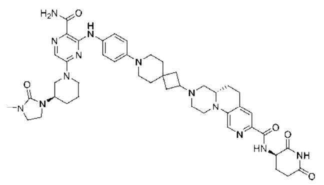 | 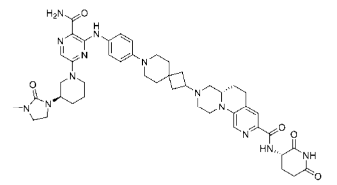 | 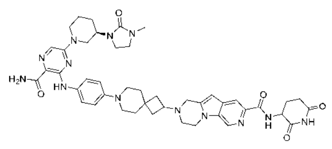 |
| 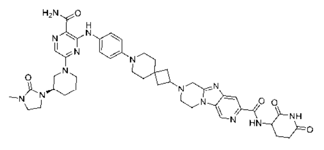 | 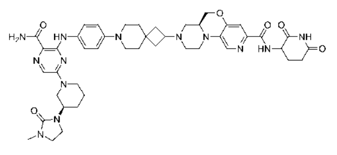 | 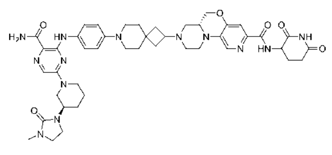 |
| 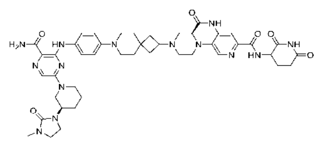 | 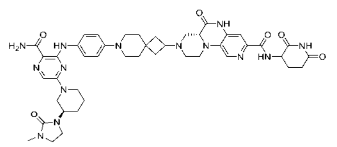 | 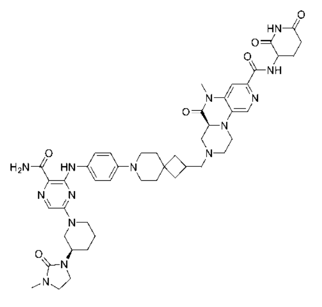 |

| | | |
|---|---|---|
| 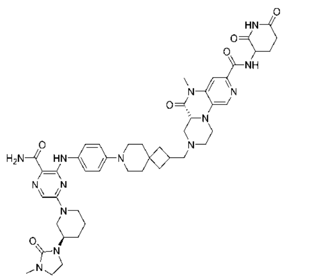 | 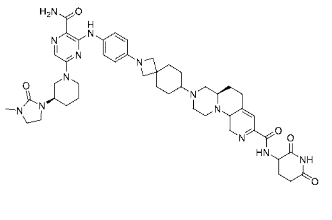 | 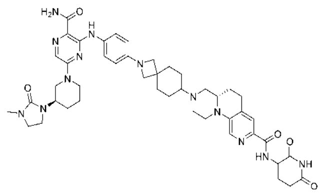 |
| 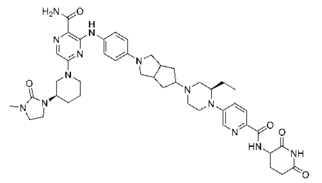 | 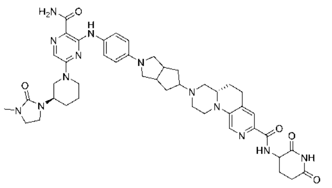 | 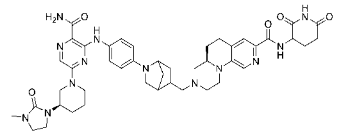 |
| 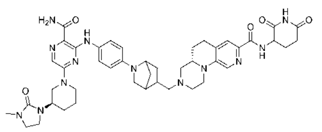 | 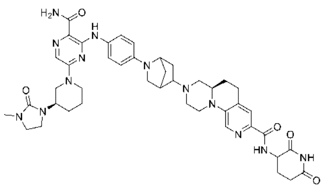 | 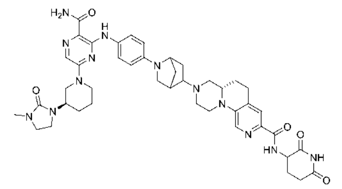 |
| 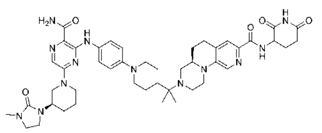 | 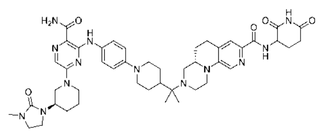 | 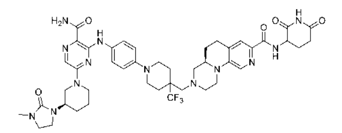 |
| 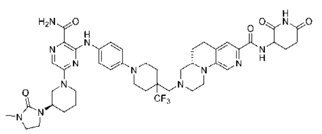 | 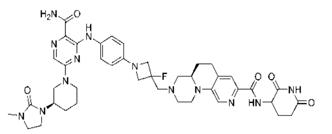 | 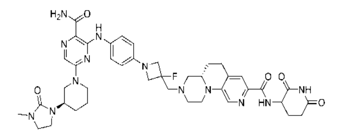 |
| 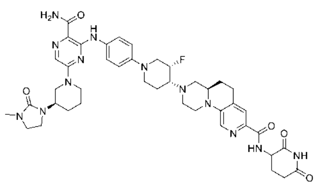 | 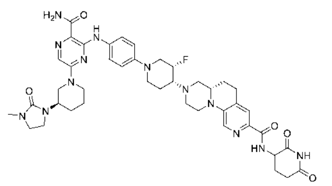 | 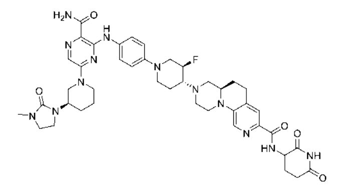 |
| 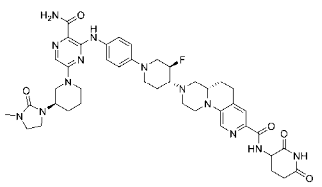 | 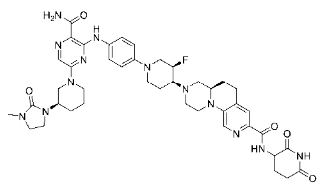 | 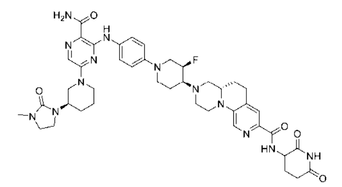 |
| 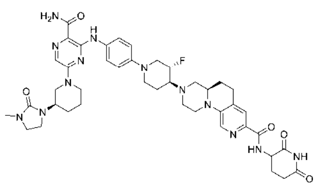 | 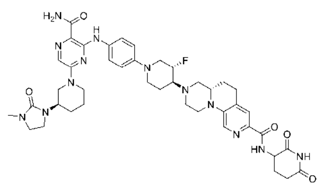 | 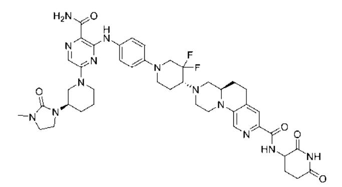 |

| | | |
|---|---|---|
| 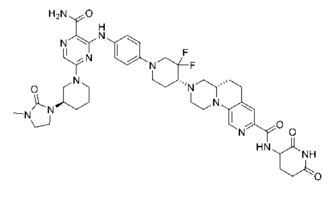 | 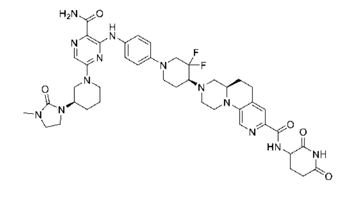 | 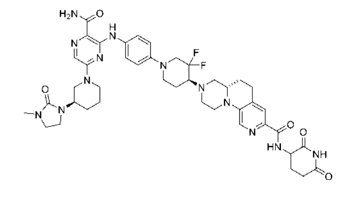 |
| 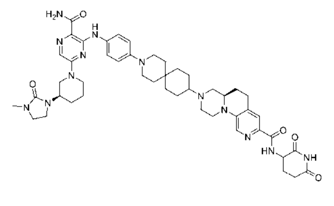 | 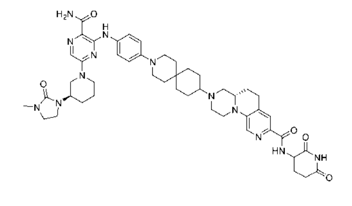 | 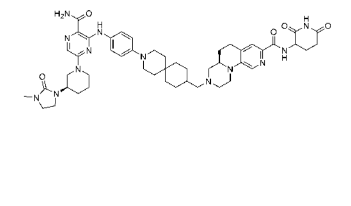 |
| 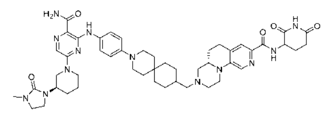 | 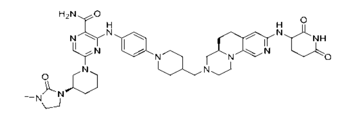 | 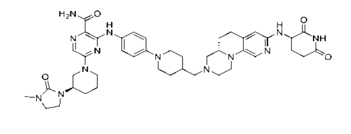 |
| 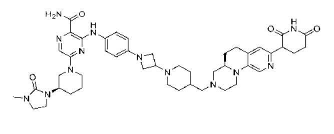 | 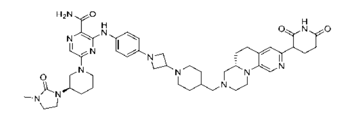 | 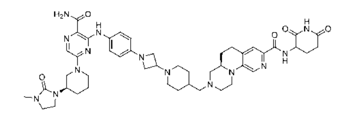 |
| 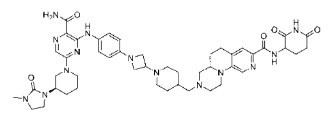 | 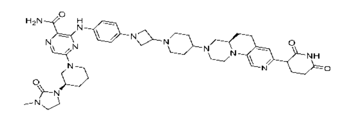 | 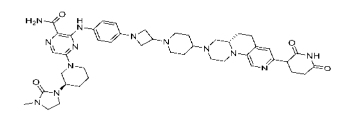 |
| 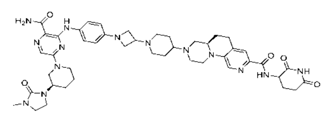 | 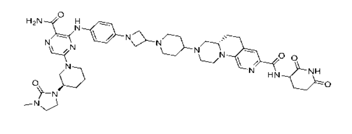 | 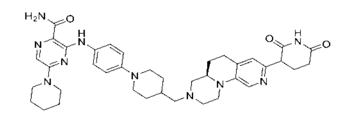 |
| 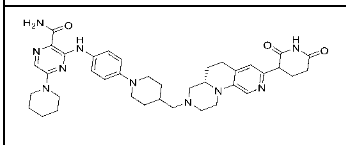 | 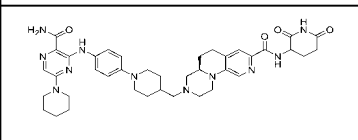 | 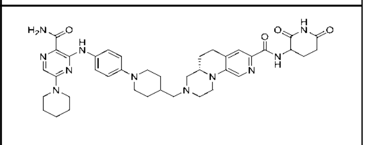 |
| 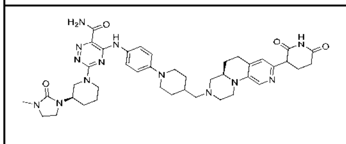 | 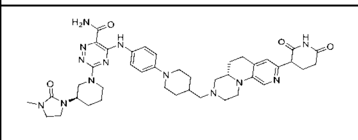 | 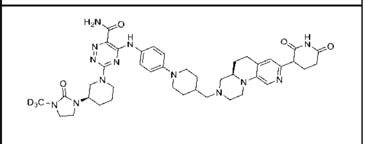 |
| 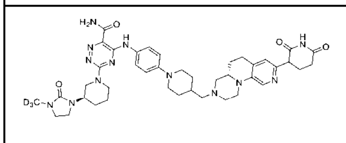 | 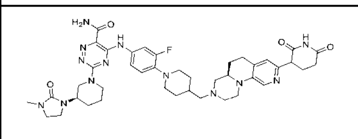 | 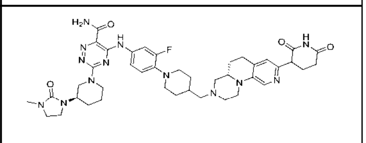 |
| 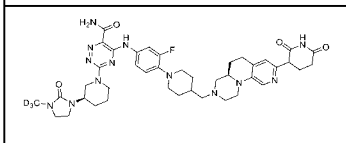 | 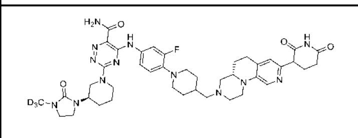 | 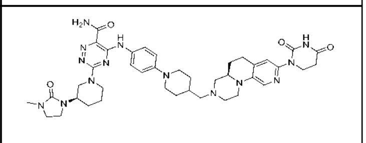 |
| 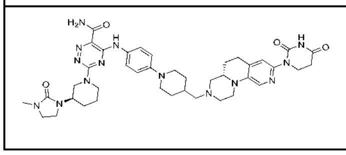 | 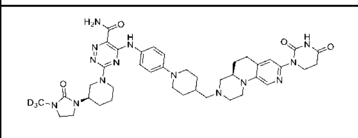 | 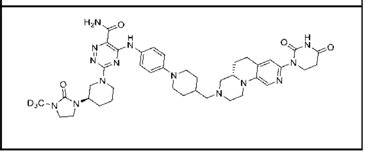 |

| | | |
|---|---|---|
| 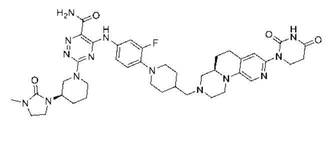 | 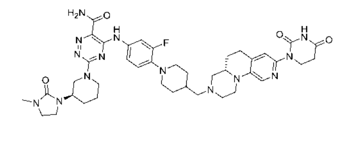 | 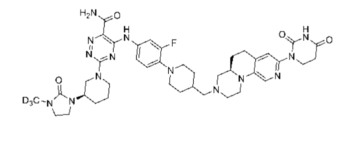 |
| 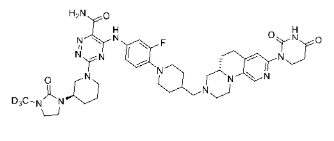 | 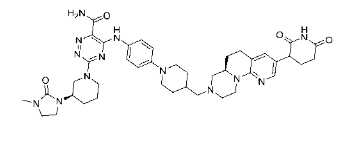 | 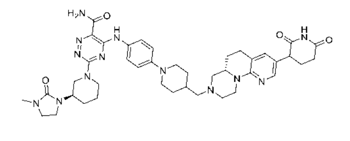 |
| 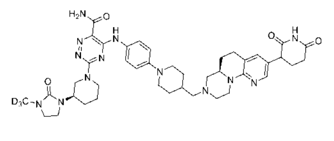 | 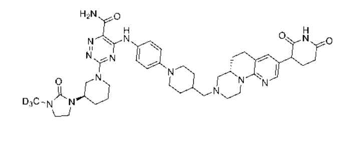 | 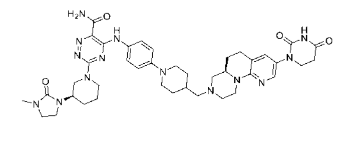 |
| 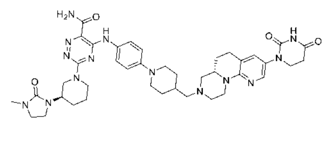 | 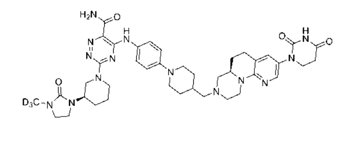 | 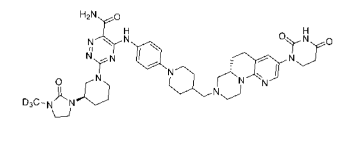 |
| 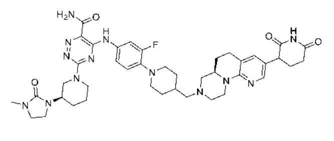 | 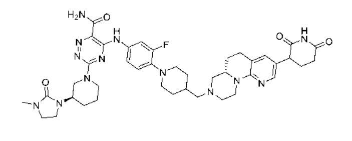 | 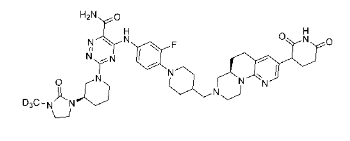 |
| 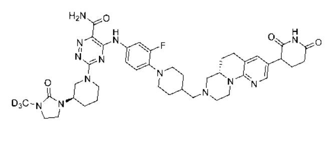 | 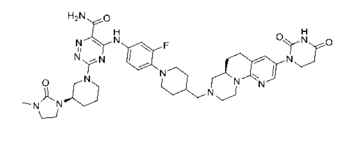 | 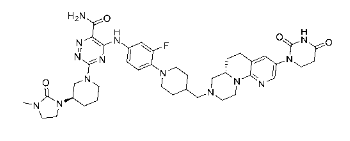 |
| 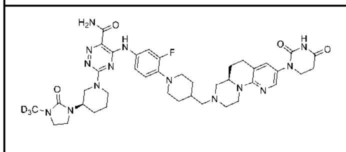 | 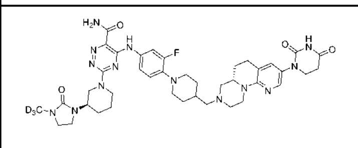 | 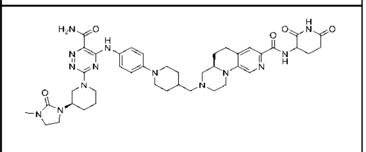 |
| 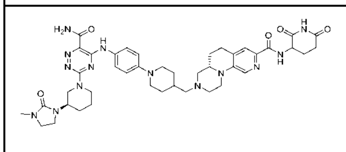 | 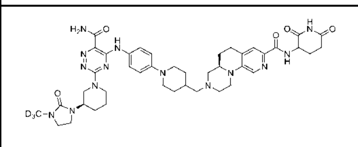 | 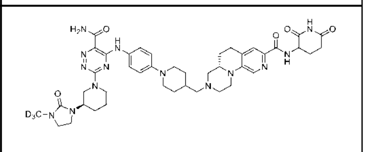 |
| 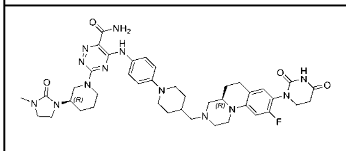 | 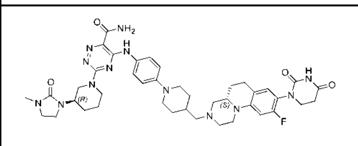 | 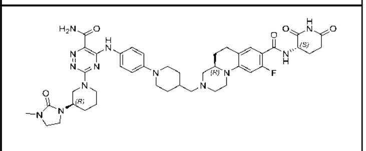 |
| 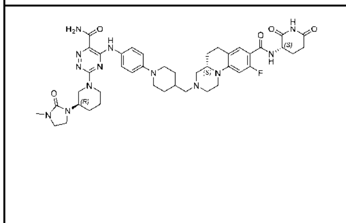 | 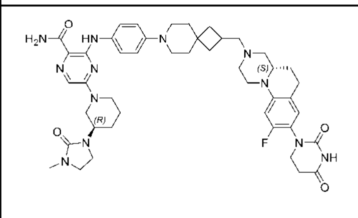 | 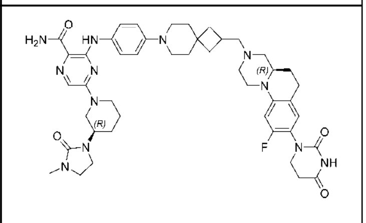 |
| 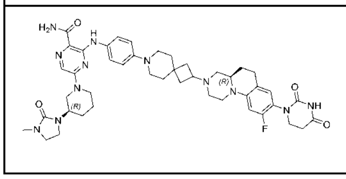 | 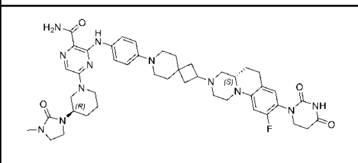 | 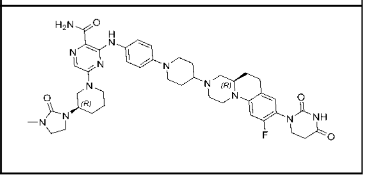 |

| | | |
|---|---|---|
| 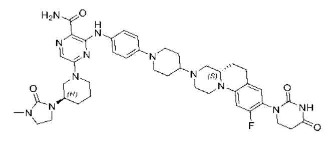 | 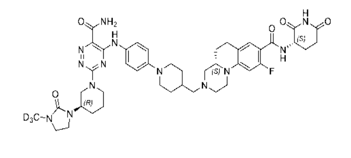 | 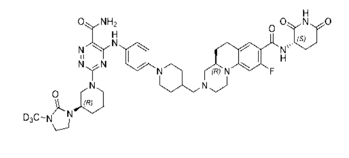 |
| 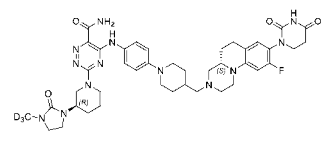 | 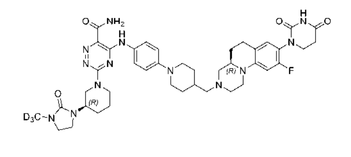 | 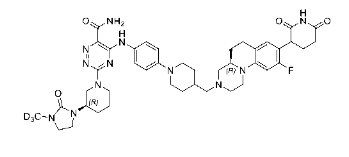 |
| 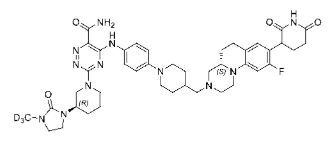 | 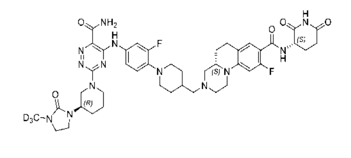 | 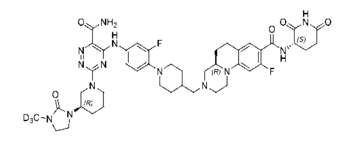 |
| 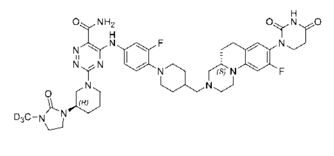 | 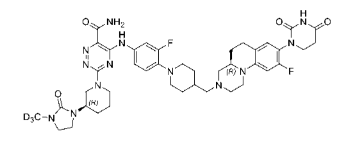 | 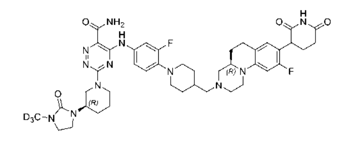 |
| 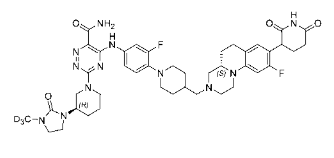 | 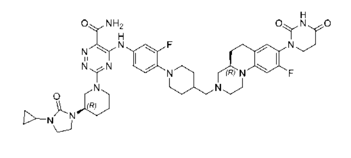 | 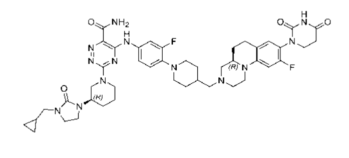 |
| 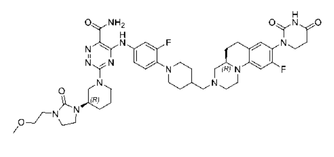 | 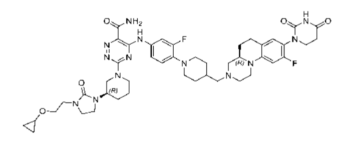 | 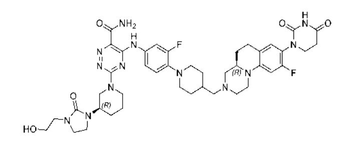 |
| 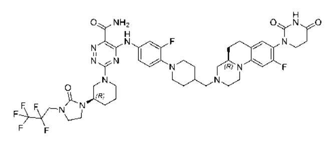 | 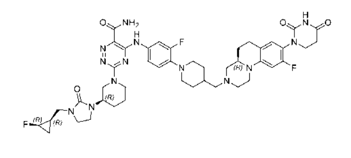 | 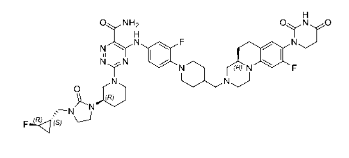 |
| 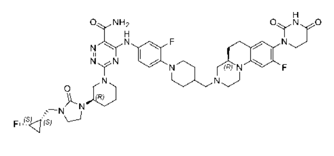 | 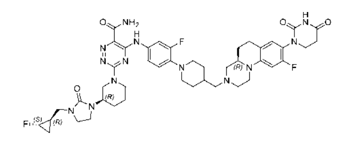 | 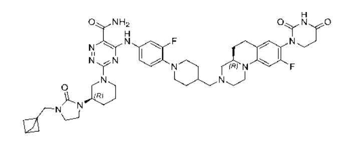 |
| 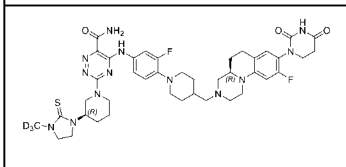 | 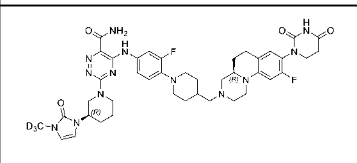 | 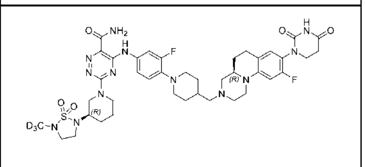 |
| 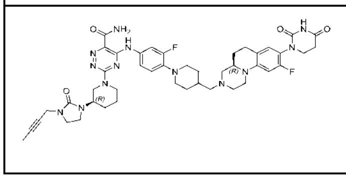 | 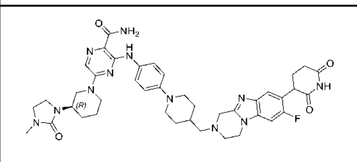 | 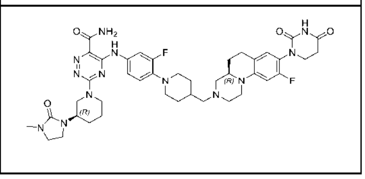 |

| | | |
|---|---|---|
| 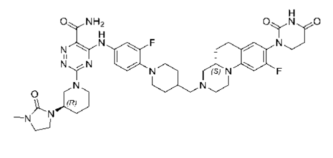 | 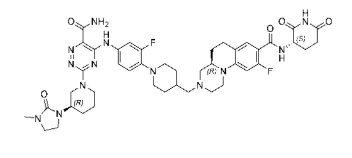 | 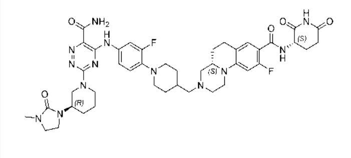 |
| 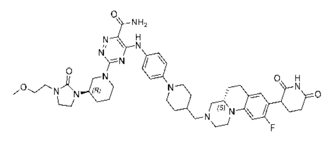 | 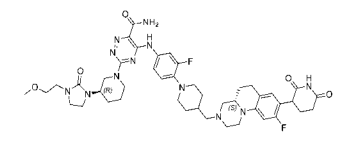 | 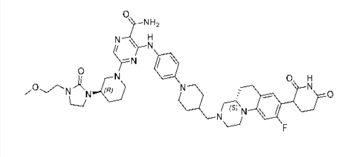 |
| 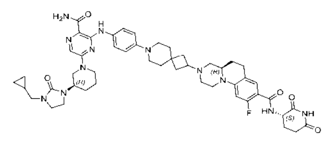 | 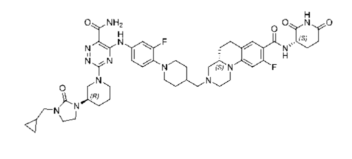 | 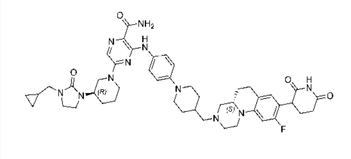 |
| 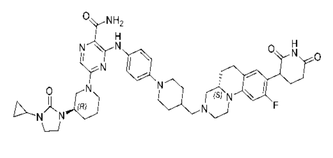 | 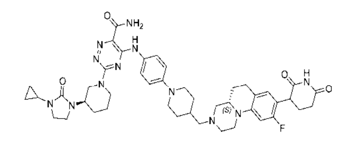 | 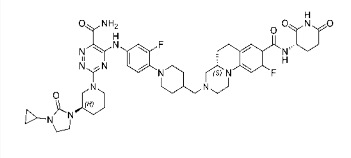 |
| 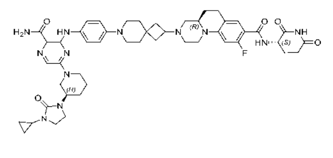 | 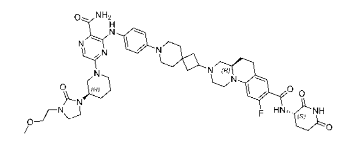 | 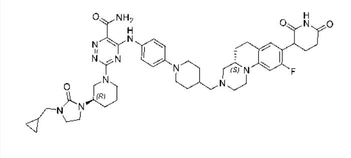 |
| 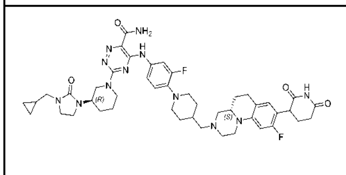 | 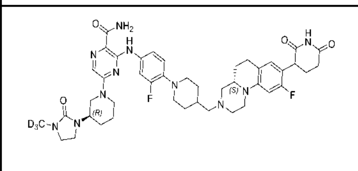 | 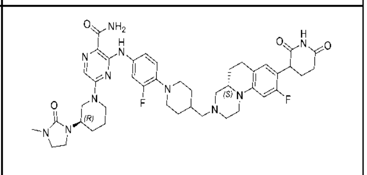 |
| 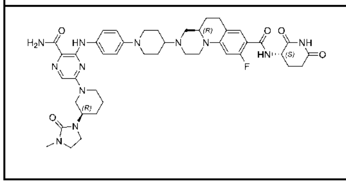 | 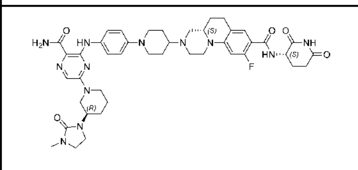 | 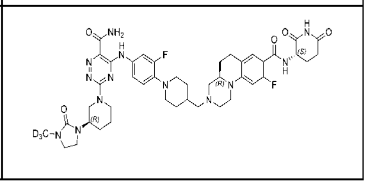 |

.

The present invention relates to a pharmaceutical composition comprising the above-mentioned compound or the stereoisomer, tautomer, deuterated compound, solvate, prodrug, metabolite, pharmaceutically acceptable salt or co-crystal thereof, and a pharmaceutically acceptable carrier.

The present invention relates to the use of the above-mentioned compound or the stereoisomer, tautomer, deuterated compound, solvate, prodrug, metabolite, pharmaceutically acceptable salt or co-crystal thereof, or the above-mentioned pharmaceutical composition in the preparation of a medicament that inhibits or degrades BTK.

The present invention relates to the use of the above-mentioned compound or the stereoisomer, tautomer, deuterated compound, solvate, prodrug, metabolite, pharmaceutically acceptable salt or co-crystal thereof, or the above-mentioned pharmaceutical composition in the preparation of a medicament for the treatment of tumours or autoimmune diseases, preferably B-cell malignancies, more preferably relapsed/refractory B-cell malignancies.

The present invention relates to a pharmaceutical composition or a pharmaceutical preparation, comprising a therapeutically effective amount of the compound or the stereoisomer, tautomer, deuterated compound, solvate, prodrug, metabolite, pharmaceutically acceptable salt or co-crystal thereof according to the present invention, and a pharmaceutically acceptable excipient. The pharmaceutical composition can be in a unit dosage form (the amount of the active pharmaceutical ingredient per unit dosage form is also referred to as the "dosage strength").

The present invention further provides a method for treating a disease in a mammal, comprising administering to the mammal a therapeutically effective amount of the compound or the stereoisomer, tautomer, deuterated compound, solvate, prodrug, metabolite, pharmaceutically acceptable salt or co-crystal thereof, or the pharmaceutical composition

according to the present invention. In some embodiments, the mammal according to the present invention comprises human.

The term "effective amount" or "therapeutically effective amount" according to the present application refers to a sufficient amount of the compound disclosed in the present application that is administered to ameliorate, to some extent, one or more symptoms of a disease or condition (such as cancer or autoimmune diseases) being treated. In some embodiments, the outcome is the reduction and/or remission of signs, symptoms or causes of the disease, or any other desired change in the biological system. For example, an "effective amount" in terms of the therapeutic use is an amount of the included compound disclosed in the present application that is required to provide clinically significant reduction of the symptoms of the disease. Examples of the therapeutically effective amount include, but are not limited to 1-1500 mg, 1-1000 mg, 1-800 mg, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, and 150-200 mg; in some embodiments, the pharmaceutical composition comprises the compound or the stereoisomer, tautomer, deuterated compound, solvate, prodrug, metabolite, pharmaceutically acceptable salt or co-crystal thereof according to the present invention in an amount including but not limited to 1-1500 mg, 1-1000 mg, 1-800 mg, 1-600 mg, 20-400 mg, 25-200 mg, 1 mg, 5 mg, 10 mg, 15 mg, 20 mg, 25 mg, 30 mg, 35 mg, 40 mg, 45 mg, 50 mg, 55 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, and 300 mg.

Provided is a method for treating a disease in a mammal, comprising administering to a subject a therapeutically effective amount of the compound or the stereoisomer, tautomer, deuterated compound, solvate, prodrug, metabolite, pharmaceutically acceptable salt or co-crystal thereof according to the present invention, wherein the therapeutically effective amount is preferably 1-1500 mg, and the disease is preferably tumours or autoimmune diseases, more preferably relapsed/refractory B-cell malignancies.

Provided is a method for treating a disease in a mammal, comprising administering to a subject a drug, i.e., the compound or the stereoisomer, tautomer, deuterated compound, solvate, prodrug, metabolite, pharmaceutically acceptable salt or co-crystal thereof according to the present invention in a daily dose of 1-1500 mg/day, wherein the daily dose can be a single dose or divided doses; in some embodiments, the daily dose includes, but is not limited to 10-1500 mg/day, 10-1000 mg/day, 10-800 mg/day, 25-800 mg/day, 50-800 mg/day, 100-800 mg/day, 200-800 mg/day, 25-400 mg/day, 50-400 mg/day, 100-400 mg/day, and 200-400 mg/day; in some embodiments, the daily dose includes, but is not limited to 10 mg/day, 20 mg/day, 25 mg/day, 50 mg/day, 100 mg/day, 125 mg/day, 150 mg/day, 200 mg/day, 400 mg/day, 600 mg/day, and 800 mg/day.

The present invention relates to a kit, wherein the kit may comprise a composition in the form of a single dose or multiple doses and comprises the compound or the stereoisomer, tautomer, deuterated compound, solvate, prodrug, metabolite, pharmaceutically acceptable salt or co-crystal thereof according to the present invention, and the amount of the compound or the stereoisomer, tautomer, deuterated compound, solvate, prodrug, metabolite, pharmaceutically acceptable salt or co-crystal thereof according to the present invention is identical to the amount of same in the above-mentioned pharmaceutical composition.

In the present invention, the amount of the compound or the stereoisomer, tautomer, deuterated compound, solvate, prodrug, metabolite, pharmaceutically acceptable salt or co-crystal thereof according to the present invention is calculated in the form of free base in each case.

The term "dosage strength" refers to the amount of the active pharmaceutical ingredient contained in each vial, tablet or any other single unit dosage form.

The compound of the present invention can be prepared according to the following method:

General synthetic method I:

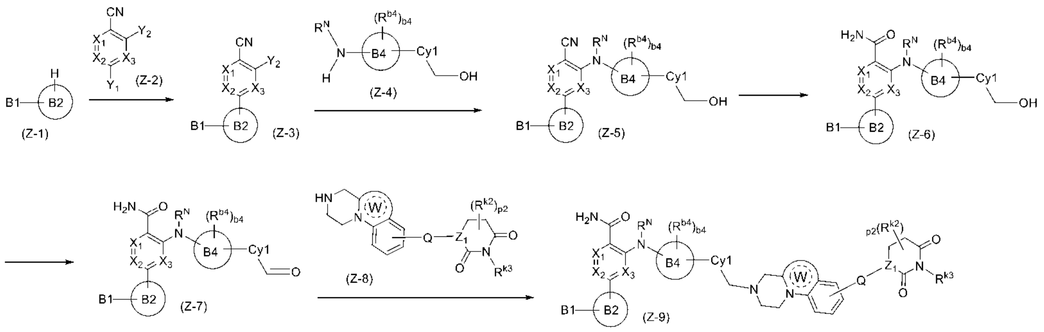

a compound of general formula (Z-1) and a compound of general formula (Z-2) are subjected to a nucleophilic substitution or coupling reaction to obtain a compound of general formula (Z-3);
the compound of general formula (Z-3) and a compound of general formula (Z-4) are subjected to a nucleophilic substitution or coupling reaction to obtain a compound of general formula (Z-5);
the compound of general formula (Z-5) is subjected to a hydrolysis reaction to obtain a compound of general formula (Z-6);
the compound of general formula (Z-6) is subjected to an oxidation reaction to obtain a compound of general formula (Z-7);
the compound of general formula (Z-7) and a compound of general formula (Z-8) are subjected to a reductive amination reaction to obtain a compound of general formula (Z-9), i.e., a compound of general formula (I).

The definitions of the remaining groups are the same as those in the description and claims.

Unless stated to the contrary, the terms used in the description and claims of the present application have the following meanings.

The carbon, hydrogen, oxygen, sulphur, nitrogen or F, Cl, Br, I involved in the groups and compounds of the present invention all comprise their isotopes, and the carbon, hydrogen, oxygen, sulphur or nitrogen involved in the groups and compounds of the present invention is optionally replaced with one or more of their corresponding isotopes, wherein the isotopes of carbon comprise $^{12}C$, $^{13}C$ and $^{14}C$, the isotopes of hydrogen comprise protium (H), deuterium (D, also known as heavy hydrogen), tritium (T, also known as superheavy hydrogen), the isotopes of oxygen comprise $^{16}O$, $^{17}O$ and $^{18}O$, the isotopes of sulphur comprise $^{32}S$, $^{33}S$, $^{34}S$ and $^{36}S$, the isotopes of nitrogen comprise $^{14}N$ and $^{15}N$, the isotopes of fluorine comprise $^{17}F$ and $^{19}F$, the isotopes of chlorine comprise $^{35}Cl$ and $^{37}Cl$, and the isotopes of bromine comprise $^{79}Br$ and $^{81}Br$.

"Halogen" refers to F, Cl, Br or I.

"Halogen-substituted" refers to a substitution with F, Cl, Br, or I, including but not limited to a substitution with 1 to 10 substituents selected from F, Cl, Br, or I, or a substitution with 1 to 6 substituents selected from F, Cl, Br, or I, preferably a substitution with 1 to 4 substituents selected from F, Cl, Br, or I. "Halogen-substituted" is referred to simply as "halo".

"Alkyl" refers to a substituted or unsubstituted linear or branched saturated aliphatic hydrocarbon group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl and various branched isomers thereof. The definition of the alkyl herein is consistent with this definition. The alkyl can be monovalent, divalent, trivalent or tetravalent.

"Alkylene" refers to a substituted or unsubstituted linear or branched divalent saturated hydrocarbon group, including $-(CH_2)_v-$ (v is an integer from 1 to 10). Examples of the alkylene include, but are not limited to, methylene, ethylene, propylene, and butylene.

"Cycloalkyl" refers to a substituted or unsubstituted saturated carbocyclic hydrocarbon group, typically having 3 to 10 carbon atoms, and non-limiting examples include cyclopropyl, cyclobutyl, cyclopentyl, cyclohexyl, cycloheptyl, etc. The "cycloalkyl" herein is as defined above. The cycloalkyl can be monovalent, divalent, trivalent or tetravalent.

"Heterocycloalkyl" refers to a substituted or unsubstituted saturated heteroatom-containing cyclic hydrocarbon group, including but not limited to 3 to 10 atoms, 3 to 8 atoms, or 1 to 3 heteroatoms selected from N, O or S. The selectively substituted N and S in the ring of the heterocycloalkyl can be oxidised to various oxidation states. Heterocycloalkyl can be connected to a heteroatom or a carbon atom; heterocycloalkyl can be connected to an aromatic ring or a non-aromatic ring; and heterocycloalkyl can be connected to a bridged ring or a spiro ring. Non-limiting examples include oxiranyl, aziridinyl, oxetanyl, azetidinyl, tetrahydrofuryl, tetrahydro-2*H*-pyranyl, dioxolanyl, dioxanyl, pyrrolidyl, piperidyl, imidazolidinyl, oxazolidinyl, oxazinanyl, morpholinyl, hexahydropyrimidyl or piperazinyl. The heterocycloalkyl can be monovalent, divalent, trivalent or tetravalent.

"Alkenyl" refers to a substituted or unsubstituted linear or branched unsaturated hydrocarbon group, having at least 1, typically 1, 2 or 3 carbon-carbon double bonds, with a main chain including but not limited to, 2 to 10, 2 to 6, or 2 to 4 carbon atoms. Examples of the alkenyl include, but are not limited to, vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, and 5-hexenyl. The definition of the alkenyl herein is consistent with this definition. The alkenyl can be monovalent, divalent, trivalent or tetravalent.

"Alkynyl" refers to a substituted or unsubstituted linear or branched unsaturated hydrocarbon group, having at least 1, typically 1, 2 or 3 carbon-carbon triple bonds, with a main chain including 2 to 10 carbon atoms, including but not limited to a main chain including 2 to 6 carbon atoms, or a main chain including 2 to 4 carbon atoms. Examples of the alkynyl include, but are not limited to, ethynyl, propargyl, 1-propynyl, 2-propynyl, 1-butynyl, 2-butynyl, 3-butynyl, 1-pentynyl, 2-pentynyl, 3-pentynyl, and 4-pentynyl. The alkynyl can be monovalent, divalent, trivalent or tetravalent.

"Propynyl" refers to 1-propynyl or 2-propynyl.

"Alkoxy" refers to substituted or unsubstituted -O-alkyl. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocyclic ring" refers to a substituted or unsubstituted saturated or unsaturated aromatic ring or non-aromatic ring, wherein the aromatic ring or non-aromatic ring can be a 3- to 8-membered monocyclic ring, 4- to 12-membered bicyclic ring, or 10- to 15-membered tricyclic ring system. Carbocyclyl can be connected to an aromatic ring or non-aromatic ring, wherein the aromatic ring or non-aromatic ring is optionally a monocyclic ring, a bridged ring, or a spiro ring. Non-limiting examples include cyclopropane, cyclobutane, cyclopentane, cyclohexane, cycloheptane, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, a benzene ring, a naphthalene ring,

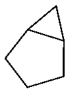, 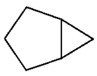, 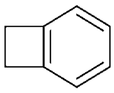,

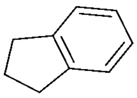 or 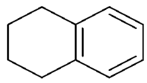.

The "carbocyclyl" or "carbocyclic ring" can be monovalent, divalent, trivalent or tetravalent.

"Heterocyclyl" or "heterocyclic ring" refers to a substituted or unsubstituted saturated or unsaturated aromatic ring or non-aromatic ring, wherein the aromatic ring or non-aromatic ring can be a 3- to 8-membered monocyclic ring, a 4- to 12-membered bicyclic ring or a 10- to 15-membered tricyclic ring system, and contains one or more (including but not limited to 2, 3, 4 or 5) heteroatoms selected from N, O or S. The selectively substituted N and S in the ring of the heterocyclyl can be oxidised to various oxidation states. Heterocyclyl can be connected to a heteroatom or a carbon atom; heterocyclyl can be connected to an aromatic ring or a non-aromatic ring; and heterocyclyl can be connected to a bridged ring or a spiro ring. Non-limiting examples include oxiranyl, aziridinyl, oxetanyl, azetidinyl, 1,3-dioxolanyl, 1,4-dioxolanyl, 1,3-dioxanyl, azepanyl, pyridyl, furyl, thienyl, pyranyl, N-alkylpyrrolyl, pyrimidyl, pyrazinyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, tetrahydrothiazolyl, tetrahydropyranyl, benzoimidazolyl, benzopyridyl, pyrrolopyridyl, benzodihydrofuryl, pyrrolyl, pyrazolyl, thiazolyl, oxazolyl, pyrazinyl, indazolyl, benzothienyl, benzofuryl, benzopyrrolyl, benzoimidazolyl, benzothiazolyl, benzoxazolyl, benzopyridyl, benzopyrimidyl, benzopyrazinyl, piperazinyl, azabicyclo[3.2.1]octanyl, azabicyclo[5.2.0]nonanyl, oxatricyclo[5.3.1.1]dodecyl, azaadamantyl, oxaspiro[3.3]heptanyl,

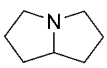,

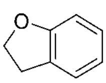, 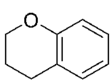, 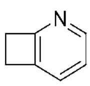, 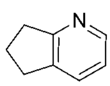, 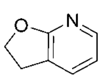, 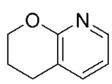, 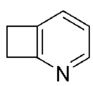, 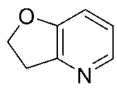,

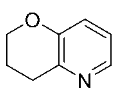, 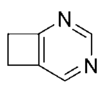, 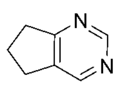, 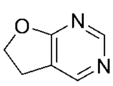 or 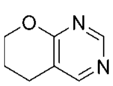.

The "heterocyclyl" or "heterocyclic ring" can be monovalent, divalent, trivalent or tetravalent.

"Spiro ring" or "spiro ring group" refers to a polycyclic group that shares one atom (called a spiro atom) between substituted or unsubstituted monocyclic rings. The number of ring atoms in the spiro ring system includes but is not limited to 5 to 20, 6 to 14, 6 to 12, or 6 to 10, wherein one or more rings may contain 0 or more (including but not limited to 1, 2, 3 or 4) double bonds, and may optionally contain 1 to 5 heteroatoms selected from N, O or $S(=O)_n$.

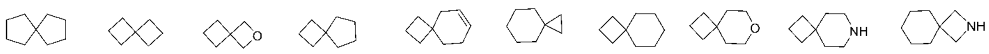

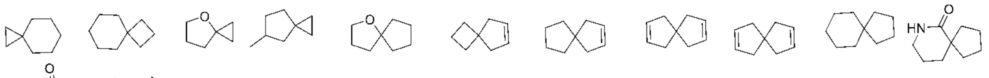

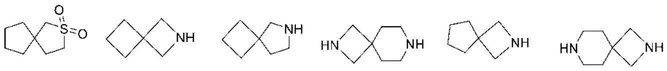

The "spiro ring" or "spiro ring group" can be monovalent, divalent, trivalent or tetravalent.

"Fused ring" or "fused ring group" refers to a polycyclic group in which each ring in the system shares an adjacent pair of atoms with other rings in the system, wherein one or more rings may contain 0 or more (including but not limited to 1, 2, 3 or 4) double bonds, and may be substituted or unsubstituted, and each ring in the fused ring system may contain 0 to 5 heteroatoms or groups containing heteroatoms (including but not limited to N, $S(=O)_n$ or O, wherein n is 0, 1 or 2). The number of ring atoms in the fused ring system includes, but is not limited to 5 to 20, 5 to 14, 5 to 12, or 5 to 10. Non-limiting examples include:

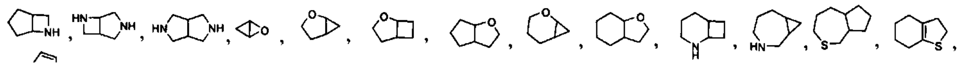

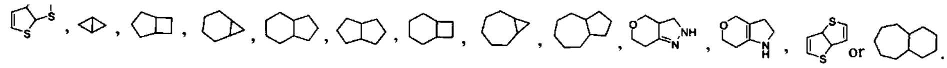

or

The "fused ring" or "fused ring group" can be monovalent, divalent, trivalent or tetravalent.

"Bridged ring" or "bridged ring group" refers to a substituted or unsubstituted polycyclic group containing any two rings sharing two atoms that are not directly connected, and may contain 0 or more double bonds. Any ring in the bridged ring system may contain 0 to 5 groups selected from heteroatoms or groups containing heteroatoms (including but not limited to N, S(=O)n or O, wherein n is 0, 1 or 2). The number of ring atoms includes, but is not limited to 5 to 20, 5 to 14, 5 to 12, or 5 to 10. Non-limiting examples include

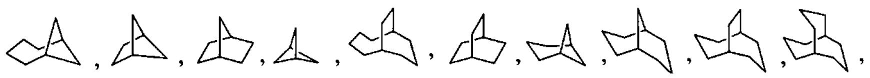

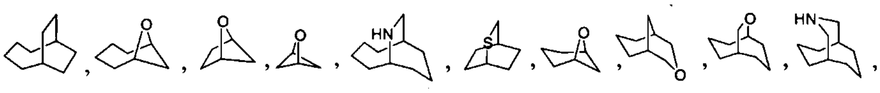

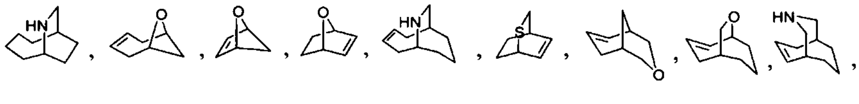

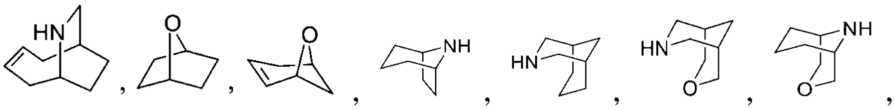

cubane and adamantane. The "bridged ring" or "bridged ring group" can be monovalent, divalent, trivalent or tetravalent.

"Carbospiro ring", "spiro ring carbocyclyl", "spirocarbocyclyl" or "carbospiro ring group" refers to a "spiro ring" with a ring system consisting only of carbon atoms.

"Carbo-fused ring", "fused ring carbocyclyl", "fused carbocyclyl" or "carbo-fused ring group" refers to a "fused ring" with a ring system consisting only of carbon atoms.

"Carbo-bridged ring", "bridged ring carbocyclyl", "bridged carbocyclyl" or "carbo-bridged ring group" refers to a "bridged ring" with a ring system consisting only of carbon atoms.

"Mono-heterocyclic ring", "monocyclic heterocyclyl" or "mono-heterocyclyl" refers to "heterocyclyl" or "heterocyclic ring" with a monocyclic system.

"Fused heterocyclic ring", "fused heterocyclic ring group", "fused heterocyclyl" or "fused heterocyclic ring group" refers to a "fused ring" containing a heteroatom.

"Spiro-heterocyclic ring", "spiro-heterocyclic ring group", "spiroheterocyclyl" or "spiro-heterocyclic ring group" refers to a "spiro ring" containing a heteroatom.

"Bridged heterocyclic ring", "bridged heterocyclic ring group", "bridged heterocyclyl" or "bridged heterocyclic ring group" refers to a "bridged ring" containing a heteroatom.

"Aryl" or "aromatic ring" refers to a substituted or unsubstituted aromatic hydrocarbon group with a monocyclic ring or a fused ring, wherein the number of ring atoms in the aromatic ring includes, but is not limited to 6 to 18, 6 to 12, or 6 to 10 carbon atoms. The aryl ring can be fused to a saturated or unsaturated carbocyclic ring or heterocyclic ring, wherein the ring connected to the parent structure is the aryl ring. Non-limiting examples include a benzene ring, a naphthalene ring, or

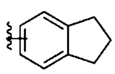, 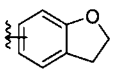, 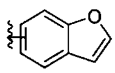, 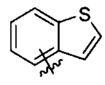, 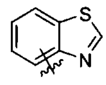,

and the "aryl" or "aromatic ring" can be monovalent, divalent, trivalent or tetravalent. When divalent, trivalent or tetravalent, the point of connection is on the aryl ring.

"Heteroaryl" or "heteroaromatic ring" refers to a substituted or unsubstituted aromatic hydrocarbon group containing 1 to 5 heteroatoms or groups containing heteroatoms (including but not limited to N, O or S(=O)n, wherein n is 0, 1 or 2), wherein the number of ring atoms in the heteroaromatic ring includes, but is not limited to, 5 to 15, 5 to 10, or 5 to 6. Non-limiting examples of the heteroaryl include, but are not limited to pyridyl, furyl, thienyl, pyridyl, pyranyl, N-alkylpyrrolyl, pyrimidyl, pyrazinyl, pyridazinyl, imidazolyl, benzopyrazole, benzoimidazole, benzopyridine, pyrrolopyridine, etc. The heteroaryl ring can be fused to a saturated or unsaturated carbocyclic ring or heterocyclic ring, wherein the ring connected to the parent structure is the heteroaryl ring. Non-limiting examples include

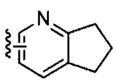, 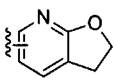, 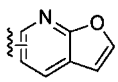, 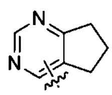, 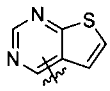, 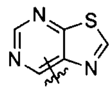, 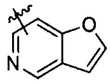

and

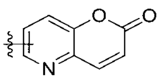.

The definition of the heteroaryl herein is consistent with this definition. The heteroaryl can be monovalent, divalent, trivalent or tetravalent. When divalent, trivalent or tetravalent, the point of connection is on the heteroaryl ring.

A "5-membered ring-fused-5-membered heteroaromatic ring" refers to a 5,5-fused heteroaromatic ring, wherein at least one of the two fused rings contains at least one heteroatom (including but not limited to O, S or N), and the entire group is aromatic. Non-limiting examples include a pyrrolopyrrole ring, a pyrazolopyrrole ring, a pyrazolopyrazole ring, a pyrrolofuran ring, a pyrazolofuran ring, a pyrrolothiophene ring and a pyrazolothiophene ring.

A "5-membered ring-fused-6-membered heteroaromatic ring" refers to a 5,6-fused heteroaromatic ring, wherein at least one of the two fused rings contains at least one heteroatom (including but not limited to O, S or N), and the entire group is aromatic. Non-limiting examples include benzofused 5-membered heteroaryl and a 6-membered heteroaromatic ring-fused-5-membered heteroaromatic ring.

"Substitution" or "substituted" refers to a substitution with 1 or more (including, but not limited to 2, 3, 4 or 5) substituents including, but not limited to H, F, Cl, Br, I, alkyl, cycloalkyl, alkoxy, haloalkyl, mercaptan, hydroxyl, nitro, mercapto, amino, cyano, isocyano, aryl, heteroaryl, heterocyclyl, bridged ring group, spiro ring group, fused ring group, hydroxyalkyl, =O, carbonyl, aldehyde, carboxylic acid, carboxylate, $-(CH_2)_m-C(=O)-R^a$, $-O-(CH_2)_m-C(=O)-R^a$, $-(CH2)_m-C(=O)-NR^bR^c$, $-(CH_2)_mS(=O)_nR^a$, $-(CH_2)_m$-alkenyl-$R^a$, $OR^d$ or $-(CH_2)_m$-alkynyl-$R^a$ (wherein m and n are 0, 1 or 2), arylthio, thiocarbonyl, silyl, $-NR^bR^c$, etc., wherein $R^b$ and $R^c$ are independently selected from H, hydroxyl, amino, carbonyl, alkyl, alkoxy, cycloalkyl, heterocyclyl, aryl, heteroaryl, sulphonyl, or trifluoromethylsulphonyl. Alternatively, $R^b$ and $R^c$ can form a five- or six-membered cycloalkyl or heterocyclyl. $R^a$ and $R^d$ are each independently selected from aryl, heteroaryl, alkyl, alkoxy, cycloalkyl, heterocyclyl, carbonyl, ester group, bridged ring group, spiro ring group or bicyclic ring group.

"Containing 1 to 5 heteroatoms selected from O, S and N" means containing 1, 2, 3, 4 or 5 heteroatoms selected from O, S and N.

"Substituted with 1 to X substituents selected from ..." refers to a substitution with 1, 2, 3 ... X substituents, wherein X is any integer from 1 to 10. For example, "substituted with 1 to 4 $R^k$" refers to a substitution with 1, 2, 3 or 4 $R^k$. For example, "substituted with 1 to 5 substituents selected from ..." refers to a substitution with 1, 2, 3, 4 or 5 substituents selected from ... For example, "bridged heterocyclic ring is optionally substituted with 1 to 4 substituents selected from D or F" means that the bridged heterocyclic ring is optionally substituted with 1, 2, 3 or 4 substituents selected from D or F.

An X- to Y-membered ring ($3 \leq X < Y$, and Y is selected from any integer between 4 and 12) includes X, X+1-, X+2-, X+3-, X+4-,...to Y-membered rings. Rings include a heterocyclic ring, a carbocyclic ring, an aromatic ring, aryl, heteroaryl, cycloalkyl, a mono-heterocyclic ring, a fused heterocyclic ring, a spiro-heterocyclic ring, or a bridged heterocyclic ring. For example, a "4- to 7-membered mono-heterocyclic ring" refers to a 4-, 5-, 6- or 7-membered mono-heterocyclic ring, and a "5- to 10-membered fused heterocyclic ring" refers to a 5-, 6-, 7-, 8-, 9- or 10-membered fused heterocyclic ring.

The term "optional" or "optionally" means that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutically acceptable salt" or "pharmaceutically acceptable salt thereof" refers to a salt of the compound of the present invention, which salt maintains the biological effectiveness and characteristics of a free acid or a free base, and is obtained by reacting the free acid with a non-toxic inorganic base or organic base, or reacting the free base with a non-toxic inorganic acid or organic acid.

"Pharmaceutical composition" refers to a mixture of one or more compounds of the present invention, or stereoisomers, tautomers, deuterated compounds, solvates, prodrugs, metabolites, pharmaceutically acceptable salts or cocrystals thereof and other chemical components, wherein "other chemical components" refer to pharmaceutically acceptable carriers, excipients and/or one or more other therapeutic agents.

"Carrier" refers to a material that does not cause significant irritation to an organism and does not eliminate the biological activity and characteristics of a compound administered.

"Prodrug" refers to a compound that can be converted into the compound of the present invention with the biological activity by metabolism *in vivo.* The prodrug of the present invention is prepared by modifying an amino or carboxyl group in the compound of the present invention, and the modification can be removed by conventional operations or removed in vivo to obtain a parent compound. When the prodrug of the present invention is administered to a mammalian individual, the prodrug is split to form a free amino or carboxyl group.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

"Animal" is meant to include mammals, such as humans, companion animals, zoo animals, and domestic animals, preferably humans, horses, or dogs.

"Stereoisomer" refers to an isomer produced as a result of different spatial arrangement of atoms in molecules, including cis-trans isomers, enantiomers, diastereomers and conformational isomers.

"Tautomer" refers to a functional group isomer produced by the rapid movement of an atom in two positions in a molecule, such as keto-enol isomerisation and amide-imino alcohol isomerisation.

Detailed Description of Embodiments

The technical solutions of the present invention will be described in detail by the following examples, but the scope of protection of the present invention includes but is not limited thereto.

The structures of the compounds are determined by nuclear magnetic resonance (NMR) or (and) mass spectrometry (MS). The NMR shift ($\delta$) is given in the unit of $10^{-6}$ (ppm). NMR is determined with NMR instruments (Bruker Avance III 400 and Bruker Avance 300); the solvent for determination is deuterated dimethylsulphoxide (DMSO-$d_6$), deuterated chloroform ($CDCl_3$) or deuterated methanol ($CD_3OD$); and the internal standard is tetramethylsilane (TMS).

MS is determined with Agilent 6120B (ESI) and Agilent 6120B (APCI); and

HPLC is determined with Agilent 1260DAD high pressure liquid chromatograph (Zorbax SB-C18 100 $\times$ 4.6 mm, 3.5 $\mu$M).

For thin layer chromatography (TLC), silica gel plates from Yantai Huanghai $HSGF_{254}$ or Qingdao $GF_{254}$ are used. Silica gel plates with a thickness of 0.15 mm-0.20 mm are employed for TLC, while plates with a thickness of 0.4 mm-0.5 mm are used for TLC separation and purification.

For column chromatography, Yantai Huanghai silica gel of 200-300 mesh is typically used as a carrier.

Boc: tert-butoxycarbonyl; Ts: p-toluenesulphonyl; Cbz: benzyloxycarbonyl; TMS: trimethylsilane.

**Synthesis of intermediate 1:**

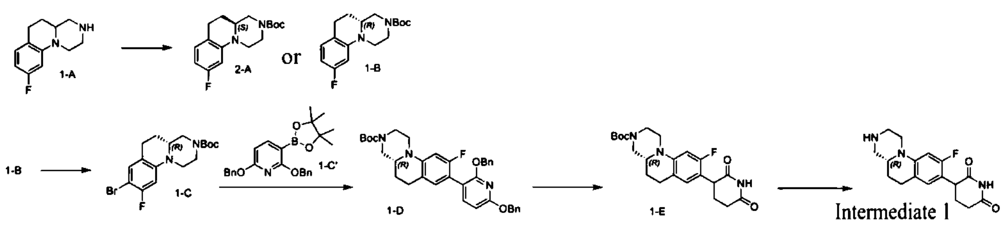

1-A, 2-A or 1-B

1-B, 1-C, 1-C', 1-D, 1-E, Intermediate 1

Step 1: Preparation of 1-B

1-A (2.8 g, 13.58 mmol) (see Bioorganic & Medicinal Chemistry Letters, 2016, 26, 5877-5882 for the synthetic method) was dissolved in dichloromethane (50 mL), $Boc_2O$ (5.93 g, 27.17 mmol) and DMAP (3.32 g, 27.18 mmol) were added, and the mixture was reacted at room temperature for 16 h. The reaction system was washed with 0.5 mol/L hydrochloric acid (50 mL), dried over anhydrous sodium sulphate, and concentrated under reduced pressure. The crude product was separated and purified by silica gel column chromatography (ethyl acetate : petroleum ether (v/v) = 0 : 1-1 : 9) to obtain the racemate of 1-B (3.4 g, yield: 82%).

The racemate of 1-B was subjected to chiral resolution, and the chiral resolution method was as follows:

1. Instrument: SFC Prep 150 AP; chromatography column: Daicel IC-H (19 mm × 250 mm).
2. The sample was dissolved in methanol and filtered through a 0.45 μm filter head to prepare a sample solution.
3. Preparative chromatography conditions: a. the mobile phase consists of systems A and B: mobile phase A: $CO_2$; mobile phase B: methanol/isopropanol (v/v) = 1 : 1; b. isocratic elution, mobile phase B: 20%; c. flow rate: 40 mL/min.

Retention time: chiral isomer 1 (compound 1-B): 5.7 min, chiral isomer 2 (compound 2-A): 6.47 min.

According to the MicroED structure determination of compound 2-B, compound 1-B is in the R configuration, and compound 2-A is in the S configuration.

LCMS m/z = 307.3 $[M+1]^+$.

Step 2: Preparation of 1-C

1-B (1.8 g, 5.88 mmol) was dissolved in acetonitrile (50 mL), NBS (1.05 g, 5.90 mmol) was added, and the mixture was reacted at room temperature for 1 h. The reaction system was concentrated under reduced pressure, and the crude product was separated and purified by silica gel column chromatography (ethyl acetate : petroleum ether (v/v) = 0 : 1-1 : 9) to obtain 1-C (1.6 g, yield: 71%).

LCMS m/z = 385.3 $[M+1]^+$.

Step 3: Preparation of 1-D

1-C (0.77 g, 2.0 mmol), 1-C' (1.67 g, 4.0 mmol) (see WO 2022235945 for the synthetic method), Pd(dppf)$Cl_2$·DCM (0.16 g, 0.20 mmol) and caesium carbonate (1.30 g, 4.0 mmol) were added to a reaction flask, followed by 1,4-dioxane (30 mL) and water (3 mL), and the mixture was reacted at 100°C under nitrogen atmosphere for 20 h. The reaction system was cooled to room temperature, and water (50 mL) and ethyl acetate (50 mL) were added. The aqueous phase was extracted with ethyl acetate (50 mL), and the organic phase was washed with a saturated aqueous sodium chloride solution (30 mL), dried over anhydrous sodium sulphate, and concentrated under reduced pressure. The crude product was separated and purified by silica gel column chromatography (petroleum ether/ethyl acetate (v/v) = 1 : 0-9 : 1) to obtain 1-D (0.70 g, yield: 59%).

Step 4: Preparation of 1-E

1-D (0.70 g, 1.18 mmol) was dissolved in THF (20 mL), and 10% palladium on carbon (0.63 g) was added. The mixture was reacted at 45°C under hydrogen balloon atmosphere for 20 h. The reaction system was cooled to room temperature, and filtered by suction. The filtrate was concentrated under reduced pressure, and the crude product was

separated and purified by silica gel column chromatography (MeOH : DCM (v/v) = 0 : 1-5 : 95) to obtain 1-E (0.25 g, yield: 51%).

LCMS m/z = 418.1 $[M+1]^+$.

Step 5: Preparation of intermediate 1

1-E (250 mg, 0.6 mmol) was dissolved in dichloromethane (2 mL), trifluoroacetic acid (2 mL) was added, and the mixture was reacted at room temperature for 3 h. The reaction system was concentrated under reduced pressure. Dichloromethane (5 mL) and triethylamine (1 mL) were added, and the mixture was concentrated under reduced pressure to obtain crude intermediate 1 (190 mg).

LCMS m/z = 318.3 $[M+1]^+$.

Synthesis of intermediate 2:

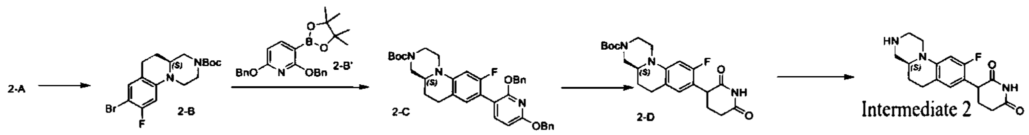


Step 1: Preparation of 2-B

2-A (1.4 g, 4.57 mmol) was dissolved in acetonitrile (50 mL), NBS (0.81 g, 4.55 mmol) was added, and the mixture was reacted at room temperature for 1 h. The reaction system was concentrated under reduced pressure, and the crude product was separated and purified by silica gel column chromatography (ethyl acetate : petroleum ether (v/v) = 0 : 1-1 : 9) to obtain 2-B (1.6 g, yield: 92%).

LCMS m/z = 385.3 $[M+1]^+$.

According to the MicroED structure determination, compound 2-B is in the S configuration.

Step 2: Preparation of 2-C

2-B (1.6 g, 4.16 mmol), 2-B' (3.46 g, 8.29 mmol), $Pd(dppf)Cl_2 \cdot DCM$ (0.34 g, 0.42 mmol) and caesium carbonate (2.70 g, 8.3 mmol) were added to a reaction flask, followed by 1,4-dioxane (50 mL) and water (5 mL), and the mixture was reacted at 100°C under nitrogen atmosphere for 20 h. The reaction system was cooled to room temperature, and water (50 mL) and ethyl acetate (50 mL) were added. The aqueous phase was extracted with ethyl acetate (50 mL), and the organic phase was washed with a saturated aqueous sodium chloride solution (30 mL), dried over anhydrous sodium sulphate, and concentrated under reduced pressure. The crude product was separated and purified by silica gel column chromatography (petroleum ether/ethyl acetate (v/v) = 1 : 0-9 : 1) to obtain 2-C (1.4 g, yield: 56%).

Step 3: Preparation of 2-D

2-C (1.4 g, 2.35 mmol) was dissolved in THF (50 mL), and 10% palladium on carbon (1.25 g) was added. The mixture was reacted at 45°C under hydrogen balloon atmosphere for 20 h. The reaction system was cooled to room temperature, and filtered by suction. The filtrate was concentrated under reduced pressure, and the crude product was separated and purified by silica gel column chromatography (MeOH : DCM (v/v) = 0 : 1-5 : 95) to obtain 2-D (0.85 g, yield: 87%).

LCMS m/z = 418.1 $[M+1]^+$.

Step 4: Preparation of intermediate 2

2-D (620 mg, 1.49 mmol) was dissolved in dichloromethane (5 mL), trifluoroacetic acid (3 mL) was added, and the mixture was reacted at room temperature for 3 h. The reaction system was concentrated under reduced pressure. Dichloromethane (5 mL) and triethylamine (1 mL) were added, and the mixture was concentrated under reduced pressure to obtain crude intermediate 2 (460 mg).

LCMS m/z = 318.3 $[M+1]^+$.

**Preparation of intermediate 3:**

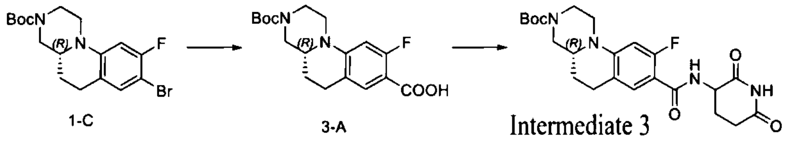

Step 1: Preparation of 3-A

Under nitrogen atmosphere, 1-C (7.00 g, 18.17 mmol) and tetrahydrofuran (70 mL) were respectively added to a reaction flask, followed by slowly dropwise addition of 2.5 mol/L solution of n-butyllithium in n-hexane (14.50 mL, 36.25 mmol) at -78°C, and the mixture was stirred for another 1.5 h at -78°C. Subsequently, the system was purged three times with carbon dioxide, and reacted for 0.5 h under carbon dioxide balloon atmosphere with the system temperature maintained below -40°C. The reaction system was allowed to warm to room temperature, and ethyl acetate (20 mL) was added. The mixture was adjusted to pH 2 with 1 mol/L hydrochloric acid, and extracted with ethyl acetate (30 mL×3). The organic phase was dried over anhydrous sodium sulphate, and concentrated under reduced pressure, and the crude product was separated and purified by silica gel column chromatography (petroleum ether/ethyl acetate (v/v) = 10 : 1-2 : 1) to obtain 3-A (2.4 g, yield: 38%).

$^1$H NMR (400 MHz, DMSO-$d_6$) δ 7.42 (d, 1H), 6.64 (d, 1H), 4.00 - 3.82 (m, 3H), 3.23 - 3.09 (m, 1H), 3.08 - 2.90 (m, 1H), 2.90 - 2.77 (m, 1H), 2.76 - 2.60 (m, 3H), 2.05 - 1.90 (m, 1H), 1.65 - 1.50 (m, 1H), 1.49 - 1.35 (m, 9H).

Step 2: Preparation of intermediate 3

To a reaction flask were respectively added 3-A (2.4 g, 6.85 mmol), HATU (3.13 g, 8.23 mmol), diisopropylethylamine (2.66 g, 20.58 mmol) and DMF (10 mL), and the mixture was stirred at room temperature for 10 min. Subsequently, 3-amino-2,6-piperidinedione hydrochloride (1.35 g, 8.20 mmol) was added, and the resulting mixture was reacted at room temperature for 30 min. The reaction solution was poured into water (100 mL), and filtered. The filter cake was washed with water (50 mL), and dried under reduced pressure to obtain intermediate 3 (2.0 g, yield: 63%).

LCMS m/z = 461.2 $[M+1]^+$

**Preparation of intermediate 4:**

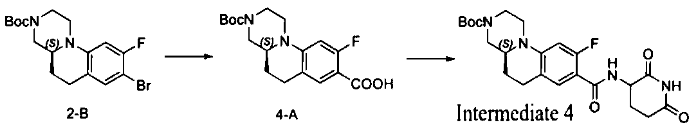

Starting from compound 2-B and following the synthetic method of intermediate 3, intermediate 4 was obtained.

LCMS m/z = 461.2 $[M+1]^+$

**Preparation of intermediate 5:**

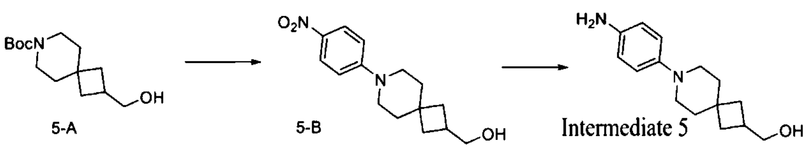

Step 1: Preparation of 5-B

5-A (2.2 g, 8.6 mmol) was added to a single-necked flask, followed by 1,4-dioxane (20 mL) and 4 mol/L solution of

hydrogen chloride in 1,4-dioxane (20 mL), and the mixture was reacted at room temperature for 2 h. The reaction system was concentrated under reduced pressure, p-fluoronitrobenzene (1.213 g, 8.6 mmol), N,N-dimethylformamide (30 mL) and potassium carbonate (4.7 g, 34.0 mmol) were added, and the mixture was reacted at 80°C for 16 h. The reaction system was cooled to room temperature, and poured into water (300 mL), with the solids precipitated out. The mixture was filtered, and the filter cake was washed with water (50 mL), and dried under reduced pressure to obtain crude 5-B (2 g).

LCMS m/z = 277.1 $[M+1]^+$

Step 2: Preparation of intermediate 5

The above-mentioned crude 5-B (2 g) was dissolved in methanol (50 mL), 5% palladium on carbon (100 mg) was added, and the mixture was reacted at room temperature under hydrogen balloon atmosphere for 4 h. The reaction system was filtered through a pad of diatomaceous earth, the filter cake was washed with dichloromethane (50 mL), and the filtrate was concentrated under reduced pressure to obtain crude intermediate 5 (1.7 g).

LCMS m/z = 247.1 $[M+1]^+$

**Preparation of intermediate 6:**

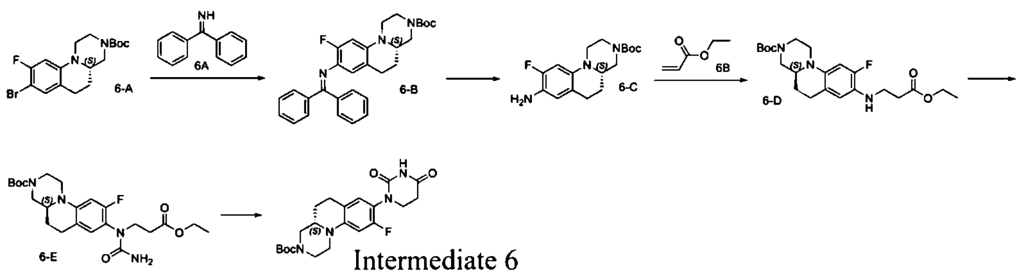


Step 1: Preparation of 6-B

6-A (6.29 g, 16.33 mmol) (see WO 2023232133 for the synthetic method), 6A (4.14 g, 22.84 mmol), caesium carbonate (10.63 g, 32.63 mmol), palladium acetate (0.73 g, 3.25 mmol), and XantPhos (0.94 g, 1.62 mmol) were added to a 1,4-dioxane solution (100 mL), and the mixture was reacted at 105°C under nitrogen atmosphere for 16 h. The reaction solution was cooled to room temperature, and filtered through a pad of diatomaceous earth. The filter cake was washed with dichloromethane (50 mL), and the filtrate was concentrated under reduced pressure. The crude product was separated and purified by silica gel column chromatography (ethyl acetate/petroleum ether (v/v) = 1 : 5) to obtain 6-B (6.94 g, yield: 88%).

LCMS m/z = 486.2 $[M+1]^+$

Step 2: Preparation of 6-C

6-B (6.94 g, 14.30 mmol) was added to methanol (200 mL), followed by 10% palladium on carbon (6.92 g) and ammonium acetate (6.79 g, 88.09 mmol), and the mixture was reacted at room temperature under hydrogen balloon atmosphere for 12 h. The reaction solution was filtered through a pad of diatomaceous earth, the filtrate was concentrated under reduced pressure, and the crude product was separated and purified by silica gel column chromatography (ethyl acetate/petroleum ether (v/v) = 1 : 1) to obtain 6-C (4.15 g, yield: 90%).

LCMS m/z = 322.2 $[M+1]^+$

Step 3: Preparation of 6-D

6-C (4.15 g, 12.92 mmol), 6B (3.88 g, 38.75 mmol) and N,N-diisopropylethylamine (5.01 g, 38.76 mmol) were successively added to ethanol (60 mL), and the mixture was reacted at 100°C for 72 h. The reaction system was cooled to room temperature, and concentrated under reduced pressure, and the crude product was separated and purified by silica gel column chromatography (methanol/dichloromethane (v/v) = 1 : 20) to obtain 6-D (3.91 g, yield: 72%).

LCMS m/z = 422.3 $[M+1]^+$

Step 4: Preparation of 6-E

6-D (0.50 g, 1.19 mmol) and N,N-diisopropylethylamine (0.46 g, 3.56 mmol) were added to tetrahydrofuran (20 mL), and then triphosgene (0.39 g, 1.31 mmol) was slowly added, and the mixture was reacted at room temperature for 1 h. Aqueous ammonia (5 mL) was added, and the resulting mixture was reacted at 50°C for 2 h. The reaction system was cooled to room temperature, and ethyl acetate (100 mL) was added. The organic phase was washed with water (50 mL×3) and a saturated aqueous sodium chloride solution (50 mL), dried over anhydrous sodium sulphate, and concentrated under reduced pressure. The crude product was separated and purified by silica gel column chromatography (methanol/dichloromethane (v/v) = 1 : 15) to obtain 6-E (0.46 g, yield: 83%).

LCMS m/z = 465.3 $[M+1]^+$

Step 5: Preparation of intermediate 6

6-E (0.46 g, 0.99 mmol) was added to acetonitrile (10 mL), a 40% solution of benzyltrimethylammonium hydroxide in methanol (1.2 mL) was added, and the mixture was reacted at 60°C for 2 h. The reaction system was cooled to room temperature, and concentrated under reduced pressure, and the crude product was separated and purified by silica gel column chromatography (methanol/dichloromethane (v/v) = 1 : 15) to obtain intermediate 6 (0.21 g, yield: 51%).

**Preparation of intermediate 7:**

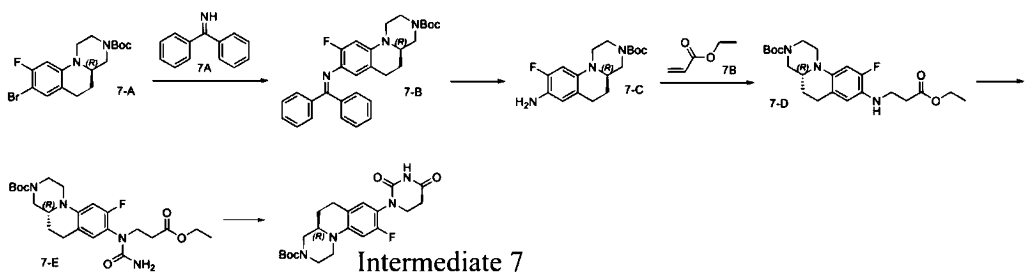


Starting from compound 7-A (see WO 2023232133 for the synthetic method) and following the synthetic method of intermediate 6, intermediate 7 was obtained.

**Preparation of intermediate 8:**

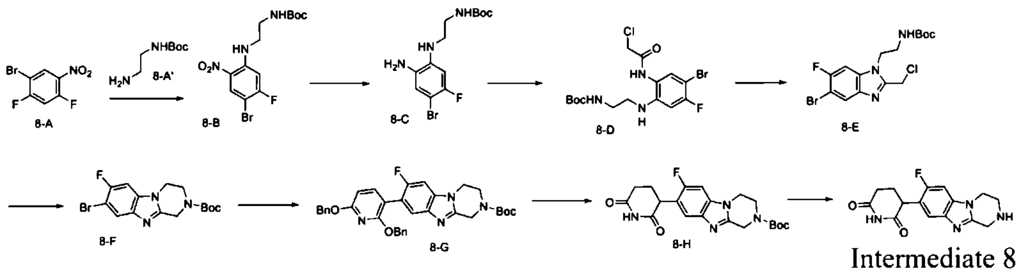


Step 1: Preparation of 8-B

8-A (10 g, 42.02 mmol) was dissolved in DMSO (80 mL), 8-A' (7.41 g, 46.25 mmol) and DIPEA (16.29 g, 126.03 mmol) were added, and the mixture was reacted at 50°C for 3 h. The reaction system was cooled to room temperature, and water (300 mL) and ethyl acetate (400 mL) were added. The organic phase was washed with a saturated aqueous sodium chloride solution (300 mL), dried over anhydrous sodium sulphate, and concentrated under reduced pressure to obtain crude 8-B (6.2 g).

Step 2: Preparation of 8-C

The above-mentioned crude 8-B (6.2 g) was added to methanol (150 mL) and water (30 mL), followed by iron powder (9.15 g, 163.4 mmol) and ammonium chloride (8.77 g, 163.96 mmol), and the mixture was reacted at 70°C for 20 h. The reaction system was cooled to room temperature, and filtered through a pad of diatomaceous earth. The filtrate was concentrated under reduced pressure, and water (300 mL) and ethyl acetate (400 mL) were added to the residue. The organic phase was washed with a saturated aqueous sodium chloride solution (300 mL), dried over anhydrous sodium sulphate, and concentrated under reduced pressure. The crude product was separated and purified by silica gel column chromatography (methanol : dichloromethane (v/v) = 0 : 1-1 : 9) to obtain 8-C (1.9 g, two-step yield from compound 8-A: 13%).

Step 3: Preparation of 8-D

8-C (1.9 g, 5.46 mmol) and TEA (1.66 g, 16.40 mmol) were dissolved in dichloromethane (20 mL), chloroacetyl chloride (0.99 g, 8.77 mmol) was added dropwise, and the mixture was reacted at room temperature for 20 h. To the reaction solution was added water (50 mL). The organic phase was dried over anhydrous sodium sulphate, and concentrated under reduced pressure to obtain crude 8-D (2.1 g).

Step 4: Preparation of 8-E

The above-mentioned crude 8-D (0.95 g) was added to acetic acid (15 mL), and the mixture was reacted at 50°C for 20 h. The reaction system was cooled to 0°C, adjusted to pH 8 with 25% aqueous ammonia, and extracted with ethyl acetate (150 mL). The organic phase was dried over anhydrous sodium sulphate, and concentrated under reduced pressure. The crude product was separated and purified by silica gel column chromatography (methanol : dichloromethane (v/v) = 0 : 1-1 : 9) to obtain 8-E (0.85 g, two-step yield from compound 8-C: 85%).

Step 5: Preparation of 8-F

8-E (7.3 g, 17.95 mmol) was dissolved in THF (80 mL), 1 mol/L solution of potassium tert-butoxide in tetrahydrofuran (20 mL) was added, and the mixture was reacted at room temperature for 19 h. To the reaction system were added water (30 mL) and ethyl acetate (50 mL). The organic phase was washed with a saturated aqueous sodium chloride solution (300 mL), dried over anhydrous sodium sulphate, and concentrated under reduced pressure. The crude product was separated and purified by silica gel column chromatography (methanol : dichloromethane (v/v) = 0 : 1-1 : 9) to obtain 8-F (3.6 g, yield: 54%).

Step 6: Preparation of 8-G

8-F (2.0 g, 5.4 mmol), 2,6-bisbenzyloxypyridine-3-boronic acid pinacol ester (3.4 g, 8.15 mmol), Pd(dppf)$Cl_2$·DCM (0.44 g, 0.54 mmol) and caesium carbonate (3.5 g, 10.74 mmol) were successively added to a reaction flask, followed by 1,4-dioxane (100 mL) and water (10 mL), and the mixture was reacted at 100°C under nitrogen atmosphere for 20 h. The reaction system was cooled to room temperature, and water (300 mL) and ethyl acetate (300 mL) were added. The organic phase was washed with a saturated aqueous sodium chloride solution (100 mL), dried over anhydrous sodium sulphate, and concentrated under reduced pressure. The crude product was separated and purified by silica gel column chromatography (petroleum ether/ethyl acetate (v/v) = 1 : 0-9 : 1) to obtain 8-G (2.6 g, yield: 83%).

LCMS m/z = 581.4 $[M+1]^+$.

Step 7: Preparation of 8-H

8-G (2.6 g, 4.48 mmol) was dissolved in THF (10 mL) and methanol (30 mL), 10% palladium on carbon (2.5 g) was added, and the mixture was reacted at 40°C under hydrogen balloon atmosphere for 20 h. The reaction system was cooled to room temperature, and filtered. The filtrate was concentrated under reduced pressure to obtain crude 8-H (0.98 g).

LCMS m/z = 403.3 $[M+1]^+$.

Step 8: Preparation of intermediate 8

The above-mentioned crude 8-H (100 mg) was dissolved in dichloromethane (2 mL), trifluoroacetic acid (1 mL) was added, and the mixture was reacted at room temperature for 3 h. The reaction system was concentrated under reduced pressure. Methanol (5 mL) and sodium bicarbonate (solid, 150 mg) were added, and the mixture was stirred for 10

min. Subsequently, the system was filtered by suction, and the filtrate was concentrated under reduced pressure to obtain crude intermediate 8 (80 mg).

**Example 1: Preparation of compound 1**

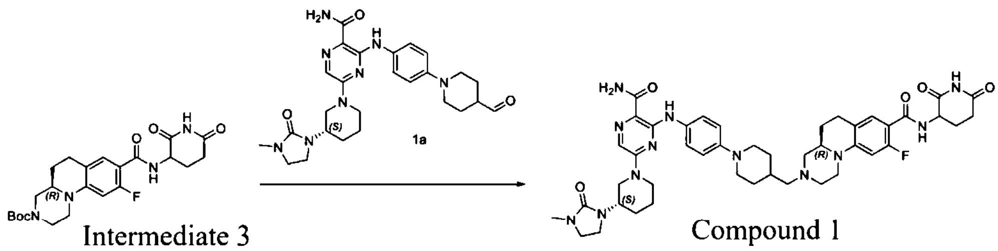

Intermediate 3 (45 mg, 0.098 mmol) was added to a single-necked flask, followed by dichloromethane (10 mL) and 4 mol/L solution of hydrogen chloride in 1,4-dioxane (10 mL), and the mixture was reacted at room temperature for 2 h. The reaction system was concentrated under reduced pressure, dichloromethane (15 mL) and sodium bicarbonate (25 mg, 0.3 mmol) were added, and the mixture was stirred at room temperature for 30 min. 1a (50 mg, 0.099 mmol) (see WO 2023088406 for the synthetic method), anhydrous sodium sulphate (200 mg) and acetic acid (0.02 mL) were added, and the mixture was reacted at room temperature for 16 h. Sodium triacetoxyborohydride (42 mg, 0.198 mmol) was then added, and the resulting mixture was reacted at room temperature for 2 h. To the reaction system was added water (120 mL), and the mixture was extracted with dichloromethane (50 mL×3). The organic phase was washed with a saturated aqueous sodium chloride solution (50 mL×2), dried over anhydrous sodium sulphate, and concentrated under reduced pressure. The crude product was separated and purified by prep-TLC (dichloromethane : methanol (v/v) = 93 : 7) to obtain compound 1 (36 mg, yield: 43%).

$^1$H NMR (400 MHz, $CDCl_3$) δ 10.63 (s, 1H), 7.97 (s, 1H), 7.66 (d, 1H), 7.56 - 7.32 (m, 5H), 7.05 - 6.80 (m, 2H), 6.41 (d, 1H), 5.22 (s, 1H), 4.85 - 4.70 (m, 1H), 4.40 - 4.22 (m, 2H), 3.86 - 3.74 (m, 1H), 3.73 - 3.51 (m, 3H), 3.44 - 3.15 (m, 5H), 3.15 - 2.60 (m, 16H), 2.40 - 2.05 (m, 3H), 2.04 - 1.55 (m, 12H).

LCMS m/z = 851.4 $[M+1]^+$

**Example 3: Preparation of compound 3**

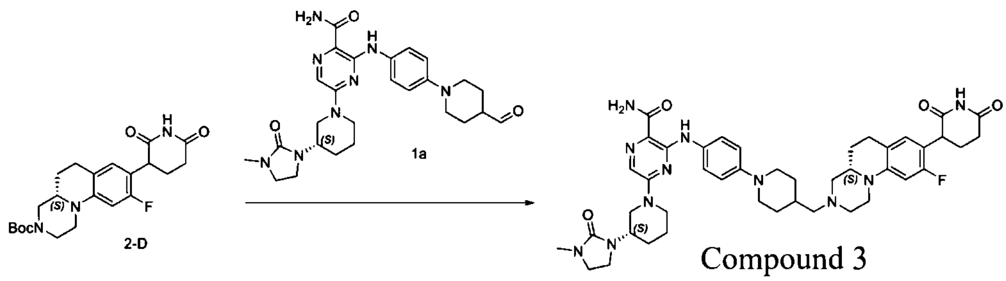

2-D (41 mg, 0.098 mmol) was added to a single-necked flask, followed by dichloromethane (10 mL) and 4 mol/L solution of hydrogen chloride in 1,4-dioxane (10 mL), and the mixture was reacted at room temperature for 2 h. The reaction system was concentrated under reduced pressure, dichloromethane (15 mL) and sodium bicarbonate (25 mg, 0.3 mmol) were added, and the mixture was stirred at room temperature for 30 min. 1a (50 mg, 0.099 mmol) (see WO 2023088406 for the synthetic method), anhydrous sodium sulphate (200 mg) and acetic acid (0.02 mL) were added, and the mixture was reacted at room temperature for 16 h. Sodium triacetoxyborohydride (42 mg, 0.198 mmol) was then added, and the resulting mixture was reacted at room temperature for 2 h. To the reaction system was added water (120 mL), and the mixture was extracted with dichloromethane (50 mL×3). The organic phase was washed with a saturated aqueous sodium chloride solution (50 mL×2), dried over anhydrous sodium sulphate, and concentrated under reduced pressure. The crude product was separated and purified by prep-TLC (dichloromethane : methanol (v/v) = 93 : 7) to obtain compound 3 (35 mg, yield: 44%).

$^1$H NMR (400 MHz, $CDCl_3$) δ 10.63 (s, 1H), 7.96 (s, 1H), 7.56 - 7.32 (m, 4H), 7.00 - 6.85 (m, 2H), 6.71 (d, 1H), 6.49

(d, 1H), 5.16 (s, 1H), 4.40 - 4.22 (m, 2H), 3.88 - 3.70 (m, 2H), 3.70 - 3.53 (m, 3H), 3.44 - 3.22 (m, 4H), 3.18 - 3.00 (m, 2H), 3.00 - 2.55 (m, 13H), 2.35 - 2.07 (m, 5H), 2.05 - 1.55 (m, 10H), 1.48 - 1.28 (m, 2H).

LCMS m/z = 808.6 $[M+1]^+$

**Example 5: Preparation of compound 5**

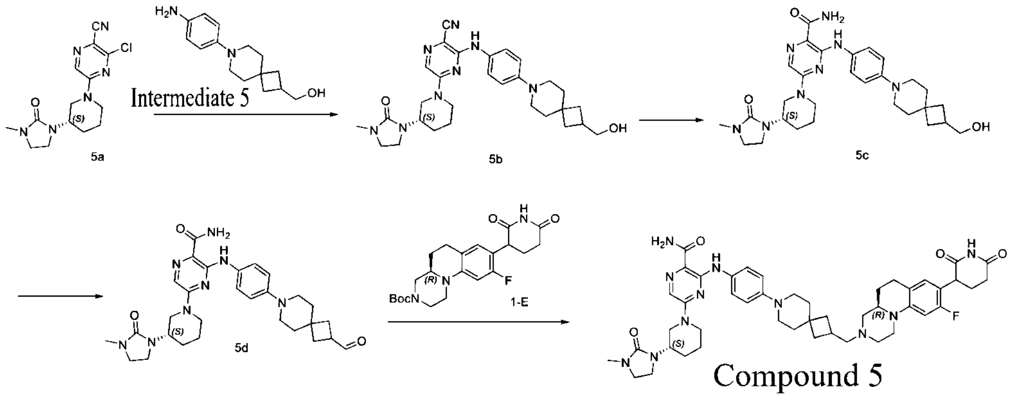


Step 1: Preparation of 5b

5a (1.1 g, 3.43 mmol) (see WO 2023088406 for the synthetic method) was dissolved in 1,4-dioxane (30 mL), the above-mentioned crude intermediate 5 (1.27 g), caesium carbonate (3.35 g, 10.28 mmol), palladium acetate (76 mg, 0.34 mmol) and 2,2'-bis(diphenylphosphino)-1,1'-binaphthyl (321 mg, 0.52 mmol) were added, and the mixture was reacted at 80°C under nitrogen atmosphere for 6 h. The reaction system was cooled to room temperature, and filtered. The filter cake was washed with dichloromethane (150 mL), and the filtrate was concentrated under reduced pressure. The crude product was separated and purified by silica gel column chromatography (dichloromethane : methanol (v/v) = 91 : 9) to obtain 5b (1.2 g, yield: 66%).

LCMS m/z = 531.3 $[M+1]^+$

Step 2: Preparation of 5c

5b (1.2 g, 2.26 mmol) was dissolved in DMSO (15 mL), and then caesium carbonate (735 mg, 2.23 mmol) was added, followed by slowly dropwise addition of 30% hydrogen peroxide (6 mL) at 0°C, and the mixture was stirred at 0°C for 5 min. Subsequently, the resulting system was allowed to warm to room temperature and reacted for 3 h. To the reaction system were added a saturated sodium sulphite solution (50 mL) and ethyl acetate (70 mL). The aqueous phase was extracted with ethyl acetate (50 mL×3), and the organic phase was washed with a saturated aqueous sodium chloride solution (60 mL×2), dried over anhydrous sodium sulphate, and concentrated under reduced pressure. The crude product was separated and purified by silica gel column chromatography (dichloromethane : methanol (v/v) = 91 : 9) to obtain 5c (0.92 g, yield: 74%).

LCMS m/z = 549.3 $[M+1]^+$

Step 3: Preparation of 5d

5c (820 mg, 1.50 mmol) was dissolved in a mixed solvent of dichloromethane (35 mL) and acetonitrile (5 mL), 4 Å molecular sieve (600 mg), N-methylmorpholine oxide (260 mg, 2.22 mmol) and tetrapropylammonium perruthenate (26 mg, 0.074 mmol) were added, and the mixture was reacted at room temperature for 6 h. The reaction system was filtered, the filtrate was concentrated under reduced pressure, and the crude product was separated and purified by silica gel column chromatography (dichloromethane : methanol (v/v) = 92 : 8) to obtain 5d (260 mg, yield: 32%).

LCMS m/z = 547.3 $[M+1]^+$

Step 4: Preparation of compound 5

1-E (41 mg, 0.098 mmol) was added to a single-necked flask, followed by dichloromethane (10 mL) and 4 mol/L solution of hydrogen chloride in 1,4-dioxane (10 mL), and the mixture was reacted at room temperature for 2 h. The

reaction system was concentrated under reduced pressure, dichloromethane (15 mL) and sodium bicarbonate (25 mg, 0.3 mmol) were added, and the mixture was stirred at room temperature for 30 min. 5d (54 mg, 0.099 mmol), anhydrous sodium sulphate (200 mg) and acetic acid (0.02 mL) were added, and the mixture was reacted at room temperature for 16 h. Sodium triacetoxyborohydride (42 mg, 0.198 mmol) was then added, and the resulting mixture was reacted at room temperature for 2 h. To the reaction system was added water (120 mL), and the mixture was extracted with dichloromethane (50 mL×3). The organic phase was washed with a saturated aqueous sodium chloride solution (50 mL×2), dried over anhydrous sodium sulphate, and concentrated under reduced pressure. The crude product was separated and purified by prep-TLC (dichloromethane : methanol (v/v) = 93 : 7) to obtain compound 5 (37 mg, yield: 45%).

$^1H$ NMR (400 MHz, $CDCl_3$) δ 10.61 (s, 1H), 7.95 (s, 1H), 7.56 - 7.32 (m, 4H), 6.98 - 6.85 (m, 2H), 6.71 (d, 1H), 6.48 (d, 1H), 5.14 (s, 1H), 4.38 - 4.22 (m, 2H), 3.87 - 3.70 (m, 2H), 3.68 - 3.52 (m, 1H), 3.43 - 3.22 (m, 4H), 3.20 - 3.05 (m, 4H), 3.05 - 2.70 (m, 10H), 2.70 - 2.43 (m, 5H), 2.35 - 1.45 (m, 19H).

LCMS m/z = 848.6 $[M+1]^+$

**Example 7: Preparation of compound 7**

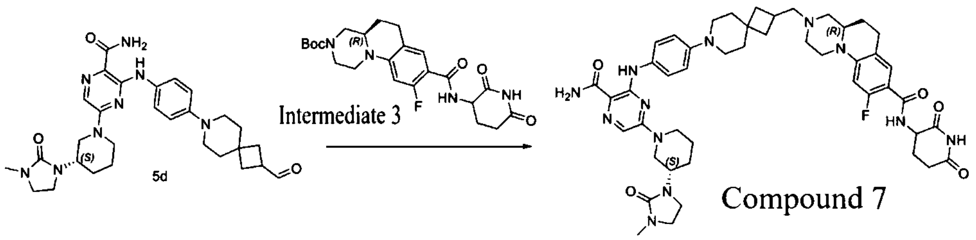


Compound 7

Intermediate 3 (45 mg, 0.098 mmol) was added to a single-necked flask, followed by dichloromethane (10 mL) and 4 mol/L solution of hydrogen chloride in 1,4-dioxane (10 mL), and the mixture was reacted at room temperature for 2 h. The reaction system was concentrated under reduced pressure, dichloromethane (15 mL) and sodium bicarbonate (25 mg, 0.3 mmol) were added, and the mixture was stirred at room temperature for 30 min. 5d (54 mg, 0.099 mmol), anhydrous sodium sulphate (200 mg) and acetic acid (0.02 mL) were added, and the mixture was reacted at room temperature for 16 h. Sodium triacetoxyborohydride (42 mg, 0.198 mmol) was then added, and the resulting mixture was reacted at room temperature for 2 h. To the reaction system was added water (120 mL), and the mixture was extracted with dichloromethane (50 mL×3). The organic phase was washed with a saturated aqueous sodium chloride solution (50 mL×2), dried over anhydrous sodium sulphate, and concentrated under reduced pressure. The crude product was separated and purified by prep-TLC (dichloromethane : methanol (v/v) = 93 : 7) to obtain compound 7 (33 mg, yield: 38%).

$^1H$ NMR (400 MHz, $CDCl_3$) δ 10.61 (s, 1H), 7.91 (s, 1H), 7.66 (d, 1H), 7.53 - 7.30 (m, 5H), 7.00 - 6.85 (m, 2H), 6.40 (d, 1H), 5.14 (s, 1H), 4.84 - 4.70 (m, 1H), 4.38 - 4.20 (m, 2H), 3.88 - 3.60 (m, 2H), 3.45 - 2.38 (m, 25H), 2.30 - 1.45 (m, 17H).

LCMS m/z = 891.5 $[M+1]^+$

Following the synthetic method of other examples, the following compounds were obtained:

| Example No. | Structure | Starting material | LCMS / $^1H$ NMR |
|---|---|---|---|
| Example 2 | 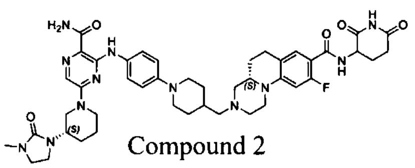 Compound 2 | Intermediate 4+1a (following the synthetic method of example 1) | $^1H$ NMR (400 MHz, $CDCl_3$) δ 10.64 (s, 1H), 7.96 (s, 1H), 7.66 (d, 1H), 7.56 - 7.32 (m, 5H), 7.05 - 6.80 (m, 2H), 6.41 (d, 1H), 5.17 (s, 1H), 4.85 - 4.70 (m, 1H), 4.40 - 4.22 (m, 2H), 3.86 - 3.74 (m, 1H), 3.73 - 3.51 (m, 3H), 3.44 - 3.15 (m, 5H), 3.15 - 2.60 (m, 16H), 2.40 - 2.05 (m, 3H), 2.05 - 1.50 (m, 12H). LCMS m/z = 851.4 $[M+1]^+$ |

(continued)

| Example No. | Structure | Starting material | LCMS / $^1$H NMR |
|---|---|---|---|
| Example 4 | 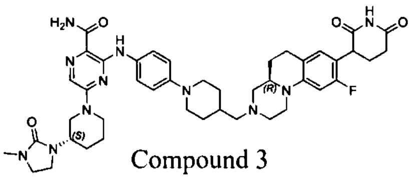 Compound 3 | 1-E+1a (following the synthetic method of example 3) | $^1$H NMR (400 MHz, $CDCl_3$) $\delta$ 10.64 (s, 1H), 7.95 (s, 1H), 7.56 - 7.32 (m, 4H), 7.05 - 6.85 (m, 2H), 6.72 (d, 1H), 6.49 (d, 1H), 5.16 (s, 1H), 4.40 - 4.22 (m, 2H), 3.88 - 3.70 (m, 2H), 3.70 - 3.53 (m, 3H), 3.44 - 3.22 (m, 4H), 3.18 - 3.00 (m, 2H), 3.00 - 2.55 (m, 13H), 2.35 - 2.07 (m, 5H), 2.05 - 1.55 (m, 10H), 1.48 - 1.28 (m, 2H). LCMS m/z = 808.6 [M+1]$^+$ |
| Example 6 | 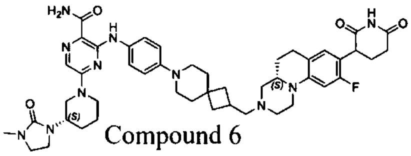 Compound 6 | 2-D+5d (following the synthetic method of example 5) | $^1$H NMR (400 MHz, $CDCl_3$) $\delta$ 10.61 (s, 1H), 8.01 (s, 1H), 7.56 - 7.32 (m, 4H), 6.98 - 6.85 (m, 2H), 6.71 (d, 1H), 6.48 (d, 1H), 5.19 (s, 1H), 4.38 - 4.22 (m, 2H), 3.87 - 3.70 (m, 2H), 3.68 - 3.52 (m, 1H), 3.43 - 3.22 (m, 4H), 3.20 - 3.04 (m, 4H), 3.04 - 2.70 (m, 10H), 2.70 - 2.43 (m, 5H), 2.35 - 1.45 (m, 19H). LCMS m/z = 848.6 [M+1]$^+$ |
| Example 8 | 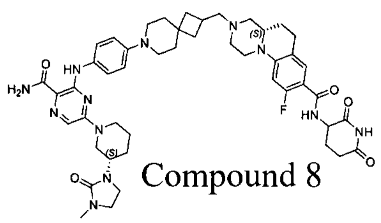 Compound 8 | Intermediate 4+5d (following the synthetic method of example 7) | $^1$H NMR (400 MHz, $CDCl_3$) $\delta$ 10.61 (s, 1H), 7.90 (s, 1H), 7.65 (d, 1H), 7.53 - 7.30 (m, 5H), 7.00 - 6.85 (m, 2H), 6.40 (d, 1H), 5.13 (s, 1H), 4.84 - 4.70 (m, 1H), 4.38 - 4.20 (m, 2H), 3.88 - 3.60 (m, 2H), 3.45 - 2.38 (m, 25H), 2.30 - 1.45 (m, 17H). LCMS m/z = 891.5 [M+1]$^+$ |
| Example 9 | 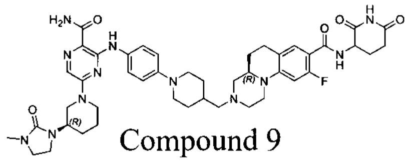 Compound 9 | Intermediate 3+9a (following the synthetic method of example 1) | $^1$H NMR (400 MHz, $CDCl_3$) $\delta$ 10.67 (s, 1H), 7.97 (s, 1H), 7.66 (d, 1H), 7.60 - 7.32 (m, 5H), 7.15 - 6.85 (m, 2H), 6.41 (d, 1H), 5.17 (s, 1H), 4.84 - 4.70 (m, 1H), 4.40 - 4.24 (m, 2H), 3.88 - 3.75 (m, 1H), 3.75 - 3.55 (m, 3H), 3.50 - 3.25 (m, 5H), 3.25 - 2.50 (m, 16H), 2.50 - 2.11 (m, 3H), 2.10 - 1.45 (m, 12H). |
| Example 10 | 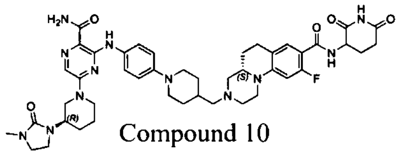 Compound 10 | Intermediate 4+9a (following the synthetic method of example 1) | $^1$H NMR (400 MHz, $CDCl_3$) $\delta$ 10.72 (s, 1H), 7.94 (s, 1H), 7.67 (d, 1H), 7.60 - 7.32 (m, 5H), 7.20 - 6.85 (m, 2H), 6.42 (d, 1H), 5.17 (s, 1H), 4.84 - 4.70 (m, 1H), 4.40 - 4.24 (m, 2H), 3.90 - 3.75 (m, 1H), 3.75 - 3.55 (m, 3H), 3.50 - 3.25 (m, 5H), 3.25 - 2.50 (m, 16H), 2.50 - 2.11 (m, 3H), 2.10 - 1.42 (m, 12H). |
| Example 11 | 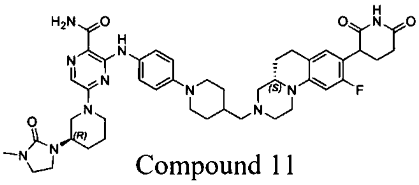 Compound 11 | 2-D+9a (following the synthetic method of example 3) | $^1$H NMR (400 MHz, $CDCl_3$) $\delta$ 10.64 (s, 1H), 8.01 (s, 1H), 7.58 - 7.31 (m, 4H), 7.05 - 6.85 (m, 2H), 6.72 (d, 1H), 6.49 (d, 1H), 5.18 (s, 1H), 4.42 - 4.24 (m, 2H), 3.88 - 3.72 (m, 2H), 3.70 - 3.52 (m, 3H), 3.45 - 3.23 (m, 4H), 3.20 - 3.03 (m, 2H), 3.03 - 2.85 (m, 3H), 2.85 - 2.55 (m, 10H), 2.50 - 2.08 (m, 5H), 2.05 - 1.55 (m, 10H), 1.53 - 1.20 (m, 2H). LCMS m/z = 808.4 [M+1]$^+$ |

(continued)

| Example No. | Structure | Starting material | LCMS / $^1$H NMR |
|---|---|---|---|
| Example 12 | 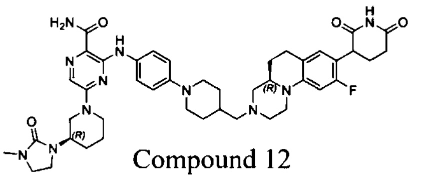 Compound 12 | 1-E +9a (following the synthetic method of example 3) | $^1$H NMR (400 MHz, $CDCl_3$) δ 10.64 (s, 1H), 8.12 (s, 1H), 7.58 - 7.31 (m, 4H), 7.00 - 6.85 (m, 2H), 6.71 (d, 1H), 6.49 (d, 1H), 5.23 (s, 1H), 4.40 - 4.22 (m, 2H), 3.88 - 3.70 (m, 2H), 3.68 - 3.52 (m, 3H), 3.44 - 3.24 (m, 4H), 3.22 - 2.55 (m, 15H), 2.40 - 2.08 (m, 5H), 2.05 - 1.55 (m, 10H), 1.50 - 1.32 (m, 2H).<br>LCMS m/z = 808.4 $[M+1]^+$ |
| Example 14 | 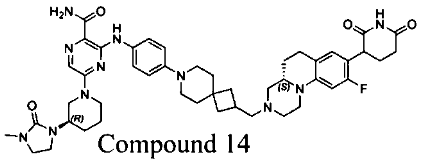 Compound 14 | 2-D+13d (following the synthetic method of example 5) | $^1$H NMR (400 MHz, $CDCl_3$) δ 10.61 (s, 1H), 8.03 (s, 1H), 7.54 - 7.32 (m, 4H), 6.98 - 6.85 (m, 2H), 6.71 (d, 1H), 6.48 (d, 1H), 5.17 (s, 1H), 4.38 - 4.20 (m, 2H), 3.89 - 3.50 (m, 3H), 3.42 - 3.24 (m, 4H), 3.20 - 2.40 (m, 18H), 2.35 - 1.44 (m, 20H).<br>LCMS m/z = 424.8 $[M/2 +1]^+$ |
| Example 15 | 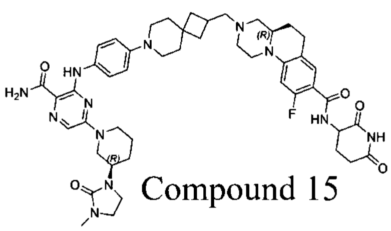 Compound 15 | Intermediate 3+13d (following the synthetic method of example 7) | $^1$H NMR (400 MHz, $CDCl_3$) δ 10.63 (s, 1H), 7.93 (s, 1H), 7.67 (d, 1H), 7.54 - 7.32 (m, 5H), 7.00 - 6.85 (m, 2H), 6.40 (d, 1H), 5.14 (s, 1H), 4.85 - 4.72 (m, 1H), 4.40 - 4.20 (m, 2H), 3.85 - 3.65 (m, 2H), 3.45 - 3.23 (m, 5H), 3.18 - 2.55 (m, 20H), 2.50 - 1.45 (m, 17H). |
| Example 16 | 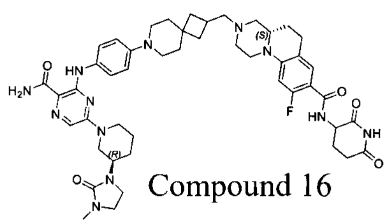 Compound 16 | Intermediate 4+13d (following the synthetic method of example 8) | $^1$H NMR (400 MHz, $CDCl_3$) δ 10.63 (s, 1H), 7.93 (s, 1H), 7.67 (d, 1H), 7.54 - 7.32 (m, 5H), 7.00 - 6.85 (m, 2H), 6.40 (d, 1H), 5.14 (s, 1H), 4.85 - 4.70 (m, 1H), 4.40 - 4.20 (m, 2H), 3.85 - 3.65 (m, 2H), 3.45 - 3.23 (m, 5H), 3.20 - 2.55 (m, 20H), 2.50 - 1.45 (m, 17H). |
| Example 22 | 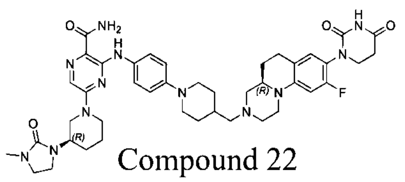 Compound 22 | 9a+ intermediate 7 (following the synthetic method of example 21) | $^1$H NMR (400 MHz, $CDCl_3$) δ 10.58 (s, 1H), 7.58 - 7.25 (m, 5H), 7.00 - 6.70 (m, 3H), 6.47 (d, 1H), 5.10 (s, 1H), 4.38 - 4.15 (m, 2H), 3.80 - 3.46 (m, 6H), 3.38 - 3.17 (m, 4H), 3.15 - 2.95 (m, 2H), 2.95 - 2.52 (m, 12H), 2.35 - 2.00 (m, 2H), 2.00 - 1.25 (m, 14H).<br>LCMS m/z = 809.6 $[M+1]^+$ |

**Example 13: Preparation of compound 13**

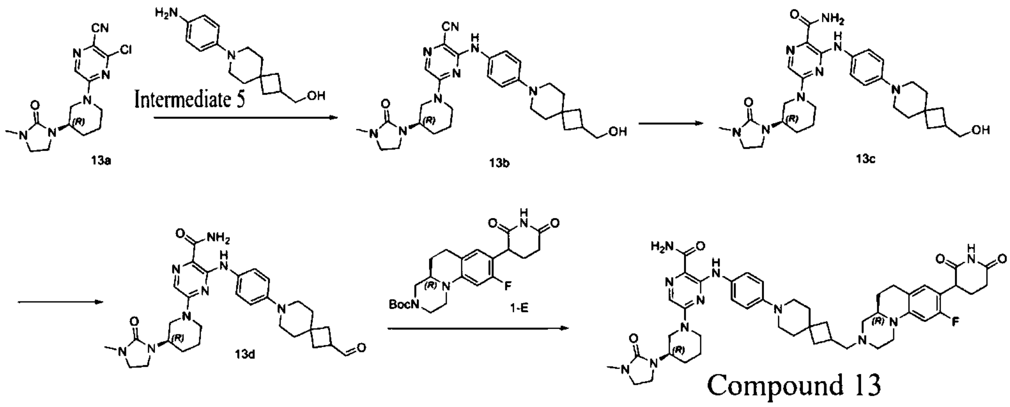

Compound 13

Starting from compound 13a (see WO 2023088406 for the synthetic method) and intermediate 5 and following the synthetic method of example 5, compound 13 was obtained.

$^1H$ NMR (400 MHz, $CDCl_3$) δ 10.62 (s, 1H), 7.97 (s, 1H), 7.55 - 7.31 (m, 4H), 7.00 - 6.82 (m, 2H), 6.79 - 6.65 (m, 1H), 6.48 (d, 1H), 5.16 (s, 1H), 4.40 - 4.20 (m, 2H), 3.90 - 3.52 (m, 3H), 3.45 - 3.22 (m, 4H), 3.18 - 2.40 (m, 18H), 2.35 - 1.44 (m, 20H).

LCMS m/z = 848.4 $[M+1]^+$

**Example 17: Preparation of compound 17**

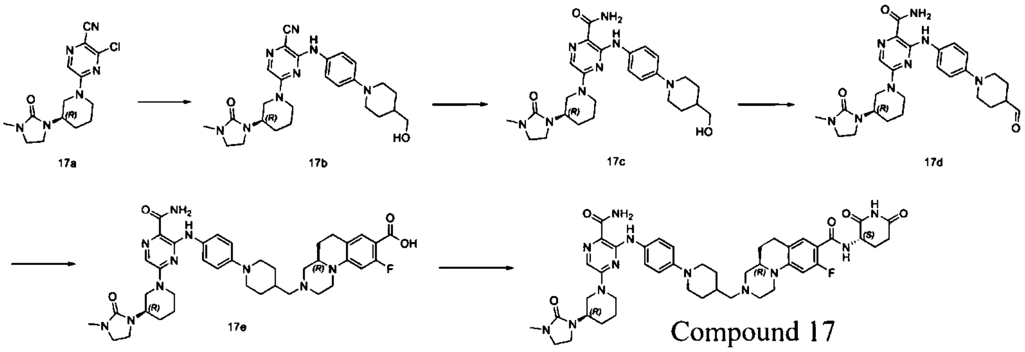

Compound 17

Step 1: Preparation of 17b

17a (2 g, 6.23 mmol) (see WO 2023088406 for the synthetic method) was dissolved in 1,4-dioxane (30 mL), (1-(4-aminophenyl)piperidin-4-yl)methanol (1.9 g, 9.21 mmol), caesium carbonate (6.1 g, 18.72 mmol), palladium acetate (140 mg, 0.624 mmol) and 2,2'-bis(diphenylphosphino)-1,1'-binaphthyl (578 mg, 0.93 mmol) were added, and the mixture was purged three times with nitrogen and reacted at 80°C for 6 h. The reaction system was cooled to room temperature, and filtered. The filter cake was washed twice with dichloromethane (200 mL), and the filtrate was concentrated under reduced pressure. The crude product was separated and purified by silica gel column chromatography (dichloromethane : methanol (v/v) = 91 : 9) to obtain 17b (1.2 g, yield: 39%).

LCMS m/z = 491.3 $[M+1]^+$

Step 2: Preparation of 17c

17b (1.2 g, 2.45 mmol) was dissolved in DMSO (20 mL), caesium carbonate (796 mg, 2.44 mmol) was added, followed by slowly dropwise addition of 30% hydrogen peroxide (8 mL) in an ice-water bath, and the mixture was stirred for 5 min. Subsequently, the ice-water bath was removed, and the mixture was reacted at room temperature for 3 h. To the reaction system were added a saturated sodium sulphite solution (70 mL) and ethyl acetate (70 mL). The aqueous phase was extracted with ethyl acetate (60 mL×3), and the organic phase was washed with a saturated aqueous sodium chloride solution (50 mL×2), dried over anhydrous sodium sulphate, and concentrated under reduced pressure. The crude product was separated and purified by silica gel column chromatography (dichloromethane : methanol (v/v) = 92 : 8) to obtain 17c

(1 g, yield: 80%).

LCMS m/z = 509.3 [M+1]+

Step 3: Preparation of 17d

17c (700 mg, 1.38 mmol) was dissolved in a mixed solvent of dichloromethane (35 mL) and acetonitrile (5 mL), 4 Å molecular sieve (500 mg), N-methylmorpholine oxide (242 mg, 2.07 mmol) and tetrapropylammonium perruthenate (24 mg, 0.068 mmol) were added, and the mixture was reacted at room temperature for 6 h. The reaction system was filtered, the filtrate was concentrated under reduced pressure, and the crude product was separated and purified by silica gel column chromatography (dichloromethane : methanol (v/v) = 92 : 8) to obtain 17d (150 mg, yield: 21%).

LCMS m/z = 507.3 [M+1]+

Step 4: Preparation of 17e

3-A (60 mg, 0.17 mmol) was added to a single-necked flask (50 mL), followed by dichloromethane (10 mL) and trifluoroacetic acid (3 mL), and the mixture was reacted at room temperature for 2 h. The reaction system was concentrated under reduced pressure, N,N-dimethylacetamide (6 mL) and 17d (86 mg, 0.17 mmol) were added, and the mixture was reacted at room temperature for 16 h. Sodium triacetylborohydride (60 mg, 0.28 mmol) was then added, and the resulting mixture was reacted at room temperature for 2 h. To the reaction system was added water (60 mL), and the mixture was extracted with dichloromethane (60 mL×3). The organic phase was washed with a saturated aqueous sodium chloride solution (60 mL×2), dried over anhydrous sodium sulphate, and concentrated under reduced pressure. The crude product was separated and purified by silica gel column chromatography (dichloromethane : methanol (v/v) = 91 : 9) to obtain 17e (50 mg, yield: 40%).

LCMS m/z = 741.5 [M+1]+

Step 5: Preparation of compound 17

17e (25 mg, 0.034 mmol) was added to a single-necked flask (50 mL), followed by N,N-dimethylformamide (6 mL), EDCI (10 mg, 0.052 mol), HOBt (8 mg, 0.0592 mmol) and N-methylimidazole (15 mg, 0.18 mmol), and the mixture was stirred at room temperature for 30 min. (S)-3-Aminopiperidine-2,6-dione hydrochloride (7 mg, 0.043 mmol) was then added, and the resulting mixture was reacted at room temperature for 16 h. To the reaction system was added water (70 mL), and the mixture was extracted with dichloromethane (50 mL×3). The organic phase was washed with a saturated aqueous sodium chloride solution (50 mL×2), dried over anhydrous sodium sulphate, and concentrated under reduced pressure. The crude product was separated and purified by preparative thick-layer chromatography (dichloromethane : methanol (v/v) = 92 : 8) to obtain compound 17 (20 mg, yield: 69%).

$^1H$ NMR (400 MHz, $CDCl_3$) δ 10.70 (s, 1H), 7.95 (s, 1H), 7.66 (d, 1H), 7.60 - 7.32 (m, 5H), 7.14 - 6.85 (m, 2H), 6.41 (d, 1H), 5.17 (s, 1H), 4.84 - 4.70 (m, 1H), 4.40 - 4.24 (m, 2H), 3.88 - 3.56 (m, 4H), 3.45 - 2.60 (m, 21H), 2.50 - 2.12 (m, 3H), 2.10 - 1.50 (m, 12H).

LCMS m/z = 851.4 [M+1]+

**Example 18: Preparation of compound 18**

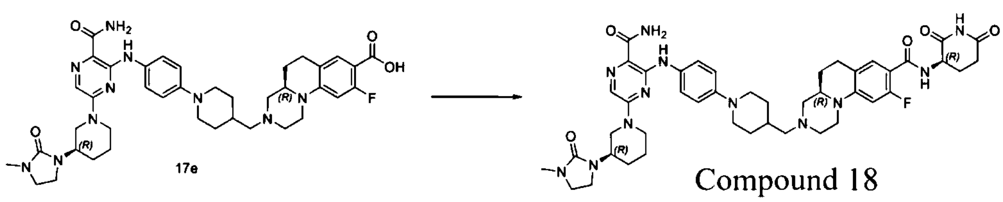

17e

Compound 18

Starting from compound 17e and (R)-3-aminopiperidine-2,6-dione hydrochloride and following the synthetic method of example 17, compound 18 was obtained.

$^1H$ NMR (400 MHz, $CDCl_3$) δ 10.67 (s, 1H), 7.97 (s, 1H), 7.66 (d, 1H), 7.58 - 7.30 (m, 5H), 7.10 - 6.85 (m, 2H), 6.41 (d, 1H), 5.17 (s, 1H), 4.87 - 4.70 (m, 1H), 4.41 - 4.20 (m, 2H), 3.90 - 3.55 (m, 4H), 3.45 - 2.55 (m, 21H), 2.50 - 2.10 (m, 3H), 2.09 - 1.50 (m, 12H).

LCMS m/z = 851.4 [M+1]+

**Example 19: Preparation of compound 19**

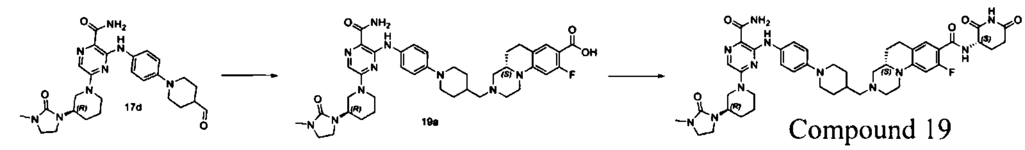

17d

19a

Compound 19

Starting from compounds 17d and 4-A and following the synthetic method of example 17, compound 19 was obtained.

$^1$H NMR (400 MHz, $CDCl_3$) δ 10.69 (s, 1H), 7.94 (s, 1H), 7.67 (d, 1H), 7.58 - 7.32 (m, 5H), 7.13 - 6.80 (m, 2H), 6.41 (d, 1H), 5.17 (s, 1H), 4.84 - 4.72 (m, 1H), 4.40 - 4.22 (m, 2H), 3.88 - 3.57 (m, 4H), 3.50 - 2.55 (m, 21H), 2.50 - 2.10 (m, 3H), 2.08 - 1.50 (m, 12H).

LCMS m/z = 851.4 $[M+1]^+$

**Example 20: Preparation of compound 20**

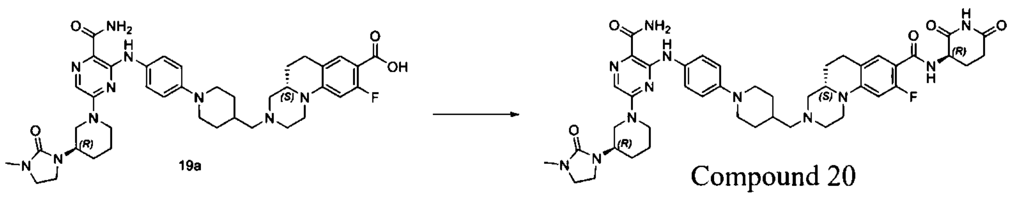

19a

Compound 20

Starting from compound 19a and (R)-3-aminopiperidine-2,6-dione hydrochloride and following the synthetic method of example 17, compound 20 was obtained.

$^1$H NMR (400 MHz, $CDCl_3$) δ 10.72 (s, 1H), 7.94 (s, 1H), 7.67 (d, 1H), 7.61 - 7.32 (m, 5H), 7.18 - 6.85 (m, 2H), 6.42 (d, 1H), 5.17 (s, 1H), 4.85 - 4.66 (m, 1H), 4.43 - 4.21 (m, 2H), 3.90 - 3.55 (m, 4H), 3.45 - 3.25 (m, 5H), 3.20 - 3.15 (m, 19H), 2.10 - 1.55 (m, 12H).

LCMS m/z = 851.4 $[M+1]^+$

**Example 21: Preparation of compound 21**

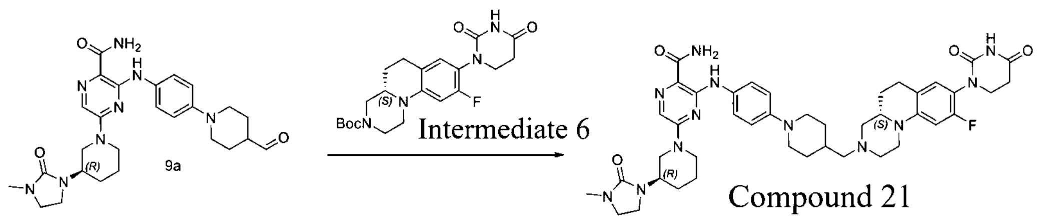


9a

Compound 21

Intermediate 6 (80 mg, 0.19 mmol) was added to a single-necked flask (50 mL), followed by dichloromethane (10 mL) and 4 mol/L solution of hydrogen chloride in 1,4-dioxane (10 mL), and the mixture was reacted at room temperature for 2 h. The reaction system was concentrated under reduced pressure, dichloromethane (20 mL) and sodium bicarbonate (48 mg, 0.59 mmol) were added, and the mixture was stirred at room temperature for 30 min. 9a (96 mg, 0.19 mmol), anhydrous sodium sulphate (200 mg) and acetic acid (0.02 mL) were added, and the mixture was stirred and reacted at room temperature for 16 h. Sodium triacetoxyborohydride (81 mg, 0.38 mmol) was then added, and the resulting mixture was reacted at room temperature for 2 h. To the reaction system was added water (120 mL), and the mixture was extracted with dichloromethane (70 mL×3). The organic phase was washed with a saturated aqueous sodium chloride solution (60 mL×2), dried over anhydrous sodium sulphate, and concentrated under reduced pressure. The crude product was separated and purified by preparative thick-layer chromatography (dichloromethane : methanol (v/v) = 93 : 7) to obtain compound 21 (70 mg, yield: 46%).

$^1H$ NMR (400 MHz, $CDCl_3$) δ 10.64 (s, 1H), 7.60 - 7.32 (m, 5H), 7.10 - 6.75 (m, 3H), 6.53 (d, 1H), 5.16 (s, 1H), 4.40 - 4.21 (m, 2H), 3.89 - 3.51 (m, 6H), 3.44 - 3.23 (m, 4H), 3.19 - 2.55 (m, 14H), 2.40 - 2.06 (m, 2H), 2.05 - 1.55 (m, 12H), 1.50 - 1.32 (m, 2H).
LCMS m/z = 809.6 $[M+1]^+$

**Example 23: Preparation of compound 23**

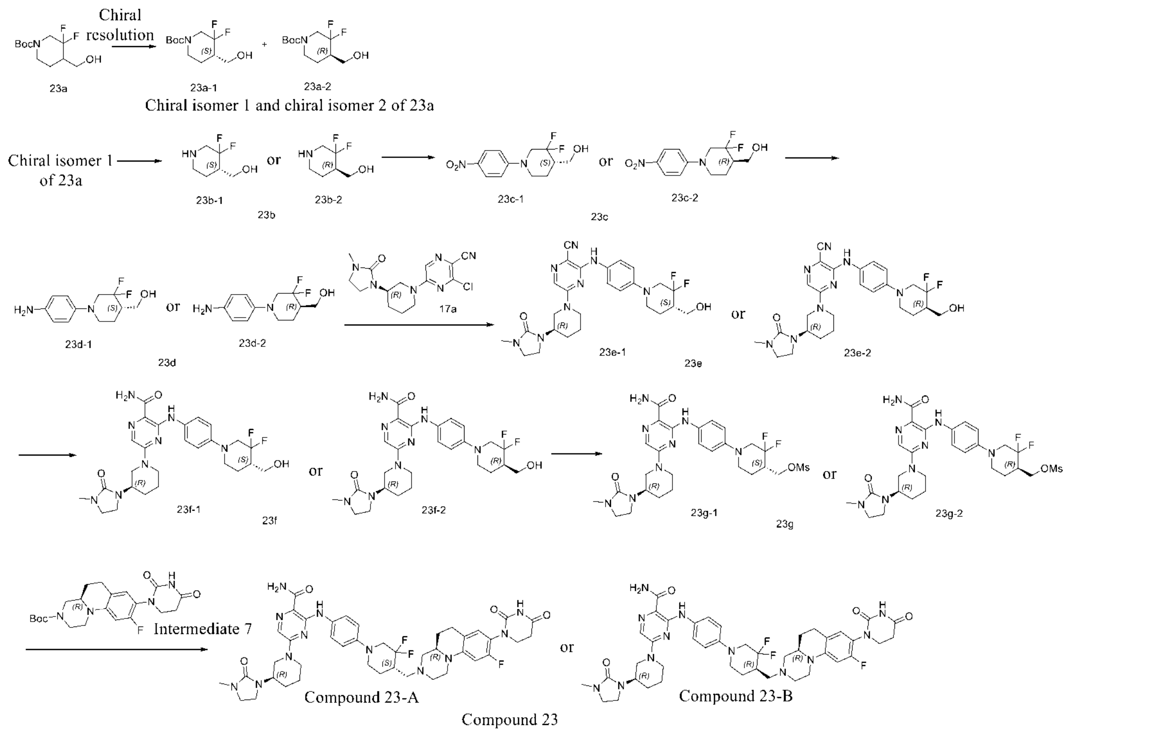


Step 1: Preparation of chiral isomer 1 and chiral isomer 2 of 23a

23a (see WO 2021023105 for the synthetic method) was subjected to chiral resolution, and the preparation conditions were as follows:

1. Instrument: Waters 150 Prep-SFC E; chromatography column: Chiralcel AD column.
2. The sample was dissolved in acetonitrile and filtered through a 0.45 μm filter head to prepare a sample solution.
3. Preparative chromatography conditions: a. the mobile phase consists of systems A and B: mobile phase A: $CO_2$; mobile phase B: methanol; b. isocratic elution, mobile phase B: 10%; c. flow rate: 120 mL/min.

Chiral analysis conditions:

1. Instrument: SHIMADZU LC-30AD; chromatography column: Chiralcel AD column.

2. Analytical chromatography conditions: a. the mobile phase consists of systems A and B: mobile phase A: $CO_2$; mobile phase B: methanol (containing 0.05% diethylamine); b. isocratic elution, mobile phase B: 5%-40%; c. flow rate: 3 mL/min.

Retention time: chiral isomer 1: 0.60 min, chiral isomer 2: 0.78 min.
Chiral isomer 1 and chiral isomer 2 of compound 23a are respectively one of the isomers of structure 23a-1 or 23a-2.

Step 2: Preparation of trifluoroacetate of 23b

Chiral isomer 1 of 23a (6.3 g, 25.07 mmol) was dissolved in dichloromethane (80 mL), trifluoroacetic acid (20 mL) was slowly added, and the mixture was reacted at 25°C for 2 h. The reaction solution was concentrated under reduced pressure to obtain the trifluoroacetate of crude 23b (5.6 g).
Compound 23b is one of the isomers of structure 23b-1 or 23b-2.

Step 3: Preparation of 23c

The above-mentioned trifluoroacetate of crude 23b (5.6 g) was dissolved in DMF (100 mL), potassium carbonate (13.8 g, 100 mmol) and p-fluoronitrobenzene (4.2 g, 29.8 mmol) were added, and the mixture was reacted at 80°C for 16 h. The reaction solution was cooled to room temperature, purified water (500 mL) was added, and the mixture was extracted twice with dichloromethane (300 mL). The organic phases were combined, washed with purified water (600 mL), and concentrated under reduced pressure. The crude product was separated and purified by silica gel column chromatography (petroleum ether : ethyl acetate (v/v) = 1 : 1) to obtain 23c (4.5 g, yield: 55%).
LCMS m/z = 273.1 $[M+1]^+$
Compound 23c is one of the isomers of structure 23c-1 or 23c-2.

Step 4: Preparation of 23d

23c (4.5 g, 16.54 mmol) was dissolved in ethanol (100 mL), iron powder (4.5 g, 80.36 mmol) and a saturated ammonium chloride solution (20 mL) were added, and the mixture was reacted under reflux for 3 h. The reaction system was cooled to room temperature, dichloromethane (300 mL) was added, and the mixture was filtered. The filtrate was concentrated under reduced pressure to obtain crude 23d (3.9 g).
Compound 23d is one of the isomers of structure 23d-1 or 23d-2.

Step 5: Preparation of 23e

The above-mentioned crude 23d (3.9 g) was dissolved in 1,4-dioxane (100 mL), 17a (5.2 g, 16.21 mmol), palladium acetate (0.36 g, 1.6 mmol), BINAP (2.0 g, 3.2 mmol) and caesium carbonate (13 g, 39.9 mmol) were added, and the mixture was reacted under reflux for 12 h. The reaction system was cooled to room temperature, dichloromethane (300 mL) was added, and the mixture was filtered. The filtrate was concentrated under reduced pressure, and the crude product was separated and purified by silica gel column chromatography (dichloromethane : methanol (v/v) = 15 : 1) to obtain 23e (4.6 g, yield: 54%).
LCMS m/z = 527.5 $[M+1]^+$
Compound 23e is one of the isomers of structure 23e-1 or 23e-2.

Step 6: Preparation of 23f

23e (4.6 g, 8.7 mmol) was dissolved in methanol (180 mL), and DMSO (10 mL) and caesium carbonate (3.3 g, 10.13 mmol) were added. 30% aqueous hydrogen peroxide solution (50 mL) was then slowly added, and the mixture was reacted at 25°C for 3 h. The reaction solution was concentrated under reduced pressure, and purified water (300 mL) was added, with the yellow solids precipitated out. The mixture was filtered, and the filter cake was dried under reduced pressure to obtain crude 23f (3.8 g).
LCMS m/z = 545.5 $[M+1]^+$
Compound 23f is one of the isomers of structure 23f-1 or 23f-2.

Step 7: Preparation of 23g

The above-mentioned crude 23f (0.33 g) was dissolved in dichloromethane (20 mL), triethylamine (0.12 g, 1.19 mmol) and methanesulphonic anhydride (0.14 g, 0.8 mmol) were added, and the mixture was reacted at 25°C for 3 h. The reaction solution was concentrated under reduced pressure, and the crude product was separated and purified by silica gel column chromatography (dichloromethane : methanol (v/v) = 15 : 1) to obtain 23g (0.36 g, two-step yield from compound 23e: 77%).
Compound 23g is one of the isomers of structure 23g-1 or 23g-2.

Step 8: Preparation of compound 23

Intermediate 7 (0.21 g, 0.50 mmol) was dissolved in dichloromethane (5 mL), trifluoroacetic acid (5 mL) was added, and the mixture was reacted at room temperature for 1 h. The reaction solution was concentrated under reduced pressure, and dichloromethane (10 mL) and isopropanol (1 mL) were added. The mixture was adjusted to pH 9 with a saturated aqueous sodium bicarbonate solution, and extracted three times with a mixed solvent of dichloromethane/isopropanol (v/v) = 10 : 1 (100 mL). The organic phases were combined, dried over anhydrous sodium sulphate, and concentrated under reduced pressure to obtain a crude product (0.158 g). 23g (0.125 g, 0.2 mmol) was dissolved in a mixed solvent of acetonitrile (10 mL) and tetrahydrofuran (5 mL), potassium carbonate (0.07 g, 0.51 mmol) and the above-mentioned crude product (0.064 g) were added, and the mixture was reacted under reflux for 8 h. The reaction solution was cooled to room temperature, and concentrated under reduced pressure. The crude product was separated and purified by silica gel column chromatography (dichloromethane : methanol (v/v) = 10 : 1) to obtain compound 23 (22 mg, yield: 13%).

$^{1}H$ NMR (400 MHz, $CDCl_3$) δ 10.48 (s, 1H), 7.56 - 7.34 (m, 4H), 7.28 - 7.24 (m, 1H), 6.87 (d, 1H), 6.60 - 6.43 (m, 3H), 5.35 (s, 1H), 4.38 - 4.20 (m, 2H), 4.10 - 3.50 (m, 9H), 3.42 - 2.72 (m, 18H), 2.72 - 2.57 (m, 3H), 2.25 - 2.07 (m, 1H), 2.05 - 1.58 (m, 7H).

LCMS m/z = 845.4 [M+1] $^{+}$

Compound 23 is one of the isomers of compound 23-A or 23-B.

**Example 24: Preparation of compound 24**

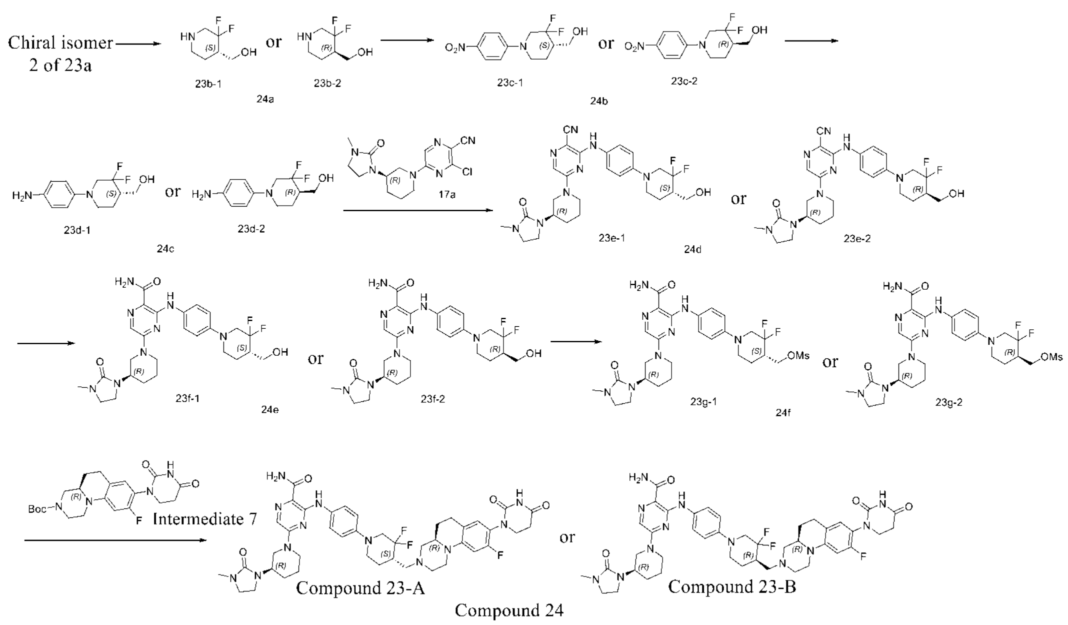


Compound 23-A or Compound 23-B

Compound 24

Starting from chiral isomer 2 of compound 23a and following the synthetic method of example 23, compound 24 was obtained.

$^{1}H$ NMR (400 MHz, $CDCl_3$) δ 10.48 (s, 1H), 7.50 - 7.32 (m, 4H), 7.27 - 7.23 (m, 1H), 6.89 (d, 1H), 6.60 - 6.43 (m, 3H), 5.39 (s, 1H), 4.38 - 4.20 (m, 2H), 4.10 - 3.50 (m, 9H), 3.42 - 2.50 (m, 21H), 2.25 - 2.07 (m, 1H), 2.05 - 1.58 (m, 7H).

LCMS m/z = 845.4 [M+1] $^{+}$

Compound 24 is one of the isomers of compound 23-A or 23-B.

**Example 25: Preparation of compound 25**

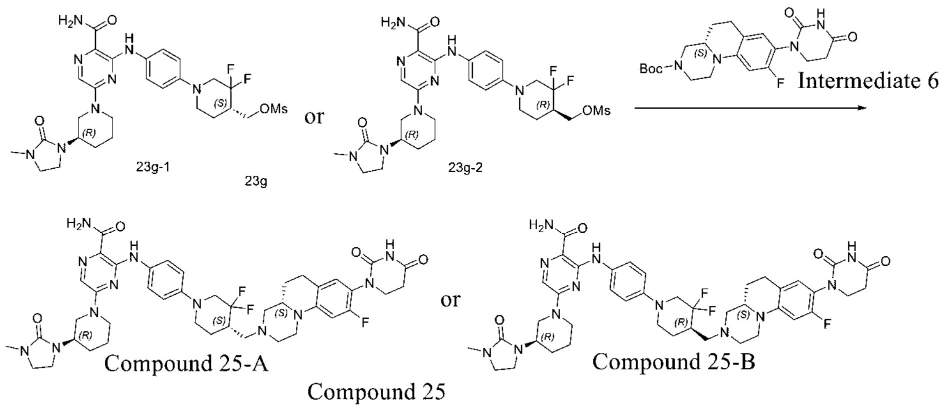
23g-1 or 23g-2

23g

Intermediate 6

Compound 25-A or Compound 25-B

Compound 25

Starting from compound 23g and intermediate 6 and following the synthetic method of example 23, compound 25 was obtained.

$^1H$ NMR (400 MHz, $CDCl_3$) δ 10.48 (s, 1H), 7.54 - 7.30 (m, 4H), 7.29 - 7.23 (m, 1H), 6.95 - 6.82 (m, 1H), 6.60 - 6.43 (m, 3H), 5.35 (s, 1H), 4.38 - 4.22 (m, 2H), 4.10 - 3.50 (m, 9H), 3.42 - 2.50 (m, 21H), 2.25 - 2.07 (m, 1H), 2.05 - 1.55 (m, 7H).

LCMS m/z = 845.3 $[M+1]^+$

Compound 25 is one of the isomers of compound 25-A or 25-B.

**Example 26: Preparation of compound 26**

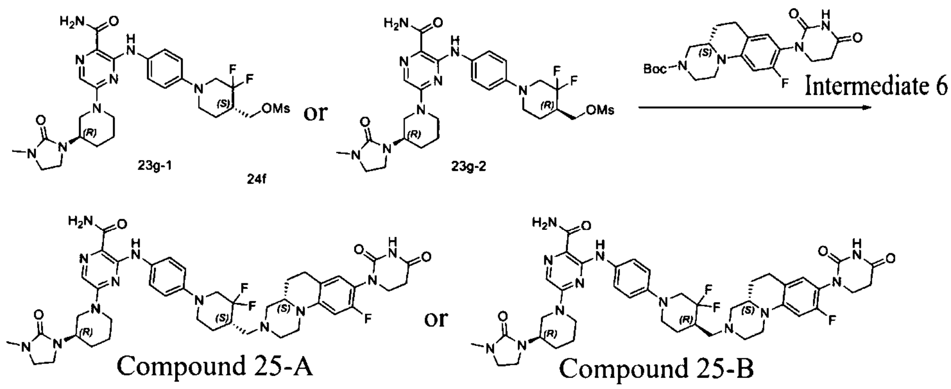
23g-1 or 23g-2

24f

Intermediate 6

Compound 25-A or Compound 25-B

Compound 26

Starting from compound 24f and intermediate 6 and following the synthetic method of example 23, compound 26 was obtained.

$^1H$ NMR (400 MHz, $CDCl_3$) δ 10.49 (s, 1H), 7.55 - 7.30 (m, 4H), 7.29 - 7.23 (m, 1H), 6.95 - 6.82 (m, 1H), 6.60 - 6.43 (m, 3H), 5.37 (s, 1H), 4.38 - 4.20 (m, 2H), 4.10 - 3.50 (m, 9H), 3.45 - 2.50 (m, 21H), 2.25 - 2.07 (m, 1H), 2.05 - 1.55 (m, 7H).

LCMS m/z = 423.3 $[M/2 +1]^+$

Compound 26 is one of the isomers of compound 25-A or 25-B.

**Example 27: Preparation of compound 27**

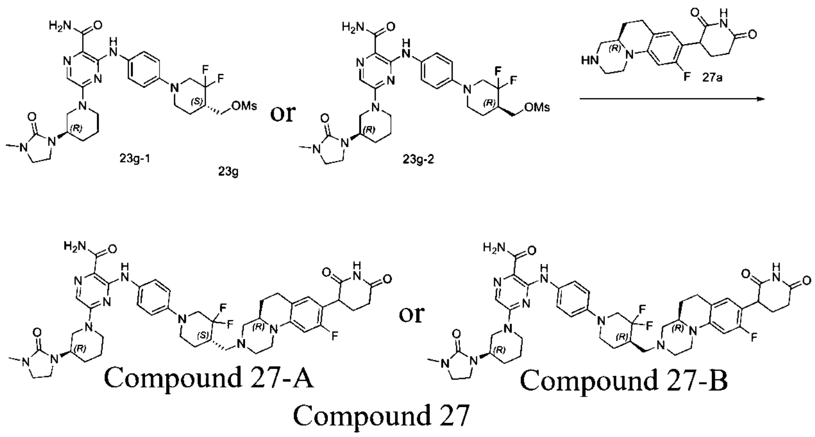

23g-1 or 23g-2

23g

27a

Compound 27-A or Compound 27-B

Compound 27

23g (0.125 g, 0.2 mmol) was dissolved in a mixed solvent of acetonitrile (10 mL) and tetrahydrofuran (5 mL), potassium carbonate (0.07 g, 0.51 mmol) and 27a (0.064 g, 0.2 mmol) (see WO 2023232133 for the synthetic method) were added, and the mixture was reacted under reflux for 8 h. The reaction solution was cooled to room temperature, and concentrated under reduced pressure. The crude product was separated and purified by silica gel column chromatography (dichloromethane : methanol (v/v) = 10 : 1) to obtain compound 27 (47 mg, yield: 28%).

$^1$H NMR (400 MHz, $CDCl_3$) δ 10.55 (s, 1H), 8.02 (d, 1H), 7.55 - 7.28 (m, 4H), 6.80 - 6.66 (m, 1H), 6.58 - 6.42 (m, 3H), 5.17 (s, 1H), 4.40 - 4.20 (m, 2H), 3.90 - 3.52 (m, 6H), 3.45 - 2.50 (m, 20H), 2.45 - 1.40 (m, 13H).

LCMS m/z = 844.4 [M+1] $^+$

Compound 27 is one of the isomers of compound 27-A or 27-B.

**Example 28: Preparation of compound 28**

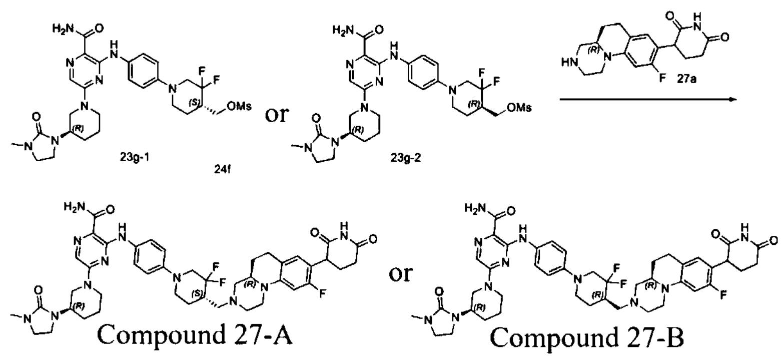

23g-1 or 23g-2

24f

27a

Compound 27-A or Compound 27-B

Compound 28

Starting from compounds 27a and 24f and following the synthetic method of example 27, compound 28 was obtained.

$^1$H NMR (400 MHz, $CDCl_3$) δ 10.56 (s, 1H), 8.06 - 7.96 (m, 1H), 7.56 - 7.30 (m, 4H), 6.80 - 6.66 (m, 1H), 6.58 - 6.42 (m, 3H), 5.17 (s, 1H), 4.40 - 4.20 (m, 2H), 3.90 - 3.52 (m, 6H), 3.45 - 2.50 (m, 20H), 2.35 - 1.40 (m, 13H).

Compound 28 is one of the isomers of compound 27-A or 27-B.

**Example 29: Preparation of compound 29**

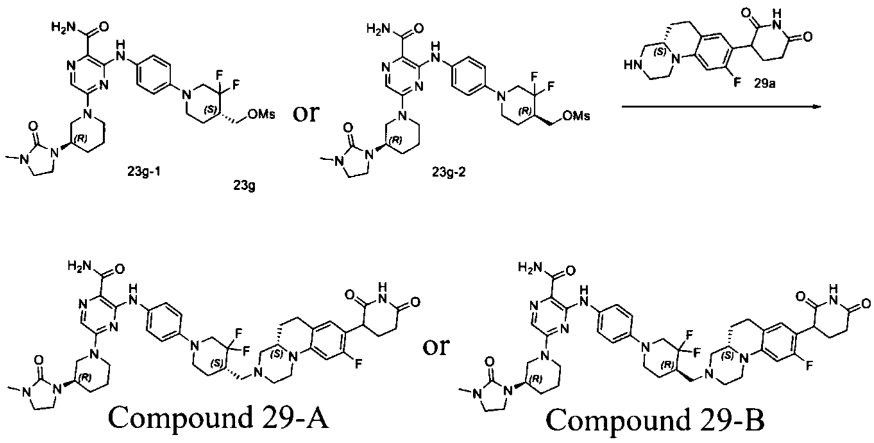

23g-1 or 23g-2

23g

29a

Compound 29-A or Compound 29-B

Compound 29

Starting from compounds 23g and 29a (see WO 2023232133 for the synthetic method) and following the synthetic method of example 27, compound 29 was obtained.

$^1$H NMR (400 MHz, $CDCl_3$) δ 10.54 (s, 1H), 8.04 - 7.91 (m, 1H), 7.55 - 7.30 (m, 4H), 6.80 - 6.66 (m, 1H), 6.58 - 6.42 (m, 3H), 5.15 (s, 1H), 4.40 - 4.20 (m, 2H), 3.90 - 3.52 (m, 6H), 3.45 - 2.45 (m, 20H), 2.37 - 1.40 (m, 13H).

Compound 29 is one of the isomers of compound 29-A or 29-B.

**Example 30: Preparation of compound 30**

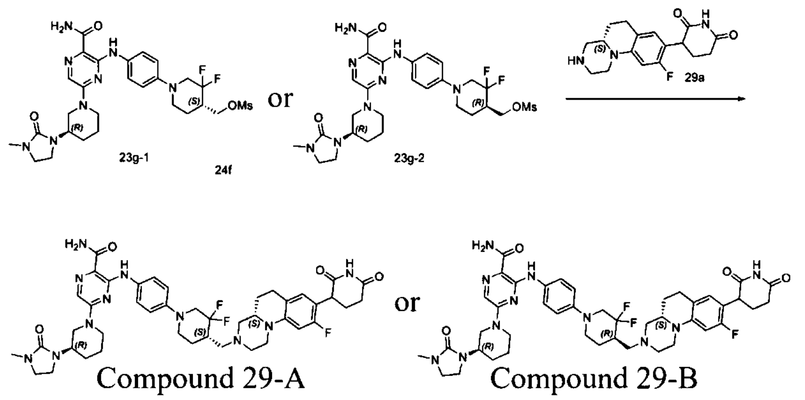

23g-1 or 23g-2

24f

29a

Compound 29-A or Compound 29-B

Compound 30

Starting from compounds 24f and 29a and following the synthetic method of example 27, compound 30 was obtained.

$^1$H NMR (400 MHz, $CDCl_3$) δ 10.55 (s, 1H), 8.06 - 7.88 (m, 1H), 7.62 - 7.30 (m, 4H), 6.80 - 6.66 (m, 1H), 6.58 - 6.42 (m, 3H), 5.15 (s, 1H), 4.40 - 4.16 (m, 2H), 3.90 - 3.52 (m, 6H), 3.45 - 2.40 (m, 20H), 2.35 - 1.40 (m, 13H).

Compound 30 is one of the isomers of compound 29-A or 29-B.

**Example 31: Preparation of compound 31**

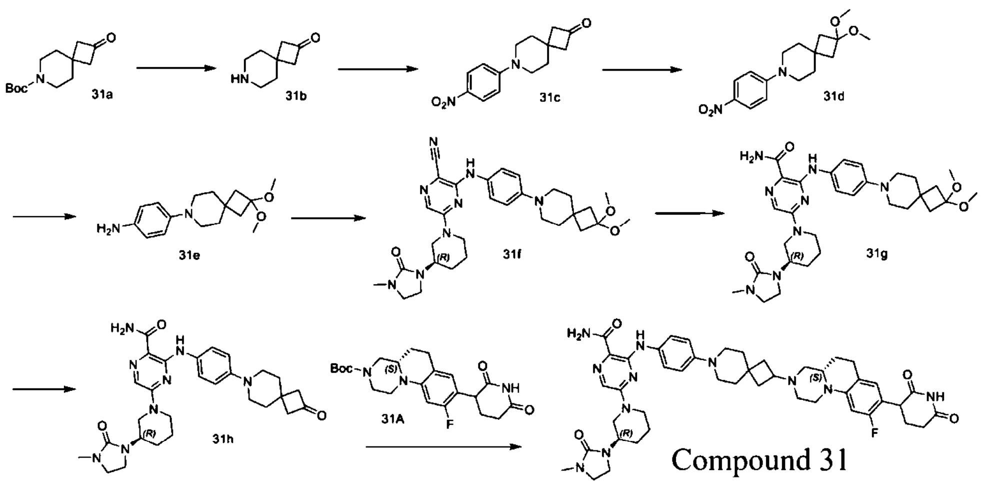

31a 31b 31c 31d

31e 31f 31g

31h 31A Compound 31

Step 1: Preparation of trifluoroacetate of 31b

31a (10 g, 41.79 mmol) was dissolved in dichloromethane (100 mL), trifluoroacetic acid (30 mL) was added, and the mixture was reacted at room temperature for 5 h. The reaction system was concentrated under reduced pressure to obtain the trifluoroacetate of crude 31b (12 g).

Step 2: Preparation of 31c

4-Fluoronitrobenzene (5.36 g, 37.99 mmol) and the above-mentioned trifluoroacetate of crude 31b (12 g) were dissolved in DMF (100 mL), potassium carbonate (15.75 g, 113.97 mmol) was added, and the mixture was reacted at 80°C for 18 h. The reaction system was cooled to room temperature, and slowly poured into water (1000 mL), with the solids precipitated out. The mixture was filtered, and the filter cake was washed with water (20 mL×3), and dried under reduced pressure to obtain crude 31c (9.5 g).
LCMS m/z = 261.1 $[M+1]^+$

Step 3: Preparation of 31d

The above-mentioned crude 31c (3.0 g) was dissolved in methanol (50 mL), trimethyl orthoformate (4.89 g, 46.08 mmol) and p-toluenesulphonic acid (0.2 g, 1.16 mmol) were added, and the mixture was reacted at 40°C for 16 h. The reaction system was concentrated under reduced pressure to obtain crude 31d (3.8 g).

Step 4: Preparation of 31e

The above-mentioned crude 31d (3.5 g) was dissolved in methanol (60 mL), 10% palladium on carbon (1.0 g) was added, and the mixture was reacted at room temperature under hydrogen balloon atmosphere for 5 h. The reaction system was filtered through a pad of diatomaceous earth, the filter cake was washed with DCM (50 mL), and the filtrate was concentrated under reduced pressure to obtain crude 31e (3.0 g).

Step 5: Preparation of 31f

17a (1.5 g, 4.68 mmol) was dissolved in 1,4-dioxane (60 mL), the above-mentioned crude 31e (1.45 g), caesium carbonate (4.87 g, 14.95 mmol), palladium acetate (170 mg, 0.76 mmol) and 2,2'-bis(diphenylphosphino)-1,1'-binaphthyl (310 mg, 0.50 mmol) were added, and the mixture was purged three times with nitrogen and reacted at 100°C for 16 h. The reaction system was cooled to room temperature, and filtered. The filter cake was washed twice with dichloromethane (200 mL), and the filtrate was concentrated under reduced pressure. The crude product was separated and purified by silica gel column chromatography (dichloromethane : methanol (v/v) = 20 : 1) to obtain 31f (1.8 g, yield: 69%).
LCMS m/z = 561.3 $[M+1]^+$

Step 6: Preparation of 31g

31f (1.0 g, 3.21 mmol) was dissolved in DMSO (5 mL), caesium carbonate (580 mg, 1.78 mmol) was added,

followed by slowly dropwise addition of 30% hydrogen peroxide (5 mL) in an ice-water bath, and the mixture was stirred for 5 min. Subsequently, the ice-water bath was removed, and the mixture was reacted at room temperature for 5 h. To the reaction system was added a saturated aqueous sodium sulphite solution (50 mL), and the mixture was extracted with ethyl acetate (100 mL×3). The organic phase was washed with a saturated aqueous sodium chloride solution (100 mL), dried over anhydrous sodium sulphate, and concentrated under reduced pressure. The crude product was slurried with a mixed solvent of methyl tert-butyl ether (50 mL) and dichloromethane (5 mL), and filtered, and the filter cake was dried under reduced pressure to obtain crude 31g (0.88 g).

LCMS m/z = 579.3 $[M+1]^+$

Step 7: Preparation of 31h

The above-mentioned crude 31g (0.25 g) was dissolved in DCM (10 mL), trifluoroacetic acid (2 mL) was added, and the mixture was reacted at room temperature for 5 h. The reaction system was concentrated under reduced pressure to obtain crude 31h (0.3 g).

LCMS m/z = 533.3 $[M+1]^+$

Step 8: Preparation of compound 31

31A (90 mg, 0.22 mmol) (see WO 2023232133 for the synthetic method) was added to a single-necked flask (50 mL), followed by dichloromethane (5 mL) and trifluoroacetic acid (2 mL), and the mixture was reacted at room temperature for 5 h. The reaction system was concentrated under reduced pressure, tetrahydrofuran (10 mL), the above-mentioned crude 31h (150 mg) and sodium bicarbonate (100 mg, 1.19 mmol) were added, and the mixture was stirred at room temperature for 30 min. Acetic acid (36 mg, 0.60 mmol) was added, and the mixture was stirred at room temperature for 2 h. Sodium triacetoxyborohydride (85 mg, 0.40 mmol) was then added, and the resulting mixture was reacted at room temperature for 16 h. To the reaction system was added water (30 mL), and the mixture was extracted with dichloromethane (50 mL×3). The organic phase was washed with a saturated aqueous sodium chloride solution (50 mL×2), dried over anhydrous sodium sulphate, and concentrated under reduced pressure. The crude product was separated and purified by silica gel column chromatography (dichloromethane : methanol (v/v) = 12 : 1) to obtain compound 31 (50 mg, yield: 27%).

$^1H$ NMR (400 MHz, $CDCl_3$) δ 10.62 (s, 1H), 8.10 (s, 1H), 7.53 - 7.35 (m, 4H), 6.95 - 6.87 (m, 2H), 6.71 (d, 1H), 6.49 (d, 1H), 5.21 (s, 1H), 4.38 - 4.21 (m, 2H), 3.88 - 3.56 (m, 3H), 3.43 - 3.22 (m, 4H), 3.20 - 2.57 (m, 18H), 2.34 - 1.55 (m, 18H).

LCMS m/z = 834.7 $[M+1]^+$

**Example 32: Preparation of compound 32**

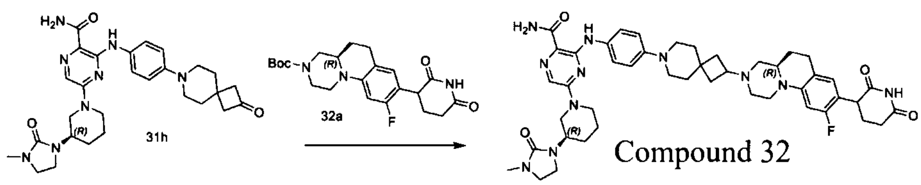

Starting from compounds 31h and 32a (see WO 2023232133 for the synthetic method) and following the synthetic method of example 31, compound 32 was obtained.

$^1H$ NMR (400 MHz, $CDCl_3$) δ 10.62 (s, 1H), 8.01 (s, 1H), 7.53 - 7.35 (m, 4H), 6.96 - 6.86 (m, 2H), 6.71 (d, 1H), 6.49 (d, 1H), 5.17 (s, 1H), 4.38 - 4.22 (m, 2H), 3.88 - 3.55 (m, 3H), 3.48 - 3.22 (m, 4H), 3.18 - 2.55 (m, 18H), 2.34 - 1.55 (m, 18H).

LCMS m/z = 834.6 $[M+1]^+$

**Example 33: Preparation of compound 33**

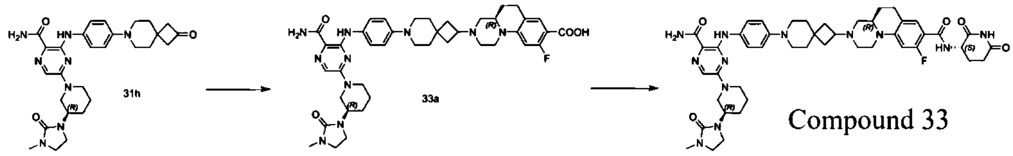


Compound 33

Step 1: Preparation of 33a

3-A (150 mg, 0.43 mmol) was added to a single-necked flask (50 mL), followed by dichloromethane (5 mL) and trifluoroacetic acid (2 mL), and the mixture was reacted at room temperature for 5 h. The reaction system was concentrated under reduced pressure, tetrahydrofuran (10 mL), the above-mentioned crude 31h (300 mg) and sodium bicarbonate (210 mg, 2.5 mmol) were added, and the mixture was stirred at room temperature for 30 min. Acetic acid (74 mg, 1.23 mmol) was added, and the mixture was stirred at 30°C for 2 h. Sodium triacetoxyborohydride (170 mg, 0.80 mmol) was then added, and the resulting mixture was reacted at room temperature for 16 h. To the reaction system was added water (30 mL), and the mixture was extracted with dichloromethane (50 mL×3). The organic phase was washed with a saturated aqueous sodium chloride solution (50 mL×2), dried over anhydrous sodium sulphate, and concentrated under reduced pressure. The crude product was separated and purified by silica gel column chromatography (dichloromethane : methanol (v/v) = 12 : 1) to obtain 33a (200 mg, yield: 61%).

LCMS m/z = 767.6 $[M+1]^+$

Step 2: Preparation of compound 33

33a (200 mg, 0.26 mmol) was added to a single-necked flask (50 mL), followed by N,N-dimethylformamide (5 mL), EDCI (100 mg, 0.52 mmol), HOBt (70 mg, 0.52 mmol) and N-methylimidazole (130 mg, 1.58 mmol), and the mixture was stirred at room temperature for 30 min. (S)-3-Aminopiperidine-2,6-dione hydrochloride (86 mg, 0.52 mmol) was then added, and the resulting mixture was reacted at room temperature for 16 h. To the reaction system was added water (70 mL), and the mixture was extracted with dichloromethane (50 mL×3). The organic phase was washed with a saturated aqueous sodium chloride solution (50 mL×2), dried over anhydrous sodium sulphate, and concentrated under reduced pressure. The crude product was subjected to Pre-HPLC (instrument and preparative column: using Waters 2767 preparative liquid phase chromatographic instrument; preparative column model: SunFire@Prep C18, 5 μm, inner diameter × length = 19 mm×250 mm). Preparative method: a solution of the crude product in DMSO was filtered through a 0.45 μm filter membrane to prepare a sample solution. Mobile phase system: water (containing 10 mmol/L ammonium bicarbonate)/acetonitrile. Gradient elution method: gradient elution with acetonitrile from 10% to 50% (elution time: 15 min). Lyophilisation was performed to obtain compound 33 (120 mg, yield: 53%).

$^1H$ NMR (400 MHz, DMSO-$d_6$) δ 10.96 (s, 1H), 10.82 (s, 1H), 7.94 (t, 1H), 7.72 - 7.66 (m, 1H), 7.60 (s, 1H), 7.45 - 7.30 (m, 3H), 7.29 - 7.21 (m, 1H), 6.94 - 6.80 (m, 2H), 6.67 (d, 1H), 4.79 - 4.65 (m, 1H), 4.45 - 4.18 (m, 2H), 3.93 - 3.80 (m, 1H), 3.68 - 3.52 (m, 1H), 3.40 - 3.20 (m, 5H), 3.15 - 2.60 (m, 16H), 2.58 - 2.45 (m, 1H), 2.20 - 1.50 (m, 18H).

LCMS m/z = 877.4 $[M+1]^+$

## Example 34: Preparation of compound 34

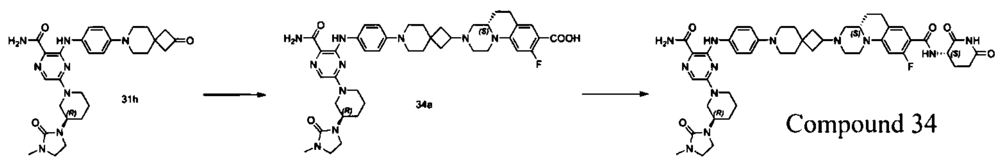


Compound 34

Starting from compounds 31h and 4-A and following the synthetic method of example 33, compound 34 was obtained.

$^1H$ NMR (400 MHz, DMSO-$d_6$) δ 10.96 (s, 1H), 10.82 (s, 1H), 7.93 (t, 1H), 7.69 (s, 1H), 7.60 (s, 1H), 7.46 - 7.31 (m, 3H), 7.26 (s, 1H), 6.94 - 6.80 (m, 2H), 6.68 (d, 1H), 4.78 - 4.65 (m, 1H), 4.43 - 4.16 (m, 2H), 3.95 - 3.80 (m, 1H), 3.68 - 3.54 (m, 1H), 3.42 - 3.18 (m, 5H), 3.17 - 2.61 (m, 16H), 2.58 - 2.50 (m, 1H), 2.20 - 1.45 (m, 18H).

LCMS m/z = 877.9 $[M+1]^+$

**Example 35: Preparation of trifluoroacetate of compound 35**

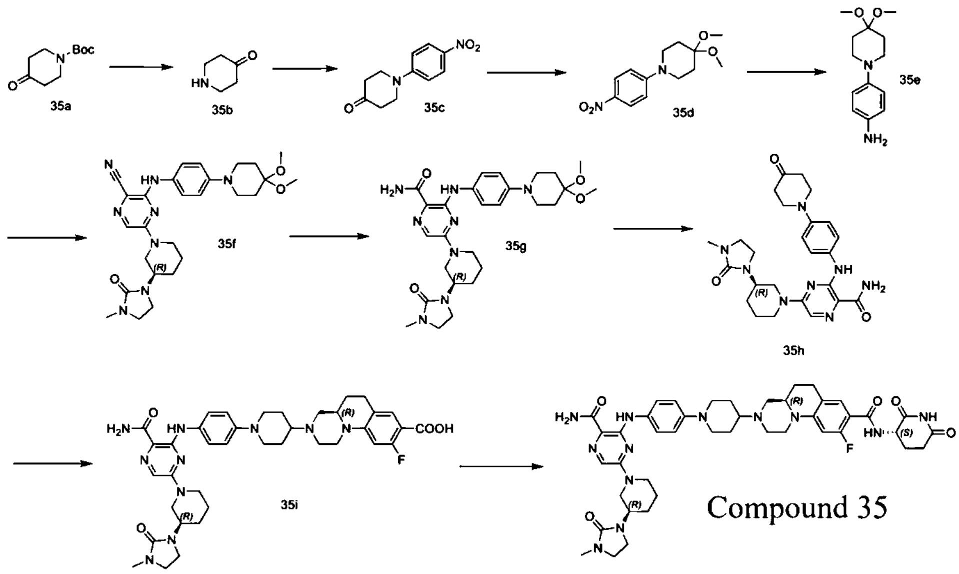

35a, 35b, 35c, 35d, 35e, 35f, 35g, 35h, 35i, Compound 35

Starting from compound 35a and following the synthetic methods of example 31 and example 33, the trifluoroacetate of compound 35 was obtained by acidic preparative chromatography [water (containing 0.1% TFA)/acetonitrile] and lyophilisation.

$^1H$ NMR (400 MHz, DMSO-$d_6$) δ 11.07 (s, 1H), 10.83 (s, 1H), 8.04 (t, 1H), 7.72 (s, 1H), 7.63 (s, 1H), 7.54 - 7.37 (m, 3H), 7.28 (s, 1H), 7.05 - 6.93 (m, 2H), 6.87 (d, 1H), 4.80 - 4.65 (m, 1H), 4.43 - 4.17 (m, 3H), 3.86 - 3.54 (m, 5H), 3.50 - 2.86 (m, 11H), 2.85 - 2.63 (m, 8H), 2.60 - 2.47 (m, 1H), 2.30 - 1.93 (m, 5H), 1.90 - 1.45 (m, 7H).

LCMS m/z = 837.8 $[M+1]^+$

**Example 36: Preparation of trifluoroacetate of compound** 36

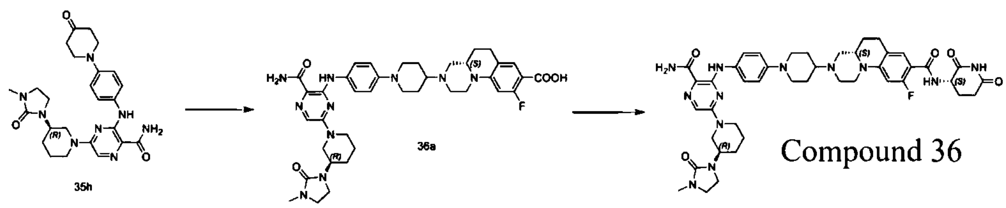

35h, 36a, Compound 36

Starting from compounds 35h and 4-A and following the synthetic method of example 35, the trifluoroacetate of compound 36 was obtained.

$^1H$ NMR (400 MHz, DMSO-$d_6$) δ 11.04 (s, 1H), 10.83 (s, 1H), 8.04 (t, 1H), 7.72 (s, 1H), 7.62 (s, 1H), 7.54 - 7.37 (m, 3H), 7.27 (s, 1H), 7.02 - 6.91 (m, 2H), 6.87 (d, 1H), 4.80 - 4.65 (m, 1H), 4.43 - 4.17 (m, 3H), 3.86 - 3.54 (m, 5H), 3.50 - 2.85 (m, 11H), 2.85 - 2.63 (m, 8H), 2.60 - 2.47 (m, 1H), 2.30 - 1.93 (m, 5H), 1.90 - 1.45 (m, 7H).

LCMS m/z = 837.6 $[M+1]^+$

**Example 37: Preparation of compound 37**

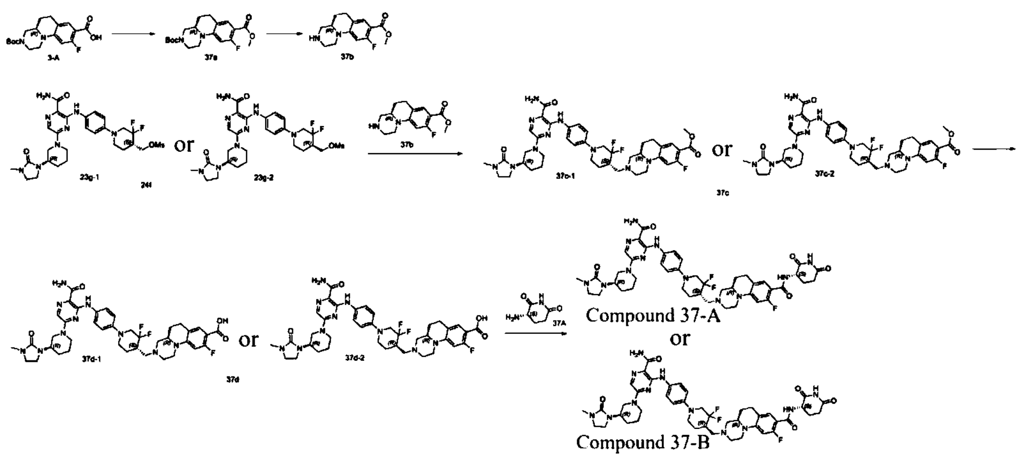


Compound 37

Step 1: Preparation of 37a

3-A (0.175 g, 0.5 mmol) was dissolved in dichloromethane (10 mL), TCFH (0.28 g, 1.0 mmol) and NMI (0.24 g, 2.92 mmol) were added, and the mixture was reacted at room temperature for 2 h. To the reaction system was added anhydrous methanol (2 mL), and the mixture was reacted at room temperature for 16 h. The reaction system was concentrated under reduced pressure, and the crude product was separated and purified by silica gel column chromatography (petroleum ether : ethyl acetate (v/v) = 3 : 1) to obtain 37a (0.17 g, yield: 93%).

Step 2: Preparation of trifluoroacetate of 37b

37a (0.17 g, 0.47 mmol) was dissolved in dichloromethane (6 mL), trifluoroacetic acid (2 mL) was slowly added, and the mixture was reacted at 25°C for 2 h. The reaction solution was concentrated under reduced pressure to obtain the trifluoroacetate of crude 37b (0.12 g).

Step 3: Preparation of 37c

24f (0.25 g, 0.4 mmol) was dissolved in a mixed solvent of acetonitrile (20 mL) and tetrahydrofuran (5 mL), potassium carbonate (0.14 g, 1.01 mmol) and the trifluoroacetate of the above-mentioned crude 37b (0.12 g) were added, and the mixture was reacted under reflux for 8 h. The reaction solution was cooled to room temperature, and concentrated under reduced pressure. The crude product was separated and purified by silica gel column chromatography (dichloromethane : methanol (v/v) = 10 : 1) to obtain 37c (0.2 g, yield: 63%).

LCMS m/z = 791.5 $[M+1]^+$

Compound 37c is one of the isomers of structure 37c-1 or 37c-2.

Step 4: Preparation of 37d

37c (0.2 g, 0.25 mmol) was dissolved in a mixed solvent of tetrahydrofuran (10 mL) and methanol (3 mL), purified water (3 mL) and lithium hydroxide monohydrate (0.63 g, 15 mmol) were added, and the mixture was reacted at 25°C for 12 h. The reaction solution was adjusted to pH 7 with 1 mol/L hydrochloric acid, and concentrated under reduced pressure to obtain crude 37d (0.15 g).

LCMS m/z = 777.3 $[M+1]^+$

Compound 37d is one of the isomers of structure 37d-1 or 37d-2.

Step 5: Preparation of compound 37

The above-mentioned crude 37d (0.15 g) was dissolved in DMF (10 mL), EDCI (0.1 g, 0.52 mmol), NMI (0.24 g, 2.92 mmol), HOBt (0.07 g, 0.52 mmol) and 37A (66.6 mg, 0.52 mmol) were added, and the mixture was reacted at room temperature for 12 h. The reaction solution was concentrated under reduced pressure, and the crude product was separated and purified by silica gel column chromatography (dichloromethane : methanol (v/v) = 10 : 1) to obtain compound 37 (62 mg, yield: 13%).

$^1H$ NMR (400 MHz, $CDCl_3$) δ 10.56 (s, 1H), 7.97 - 7.84 (m, 1H), 7.75 - 7.31 (m, 6H), 6.60 - 6.30 (m, 3H), 5.30 - 5.03

(m, 1H), 4.87 - 4.70 (m, 1H), 4.48 - 4.15 (m, 2H), 3.90 - 3.57 (m, 4H), 3.50 - 2.45 (m, 21H), 2.25 - 1.40 (m, 13H).

Compound 37 is one of the isomers of compound 37-A or 37-B.

**Example 38: Preparation of compound 38**

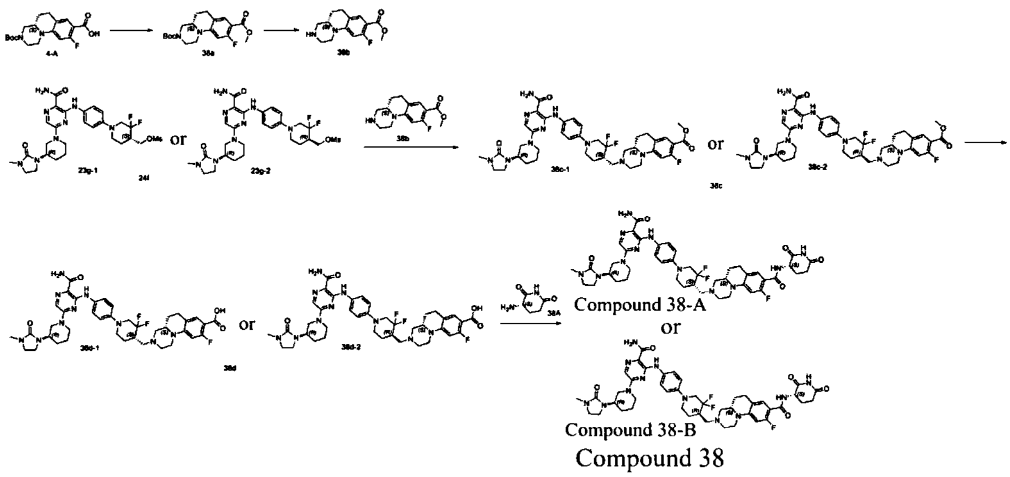


Compound 38

Starting from compound 4-A and following the synthetic method of example 37, compound 38 was obtained.

$^1$H NMR (400 MHz, $CDCl_3$) δ 10.56 (s, 1H), 8.05 - 7.92 (m, 1H), 7.75 - 7.31 (m, 6H), 6.60 - 6.30 (m, 3H), 5.12 (s, 1H), 4.87 - 4.70 (m, 1H), 4.37 - 4.20 (m, 2H), 3.90 - 3.57 (m, 5H), 3.43 - 3.23 (m, 4H), 3.20 - 2.43 (m, 16H), 2.40 - 1.55 (m, 13H).

Compound 38 is one of the isomers of compound 38-A or 38-B.

**Example 39: Preparation of compound 39**

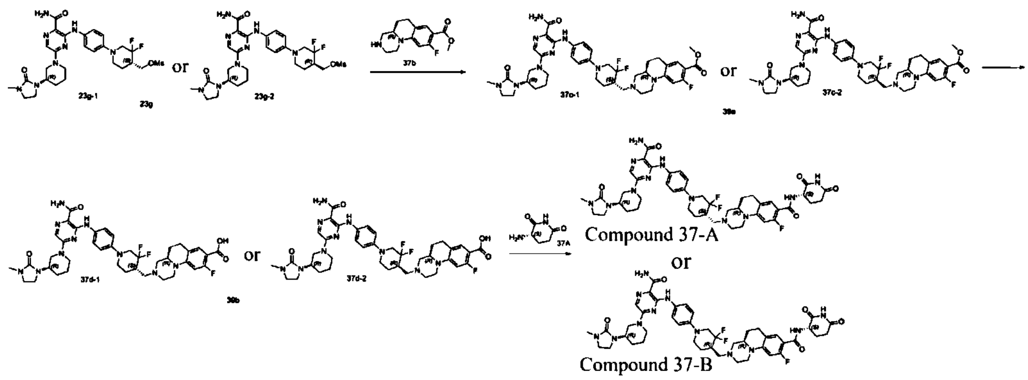


Compound 39

Starting from compound 23g and following the synthetic method of example 37, compound 39 was obtained.

$^1$H NMR (400 MHz, $CDCl_3$) δ 10.55 (s, 1H), 8.05 - 7.90 (m, 1H), 7.75 - 7.31 (m, 6H), 6.60 - 6.30 (m, 3H), 5.30 - 5.05 (m, 1H), 4.85 - 4.70 (m, 1H), 4.52 - 4.22 (m, 2H), 3.90 - 3.55 (m, 4H), 3.45 - 3.21 (m, 5H), 3.21 - 2.43 (m, 16H), 2.40 - 1.55 (m, 13H).

Compound 39 is one of the isomers of compound 37-A or 37-B.

**Example 40: Preparation of compound 40**

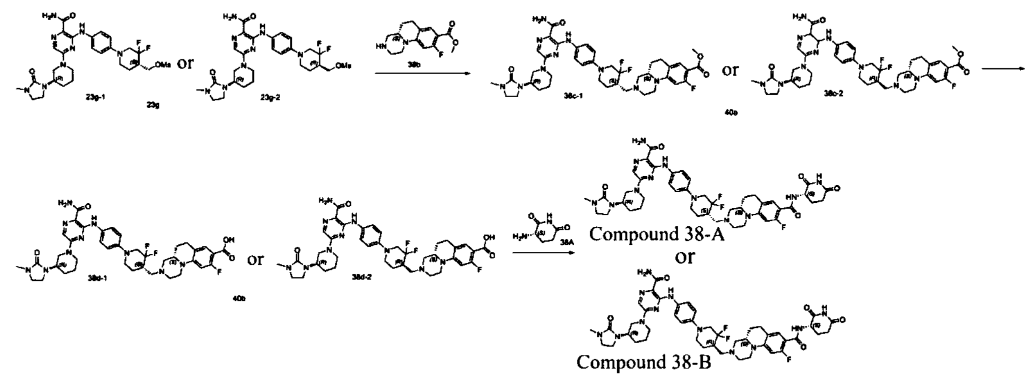

Compound 38-A

or

Compound 38-B

Compound 40

Starting from compounds 23g and 38b and following the synthetic method of example 37, compound 40 was obtained.

$^1$H NMR (400 MHz, $CDCl_3$) δ 10.57 (s, 1H), 7.93 (s, 1H), 7.75 - 7.31 (m, 6H), 6.60 - 6.30 (m, 3H), 5.15 (s, 1H), 4.87 - 4.68 (m, 1H), 4.37 - 4.20 (m, 2H), 3.90 - 3.55 (m, 6H), 3.45 - 3.23 (m, 5H), 3.21 - 2.57 (m, 16H), 2.30 - 1.55 (m, 11H).

Compound 40 is one of the isomers of compound 38-A or 38-B.

**Example 41: Preparation of trifluoroacetate of compound 41**

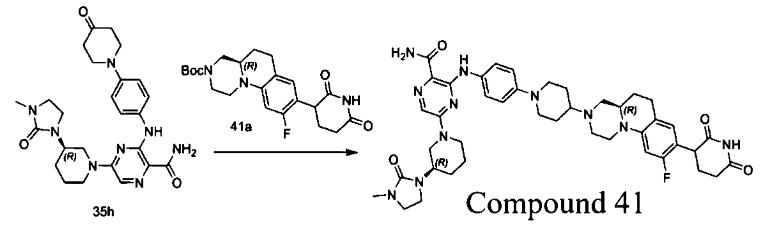

35h

41a

Compound 41

41a (90 mg, 0.22 mmol) (see WO 2023232133 for the synthetic method) was added to a single-necked flask (50 mL), followed by dichloromethane (5 mL) and trifluoroacetic acid (2 mL), and the mixture was reacted at room temperature for 5 h. The reaction system was concentrated under reduced pressure, DMA (5 mL), 35h (120 mg, 0.24 mmol) and sodium bicarbonate (100 mg, 1.19 mmol) were added, and the mixture was stirred at room temperature for 30 min. Acetic acid (36 mg, 0.60 mmol) was added, and the mixture was reacted at 30°C for 2 h. Sodium triacetoxyborohydride (85 mg, 0.40 mmol) was then added, and the resulting mixture was reacted at room temperature for 16 h. To the reaction system was added water (30 mL), and the mixture was extracted with dichloromethane (50 mL×3). The organic phase was washed with a saturated aqueous sodium chloride solution (50 mL×2), dried over anhydrous sodium sulphate, and concentrated under reduced pressure. The crude product was subjected to Pre-HPLC (instrument and preparative column: using Waters 2767 preparative liquid phase chromatographic instrument; preparative column model: SunFire@Prep C18, 5 μm, inner diameter × length = 19 mm×250 mm). Preparative method: a solution of the crude product in DMSO was filtered through a 0.45 μm filter membrane to prepare a sample solution. Mobile phase system: water (containing 0.1% TFA)/acetonitrile. Gradient elution method: gradient elution with acetonitrile from 10% to 50% (elution time: 15 min). Lyophilisation was performed to obtain the trifluoroacetate of compound 41 (12 mg).

$^1$H NMR (400 MHz, DMSO-$d_6$) δ 10.97 (s, 1H), 10.76 (s, 1H), 7.69 (s, 1H), 7.60 (s, 1H), 7.47 - 7.35 (m, 2H), 7.25 (s, 1H), 6.94 - 6.84 (m, 2H), 6.79 (d, 1H), 6.62 (d, 1H), 4.44 - 4.18 (m, 2H), 3.88 - 3.55 (m, 5H), 3.50 - 3.10 (m, 4H), 3.10 - 2.85 (m, 5H), 2.80 - 2.55 (m, 9H), 2.55 - 2.45 (m, 1H), 2.41 - 2.22 (m, 2H), 2.20 - 1.70 (m, 9H), 1.68 - 1.44 (m, 4H).

**Example 42: Preparation of compound 42**

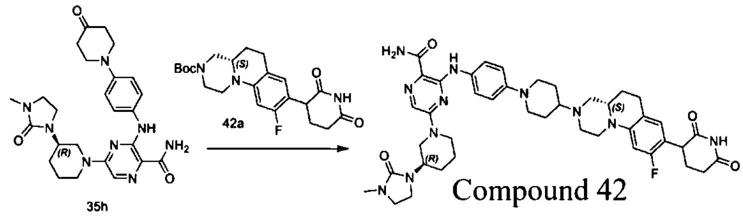


Starting from compound 42a (see WO 2023232133 for the synthetic method) and following the synthetic method of example 41, compound 42 was obtained by neutral preparative chromatography [mobile phase system: water (containing 5 mmol/L ammonium acetate)/acetonitrile] and lyophilisation.

$^1$H NMR (400 MHz, DMSO-$d_6$) δ 10.97 (s, 1H), 10.76 (s, 1H), 7.69 (s, 1H), 7.60 (s, 1H), 7.47 - 7.35 (m, 2H), 7.25 (s, 1H), 6.94 - 6.84 (m, 2H), 6.79 (d, 1H), 6.62 (d, 1H), 4.44 - 4.18 (m, 2H), 3.88 - 3.55 (m, 5H), 3.50 - 3.10 (m, 4H), 3.10 - 2.85 (m, 5H), 2.80 - 2.55 (m, 9H), 2.55 - 2.45 (m, 1H), 2.41 - 2.22 (m, 2H), 2.20 - 1.70 (m, 9H), 1.68 - 1.44 (m, 4H).

**Example 43: Preparation of compound 43**

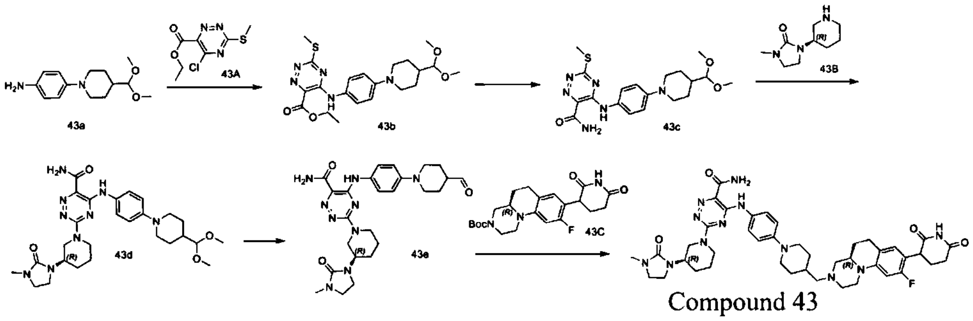


Step 1: Preparation of 43b

43A (2.34 g, 10.00 mmol) and 43a (3.01 g, 12.01 mmol) (see WO 2023083194 for the synthetic method) were dissolved in DCM (20 mL), DIPEA (1.68 g, 13.0 mmol) was slowly added dropwise, and the mixture was reacted at room temperature for 10 min. The reaction system was concentrated under reduced pressure, and the crude product was separated and purified by silica gel column chromatography (petroleum ether : ethyl acetate (v/v) = 1 : 1) to obtain 43b (4.4 g, yield: 98%).

LCMS m/z = 448.4 $[M+1]^+$

Step 2: Preparation of 43c

43b (4.4 g, 9.83 mmol) was added to methanol (30 mL), followed by slowly dropwise addition of 7 mol/L ammonia methanol solution (80 mL), and the mixture was reacted at room temperature for 3 h. The reaction system was filtered, and the filter cake was collected, washed with petroleum ether (30 mL), and dried under reduced pressure to obtain crude 43c (3.8 g).

LCMS m/z = 419.1 $[M+1]^+$

Step 3: Preparation of 43d

The above-mentioned crude 43c (1.0 g) was dissolved in NMP (20 mL), m-chloroperoxybenzoic acid (1.45 g, 8.4 mmol) was added, and the mixture was reacted at room temperature for 16 h. To the reaction system were added DIPEA (1.15 g, 8.90 mmol) and 43B (525 mg, 2.86 mmol) (see WO 2023088406 for the synthetic method), and the mixture was reacted at 80°C for 4 h. The reaction system was cooled to room temperature, water (60 mL) was added, and the mixture was extracted with ethyl acetate (80 mL×2). The organic phases were combined, washed with water (100 mL×2), dried over anhydrous sodium sulphate, and concentrated under reduced pressure. The crude product was separated and purified by silica gel column chromatography (DCM : MeOH (v/v) = 15 : 1) to obtain 43d (0.6 g, yield: 38%).

LCMS m/z = 554.7 $[M+1]^+$

Step 4: Preparation of trifluoroacetate of 43e

43d (0.3 g, 0.54 mmol) was dissolved in DCM (10 mL), trifluoroacetic acid (2 mL) was added, and the mixture was reacted at room temperature for 3 h. The reaction system was concentrated under reduced pressure to obtain the trifluoroacetate of crude 43e (0.35 g).
LCMS m/z = 508.8 $[M+1]^+$

Step 5: Preparation of compound 43

43C (120 mg, 0.29 mmol) (see WO 2023232133 for the synthetic method) was added to a single-necked flask (50 mL), followed by dichloromethane (5 mL) and trifluoroacetic acid (2 mL), and the mixture was reacted at room temperature for 5 h. The reaction system was concentrated under reduced pressure, DMA (5 mL), the above-mentioned trifluoroacetate of crude 43e (175 mg) and sodium bicarbonate (87 mg, 1.04 mmol) were added, and the mixture was stirred at room temperature for 30 min. Acetic acid (47 mg, 0.78 mmol) was added, and the mixture was reacted at 30°C for 2 h. Sodium triacetoxyborohydride (170 mg, 0.80 mmol) was then added, and the resulting mixture was reacted at room temperature for 16 h. To the reaction system was added water (30 mL), and the mixture was extracted with dichloromethane (50 mL×3). The organic phase was washed with a saturated aqueous sodium chloride solution (50 mL×2), dried over anhydrous sodium sulphate, and concentrated under reduced pressure. The crude product was subjected to Pre-HPLC (instrument and preparative column: using Waters 2767 preparative liquid phase chromatographic instrument; preparative column model: SunFire@Prep C18, 5 μm, inner diameter × length = 19 mm×250 mm). Preparative method: a solution of the crude product in DMSO was filtered through a 0.45 μm filter membrane to prepare a sample solution. Mobile phase system: water (containing 10 mmol/L ammonium bicarbonate)/acetonitrile. Gradient elution method: gradient elution with acetonitrile from 10% to 50% (elution time: 15 min). Lyophilisation was performed to obtain compound 43 (55 mg, yield: 23%).
$^1H$ NMR (400 MHz, DMSO-$d_6$) δ 11.07 (s, 1H), 10.76 (s, 1H), 8.28 (s, 1H), 7.66 (s, 1H), 7.57 - 7.42 (m, 2H), 7.00 - 6.87 (m, 2H), 6.79 (d, 1H), 6.62 (d, 1H), 5.00 - 4.17 (m, 2H), 3.88 - 3.52 (m, 5H), 3.41 - 3.18 (m, 5H), 3.17 - 2.80 (m, 5H), 2.80 - 2.54 (m, 9H), 2.54 - 2.42 (m, 1H), 2.25 - 1.40 (m, 14H), 1.32 - 1.12 (m, 2H).
LCMS m/z = 809.4 $[M+1]^+$

**Example 44: Preparation of compound 44**

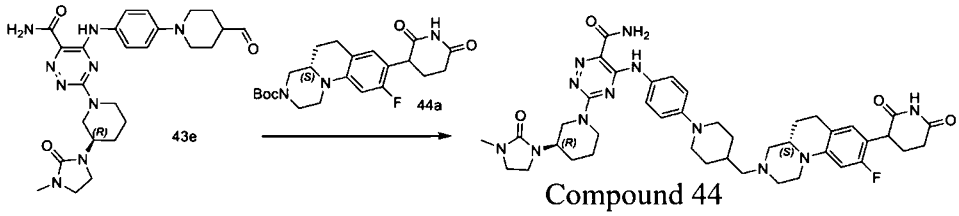


Compound 44

Starting from compound 44a (see WO 2023232133 for the synthetic method) and following the synthetic method of example 43, compound 44 was obtained.
$^1H$ NMR (400 MHz, DMSO-$d_6$) δ 11.07 (s, 1H), 10.76 (s, 1H), 8.28 (s, 1H), 7.67 (s, 1H), 7.55 - 7.44 (m, 2H), 7.00 - 6.87 (m, 2H), 6.79 (d, 1H), 6.62 (d, 1H), 5.00 - 4.20 (m, 2H), 3.88 - 3.52 (m, 5H), 3.42 - 3.18 (m, 5H), 3.16 - 2.83 (m, 5H), 2.78 - 2.54 (m, 9H), 2.54 - 2.43 (m, 1H), 2.25 - 1.99 (m, 4H), 1.99 - 1.65 (m, 8H), 1.65 - 1.43 (m, 2H), 1.33 - 1.12 (m, 2H).
LCMS m/z = 809.3 $[M+1]^+$

**Example 45: Preparation of trifluoroacetate of compound 45**

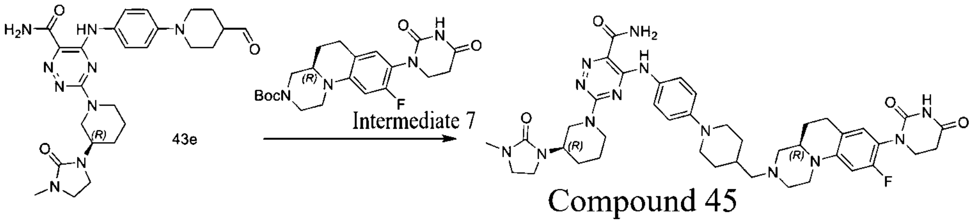

43e

Compound 45

Intermediate 7 (200 mg, 0.48 mmol) was added to a single-necked flask (50 mL), followed by dichloromethane (10 mL) and 4 mol/L solution of hydrogen chloride in 1,4-dioxane (10 mL), and the mixture was reacted at room temperature for 2 h. The reaction system was concentrated under reduced pressure, the above-mentioned trifluoroacetate of crude 43e (175 mg), sodium bicarbonate (87 mg, 1.04 mmol) and DMA (5 mL) were added, and the mixture was stirred at room temperature for 30 min. Acetic acid (47 mg, 0.78 mmol) was added, and the mixture was reacted at 30°C for 2 h. Sodium triacetoxyborohydride (170 mg, 0.80 mmol) was then added, and the resulting mixture was reacted at room temperature for 16 h. To the reaction system was added water (30 mL), and the mixture was extracted with dichloromethane (50 mL×3). The organic phase was washed with a saturated aqueous sodium chloride solution (50 mL×2), dried over anhydrous sodium sulphate, and concentrated under reduced pressure. The crude product was subjected to Pre-HPLC (instrument and preparative column: using Waters 2767 preparative liquid phase chromatographic instrument; preparative column model: SunFire@Prep C18, 5 μm, inner diameter × length = 19 mm × 250 mm). Preparative method: a solution of the crude product in DMSO was filtered through a 0.45 μm filter membrane to prepare a sample solution. Mobile phase system: water (containing 0.1% TFA)/acetonitrile. Gradient elution method: gradient elution with acetonitrile from 10% to 50% (elution time: 15 min). Lyophilisation was performed to obtain the trifluoroacetate of compound 45 (35 mg).

$^1H$ NMR (400 MHz, DMSO-$d_6$) δ 11.31 (s, 1H), 10.37 (s, 1H), 8.33 (s, 1H), 7.81 (s, 1H), 7.64 - 7.54 (m, 2H), 7.15 - 6.97 (m, 3H), 6.90 (d, 1H), 4.90 - 4.25 (m, 2H), 4.20 - 4.05 (m, 1H), 3.78 - 3.55 (m, 7H), 3.48 - 2.60 (m, 21H), 2.18 - 1.30 (m, 11H).

LCMS m/z = 810.4 $[M+1]^+$

**Example 46: Preparation of trifluoroacetate of compound 46**

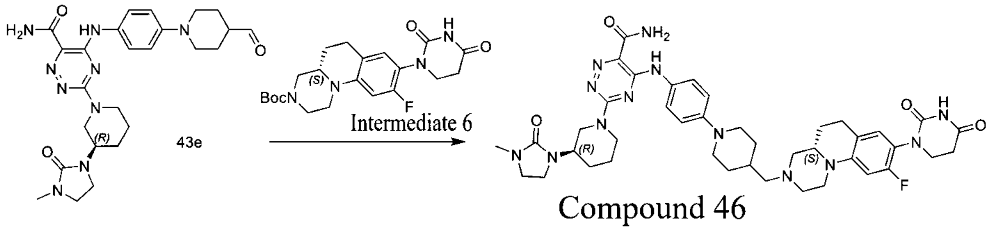

43e

Compound 46

Starting from intermediate 6 and following the synthetic method of example 45, the trifluoroacetate of compound 46 was obtained.

$^1H$ NMR (400 MHz, DMSO-$d_6$) δ 11.31 (s, 1H), 10.37 (s, 1H), 8.34 (s, 1H), 7.81 (s, 1H), 7.64 - 7.54 (m, 2H), 7.15 - 6.97 (m, 3H), 6.91 (d, 1H), 4.90 - 4.30 (m, 2H), 4.20 - 4.05 (m, 1H), 3.78 - 3.55 (m, 7H), 3.48 - 2.60 (m, 21H), 2.18 - 1.30 (m, 11H).

LCMS m/z = 810.4 $[M+1]^+$

**Example 47: Preparation of compound 47**

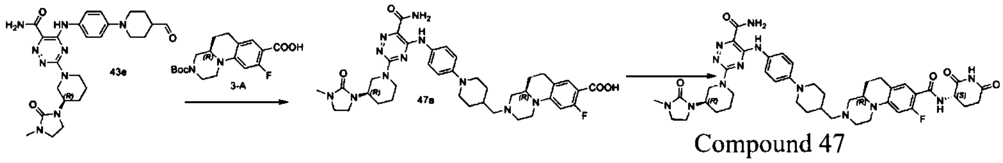
43e

3-A

47a

Compound 47

Step 1: Preparation of 47a

3-A (110 mg, 0.31 mmol) was added to a single-necked flask (50 mL), followed by dichloromethane (5 mL) and trifluoroacetic acid (2 mL), and the mixture was reacted at room temperature for 5 h. The reaction system was concentrated under reduced pressure, DMA (5 mL), the above-mentioned trifluoroacetate of crude 43e (180 mg) and sodium bicarbonate (91 mg, 1.08 mmol) were added, and the mixture was stirred at room temperature for 30 min. Acetic acid (64 mg, 1.07 mmol) was added, and the mixture was reacted at 30°C for 2 h. Sodium triacetoxyborohydride (115 mg, 0.54 mmol) was then added, and the resulting mixture was reacted at room temperature for 16 h. To the reaction system was added water (30 mL), and the mixture was extracted with dichloromethane (50 mL×3). The organic phase was washed with a saturated aqueous sodium chloride solution (50 mL×2), dried over anhydrous sodium sulphate, and concentrated under reduced pressure. The crude product was separated and purified by silica gel column chromatography (dichloromethane : methanol (v/v) = 12 : 1) to obtain 47a (150 mg, yield: 65%).
LCMS m/z = 742.4 $[M+1]^+$

Step 2: Preparation of compound 47

47a (150 mg, 0.20 mmol) was added to a single-necked flask (50 mL), followed by N,N-dimethylformamide (5 mL), EDCI (77 mg, 0.40 mol), HOBt (54 mg, 0.40 mmol) and N-methylimidazole (99 mg, 1.20 mmol), and the mixture was stirred at room temperature for 30 min. (S)-3-Aminopiperidine-2,6-dione hydrochloride (66 mg, 0.4 mmol) was then added, and the resulting mixture was reacted at room temperature for 16 h. To the reaction system was added water (70 mL), and the mixture was extracted with dichloromethane (50 mL×3). The organic phase was washed with a saturated aqueous sodium chloride solution (50 mL×2), dried over anhydrous sodium sulphate, and concentrated under reduced pressure. The crude product was subjected to Pre-HPLC (instrument and preparative column: using Waters 2767 preparative liquid phase chromatographic instrument; preparative column model: SunFire@Prep C18, 5 μm, inner diameter × length = 19 mm × 250 mm). Preparative method: a solution of the crude product in DMSO was filtered through a 0.45 μm filter membrane to prepare a sample solution. Mobile phase system: water (containing 10 mmol/L ammonium bicarbonate)/acetonitrile. Gradient elution method: gradient elution with acetonitrile from 10% to 50% (elution time: 15 min). Lyophilisation was performed to obtain compound 47 (62 mg, yield: 36%).
$^1$H NMR (400 MHz, DMSO-$d_6$) δ 11.08 (s, 1H), 10.82 (s, 1H), 8.29 (s, 1H), 7.95 (t, 1H), 7.67 (s, 1H), 7.56 - 7.45 (m, 2H), 7.36 (d, 1H), 7.04 - 6.86 (m, 2H), 6.68 (d, 1H), 5.00 - 4.25 (m, 3H), 3.94 - 3.78 (m, 1H), 3.73 - 3.55 (m, 3H), 3.40 - 3.20 (m, 5H), 3.18 - 2.60 (m, 14H), 2.59 - 2.45 (m, 1H), 2.30 - 1.88 (m, 6H), 1.88 - 1.40 (m, 8H), 1.32 - 1.14 (m, 2H).
LCMS m/z = 852.4 $[M+1]^+$

**Example 48: Preparation of compound 48**

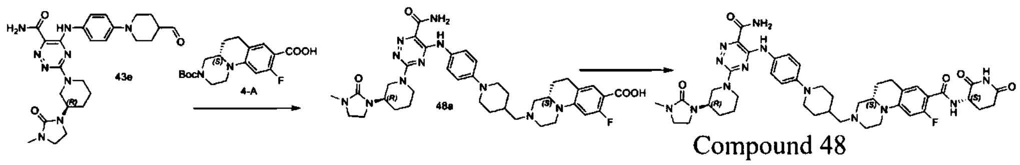

Compound 48

Starting from compound 4-A and following the synthetic method of example 47, compound 48 was obtained.
$^1$H NMR (400 MHz, DMSO-$d_6$) δ 11.07 (s, 1H), 10.82 (s, 1H), 8.28 (s, 1H), 7.95 (t, 1H), 7.67 (s, 1H), 7.56 - 7.45 (m, 2H), 7.36 (d, 1H), 7.04 - 6.86 (m, 2H), 6.68 (d, 1H), 5.00 - 4.25 (m, 3H), 3.94 - 3.78 (m, 1H), 3.73 - 3.55 (m, 3H), 3.40 - 3.20 (m, 5H), 3.18 - 2.60 (m, 14H), 2.59 - 2.45 (m, 1H), 2.30 - 1.88 (m, 6H), 1.88 - 1.40 (m, 8H), 1.32 - 1.14 (m, 2H).
LCMS m/z = 852.3 $[M+1]^+$

**Example 49: Preparation of compound 49**

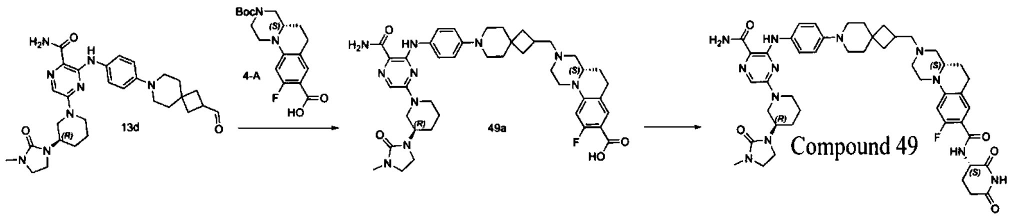


Step 1: Preparation of 49a

4-A (64 mg, 0.18 mmol) was added to a single-necked flask (50 mL), followed by dichloromethane (12 mL) and trifluoroacetic acid (4 mL), and the mixture was reacted at room temperature for 2 h. The reaction system was concentrated under reduced pressure, N,N-dimethylacetamide (10 mL) and 13d (100 mg, 0.18 mmol) were added, and the mixture was reacted at room temperature for 16 h. Sodium triacetoxyborohydride (76 mg, 0.36 mmol) was then added, and the resulting mixture was reacted at room temperature for 2 h. To the reaction system was added water (60 mL), and the mixture was extracted with dichloromethane (70 mL×3). The organic phase was washed with a saturated aqueous sodium chloride solution (80 mL×2), dried over anhydrous sodium sulphate, and filtered. The filtrate was concentrated under reduced pressure, and the crude product was separated and purified by silica gel column chromatography (dichloromethane : methanol (v/v) = 91 : 9) to obtain 49a (48 mg, yield: 34%).
LCMS m/z = 781.5 $[M+1]^+$

Step 2: Preparation of compound 49

49a (48 mg, 0.061 mmol) was added to a single-necked flask (50 mL), followed by N,N-dimethylformamide (6 mL), 1-(3-dimethylaminopropyl)-3-ethylcarbodiimide hydrochloride (17.8 mg, 0.093 mmol), 1-hydroxybenzotriazole (12 mg, 0.089 mmol), and N-methylimidazole (26 mg, 0.32 mmol), and the mixture was stirred at room temperature for 30 min. (S)-3-Aminopiperidine-2,6-dione hydrochloride (20 mg, 0.12 mmol) was then added, and the resulting mixture was reacted at room temperature for 16 h. To the reaction system was added water (80 mL), and the mixture was extracted with dichloromethane (60 mL×3). The organic phase was washed with a saturated aqueous sodium chloride solution (60 mL×2), dried over anhydrous sodium sulphate, and filtered. The filtrate was concentrated under reduced pressure, and the crude product was purified by preparative thick-layer chromatography using a silica gel plate (dichloromethane : methanol (v/v) = 93 : 7) to obtain compound 49 (30 mg, yield: 55%).
$^1H$ NMR (400 MHz, $CDCl_3$) δ 10.63 (s, 1H), 7.93 (s, 1H), 7.67 (d, 1H), 7.54 - 7.32 (m, 5H), 6.98 - 6.85 (m, 2H), 6.40 (d, 1H), 5.14 (s, 1H), 4.85 - 4.72 (m, 1H), 4.40 - 4.20 (m, 2H), 3.85 - 3.65 (m, 2H), 3.43 - 3.23 (m, 5H), 3.18 - 2.55 (m, 20H), 2.50 - 1.45 (m, 17H).
LCMS m/z = 891.5 $[M+1]^+$

**Example 50: Preparation of compound 50**

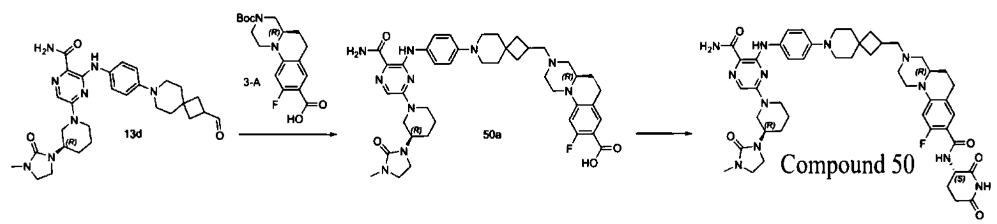


Starting from compound 3-A and following the synthetic method of example 49, compound 50 was obtained.
$^1H$ NMR (400 MHz, $CDCl_3$) δ 10.61 (s, 1H), 7.98 (s, 1H), 7.65 (d, 1H), 7.54 - 7.31 (m, 5H), 6.97 - 6.80 (m, 2H), 6.40 (d, 1H), 5.16 (s, 1H), 4.84 - 4.70 (m, 1H), 4.38 - 4.22 (m, 2H), 3.86 - 3.74 (m, 1H), 3.72 - 3.60 (m, 1H), 3.44 - 3.17 (m, 5H), 3.16 - 2.41 (m, 20H), 2.33 - 1.45 (m, 17H).
LCMS m/z = 891.5 $[M+1]^+$

**Example 51: Preparation of compound 51**

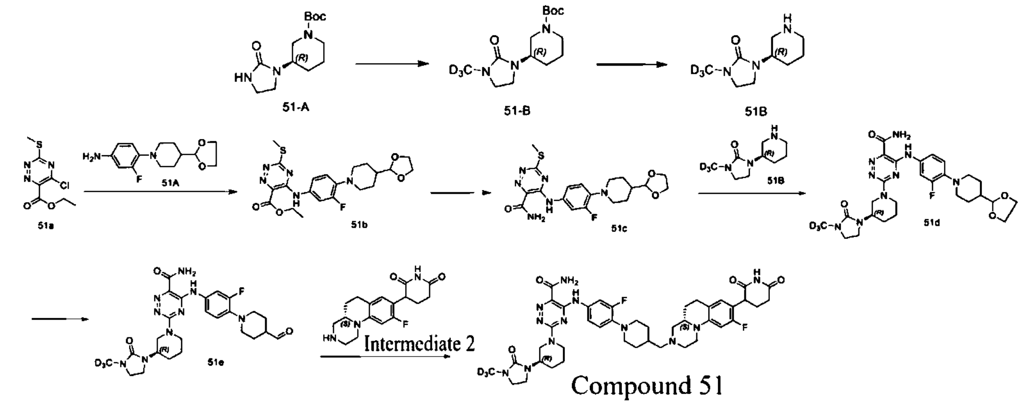


Step 1: Preparation of 51-B

51-A (21.0 g, 77.97 mmol) (see WO 2020081450 for the synthetic method) was dissolved in tetrahydrofuran (150 mL), 60% NaH (6.24 g) was added at 0°C, and the mixture was stirred for another 30 min at 0°C. Deuterated iodomethane (12.43 g, 85.75 mmol) was then added, and the resulting mixture was reacted at room temperature for 16 h. To the reaction solution was added water (200 mL), and the mixture was extracted three times with ethyl acetate (200 mL). The organic phases were combined, dried over anhydrous sodium sulphate, and concentrated under reduced pressure. The crude product was separated and purified by silica gel column chromatography (petroleum ether / ethyl acetate (v/v) = 1 : 0-1 : 1) to obtain 51-B (22.0 g, yield: 99%).

LCMS m/z = 287.3 $[M+1]^+$

Step 2: Preparation of hydrochloride of 51B

51-B (5.0 g, 17.46 mmol) was dissolved in dichloromethane (10 mL), 4 mol/L solution of hydrogen chloride in 1,4-dioxane (30 mL) was added, and the mixture was reacted at room temperature for 3 h. The reaction system was concentrated under reduced pressure to obtain the hydrochloride of crude 51B (4.0 g).

Step 3: Preparation of 51b

51a (2.15 g, 9.20 mmol) and 51A (2.94 g, 11.04 mmol) (see CN116143757 for the synthetic method) were dissolved in DCM (20 mL), DIPEA (1.55 g, 11.99 mmol) was slowly added dropwise, and the mixture was reacted at room temperature for 10 min after the addition. The reaction system was concentrated under reduced pressure, and the crude product was separated and purified by silica gel column chromatography (petroleum ether : ethyl acetate (v/v) = 1 : 1) to obtain 51b (4.0 g, yield: 94%).

LCMS m/z = 464.2 $[M+1]^+$

Step 4: Preparation of 51c

51b (4.0 g, 8.64 mmol) was added to methanol (30 mL), followed by slowly dropwise addition of 7 mol/L ammonia methanol solution (80 mL), and the mixture was reacted at room temperature for 3 h. The reaction system was filtered, and the filter cake was collected, washed with petroleum ether (30 mL), and dried under reduced pressure to obtain crude 51c (3.7 g).

Step 5: Preparation of 51d

To a reaction flask were respectively added the above-mentioned crude 51c (2.0 g), *m*-CPBA (4.38 g) and NMP (20 mL), and the mixture was stirred at room temperature for 16 h. DIPEA (2.79 g, 21.59 mmol) and the above-mentioned hydrochloride of crude 51B (1.54 g) were successively added, and the resulting mixture was reacted at 80°C for 16 h. The reaction system was cooled to room temperature, water (100 mL) was added, and the mixture was extracted with ethyl acetate (100 mL×4). The organic phases were combined, washed with a saturated aqueous sodium chloride solution (50 mL), dried over anhydrous sodium sulphate, and concentrated under reduced pressure. The crude product was separated and purified by silica gel column chromatography (dichloromethane/methanol (v/v) = 1 : 0-0 : 1) to obtain 51d (0.97 g, two-step yield from compound 51b: 36%).

LCMS m/z = 573.4 $[M+1]^+$

Step 6: Preparation of 51e

51d (0.97 g, 1.69 mmol) was dissolved in tetrahydrofuran (5 mL), 3 mol/L hydrochloric acid (7 mL) was added, and the mixture was reacted at 80°C for 3 h. The reaction system was cooled to room temperature, adjusted to pH 9 with a saturated aqueous sodium bicarbonate solution, and extracted with dichloromethane (50 mL×3). The organic phases were combined, dried over anhydrous sodium sulphate, and concentrated under reduced pressure to obtain crude 51e (1.52 g).
LCMS m/z = 529.3 $[M+1]^+$

Step 7: Preparation of compound 51

To a reaction flask were respectively added the above-mentioned crude 51e (0.25 g), the above-mentioned crude intermediate 2 (0.15 g), glacial acetic acid (0.2 mL) and DMAc (5 mL), and the mixture was reacted at room temperature for 1 h. Sodium triacetoxyborohydride (0.25 g, 1.18 mmol) was then added, and the resulting mixture was reacted at room temperature for 16 h. The reaction system was subjected to Pre-HPLC (instrument and preparative column: using SHIMADZ LC-20AP preparative liquid phase chromatographic instrument; preparative column model: C18). Preparative method: a solution of the crude product in DMSO was filtered through a 0.45 μm filter membrane to prepare a sample solution. Mobile phase system: water (containing 10 mmol/L ammonium bicarbonate)/acetonitrile. Gradient elution method: gradient elution with acetonitrile from 33% to 63% (elution time: 15 min). Lyophilisation was performed to obtain compound 51 (30.0 mg, two-step yield from compound 51d: 13%).
$^1$H NMR (400 MHz, $CDCl_3$) δ 10.88 (s, 1H), 8.38 (s, 1H), 7.73 (s, 1H), 7.54 (d, 1H), 7.21 (s, 1H), 6.95 (t, 1H), 6.70 (d, 1H), 6.48 (d, 1H), 5.73 (s, 1H), 5.25 - 4.20 (m, 2H), 3.85 - 3.67 (m, 2H), 3.67 - 3.52 (m, 1H), 3.52 - 3.24 (m, 6H), 3.24 - 2.50 (m, 12H), 2.35 - 2.07 (m, 5H), 2.05 - 1.55 (m, 10H), 1.55 - 1.30 (m, 2H).
LCMS m/z = 830.3 $[M+1]^+$

**Example 52: Preparation of compound 52**

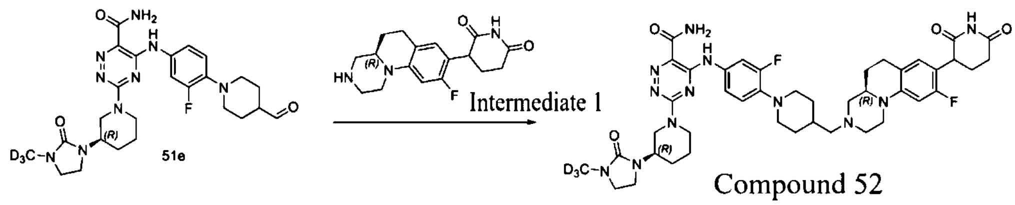


51e

Compound 52

Starting from compound 51e and intermediate 1 and following the synthetic method of example 51, compound 52 was obtained.
$^1$H NMR (400 MHz, DMSO-$d_6$) δ 11.26 (s, 1H), 10.75 (s, 1H), 8.32 (s, 1H), 7.77 - 7.22 (m, 3H), 7.03 (t, 1H), 6.79 (d, 1H), 6.62 (d, 1H), 5.10 - 4.05 (m, 2H), 3.88 - 3.76 (m, 1H), 3.76 - 3.66 (m, 1H), 3.66 - 3.52 (m, 1H), 3.40 - 3.20 (m, 6H), 3.19 - 2.83 (m, 5H), 2.82 - 2.54 (m, 6H), 2.54 - 2.44 (m, 1H), 2.28 - 2.00 (m, 4H), 2.00 - 1.44 (m, 11H), 1.38 - 1.18 (m, 2H).
LCMS m/z = 830.3 $[M+1]^+$

**Example 53: Preparation of compound 53**

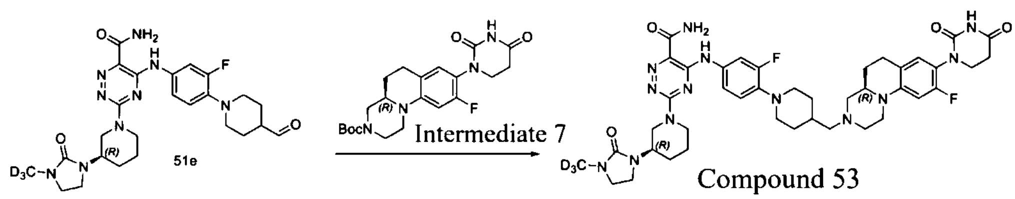


51e

Compound 53

Starting from compound 51e and intermediate 7 and following the synthetic method of example 45, compound 53 was obtained by neutral preparative chromatography [water (containing 10 mmol/L ammonium bicarbonate)/acetonitrile].
$^1$H NMR (400 MHz, DMSO-$d_6$) δ 11.26 (s, 1H), 10.33 (s, 1H), 8.32 (s, 1H), 7.72 (s, 1H), 7.65 - 7.20 (m, 2H), 7.03 (t,

1H), 6.92 (d, 1H), 6.72 (d, 1H), 5.10 - 4.20 (m, 2H), 3.80 - 3.68 (m, 1H), 3.67 - 3.50 (m, 3H), 3.40 - 3.20 (m, 6H), 3.20 - 2.82 (m, 5H), 2.82 - 2.50 (m, 7H), 2.27 - 2.14 (m, 2H), 2.12 - 1.97 (m, 1H), 1.96 - 1.43 (m, 10H), 1.38 - 1.16 (m, 2H).

LCMS m/z = 831.4 $[M+1]^+$

**Example 54: Preparation of compound 54**

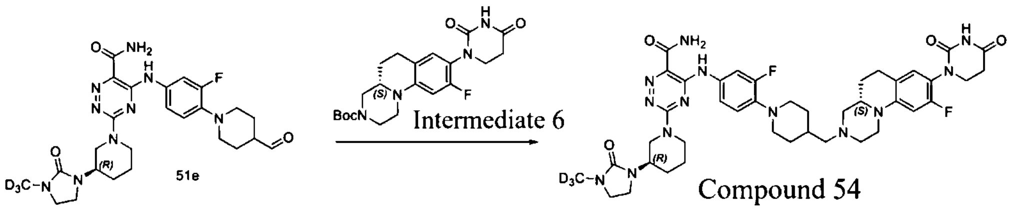


51e

Compound 54

Starting from compound 51e and intermediate 6 and following the synthetic method of example 45, compound 54 was obtained by neutral preparative chromatography [water (containing 10 mmol/L ammonium bicarbonate)/acetonitrile].

$^1$H NMR (400 MHz, DMSO-$d_6$) δ 11.26 (s, 1H), 10.32 (s, 1H), 8.32 (s, 1H), 7.71 (s, 1H), 7.65 - 7.23 (m, 2H), 7.03 (t, 1H), 6.93 (d, 1H), 6.71 (d, 1H), 5.10 - 4.20 (m, 2H), 3.80 - 3.69 (m, 1H), 3.67 - 3.54 (m, 3H), 3.40 - 3.20 (m, 6H), 3.20 - 2.85 (m, 5H), 2.82 - 2.50 (m, 7H), 2.30 - 2.15 (m, 2H), 2.14 - 1.98 (m, 1H), 1.96 - 1.45 (m, 10H), 1.40 - 1.18 (m, 2H).

LCMS m/z = 831.4 $[M+1]^+$

**Example 55: Preparation of compound 55**

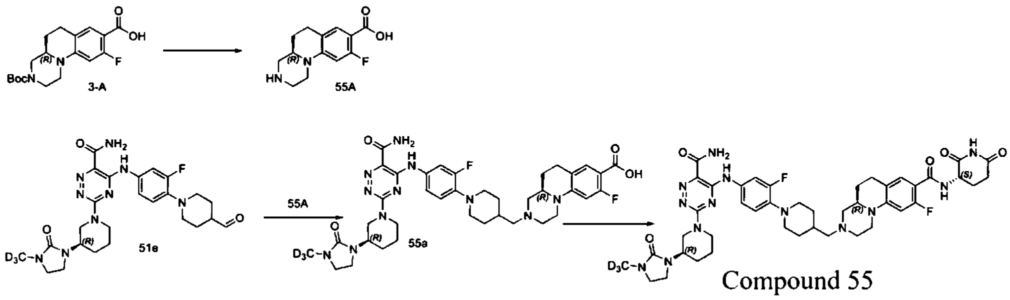


3-A, 55A, 51e, 55a

Compound 55

Step 1: Preparation of trifluoroacetate of 55A

3-A (0.329 g, 0.94 mmol) was added to a reaction flask, and dissolved in dichloromethane (10 mL), trifluoroacetic acid (3 mL) was added, and the mixture was reacted at room temperature for 2 h. The reaction system was concentrated under reduced pressure to obtain the trifluoroacetate of crude 55A (1.5 g).

Step 2: Preparation of 55a

To a reaction flask were respectively added the above-mentioned crude 51e (0.52 g), the above-mentioned trifluoroacetate of crude 55A (0.30 g), glacial acetic acid (0.2 mL) and DMAc (5 mL), and the mixture was reacted at room temperature for 1 h. Sodium triacetoxyborohydride (0.5 g, 2.36 mmol) was then added, and the resulting mixture was reacted at room temperature for 16 h. To the reaction solution was added water (20 mL), with the solids precipitated out. The mixture was filtered by suction, and the filter cake was separated and purified by silica gel column chromatography (methanol/dichloromethane (v/v) = 0 : 1-1 : 9) to obtain 55a (0.14 g, two-step yield from compound 3-A: 98%).

LCMS m/z = 763.5 $[M+1]^+$

Step 3: Preparation of compound 55

To a reaction flask were respectively added 55a (0.14 g, 0.18 mmol), (S)-3-aminopiperidine-2,6-dione hydrochloride (0.059 g, 0.36 mmol), EDCI (0.069 g, 0.36 mmol), HOBt (0.049 g, 0.36 mmol), N-methylmorpholine (0.055 g, 0.54

mmol) and DMF (4 mL), and the mixture was reacted at room temperature for 16 h. To the reaction solution was added water (20 mL), and the mixture was extracted with dichloromethane (50 mL×3). The organic phases were combined, dried over anhydrous sodium sulphate, and concentrated under reduced pressure. The crude product was subjected to Pre-HPLC (instrument and preparative column: using SHIMADZ LC-20AP preparative liquid phase chromatographic instrument; preparative column model: C18). Preparative method: a solution of the crude product in DMSO was filtered through a 0.45 μm filter membrane to prepare a sample solution. Mobile phase system: water (containing 10 mmol/L ammonium bicarbonate)/acetonitrile. Gradient elution method: gradient elution with acetonitrile from 33% to 63% (elution time: 15 min). Lyophilisation was performed to obtain compound 55 (52.0 mg, yield: 33%).

$^1$H NMR (400 MHz, DMSO-$d_6$) δ 11.26 (s, 1H), 10.81 (s, 1H), 8.38 - 8.27 (m, 1H), 7.93 (t, 1H), 7.71 (s, 1H), 7.64 - 7.25 (m, 3H), 7.03 (t, 1H), 6.67 (d, 1H), 5.00 - 4.20 (m, 3H), 3.93 - 3.76 (m, 1H), 3.70 - 3.52 (m, 1H), 3.40 - 3.20 (m, 6H), 3.19 - 2.57 (m, 12H), 2.57 - 2.47 (m, 1H), 2.28 - 1.46 (m, 14H), 1.40 - 1.20 (m, 2H).

LCMS m/z = 873.4 [M+1]$^+$

**Example 56: Preparation of compound 56**

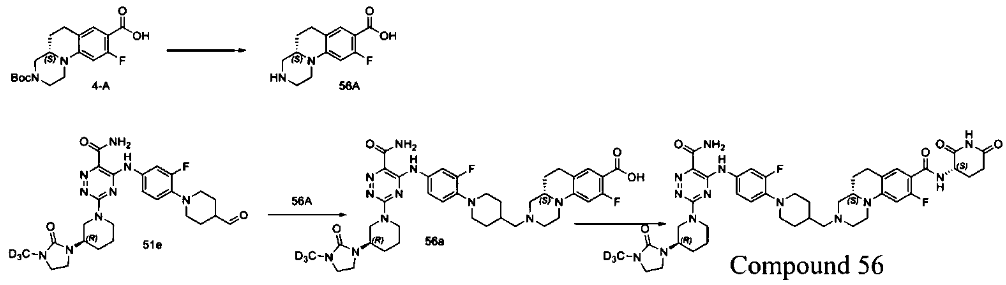


Starting from compounds 51e and 4-A and following the synthetic method of example 55, compound 56 was obtained.

$^1$H NMR (400 MHz, DMSO-$d_6$) δ 11.26 (s, 1H), 10.81 (s, 1H), 8.38 - 8.27 (m, 1H), 7.93 (t, 1H), 7.71 (s, 1H), 7.64 - 7.25 (m, 3H), 7.04 (t, 1H), 6.68 (d, 1H), 5.05 - 4.20 (m, 3H), 3.95 - 3.76 (m, 1H), 3.70 - 3.52 (m, 1H), 3.40 - 3.20 (m, 6H), 3.19 - 2.57 (m, 12H), 2.57 - 2.47 (m, 1H), 2.28 - 1.46 (m, 14H), 1.40 - 1.20 (m, 2H).

LCMS m/z = 873.4 [M+1]$^+$

**Example 57: Preparation of compound 57**

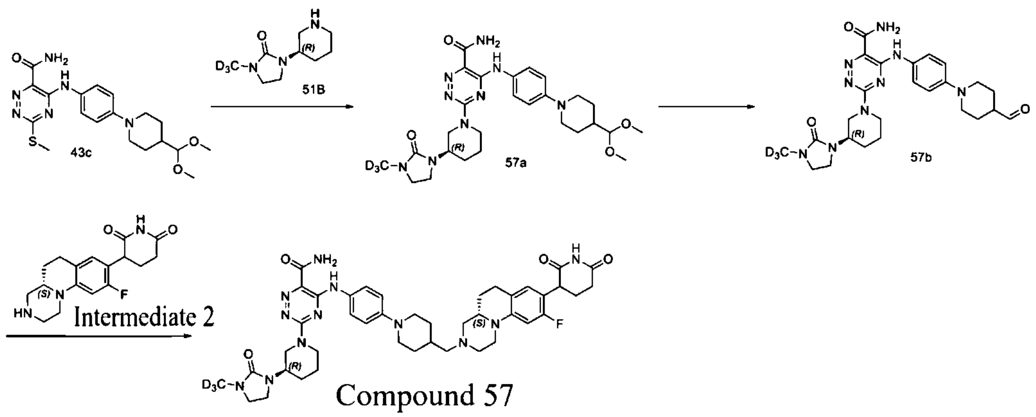


Step 1: Preparation of 57a

To a reaction flask were respectively added 43c (2.0 g, 4.78 mmol), m-CPBA (4.85 g) and NMP (20 mL), and the mixture was stirred at room temperature for 16 h. DIPEA (1.85 g, 14.31 mmol) and the above-mentioned hydrochloride of crude 51B (1.60 g) were successively added, and the resulting mixture was reacted at 80°C for 16 h. The reaction system

was cooled to room temperature, water (100 mL) was added, and the mixture was extracted with ethyl acetate (100 mL×4). The organic phases were combined, washed with a saturated aqueous sodium chloride solution (50 mL), dried over anhydrous sodium sulphate, and concentrated under reduced pressure. The crude product was separated and purified by silica gel column chromatography (dichloromethane/methanol (v/v) = 1 : 0-0 : 1) to obtain 57a (0.75 g, yield: 28%).

LCMS m/z = 557.4 $[M+1]^+$

Step 2: Preparation of trifluoroacetate of 57b

57a (0.75 g, 1.35 mmol) was dissolved in dichloromethane (5 mL), trifluoroacetic acid (2 mL) was added, and the mixture was reacted at room temperature for 3 h. The reaction system was concentrated under reduced pressure to obtain the trifluoroacetate of crude 57b (1.52 g).

Step 3: Preparation of compound 57

To a reaction flask were respectively added the above-mentioned trifluoroacetate of crude 57b (0.25 g), the above-mentioned crude intermediate 2 (0.15 g), glacial acetic acid (0.2 mL) and DMAc (5 mL), and the mixture was reacted at room temperature for 1 h. Sodium triacetoxyborohydride (0.25 g, 1.18 mmol) was then added, and the resulting mixture was reacted at room temperature for 16 h. The reaction system was subjected to Pre-HPLC (instrument and preparative column: using SHIMADZ LC-20AP preparative liquid phase chromatographic instrument; preparative column model: C18). Preparative method: a solution of the crude product in DMSO was filtered through a 0.45 μm filter membrane to prepare a sample solution. Mobile phase system: water (containing 10 mmol/L ammonium bicarbonate)/acetonitrile. Gradient elution method: gradient elution with acetonitrile from 35% to 65% (elution time: 15 min), lyophilisation was performed to obtain compound 57 (25.0 mg, two-step yield from compound 57a: 14%).

$^1H$ NMR (400 MHz, $CDCl_3$) δ 10.73 (s, 1H), 8.43 (s, 1H), 7.73 (s, 1H), 7.58 - 7.46 (m, 2H), 7.00 - 6.90 (m, 2H), 6.71 (d, 1H), 6.49 (d, 1H), 5.68 (s, 1H), 5.20 - 4.25 (m, 2H), 3.88 - 3.72 (m, 2H), 3.72 - 3.63 (m, 2H), 3.63 - 3.54 (m, 1H), 3.46 - 3.24 (m, 4H), 3.24 - 2.55 (m, 12H), 2.37 - 2.10 (m, 5H), 2.10 - 1.55 (m, 10H), 1.48 - 1.27 (m, 2H).

LCMS m/z = 406.8 $[M/2 +1]^+$

**Example 58: Preparation of compound 58**

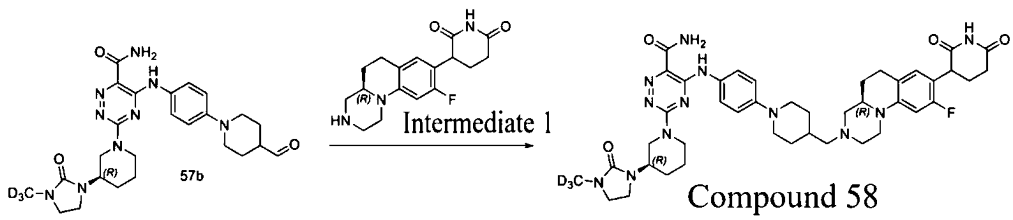


Starting from the trifluoroacetate of compound 57b and intermediate 1 and following the synthetic method of example 57, compound 58 was obtained.

$^1H$ NMR (400 MHz, DMSO-$d_6$) δ 11.08 (s, 1H), 10.76 (s, 1H), 8.28 (s, 1H), 7.67 (s, 1H), 7.55 - 7.42 (m, 2H), 7.00 - 6.87 (m, 2H), 6.79 (d, 1H), 6.61 (d, 1H), 5.10 - 4.20 (m, 2H), 3.88 - 3.76 (m, 1H), 3.76 - 3.52 (m, 4H), 3.40 - 3.17 (m, 5H), 3.15 - 2.82 (m, 5H), 2.82 - 2.54 (m, 6H), 2.54 - 2.42 (m, 1H), 2.25 - 1.40 (m, 14H), 1.30 - 1.14 (m, 2H).

LCMS m/z = 406.8 $[M/2 +1]^+$

**Example 59: Preparation of compound 59**

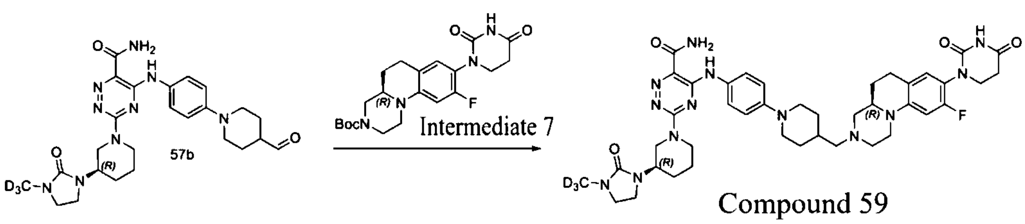

Starting from the trifluoroacetate of compound 57b and intermediate 7 and following the synthetic method of example 45, compound 59 was obtained by neutral preparative chromatography [water (containing 10 mmol/L ammonium bicarbonate)/acetonitrile].

$^1$H NMR (400 MHz, DMSO-$d_6$) δ 11.07 (s, 1H), 10.33 (s, 1H), 8.28 (s, 1H), 7.67 (s, 1H), 7.56 - 7.42 (m, 2H), 6.99 - 6.87 (m, 3H), 6.72 (d, 1H), 5.00 - 4.20 (m, 2H), 3.82 - 3.52 (m, 6H), 3.40 - 3.18 (m, 4H), 3.15 - 2.82 (m, 5H), 2.82 - 2.55 (m, 7H), 2.27 - 2.13 (m, 2H), 2.12 - 1.97 (m, 1H), 1.97 - 1.41 (m, 10H), 1.33 - 1.10 (m, 2H).

LCMS m/z = 813.4 [M+1]$^+$

**Example 60: Preparation of compound 60**

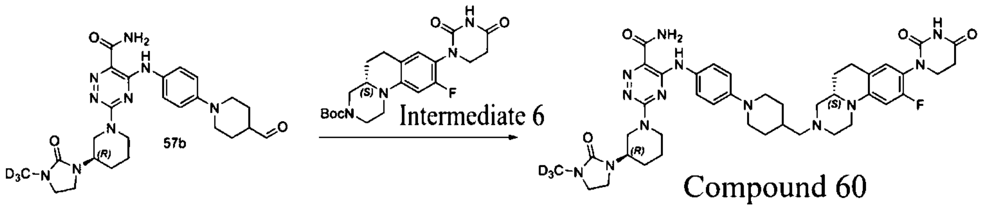


57b

Compound 60

Starting from the trifluoroacetate of compound 57b and intermediate 6 and following the synthetic method of example 45, compound 60 was obtained by neutral preparative chromatography [water (containing 10 mmol/L ammonium bicarbonate)/acetonitrile].

$^1$H NMR (400 MHz, DMSO-$d_6$) δ 11.07 (s, 1H), 10.32 (s, 1H), 8.28 (s, 1H), 7.66 (s, 1H), 7.54 - 7.46 (m, 2H), 7.00 - 6.86 (m, 3H), 6.72 (d, 1H), 5.05 - 4.15 (m, 2H), 3.82 - 3.71 (m, 1H), 3.71 - 3.53 (m, 5H), 3.39 - 3.18 (m, 4H), 3.15 - 2.83 (m, 5H), 2.81 - 2.54 (m, 7H), 2.27 - 1.43 (m, 13H), 1.33 - 1.13 (m, 2H).

LCMS m/z = 813.4 [M+1]$^+$

**Example 61: Preparation of compound 61**

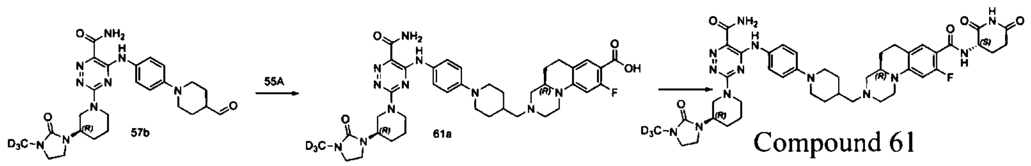


57b

61a

Compound 61

Step 1: Preparation of 61a

To a reaction flask were respectively added the trifluoroacetate of crude 57b (0.52 g), the above-mentioned trifluoroacetate of crude 55A (0.30 g), glacial acetic acid (0.2 mL) and DMAc (5 mL), and the mixture was reacted at room temperature for 1 h. Sodium triacetoxyborohydride (0.5 g, 2.36 mmol) was then added, and the resulting mixture was reacted at room temperature for 16 h. To the reaction solution was added water (20 mL), with the solids precipitated out. The mixture was filtered by suction, and the filter cake was separated and purified by silica gel column chromatography (methanol/dichloromethane (v/v) = 0 : 1-1 : 9) to obtain 61a (0.13 g, two-step yield from compound 3-A: 93%).

LCMS m/z = 745.5 [M+1]$^+$

Step 2: Preparation of compound 61

To a reaction flask were respectively added 61a (0.13 g, 0.175 mmol), (S)-3-aminopiperidine-2,6-dione hydrochloride (0.059 g, 0.36 mmol), EDCI (0.069 g, 0.36 mmol), HOBt (0.049 g, 0.36 mmol), N-methylmorpholine (0.055 g, 0.54 mmol) and DMF (4 mL), and the mixture was reacted at room temperature for 16 h. To the reaction solution was added water (20 mL), and the mixture was extracted three times with dichloromethane (50 mL). The organic phases were combined, dried over anhydrous sodium sulphate, and concentrated under reduced pressure. The crude product was subjected to Pre-HPLC (instrument and preparative column: using SHIMADZ LC-20AP preparative liquid phase chromatographic instrument; preparative column model: C18). Preparative method: a solution of the crude product in DMSO was filtered through a 0.45 μm filter membrane to prepare a sample solution. Mobile phase system: water

(containing 10 mmol/L ammonium bicarbonate)/acetonitrile. Gradient elution method: gradient elution with acetonitrile from 30% to 60% (elution time: 15 min). Lyophilisation was performed to obtain compound 61 (46.0 mg, yield: 15%).

$^{1}H$ NMR (400 MHz, DMSO-$d_6$) δ 11.07 (s, 1H), 10.80 (s, 1H), 8.27 (s, 1H), 7.93 (t, 1H), 7.66 (s, 1H), 7.55 - 7.43 (m, 2H), 7.36 (d, 1H), 7.00 - 6.88 (m, 2H), 6.67 (d, 1H), 5.10 - 4.20 (m, 3H), 3.93 - 3.77 (m, 1H), 3.73 - 3.52 (m, 3H), 3.40 - 3.18 (m, 4H), 3.18 - 2.58 (m, 11H), 2.57 - 2.47 (m, 1H), 2.25 - 1.44 (m, 15H), 1.32 - 1.13 (m, 2H).

LCMS m/z = 428.3 $[M/2 +1]^+$

**Example 62: Preparation of compound 62**

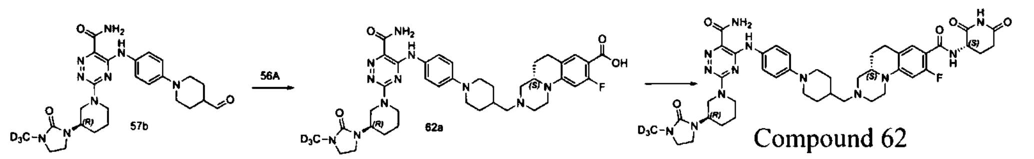


Starting from compounds 57b and 56A and following the synthetic method of example 61, compound 62 was obtained.

$^{1}H$ NMR (400 MHz, DMSO-$d_6$) δ 11.07 (s, 1H), 10.82 (s, 1H), 8.28 (s, 1H), 7.94 (t, 1H), 7.67 (s, 1H), 7.55 - 7.45 (m, 2H), 7.36 (d, 1H), 7.00 - 6.87 (m, 2H), 6.68 (d, 1H), 5.10 - 4.20 (m, 3H), 3.95 - 3.78 (m, 1H), 3.74 - 3.50 (m, 3H), 3.43 - 3.18 (m, 4H), 3.17 - 2.57 (m, 11H), 2.57 - 2.46 (m, 1H), 2.28 - 1.42 (m, 15H), 1.32 - 1.14 (m, 2H).

LCMS m/z = 428.3 $[M/2 +1]^+$

**Example 63: Preparation of trifluoroacetate of compound 63**

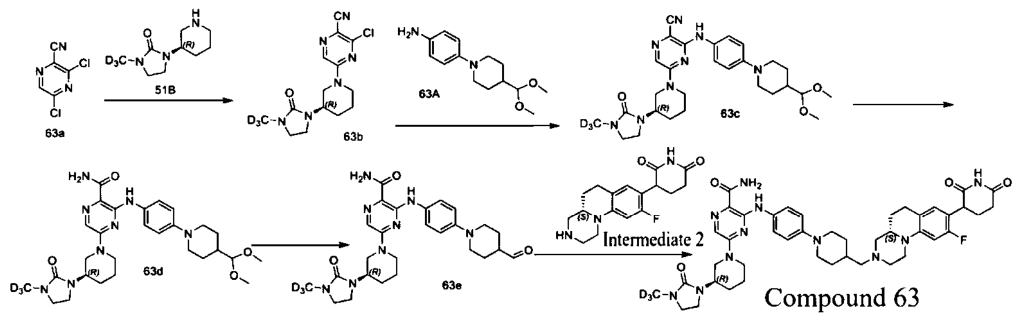


Step 1: Preparation of 63b

The above-mentioned hydrochloride of crude 51B (3.85 g) was dissolved in DMF (20 mL), DIPEA (6.68 g, 51.68 mmol) was added, and the mixture was stirred at room temperature for 10 min. 63a (3.00 g, 17.24 mmol) was then added, and the resulting mixture was stirred at room temperature for 16 h. To the reaction solution was added water (100 mL), and the mixture was extracted with ethyl acetate (80 mL×3). The organic phases were combined, dried over anhydrous sodium sulphate, and concentrated under reduced pressure. The crude product was separated and purified by silica gel column chromatography (petroleum ether / ethyl acetate (v/v) = 1 : 1-0 : 1) to obtain 63b (4.60 g, yield: 83%).

LCMS m/z = 324.2 $[M+1]^+$

Step 2: Preparation of 63c

63b (2.10 g, 6.50 mmol) and 63A (1.95 g, 7.79 mmol) (see WO 2023083194 for the synthetic method) were dissolved in 1,4-dioxane (40 mL), $Pd(OAc)_2$ (150 mg, 0.67 mmol), BINAP (604 mg, 0.97 mmol) and caesium carbonate (6.34 g, 19.46 mmol) were respectively added, and the mixture was purged three times with nitrogen and reacted at 100°C for 16 h. The reaction system was cooled to room temperature, and filtered. The filtrate was concentrated under reduced pressure, and dichloromethane (100 mL) and water (100 mL) were added to the residue. After phase separation, the aqueous phase was extracted with dichloromethane (100 mL×3), and the organic phases were combined, dried over

anhydrous sodium sulphate, and concentrated under reduced pressure. The crude product was separated and purified by silica gel column chromatography (dichloromethane/methanol (v/v) = 1 : 0-9 : 1) to obtain 63c (2.80 g, yield: 80%).

LCMS m/z = 538.3 $[M+1]^+$

Step 3: Preparation of 63d

63c (2.80 g, 5.21 mmol) was dissolved in DMSO (5 mL), caesium carbonate (1.70 g, 5.22 mmol) was added, followed by slowly dropwise addition of 30% hydrogen peroxide solution (8 mL), and the mixture was reacted at room temperature for 4 h. To the reaction system was added water (10 mL), and the mixture was filtered. The filter cake was collected, washed with water (20 mL), and dried under reduced pressure to obtain crude 63d (2.50 g).

LCMS m/z = 556.4 $[M+1]^+$

Step 4: Preparation of trifluoroacetate of 63e

The above-mentioned crude 63d (1.0 g) was dissolved in dichloromethane (5 mL), trifluoroacetic acid (3 mL) was added, and the mixture was reacted at room temperature for 3 h. The reaction solution was concentrated under reduced pressure to obtain the trifluoroacetate of crude 63e (1.5 g).

LCMS m/z = 510.3 $[M+1]^+$

Step 5: Preparation of trifluoroacetate of compound 63

The above-mentioned trifluoroacetate of crude 63e (150 mg) and the above-mentioned crude intermediate 2 (76 mg) were dissolved in DMAc (5 mL), glacial acetic acid (0.2 mL) was added, and the mixture was stirred at room temperature for 1 h. Sodium triacetoxyborohydride (130 mg, 0.61 mmol) was then added portionwise, and the resulting mixture was reacted at room temperature for 16 h. The reaction solution was subjected to Pre-HPLC (instrument and preparative column: using SHIMADZU LC-20AP preparative liquid phase chromatographic instrument, preparative column model: SunFire@Prep C18, 5 μm, inner diameter × length = 19 mm×250 mm). Preparative method: a solution of the crude product in DMSO was filtered through a 0.45 μm filter membrane to prepare a sample solution. Mobile phase system: water (containing 0.1% TFA)/acetonitrile. Gradient elution method: gradient elution with acetonitrile from 15% to 35% (elution time: 16 min). Lyophilisation was performed to obtain the trifluoroacetate of compound 63 (5.0 mg).

LCMS m/z = 811.7 $[M +1]^+$

Following step 5, the reaction solution was subjected to neutral preparative chromatography [mobile phase system: water (containing 5 mmol/L ammonium acetate)/acetonitrile] and lyophilised to obtain compound 63.

$^1$H NMR (400 MHz, $CDCl_3$) δ 10.64 (s, 1H), 8.09 (s, 1H), 7.56 - 7.33 (m, 4H), 7.00 - 6.85 (m, 2H), 6.71 (d, 1H), 6.49 (d, 1H), 5.22 (s, 1H), 4.39 - 4.23 (m, 2H), 3.88 - 3.70 (m, 2H), 3.70 - 3.52 (m, 3H), 3.47 - 3.21 (m, 4H), 3.20 - 2.55 (m, 12H), 2.40 - 2.08 (m, 5H), 2.03 - 1.55 (m, 10H), 1.52 - 1.28 (m, 2H).

LCMS m/z = 406.3 $[M/2 +1]^+$

**Example 64: Preparation of compound 64**

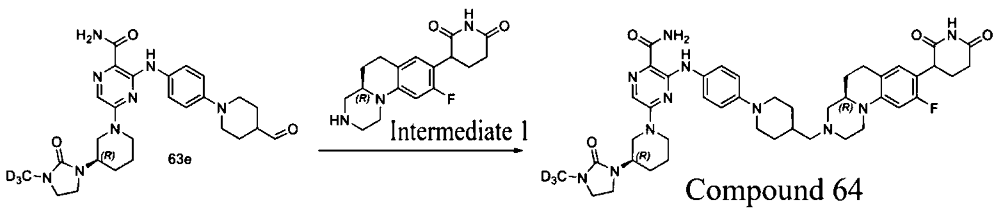


Starting from the trifluoroacetate of compound 63e and intermediate 1 and following the synthetic method of example 63, compound 64 was obtained by neutral preparative chromatography [water (10 mmol/L ammonium bicarbonate)/acetonitrile] and lyophilisation.

$^1$H NMR (400 MHz, $CDCl_3$) δ 10.64 (s, 1H), 7.95 (s, 1H), 7.56 - 7.31 (m, 4H), 7.03 - 6.85 (m, 2H), 6.72 (d, 1H), 6.49 (d, 1H), 5.15 (s, 1H), 4.39 - 4.22 (m, 2H), 3.88 - 3.70 (m, 2H), 3.70 - 3.50 (m, 3H), 3.43 - 3.21 (m, 4H), 3.20 - 2.55 (m, 12H), 2.40 - 2.08 (m, 5H), 2.03 - 1.50 (m, 10H), 1.50 - 1.28 (m, 2H).

LCMS m/z = 811.8 $[M +1]^+$

**Example 65: Preparation of compound 65**

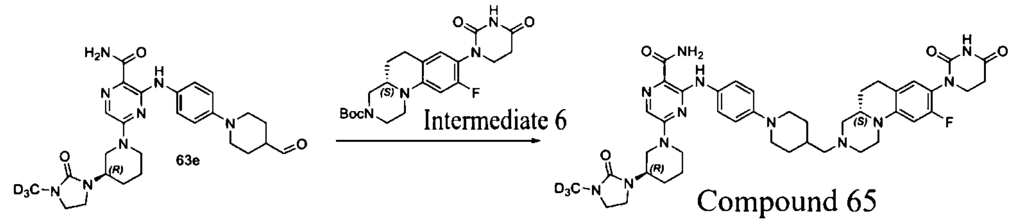


Starting from the trifluoroacetate of compound 63e and intermediate 6 and following the synthetic method of example 21, compound 65 was obtained.
$^1$H NMR (400 MHz, DMSO-$d_6$) δ 10.96 (s, 1H), 10.33 (s, 1H), 7.70 (s, 1H), 7.65 - 7.57 (m, 1H), 7.48 - 7.35 (m, 2H), 7.26 (s, 1H), 7.00 - 6.82 (m, 3H), 6.72 (d, 1H), 4.47 - 4.15 (m, 2H), 3.82 - 3.71 (m, 1H), 3.70 - 3.53 (m, 5H), 3.50 - 3.15 (m, 4H), 3.10 - 2.83 (m, 5H), 2.82 - 2.54 (m, 8H), 2.27 - 2.15 (m, 2H), 2.12 - 1.39 (m, 11H), 1.33 - 1.12 (m, 2H).
LCMS m/z = 812.6 [M+1]$^+$

**Example 66: Preparation of compound 66**

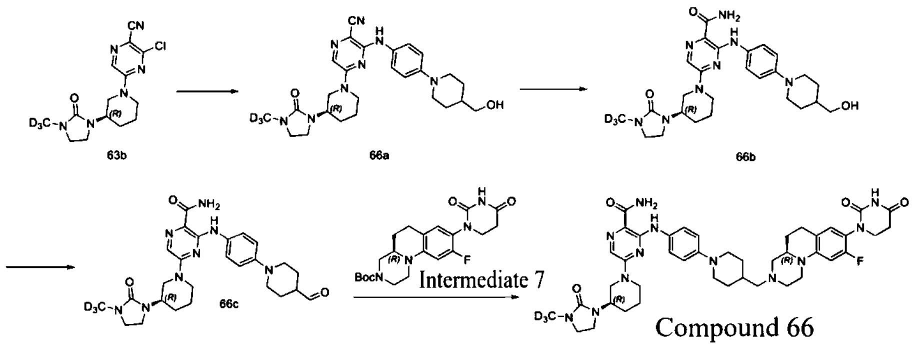


Step 1: Preparation of 66a

63b (1.8 g, 5.56 mmol) was dissolved in 1,4-dioxane (30 mL), (1-(4-aminophenyl)piperidin-4-yl)methanol (1.15 g, 5.57 mmol), caesium carbonate (5.43 g, 16.67 mmol), palladium acetate (127.7 mg, 0.57 mmol) and 2,2'-bis(diphenylphosphino)-1,1'-binaphthyl (523.1 mg, 0.84 mmol) were respectively added, and the mixture was purged three times with nitrogen and reacted at 100°C for 16 h. The reaction system was cooled to room temperature, and filtered. The filter cake was washed with dichloromethane (110 mL×2), and the filtrate was concentrated under reduced pressure. The crude product was separated and purified by silica gel column chromatography (dichloromethane : methanol (v/v) = 92 : 8) to obtain 66a (2.1 g, yield: 77%).
LCMS m/z = 494.3 [M+1]$^+$

Step 2: Preparation of 66b

66a (2.10 g, 4.26 mmol) was dissolved in DMSO (20 mL), caesium carbonate (1.38 g, 4.24 mmol) was added, followed by slowly dropwise addition of 30% hydrogen peroxide (15 mL) in an ice-water bath, and the mixture was stirred for 5 min. Subsequently, the ice-water bath was removed, and the mixture was reacted at room temperature for 3 h. To the reaction system were added a saturated sodium sulphite solution (80 mL) and ethyl acetate (200 mL). The organic phase was separated, and the aqueous phase was extracted with ethyl acetate (80 mL×3). The organic phases were combined, washed with a saturated aqueous sodium chloride solution (90 mL×2), dried over anhydrous sodium sulphate, and concentrated under reduced pressure. The crude product was separated and purified by silica gel column chromatography (dichloromethane : methanol (v/v) = 9 : 1) to obtain 66b (2.0 g, yield: 92%).
LCMS m/z = 512.3 [M+1]$^+$

Step 3: Preparation of 66c

66b (1.5 g, 2.93 mmol) was dissolved in dichloromethane (20 mL), N,N-diisopropylethylamine (1.14 g, 8.82 mmol) was added, followed by slowly dropwise addition of a solution of pyridine sulphur trioxide (1.40 g, 8.80 mmol) in DMSO (10 mL) at 0°C, and the mixture was allowed to warm to room temperature and reacted for another 2 h. To the reaction system were added water (300 mL) and ethyl acetate (100 mL). The aqueous phase was extracted with ethyl acetate (60 mL×2), and the organic phase was washed with a saturated aqueous sodium chloride solution (50 mL×2), dried over anhydrous sodium sulphate, and filtered. The filtrate was concentrated under reduced pressure, and the crude product was separated and purified by silica gel column chromatography (dichloromethane : methanol (v/v) = 9 : 1) to obtain 66c (1.05 g, yield: 70%).
LCMS m/z = 510.3 $[M+1]^+$

Step 4: Preparation of compound 66

Intermediate 7 (164 mg, 0.39 mmol) was dissolved in dichloromethane (10 mL), trifluoroacetic acid (3 mL) was added, and the mixture was reacted at room temperature for 2 h. The reaction system was concentrated under reduced pressure, dichloromethane (15 mL) and sodium bicarbonate (98 mg, 1.17 mmol) were added, and the mixture was stirred at room temperature for 30 min. 66c (200 mg, 0.39 mmol), anhydrous sodium sulphate (300 mg) and acetic acid (0.03 mL) were respectively added, and the mixture was reacted at room temperature for 16 h. Sodium triacetoxyborohydride (165 mg, 0.78 mmol) was then added, and the resulting mixture was reacted at room temperature for 2 h. To the reaction system was added water (120 mL), and the mixture was extracted with dichloromethane (80 mL×3). The organic phases were combined, washed with a saturated aqueous sodium chloride solution (70 mL×2), dried over anhydrous sodium sulphate, and filtered. The filtrate was concentrated under reduced pressure, and the crude product was separated and purified by silica gel thin-layer chromatography (dichloromethane : methanol (v/v) = 9 : 1) to obtain compound 66 (160 mg, yield: 51%).
$^1H$ NMR (400 MHz, $CDCl_3$) δ 10.65 (s, 1H), 7.60 - 7.30 (m, 5H), 7.10 - 6.80 (m, 3H), 6.53 (d, 1H), 5.17 (s, 1H), 4.40 - 4.20 (m, 2H), 3.90 - 3.52 (m, 6H), 3.45 - 3.23 (m, 4H), 3.23 - 2.57 (m, 11H), 2.45 - 2.08 (m, 2H), 2.05 - 1.30 (m, 14H).
LCMS m/z = 812.4 $[M+1]^+$

**Example 67: Preparation of compound 67**

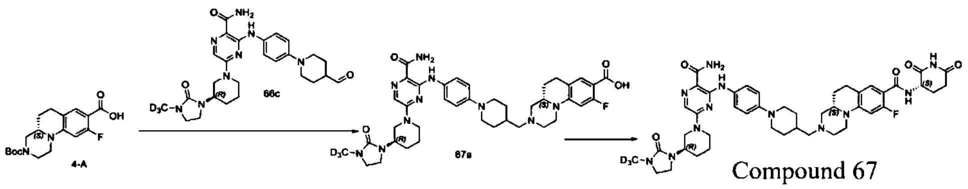
Compound 67

Step 1: Preparation of 67a

4-A (170 mg, 0.49 mmol) was dissolved in dichloromethane (10 mL), trifluoroacetic acid (3 mL) was added, and the mixture was reacted at room temperature for 2 h. The reaction system was concentrated under reduced pressure, dichloromethane (15 mL) and sodium bicarbonate (123 mg, 1.46 mmol) were added, and the mixture was stirred at room temperature for 30 min. 66c (250 mg, 0.49 mmol), anhydrous sodium sulphate (300 mg) and glacial acetic acid (0.1 mL) were respectively added, and the mixture was reacted at room temperature for 16 h. Sodium triacetoxyborohydride (208 mg, 0.98 mmol) was then added, and the resulting mixture was reacted at room temperature for 2 h. To the reaction system was added water (130 mL), and the mixture was extracted with dichloromethane (80 mL×3). The organic phases were combined, washed with a saturated aqueous sodium chloride solution (70 mL×2), dried over anhydrous sodium sulphate, and concentrated under reduced pressure. The crude product was separated and purified by silica gel thin-layer chromatography (dichloromethane : methanol (v/v) = 9 : 1) to obtain 67a (130 mg, yield: 36%).
LCMS m/z = 744.4 $[M+1]^+$

Step 2: Preparation of compound 67

67a (210 mg, 0.28 mmol) was dissolved in DMF (10 mL), EDCI (81 mg, 0.42 mmol), HOBt (57 mg, 0.42 mmol) and N-methylmorpholine (142 mg, 1.40 mmol) were respectively added, and the mixture was stirred at room temperature for 30 min. (S)-3-Aminopiperidine-2,6-dione hydrochloride (92 mg, 0.56 mmol) was then added, and the resulting mixture was

reacted at room temperature for 16 h. To the reaction solution was added water (100 mL), and the mixture was extracted with dichloromethane (60 mL×3). The organic phases were combined, washed with a saturated aqueous sodium chloride solution (70 mL×2), dried over anhydrous sodium sulphate, and filtered. The filtrate was concentrated under reduced pressure, and the crude product was separated and purified by silica gel column chromatography (dichloromethane : methanol (v/v) = 9 : 1) to obtain compound 67 (52.0 mg, yield: 22%).

$^1$H NMR (400 MHz, $CDCl_3$) δ 10.66 (s, 1H), 8.08 (s, 1H), 7.66 (d, 1H), 7.57 - 7.32 (m, 5H), 7.15 - 6.85 (m, 2H), 6.41 (d, 1H), 5.21 (s, 1H), 4.87 - 4.70 (m, 1H), 4.40 - 4.20 (m, 2H), 3.88 - 3.51 (m, 4H), 3.44 - 2.60 (m, 18H), 2.45 - 2.10 (m, 3H), 2.05 - 1.35 (m, 12H).

LCMS m/z = 854.0 [M+1]$^+$

**Example 68: Preparation of compound 68**

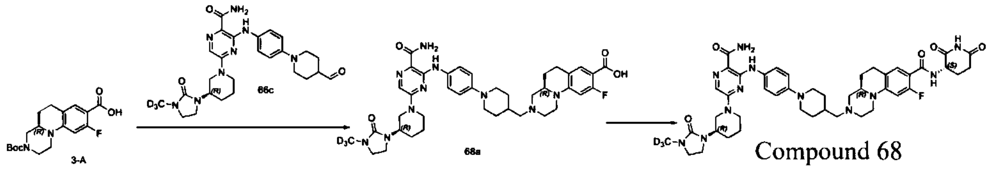

3-A
66c
68a
Compound 68

Starting from compound 3-A and following the synthetic method of example 67, compound 68 was obtained.

$^1$H NMR (400 MHz, $CDCl_3$) δ 10.75 (s, 1H), 7.92 (s, 1H), 7.67 (d, 1H), 7.62 - 7.32 (m, 5H), 7.18 - 6.90 (m, 2H), 6.42 (d, 1H), 5.19 (s, 1H), 4.84 - 4.70 (m, 1H), 4.40 - 4.24 (m, 2H), 3.88 - 3.51 (m, 4H), 3.44 - 2.60 (m, 18H), 2.58 - 2.24 (m, 3H), 2.10 - 1.35 (m, 12H).

LCMS m/z = 854.7 [M+1]$^+$

Following the synthetic method of other examples, the following compounds were obtained:

| Example No. | Structure | Starting material | LCMS / $^1$H NMR |
|---|---|---|---|
| Example 69 | 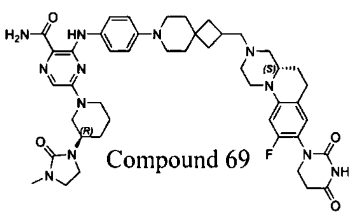 Compound 69 | Intermediate 6+13d (following the synthetic method of example 21) | $^1$H NMR (400 MHz, $CDCl_3$) δ 10.62 (s, 1H), 7.54 - 7.30 (m, 5H), 6.97 - 6.80 (m, 3H), 6.52 (d, 1H), 5.16 (s, 1H), 4.39 - 4.21 (m, 2H), 3.87 - 3.53 (m, 4H), 3.43 - 3.22 (m, 4H), 3.18 - 2.73 (m, 14H), 2.72 - 2.42 (m, 3H), 2.18 - 1.35 (m, 19H).<br>LCMS m/z = 849.5 [M+1]$^+$ |
| Example 70 | 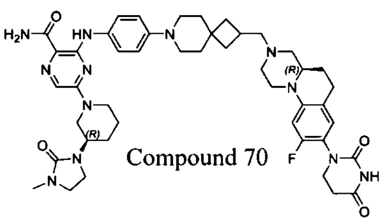 Compound 70 | Intermediate 7+13d (following the synthetic method of example 21) | $^1$H NMR (400 MHz, $CDCl_3$) δ 10.62 (s, 1H), 7.54 - 7.31 (m, 5H), 6.97 - 6.80 (m, 3H), 6.52 (d, 1H), 5.15 (s, 1H), 4.39 - 4.23 (m, 2H), 3.87 - 3.53 (m, 4H), 3.43 - 3.22 (m, 4H), 3.18 - 2.73 (m, 14H), 2.72 - 2.42 (m, 3H), 2.18 - 1.35 (m, 19H).<br>LCMS m/z = 849.5 [M+1]$^+$ |
| Example 71 | 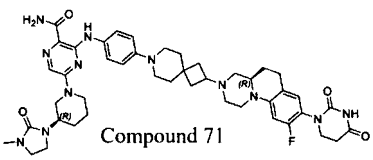 Compound 71 | Intermediate 7+31h (following the synthetic method of example 31) | $^1$H NMR (400 MHz, DMSO-$d_6$) δ 10.96 (s, 1H), 10.32 (s, 1H), 7.68 (s, 1H), 7.60 (s, 1H), 7.45 - 7.34 (m, 2H), 7.24 (s, 1H), 6.96 - 6.80 (m, 3H), 6.71 (d, 1H), 4.43 - 4.17 (m, 2H), 3.84 - 3.70 (m, 1H), 3.68 - 3.52 (m, 3H), 3.40 - 3.17 (m, 4H), 3.14 - 2.80 (m, 9H), 2.78 - 2.53 (m, 9H), 2.05 - 1.71 (m, 7H), 1.71 - 1.42 (m, 9H).<br>LCMS m/z = 835.4 [M+1]$^+$ |

(continued)

| Example No. | Structure | Starting material | LCMS / $^1$H NMR |
|---|---|---|---|
| Example 72 | 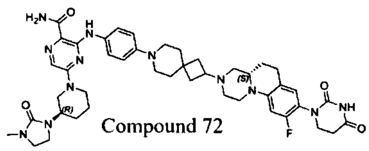 Compound 72 | Intermediate 6+31h (following the synthetic method of example 31) | $^1$H NMR (400 MHz, DMSO-$d_6$) δ 10.96 (s, 1H), 10.31 (s, 1H), 7.68 (s, 1H), 7.60 (s, 1H), 7.46 - 7.35 (m, 2H), 7.24 (s, 1H), 6.96 - 6.82 (m, 3H), 6.71 (d, 1H), 4.43 - 4.17 (m, 2H), 3.84 - 3.70 (m, 1H), 3.68 - 3.52 (m, 3H), 3.40 - 3.17 (m, 4H), 3.14 - 2.80 (m, 9H), 2.78 - 2.53 (m, 9H), 2.05 - 1.71 (m, 7H), 1.71 - 1.45 (m, 9H). LCMS m/z = 835.4 $[M+1]^+$ |
| Example 73 | 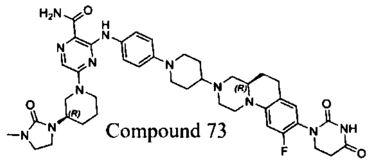 Compound 73 | Intermediate 7+35h (following the synthetic method of example 31) | $^1$H NMR (400 MHz, DMSO-$d_6$) δ 10.97 (s, 1H), 10.32 (s, 1H), 7.69 (s, 1H), 7.60 (s, 1H), 7.46 - 7.36 (m, 2H), 7.25 (s, 1H), 6.98 - 6.82 (m, 3H), 6.71 (d, 1H), 4.44 - 4.19 (m, 2H), 3.84 - 3.71 (m, 1H), 3.70 - 3.53 (m, 5H), 3.42 - 3.18 (m, 4H), 3.08 - 2.86 (m, 5H), 2.82 - 2.55 (m, 10H), 2.44 - 2.17 (m, 2H), 2.05 - 1.68 (m, 7H), 1.68 - 1.45 (m, 4H). LCMS m/z = 795.7 $[M+1]^+$ |
| Example 74 | 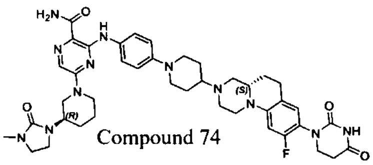 Compound 74 | Intermediate 6+35h (following the synthetic method of example 31) | $^1$H NMR (400 MHz, DMSO-$d_6$) δ 10.97 (s, 1H), 10.32 (s, 1H), 7.68 (s, 1H), 7.60 (s, 1H), 7.46 - 7.36 (m, 2H), 7.25 (s, 1H), 6.98 - 6.82 (m, 3H), 6.71 (d, 1H), 4.44 - 4.19 (m, 2H), 3.84 - 3.71 (m, 1H), 3.70 - 3.53 (m, 5H), 3.42 - 3.18 (m, 4H), 3.08 - 2.86 (m, 5H), 2.82 - 2.55 (m, 10H), 2.44 - 2.17 (m, 2H), 2.05 - 1.68 (m, 7H), 1.68 - 1.45 (m, 4H). LCMS m/z = 795.7 $[M+1]^+$ |
| Example 77 | 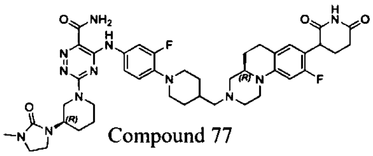 Compound 77 | Intermediate 1+76e (following the synthetic method of example 76) | $^1$H NMR (400 MHz, DMSO-$d_6$) δ 11.26 (s, 1H), 10.76 (s, 1H), 8.33 (s, 1H), 7.72 (s, 1H), 7.65 - 7.25 (m, 2H), 7.03 (d, 1H), 6.79 (d, 1H), 6.62 (d, 1H), 5.10 - 4.10 (m, 2H), 3.88 - 3.52 (m, 3H), 3.42 - 3.18 (m, 6H), 3.17 - 2.82 (m, 5H), 2.80 - 2.42 (m, 10H), 2.27 - 1.42 (m, 15H), 1.38 - 1.18 (m, 2H). LCMS m/z = 827.4 $[M+1]^+$ |
| Example 78 | 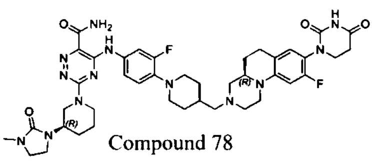 Compound 78 | Intermediate 7+76e (following the synthetic method of example 21) | $^1$H NMR (400 MHz, DMSO-$d_6$) δ 11.26 (s, 1H), 10.33 (s, 1H), 8.33 (s, 1H), 7.72 (s, 1H), 7.65 - 7.20 (m, 2H), 7.04 (t, 1H), 6.93 (d, 1H), 6.72 (d, 1H), 5.10 - 4.10 (m, 2H), 3.82 - 3.68 (m, 1H), 3.66 - 3.52 (m, 3H), 3.40 - 3.20 (m, 6H), 3.18 - 2.50 (m, 15H), 2.28 - 2.16 (m, 2H), 2.13 - 1.98 (m, 1H), 1.96 - 1.43 (m, 10H), 1.38 - 1.18 (m, 2H). |
| Example 79 | 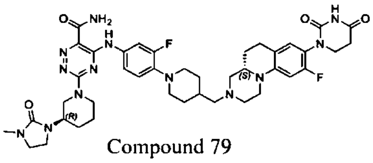 Compound 79 | Intermediate 6+76e (following the synthetic method of example 21) | $^1$H NMR (400 MHz, DMSO-$d_6$) δ 11.26 (s, 1H), 10.33 (s, 1H), 8.33 (s, 1H), 7.72 (s, 1H), 7.63 - 7.20 (m, 2H), 7.03 (t, 1H), 6.92 (d, 1H), 6.71 (d, 1H), 5.10 - 4.10 (m, 2H), 3.82 - 3.68 (m, 1H), 3.67 - 3.53 (m, 3H), 3.44 - 3.20 (m, 5H), 3.18 - 2.83 (m, 5H), 2.82 - 2.54 (m, 11H), 2.28 - 2.14 (m, 2H), 2.13 - 1.97 (m, 1H), 1.96 - 1.45 (m, 10H), 1.40 - 1.16 (m, 2H). |

**Example 75: Preparation of compound 75**

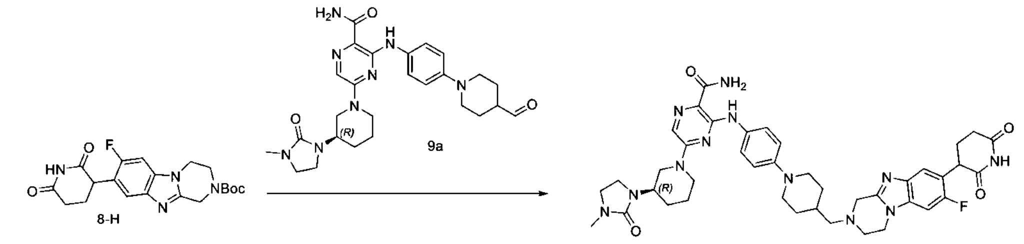


Compound 75

To the above-mentioned crude 8-H (100 mg) were added dichloromethane (12 mL) and trifluoroacetic acid (3 mL), and the mixture was reacted at room temperature for 2 h. The reaction system was concentrated under reduced pressure, dichloromethane (15 mL) and sodium bicarbonate (73 mg, 0.87 mmol) were added, and the mixture was stirred at room temperature for 30 min. 9a (147 mg, 0.29 mmol), anhydrous sodium sulphate (300 mg) and acetic acid (0.06 mL) were added, and the mixture was reacted at room temperature for 16 h. Sodium triacetoxyborohydride (123 mg, 0.58 mmol) was then added, and the resulting mixture was reacted at room temperature for 2 h. To the reaction system was added water (120 mL), and the mixture was extracted with dichloromethane (80 mL×3). The organic phase was washed with a saturated aqueous sodium chloride solution (70 mL×2), dried over anhydrous sodium sulphate, and filtered. The filtrate was concentrated under reduced pressure, and the crude product was subjected to Pre-HPLC (instrument and preparative column: using Waters 2767 preparative liquid phase chromatographic instrument; preparative column model: SunFire@Prep C18, 5 μm, inner diameter × length = 19 mm × 250 mm). Preparative method: a solution of the crude product in DMSO was filtered through a 0.45 μm filter membrane to prepare a sample solution. Mobile phase system: water (containing 10 mmol/L ammonium bicarbonate)/acetonitrile. Gradient elution method: gradient elution with acetonitrile from 10% to 50% (elution time: 15 min). Lyophilisation was performed to obtain compound 75 (52 mg, yield: 23%).

$^1$H NMR (400 MHz, DMSO-$d_6$) δ 10.96 (s, 1H), 10.82 (s, 1H), 7.68 (s, 1H), 7.60 (s, 1H), 7.51 - 7.34 (m, 4H), 7.25 (s, 1H), 6.92 - 6.83 (m, 2H), 4.46 - 4.18 (m, 2H), 4.15 - 4.04 (m, 3H), 3.80 (s, 2H), 3.68 - 3.52 (m, 3H), 3.40 - 3.18 (m, 4H), 3.08 - 2.87 (m, 4H), 2.85 - 2.40 (m, 9H), 2.38 - 2.18 (m, 1H), 2.09 - 1.97 (m, 1H), 1.90 - 1.67 (m, 6H), 1.65 - 1.45 (m, 1H), 1.38 - 1.16 (m, 2H).

LCMS m/z = 793.3 $[M+1]^+$

**Example 76: Preparation of compound 76**

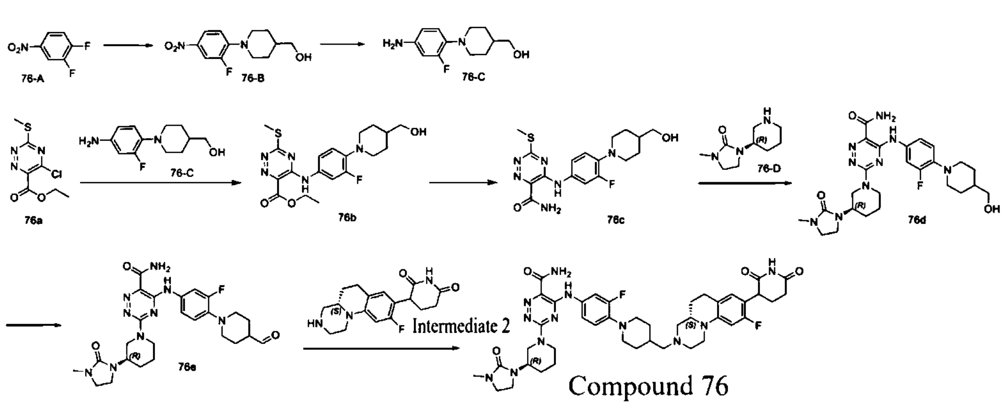


Step 1: Preparation of 76-B

76-A (10.0 g, 62.66 mmol) and 4-hydroxymethylpiperidine (7.6 g, 66.0 mmol) were dissolved in DMF (200 mL), potassium carbonate (13.03 g, 94.28 mmol) was slowly added in an ice bath, and the mixture was reacted at room temperature for 16 h. The reaction system was poured into water (500 mL), and filtered. The filter cake was dried under reduced pressure to obtain crude 76-B (15.0 g).

Step 2: Preparation of 76-C

The above-mentioned crude 76-B (5.0 g) was dissolved in methanol (100 mL), 10% palladium on carbon (3.0 g) was added, and the mixture was reacted at room temperature under hydrogen balloon atmosphere for 12 h. The reaction solution was filtered through a pad of diatomaceous earth, and the filtrate was concentrated under reduced pressure to obtain crude 76-C (4.3 g).
LCMS m/z = 225.1 $[M+1]^+$

Step 3: Preparation of 76b

76a (4.0 g, 17.12 mmol) and the above-mentioned crude 76-C (4.3 g) were dissolved in DCM (20 mL), DIPEA (2.88 g, 22.28 mmol) was slowly added dropwise, and the mixture was stirred at room temperature for 30 min after the addition. The reaction system was filtered, and the filter cake was dried under reduced pressure to obtain crude 76b (5.69 g).
LCMS m/z = 422.1 $[M+1]^+$

Step 4: Preparation of 76c

The above-mentioned crude 76b (3.8 g) was added to methanol (20 mL), followed by slowly dropwise addition of 7 mol/L ammonia methanol solution (90 mL), and the mixture was reacted at room temperature for 3 h. The reaction system was filtered, and the filter cake was collected, washed with petroleum ether (50 mL), and dried under reduced pressure to obtain crude 76c (3.1 g).
LCMS m/z = 393.0 $[M+1]^+$

Step 5: Preparation of 76d

To a reaction flask were respectively added the above-mentioned crude 76c (3.11 g), m-CPBA (8.04 g) and NMP (10 mL), and the mixture was reacted at room temperature for 16 h. Subsequently, DIPEA (5.12 g, 39.61 mmol) and 76-D (2.18 g, 11.90 mmol) (see WO 2023088406 for the synthetic method) were successively added to the reaction system, and the resulting mixture was reacted at 80°C for 16 h. The reaction solution was cooled to room temperature, water (100 mL) was added, and the mixture was extracted with ethyl acetate (100 mL×4). The organic phase was washed with a saturated aqueous sodium chloride solution (50 mL×2), dried over anhydrous sodium sulphate, and concentrated under reduced pressure. The crude product was separated and purified by silica gel column chromatography (dichloromethane/methanol (v/v) = 1 : 0-0 : 1) to obtain 76d (1.2 g, yield: 19%).
LCMS m/z = 528.7 $[M+1]^+$

Step 6: Preparation of 76e

76d (0.7 g, 1.33 mmol) was dissolved in DMSO (2 mL), triethylamine (0.67 g, 6.62 mmol) was added, followed by pyridine sulphur trioxide (1.05 g, 6.60 mmol) at room temperature with stirring, and the mixture was reacted at room temperature for 10 min. To the reaction system was added water (20 mL), and the mixture was extracted with ethyl acetate (50 mL), dried over anhydrous sodium sulphate, and concentrated under reduced pressure. The crude product was separated and purified by silica gel column chromatography (dichloromethane/methanol (v/v) = 1 : 0-0 : 1) to obtain 76e (0.59 g, yield: 84%).
LCMS m/z = 526.3 $[M+1]^+$

Step 7: Preparation of compound 76

To a reaction flask were respectively added 76e (0.15 g, 0.29 mmol), the above-mentioned crude intermediate 2 (0.12 g), glacial acetic acid (0.5 mL) and DMAc (5 mL), and the mixture was reacted at room temperature for 1 h. Sodium triacetoxyborohydride (0.18 g, 0.85 mmol) was then added, and the resulting mixture was reacted at room temperature for 16 h. The reaction solution was subjected to Pre-HPLC (instrument and preparative column: using Waters 2767 preparative liquid phase chromatographic instrument; preparative column model: SunFire@Prep C18, 5 μm, inner diameter × length = 19 mm × 250 mm). Preparative method: a solution of the crude product in DMSO was filtered through a 0.45 μm filter membrane to prepare a sample solution. Mobile phase system: water (containing 10 mmol/L ammonium bicarbonate)/acetonitrile. Gradient elution method: gradient elution with acetonitrile from 10% to 50% (elution time: 15 min). Lyophilisation was performed to obtain compound 76 (72 mg, yield: 30%).
$^1H$ NMR (400 MHz, $CDCl_3$) δ 10.88 (s, 1H), 8.19 (s, 1H), 7.71 (s, 1H), 7.55 (d, 1H), 7.22 (s, 1H), 6.96 (t, 1H), 6.71

(d, 1H), 6.49 (d, 1H), 5.59 (s, 1H), 5.20 - 4.20 (m, 2H), 3.86 - 3.70 (m, 2H), 3.66 - 3.52 (m, 1H), 3.52 - 3.26 (m, 6H), 3.26 - 2.55 (m, 15H), 2.40 - 2.07 (m, 5H), 2.05 - 1.55 (m, 10H), 1.54 - 1.35 (m, 2H).

LCMS m/z = 827.4 $[M+1]^+$

**Example 80: Preparation of compound 80**

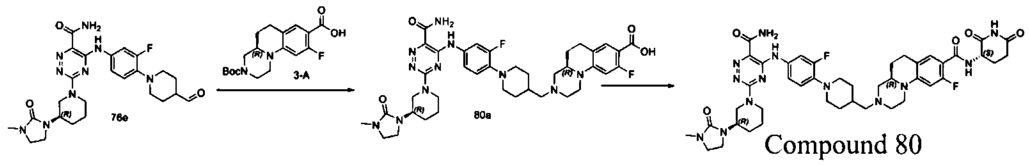


76e 80a Compound 80

Starting from compounds 76e and 3-A and following the synthetic method of example 17, compound 80 was obtained.

$^1H$ NMR (400 MHz, DMSO-$d_6$) δ 11.26 (s, 1H), 10.82 (s, 1H), 8.33 (s, 1H), 7.94 (t, 1H), 7.72 (s, 1H), 7.64 - 7.28 (m, 3H), 7.04 (t, 1H), 6.68 (d, 1H), 5.00 - 4.20 (m, 3H), 3.92 - 3.77 (m, 1H), 3.68 - 3.52 (m, 1H), 3.40 - 3.20 (m, 6H), 3.19 - 2.57 (m, 15H), 2.57 - 2.45 (m, 1H), 2.29 - 1.44 (m, 14H), 1.37 - 1.20 (m, 2H).

LCMS m/z = 870.4 $[M+1]^+$

**Example 81: Preparation of compound 81**

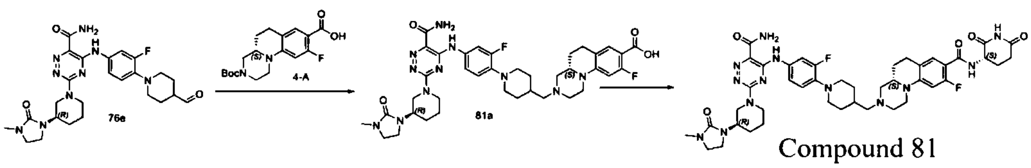


76e 81a Compound 81

Starting from compounds 76e and 4-A and following the synthetic method of example 17, compound 81 was obtained.

$^1H$ NMR (400 MHz, DMSO-$d_6$) δ 11.26 (s, 1H), 10.82 (s, 1H), 8.33 (s, 1H), 7.94 (t, 1H), 7.72 (s, 1H), 7.64 - 7.28 (m, 3H), 7.03 (t, 1H), 6.68 (d, 1H), 5.00 - 4.20 (m, 3H), 3.92 - 3.77 (m, 1H), 3.68 - 3.52 (m, 1H), 3.40 - 3.20 (m, 6H), 3.19 - 2.57 (m, 15H), 2.57 - 2.45 (m, 1H), 2.29 - 1.44 (m, 14H), 1.37 - 1.20 (m, 2H).

LCMS m/z = 870.4 $[M+1]^+$

**Example 82: Preparation of compound 82**

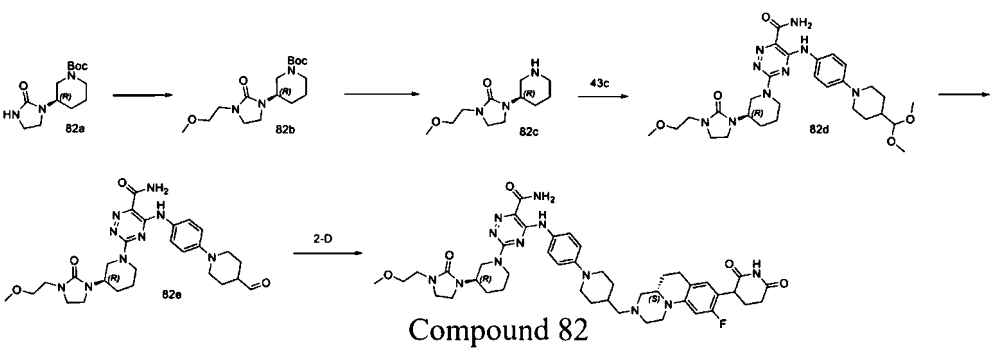


82a 82b 82c 82d 82e Compound 82

Step 1: Preparation of 82b

82a (5 g, 18.56 mmol) (see WO 2020081450 for the synthetic method) was added to a three-necked flask (250

mL), followed by dry tetrahydrofuran (80 mL), and the mixture was cooled to 0°C in an ice-water bath under nitrogen atmosphere. 60% sodium hydride (1.48 g) was added, and the mixture was stirred at this temperature for 10 min. Subsequently, the system was allowed to warm to room temperature naturally and reacted for 1 h. The reaction system was cooled to 0°C, and 1-iodo-2-methoxyethane (5.2 g, 27.96 mmol) was slowly added. The mixture was then allowed to warm to room temperature naturally and reacted for 16 h. To the reaction system was added ice water (100 mL), and the mixture was extracted with ethyl acetate (100 mL×3). The organic phase was washed with a saturated aqueous sodium chloride solution (80 mL×2), dried over anhydrous sodium sulphate, and filtered. The filtrate was concentrated under reduced pressure, and the crude product was separated and purified by silica gel column chromatography (petroleum ether : ethyl acetate (v/v) = 1 : 1) to obtain 82b (3.8 g, yield: 63%).

Starting from compounds 82b and 2-D and following the synthetic methods of examples 43 and 51, compound 82 was obtained.

$^1H$ NMR (400 MHz, $CDCl_3$) δ 10.73 (s, 1H), 7.91 (s, 1H), 7.73 - 7.64 (m, 1H), 7.59 - 7.47 (m, 2H), 7.04 - 6.87 (m, 2H), 6.78 - 6.66 (m, 1H), 6.49 (d, 1H), 5.40 - 5.28 (m, 1H), 5.10 - 4.20 (m, 2H), 3.90 - 3.58 (m, 5H), 3.58 - 3.48 (m, 2H), 3.48 - 3.30 (m, 9H), 3.27 - 2.55 (m, 13H), 2.38 - 2.07 (m, 5H), 2.05 - 1.60 (m, 9H), 1.50 - 1.22 (m, 2H).

LCMS m/z = 427.5 $[M/2 +1]^+$

**Example 83: Preparation of compound 83**

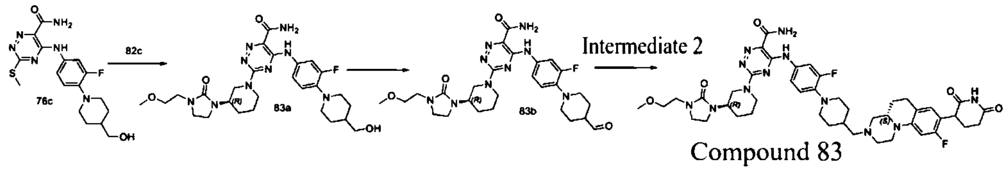


Starting from compounds 76c and 82c and following the synthetic method of example 76, compound 83 was obtained.

$^1H$ NMR (400 MHz, $CDCl_3$) δ 10.88 (s, 1H), 7.93 (s, 1H), 7.74 - 7.65 (m, 1H), 7.62 - 7.50 (m, 1H), 7.30 - 7.14 (m, 1H), 6.95 (t, 1H), 6.80 - 6.65 (m, 1H), 6.49 (d, 1H), 5.39 (s, 1H), 5.20 - 4.20 (m, 2H), 3.90 - 3.70 (m, 2H), 3.70 - 3.49 (m, 3H), 3.49 - 3.30 (m, 10H), 3.28 - 2.55 (m, 13H), 2.45 - 2.10 (m, 5H), 2.05 - 1.60 (m, 10H), 1.58 - 1.34 (m, 2H).

LCMS m/z = 436.4 $[M/2 +1]^+$

**Example 84: Preparation of compound 84**

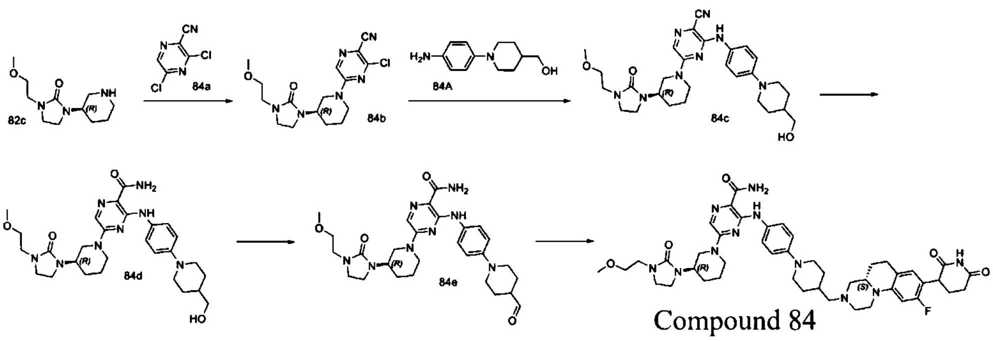


Starting from compounds 82c, 84a, 84A and 2-D and following the synthetic methods of examples 63 and 66, compound 84 was obtained.

$^1H$ NMR (400 MHz, $CDCl_3$) δ 10.67 (s, 1H), 7.94 (s, 1H), 7.56 - 7.32 (m, 4H), 7.05 - 6.83 (m, 2H), 6.72 (d, 1H), 6.49 (d, 1H), 5.15 (s, 1H), 4.40 - 4.23 (m, 2H), 3.88 - 3.72 (m, 2H), 3.70 - 3.50 (m, 5H), 3.48 - 3.27 (m, 8H), 3.20 - 2.55 (m, 13H), 2.42 - 2.08 (m, 5H), 2.05 - 1.60 (m, 10H), 1.57 - 1.30 (m, 2H).

LCMS m/z = 426.8 $[M/2 +1]^+$

**Example 85: Preparation of compound 85**

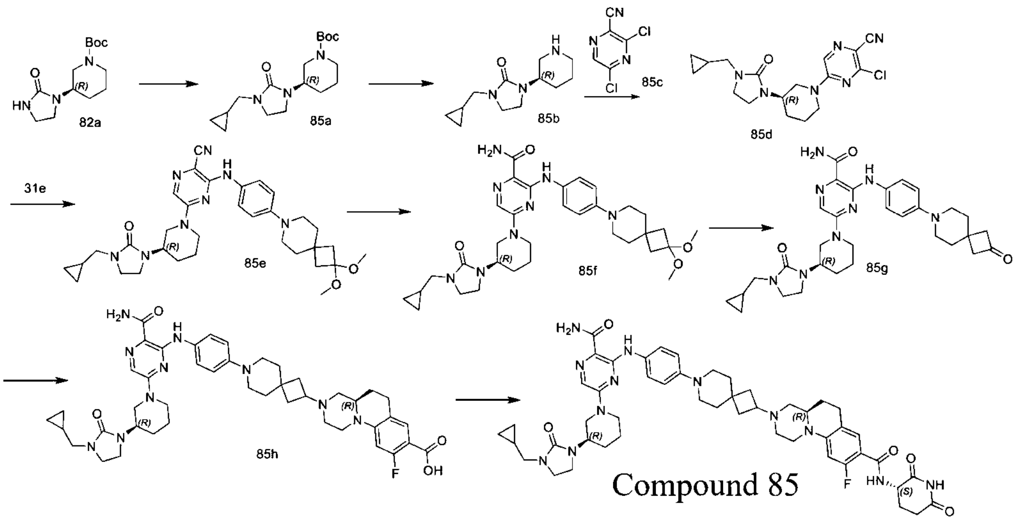


Step 1: Preparation of 85a

82a (3 g, 11.14 mmol) (see WO 2020081450 for the synthetic method) was dissolved in DMF (50 mL), 60% sodium hydride (0.89 g) was added at 0°C, and the mixture was stirred at 0°C for 30 min. Bromomethylcyclopropane (3.01 g, 22.3 mmol) was then added, and the resulting mixture was reacted at room temperature for 16 h. To the reaction solution was added water (200 mL), and the mixture was extracted three times with ethyl acetate (100 mL). The organic phases were combined, dried over anhydrous sodium sulphate, and concentrated under reduced pressure. The crude product was separated and purified by silica gel column chromatography (petroleum ether / ethyl acetate (v/v) = 1 : 0-1 : 1) to obtain 85a (2.5 g, yield: 69%).

LCMS m/z = 324.1 $[M+1]^+$

Step 2: Preparation of trifluoroacetate of 85b

85a (2.0 g, 6.19 mmol) was dissolved in dichloromethane (10 mL), trifluoroacetic acid (4 mL) was added, and the mixture was reacted at room temperature for 3 h. The reaction system was concentrated under reduced pressure to obtain the trifluoroacetate of crude 85b (4.0 g).

LCMS m/z = 224.2 $[M+1]^+$

Starting from compound 85c and the trifluoroacetate of 85b and following the synthetic methods of examples 31, 33 and 63, compound 85 was obtained.

$^1$H NMR (400 MHz, DMSO-$d_6$) δ 11.00 (s, 1H), 10.81 (s, 1H), 7.93 (t, 1H), 7.68 (s, 1H), 7.60 (s, 1H), 7.46 - 7.32 (m, 3H), 7.28 - 7.21 (m, 1H), 6.92 - 6.82 (m, 2H), 6.67 (d, 1H), 4.79 - 4.65 (m, 1H), 4.48 - 4.32 (m, 1H), 4.32 - 4.17 (m, 1H), 3.94 - 3.79 (m, 1H), 3.70 - 3.54 (m, 1H), 3.45 - 3.20 (m, 5H), 3.18 - 2.60 (m, 16H), 2.58 - 2.44 (m, 1H), 2.20 - 1.50 (m, 17H), 0.98 - 0.80 (m, 1H), 0.55 - 0.40 (m, 2H), 0.25 - 0.12 (m, 2H).

LCMS m/z = 459.5 $[M/2 +1]^+$

**Example 86: Preparation of compound 86**

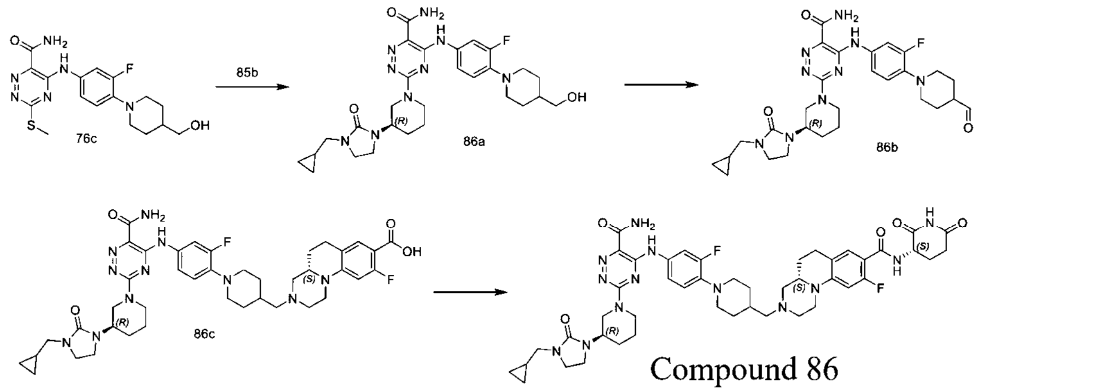


Compound 86

Starting from compound 76c, the trifluoroacetate of 85b and 4-A and following the synthetic methods of examples 76 and 17, compound 86 was obtained.

$^1$H NMR (400 MHz, DMSO-$d_6$) δ 11.26 (s, 1H), 10.81 (s, 1H), 8.32 (s, 1H), 7.93 (t, 1H), 7.71 (s, 1H), 7.63 - 7.24 (m, 3H), 7.04 (t, 1H), 6.68 (d, 1H), 5.05 - 4.20 (m, 3H), 3.93 - 3.77 (m, 1H), 3.70 - 3.52 (m, 1H), 3.47 - 3.19 (m, 8H), 3.19 - 2.58 (m, 12H), 2.58 - 2.45 (m, 1H), 2.30 - 1.45 (m, 14H), 1.38 - 1.20 (m, 2H), 0.95 - 0.77 (m, 1H), 0.55 - 0.40 (m, 2H), 0.25 - 0.12 (m, 2H).

LCMS m/z = 455.7 [M/2 +1]$^+$

**Example 87: Preparation of compound 87**

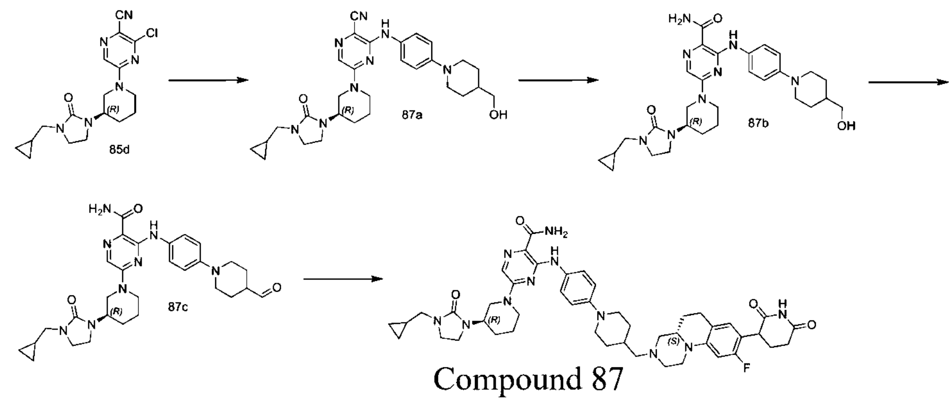


Compound 87

Starting from compounds 85d and 2-D and following the synthetic method of example 66, compound 87 was obtained.

$^1$H NMR (400 MHz, DMSO-$d_6$) δ 11.00 (s, 1H), 10.76 (s, 1H), 7.69 (s, 1H), 7.60 (s, 1H), 7.47 - 7.37 (m, 2H), 7.31 - 7.21 (m, 1H), 6.92 - 6.84 (m, 2H), 6.79 (d, 1H), 6.62 (d, 1H), 4.50 - 4.15 (m, 2H), 3.88 - 3.50 (m, 5H), 3.45 - 3.25 (m, 4H), 3.14 - 2.83 (m, 7H), 2.82 - 2.55 (m, 6H), 2.55 - 2.42 (m, 1H), 2.27 - 1.45 (m, 15H), 1.34 - 1.15 (m, 2H), 0.95 - 0.80 (m, 1H), 0.54 - 0.42 (m, 2H), 0.24 - 0.12 (m, 2H).

LCMS m/z = 424.8 [M/2 +1]$^+$

**Example 88: Preparation of compound 88**

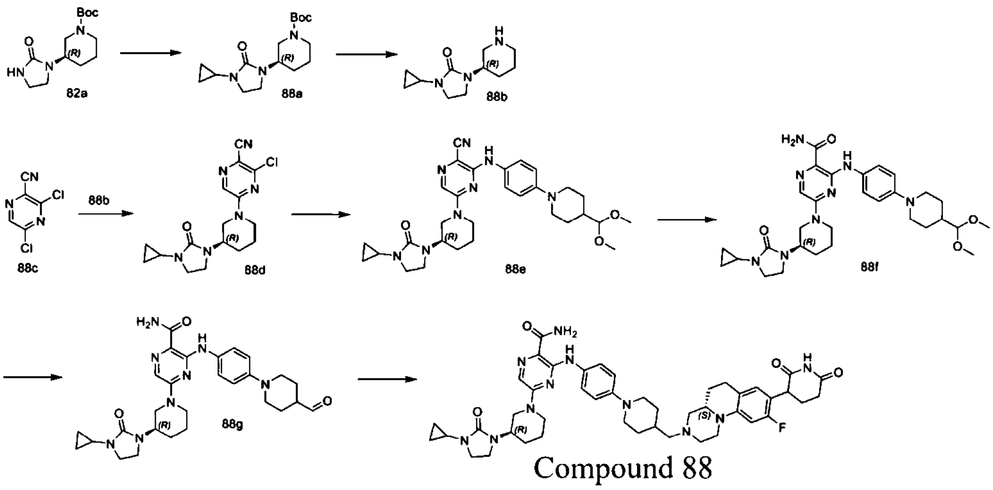


Compound 88

Step 1: Preparation of 88a

82a (5 g, 18.56 mmol) (see WO 2020081450 for the synthetic method) was dissolved in DMF (50 mL), cyclopropyl boronic acid (5.74 g, 66.82 mmol), pyridine (7.05 g, 89.13 mmol) and copper acetate (6.07 g, 33.42 mmol) were added, and the mixture was reacted at 45°C under open atmosphere for 72 h. To the reaction solution was added water (200 mL), and the mixture was extracted three times with ethyl acetate (100 mL). The organic phases were combined, dried over anhydrous sodium sulphate, and concentrated under reduced pressure. The crude product was separated and purified by silica gel column chromatography (petroleum ether / ethyl acetate (v/v) = 1 : 0-1 : 1) to obtain 88a (2.0 g, yield: 35%).

Step 2: Preparation of trifluoroacetate of 88b

88a (2.0 g, 6.46 mmol) was dissolved in dichloromethane (10 mL), trifluoroacetic acid (4 mL) was added, and the mixture was reacted at room temperature for 3 h. The reaction system was concentrated under reduced pressure to obtain the trifluoroacetate of crude 88b (4.0 g).

LCMS m/z = 210.2 $[M+1]^+$

Starting from the trifluoroacetate of compound 88b and following the synthetic method of example 63, compound 88 was obtained by neutral preparative chromatography [water (containing 10 mmol/L ammonium bicarbonate)/acetonitrile] and lyophilisation.

**Example 89: Preparation of compound 89**

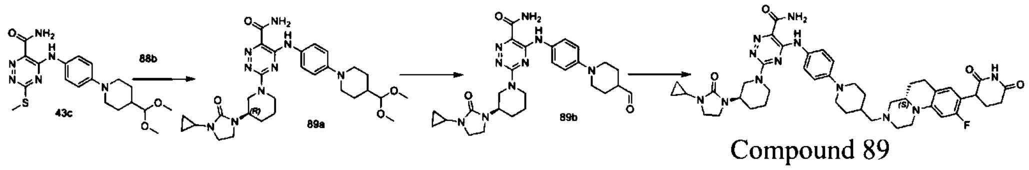


Compound 89

Starting from compound 43c, the trifluoroacetate of 88b and 2-D and following the synthetic method of example 43, compound 89 was obtained.

$^1H$ NMR (400 MHz, DMSO-$d_6$) δ 11.11 (s, 1H), 10.75 (s, 1H), 8.27 (s, 1H), 7.66 (s, 1H), 7.55 - 7.45 (m, 2H), 7.00 - 6.87 (m, 2H), 6.84 - 6.74 (m, 1H), 6.68 - 6.56 (m, 1H), 5.15 - 3.95 (m, 2H), 3.88 - 3.54 (m, 5H), 3.35 - 3.14 (m, 5H), 3.10 - 2.83 (m, 5H), 2.80 - 2.55 (m, 6H), 2.50 - 2.30 (m, 2H), 2.26 - 1.40 (m, 14H), 1.30 - 1.12 (m, 2H), 0.70 - 0.50 (m, 4H).

LCMS m/z = 418.3 $[M/2 +1]^+$

**Example 90: Preparation of compound 90**

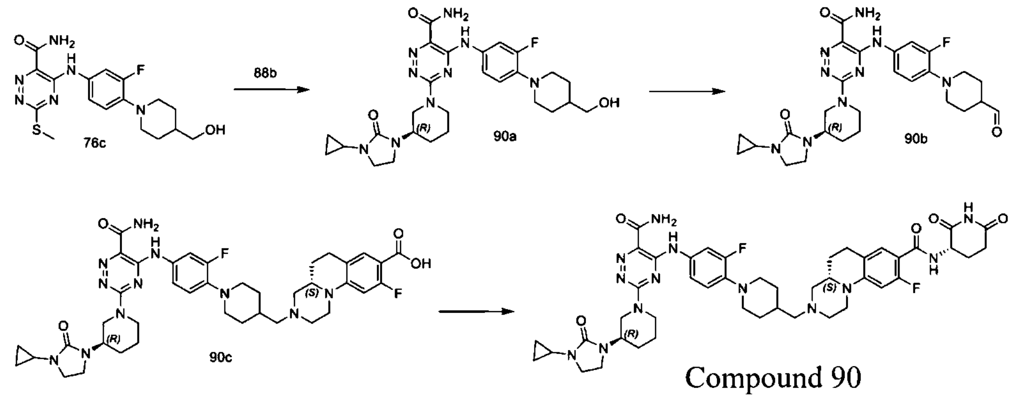


76c 90a 90b 90c Compound 90

Starting from compound 76c, the trifluoroacetate of 88b and 4-A and following the synthetic methods of examples 76 and 17, compound 90 was obtained.

$^{1}$H NMR (400 MHz, DMSO-$d_6$) δ 11.28 (s, 1H), 10.81 (s, 1H), 8.32 (s, 1H), 7.93 (t, 1H), 7.75 - 7.67 (m, 1H), 7.64 - 7.45 (m, 1H), 7.42 - 7.30 (m, 2H), 7.04 (t, 1H), 6.68 (d, 1H), 5.00 - 4.10 (m, 3H), 3.94 - 3.77 (m, 1H), 3.70 - 3.52 (m, 1H), 3.40 - 3.18 (m, 6H), 3.17 - 2.57 (m, 12H), 2.54 - 2.32 (m, 2H), 2.27 - 2.17 (m, 2H), 2.15 - 1.45 (m, 12H), 1.38 - 1.17 (m, 2H), 0.70 - 0.47 (m, 4H).

LCMS m/z = 448.8 [M/2 +1]$^+$

**Example 91: Preparation of compound 91**

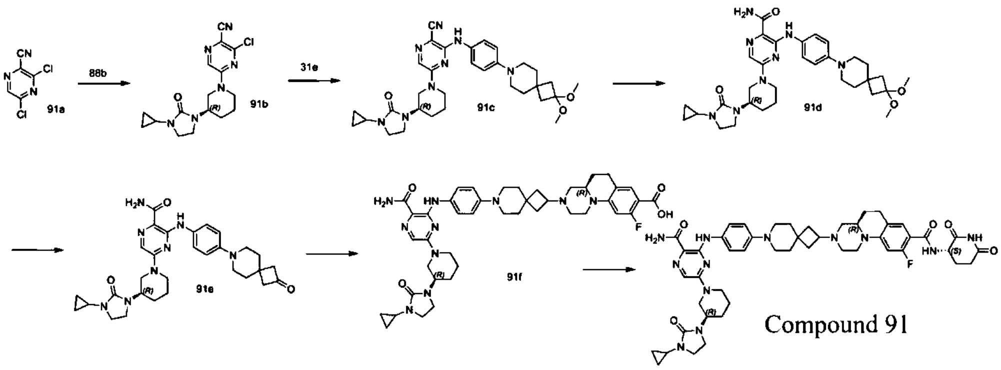


91a 91b 91c 91d 91e 91f Compound 91

Starting from compound 91a and the trifluoroacetate of 88b and following the synthetic methods of examples 31, 33 and 63, compound 91 was obtained.

$^{1}$H NMR (400 MHz, DMSO-$d_6$) δ 11.00 (s, 1H), 10.81 (s, 1H), 7.93 (t, 1H), 7.69 (s, 1H), 7.60 (s, 1H), 7.46 - 7.32 (m, 3H), 7.25 (s, 1H), 6.94 - 6.82 (m, 2H), 6.67 (d, 1H), 4.80 - 4.65 (m, 1H), 4.44 - 4.18 (m, 2H), 3.94 - 3.80 (m, 1H), 3.70 - 3.52 (m, 1H), 3.38 - 3.18 (m, 5H), 3.16 - 2.60 (m, 13H), 2.58 - 2.35 (m, 2H), 2.20 - 1.45 (m, 18H), 0.68 - 0.54 (m, 4H).

LCMS m/z = 452.4 [M/2 +1]$^+$

**Example 92: Preparation of compound 92**

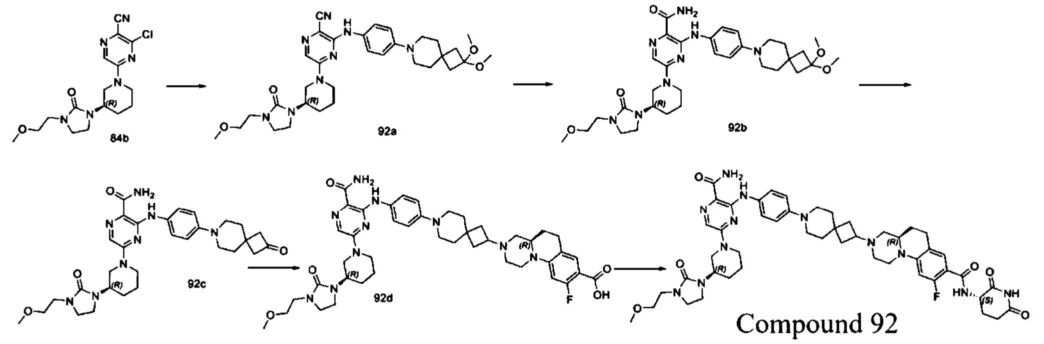

84b 92a 92b 92c 92d

Compound 92

Starting from compounds 84b and 31e and following the synthetic methods of examples 31 and 33, compound 92 was obtained.

$^1$H NMR (400 MHz, DMSO-$d_6$) δ 11.00 (s, 1H), 10.82 (s, 1H), 7.94 (t, 1H), 7.69 (s, 1H), 7.60 (s, 1H), 7.46 - 7.31 (m, 3H), 7.26 (s, 1H), 6.92 - 6.82 (m, 2H), 6.68 (d, 1H), 4.80 - 4.65 (m, 1H), 4.44 - 4.18 (m, 2H), 3.94 - 3.80 (m, 1H), 3.70 - 3.52 (m, 1H), 3.50 - 3.42 (m, 2H), 3.38 - 3.20 (m, 10H), 3.16 - 2.60 (m, 13H), 2.58 - 2.41 (m, 1H), 2.20 - 1.45 (m, 18H).

LCMS m/z = 461.4 $[M/2 +1]^+$

**Example 93: Preparation of trifluoroacetate of compound 93**

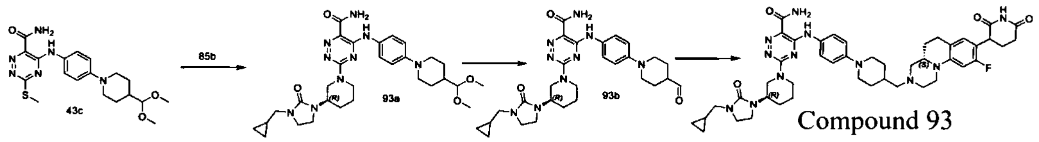


43c 93a 93b

Compound 93

Starting from compound 43c, the trifluoroacetate of 85b and 2-D and following the synthetic method of example 43, the trifluoroacetate of compound 93 was obtained by acidic preparative chromatography [water (containing 0.1 % TFA)/acetonitrile] and lyophilisation.

$^1$H NMR (400 MHz, DMSO-$d_6$) δ 11.24 (s, 1H), 10.79 (s, 1H), 8.32 (s, 1H), 7.74 (s, 1H), 7.63 - 7.51 (m, 2H), 7.10 - 6.72 (m, 4H), 5.00 - 4.30 (m, 2H), 4.20 - 4.00 (m, 1H), 3.95 - 3.77 (m, 1H), 3.77 - 3.55 (m, 4H), 3.47 - 3.25 (m, 6H), 3.23 - 2.57 (m, 14H), 2.57 - 2.45 (m, 1H), 2.24 - 1.22 (m, 13H), 0.98 - 0.80 (m, 1H), 0.58 - 0.40 (m, 2H), 0.28 - 0.10 (m, 2H).

LCMS m/z = 425.3 $[M/2 +1]^+$

**Example 94: Preparation of compound 94**

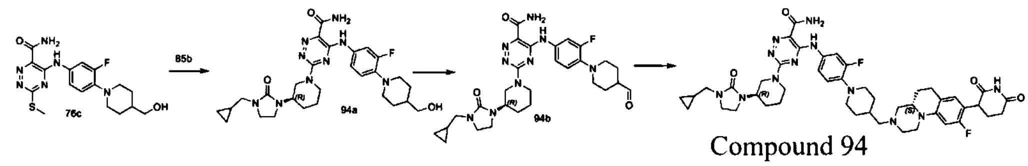


76c 94a 94b

Compound 94

Starting from compound 76c and the trifluoroacetate of 85b and following the synthetic method of example 76, compound 94 was obtained.

$^1$H NMR (400 MHz, DMSO-$d_6$) δ 11.25 (s, 1H), 10.75 (s, 1H), 8.31 (s, 1H), 7.71 (s, 1H), 7.63 - 7.20 (m, 2H), 7.04 (t, 1H), 6.79 (d, 1H), 6.62 (d, 1H), 5.20 - 4.10 (m, 2H), 3.88 - 3.55 (m, 3H), 3.45 - 3.22 (m, 6H), 3.20 - 2.55 (m, 13H), 2.54 - 2.40 (m, 1H), 2.28 - 1.45 (m, 15H), 1.37 - 1.20 (m, 2H), 0.95 - 0.80 (m, 1H), 0.52 - 0.42 (m, 2H), 0.22 - 0.12 (m, 2H).

LCMS m/z = 434.3 $[M/2 +1]^+$

**Example 95: Preparation of compound 95**

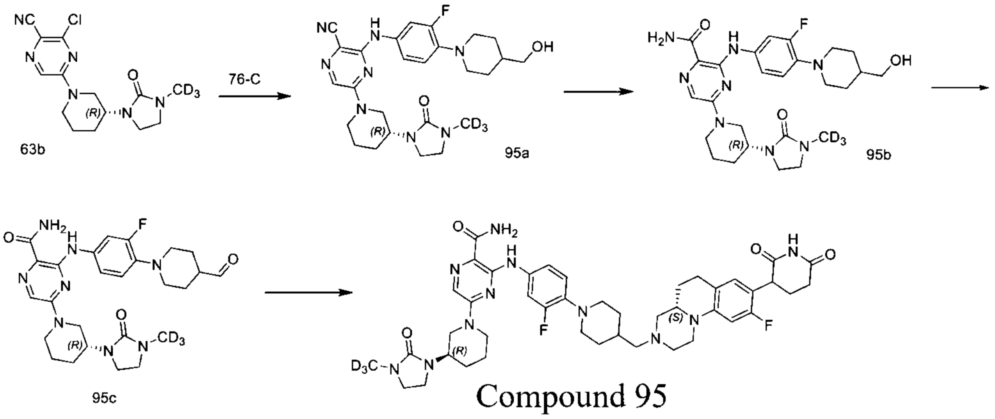


63b 95a 95b 95c

Compound 95

Starting from compounds 63b, 76-C and 2-D and following the synthetic method of example 66, compound 95 was obtained.

$^1H$ NMR (400 MHz, DMSO-$d_6$) δ 11.22 (s, 1H), 10.75 (s, 1H), 7.75 (s, 1H), 7.67 (s, 1H), 7.49 (dd, 1H), 7.33 (s, 1H), 7.26 - 7.18 (m, 1H), 6.97 (t, 1H), 6.79 (d, 1H), 6.62 (d, 1H), 4.38 - 4.20 (m, 2H), 3.88 - 3.52 (m, 3H), 3.40 - 3.20 (m, 6H), 3.15 - 2.82 (m, 5H), 2.80 - 2.55 (m, 6H), 2.55 - 2.45 (m, 1H), 2.30 - 1.46 (m, 15H), 1.38 - 1.20 (m, 2H).

LCMS m/z = 829.4 $[M+1]^+$

**Example 96: Preparation of compound 96**

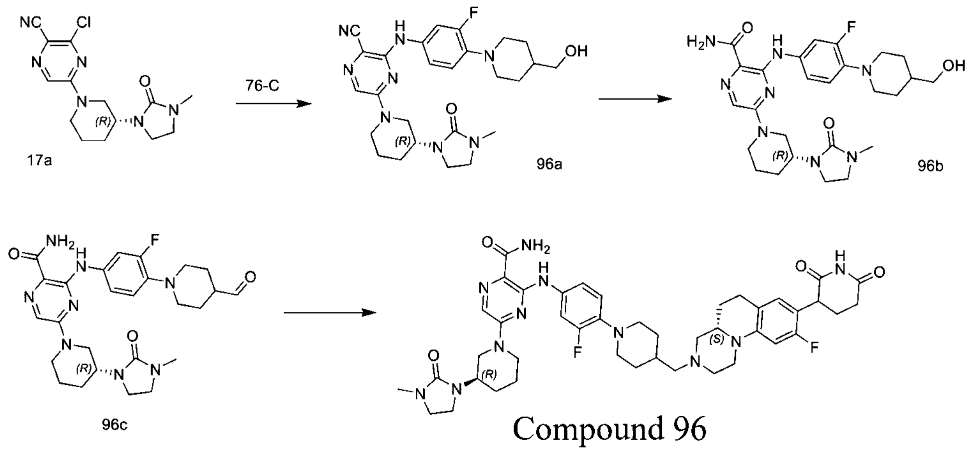


17a 96a 96b 96c

Compound 96

Starting from compounds 17a, 76-C and 2-D and following the synthetic method of example 66, compound 96 was obtained.

$^1H$ NMR (400 MHz, DMSO-$d_6$) δ 11.22 (s, 1H), 10.75 (s, 1H), 7.75 (s, 1H), 7.67 (s, 1H), 7.49 (dd, 1H), 7.33 (s, 1H), 7.26 - 7.18 (m, 1H), 6.97 (t, 1H), 6.79 (d, 1H), 6.62 (d, 1H), 4.38 - 4.20 (m, 2H), 3.88 - 3.52 (m, 3H), 3.40 - 3.20 (m, 6H), 3.15 - 2.82 (m, 5H), 2.80 - 2.55 (m, 9H), 2.55 - 2.45 (m, 1H), 2.30 - 1.46 (m, 15H), 1.38 - 1.20 (m, 2H).

LCMS m/z = 826.4 $[M+1]^+$

**Biological test examples**

**1. Cell proliferation inhibition experiment**

SU-DHL-4 cells were seeded when the density reached $5 \times 10^6$ cells/mL, and Mino cells were seeded at $2 \times 10^6$ cells/mL. The cells in the culture dish/flask were uniformly mixed using a pipette, transferred to a 15 mL (50 mL) sterile centrifuge tube, and centrifuged at 1500 rpm for 3 minutes. The centrifuge tube was taken out after centrifugation, and the supernatant was discarded. The cells were resuspended by adding 5 mL of a culture medium to the centrifuge tube and then counted. Based on the counting results, the SU-DHL-4 cells were diluted into 20,000 cells/90 μL, and the Mino cells were diluted into 5,000 cells/90 μL. The cells were seeded using a multi-channel pipette, with 90 μL per well. During

seeding, the cells in the loading slot should be mixed uniformly, and the cell information was labelled on the 96-well plate.

For SU-DHL-4 cells: P13I and the test compound stock solutions (10 mM) were used. A 3-fold serial dilution was performed over 9 concentration points, starting from an initial concentration of 10,000 nM. The compound concentrations were: 10,000, 3,333.33, 1,111.11, 370.37, 123.46, 41.15, 13.72, 4.57, and 1.52 nM. Dilution method: In well 1, 5 μL of the compound stock solution was added to 495 μL of culture medium. In wells 2 through 9, 80 μL of culture medium containing 1% DMSO was added to each well, followed by the addition of 40 μL of the dilution from the preceding well.

For Mino cells: P13I and the test compound stock solutions (10 mM) were used. A 5-fold serial dilution was performed over 6 concentration points, starting from an initial concentration of 10,000 nM. The compound concentrations were: 10,000, 2,000, 400, 80, 16, and 3.2 nM. Dilution method: In well 1, 5 μL of the compound stock solution was added to 495 μL of culture medium. In wells 2 through 6, 80 μL of culture medium containing 1% DMSO was added to each well, followed by the addition of 20 μL of the dilution from the preceding well.

10 μL/well of the compounds were added to the 96-well plate in which the cells were already seeded. Each concentration was tested in triplicate wells. The last column contained the DMSO vehicle control group. The plate was then incubated for 72 hours at 37°C under 5% $CO_2$.

After 72 hours, 100 μL of a detection reagent (Cell Viability Assay, Promega, G7573) was added to each well, and the plate was uniformly mixed for 2 minutes and incubated at room temperature for 10 minutes. The chemiluminescence reading was then measured using a microplate reader.

Cell proliferation inhibition determination ($IC_{50}$): The $IC_{50}$ values of the compounds for inhibiting cell proliferation were calculated using origin9.2 software.

**Conclusion:** The compounds of the present invention, such as the example compounds, have a good inhibitory effect on the proliferation of Mino cells (mantle cell lymphoma cells).

**2. TMD-8 cell proliferation inhibitory activity test:**

TMD-8 is a human lymphoma cell line that expresses BTK protein and is used to evaluate the efficacy of compounds. TMD-8 cells were maintained in RPMI-1640 + 10% FBS + 1% penicillin-streptomycin solution, and cultured in a 37°C, 5% $CO_2$ incubator. The cells were seeded into a 96-well culture plate (Biosharp, BS-MP-96W) at a density of 3,000 cells per well. After seeding, compounds at various concentrations were added, with a final volume of 100 μL per well, and the plate was incubated for 72 hours in the 37°C, 5% $CO_2$ incubator. Following incubation, 50 μL of a detection solution (CellTiter-Glo®, Promega, G7573) was added to each well, and the plate was uniformly mixed for 2 minutes and incubated at room temperature for 10 minutes. The chemiluminescence reading was then measured using a microplate reader (Molecular Devices, SpectraMax Paradigm). The inhibition rate of cell proliferation by the compounds was calculated according to Equation (1), where $RLU_{compound}$ represents the readout from the compound-treated well, $RLU_{control}$ represents the readout from the DMSO vehicle control well, and $RLU_{blank}$ represents the readout from the cell-free well. Data analysis was performed using Graphpad Prism 8.0 software. The concentration-response curve was generated by fitting the data to a four-parameter equation, and the $IC_{50}$ values for the compound's inhibition of cell proliferation were calculated.

$$Inh\% = 100\% - (RLU_{compound} - RLU_{blank})/(RLU_{control} - RLU_{blank}) * 100\% \quad \text{Equation (1)}$$

Table 1. $IC_{50}$ values for inhibiting TMD-8 cell proliferation

| Serial No. | Compound No. | $IC_{50}$ (nM) |
|---|---|---|
| 1 | Compound 11 | <20 |
| 2 | Compound 12 | <20 |
| 3 | Compound 17 | <20 |
| 4 | Compound 18 | <20 |
| 5 | Compound 19 | <20 |
| 6 | Compound 20 | <20 |

**Conclusion:** The compounds of the present invention, such as the example compounds, have a good inhibitory effect on the proliferation of TMD-8 cells.

### 3. Detection of BTK degradation in Mino cells

Mino is a human mantle cell lymphoma cell line purchased from ATCC and cultured under the following conditions: RPMI-1640 + 15% FBS + 1% penicillin-streptomycin solution. The cells were cultured in a 37°C, 5% $CO_2$ incubator. The cells were seeded in a 6-well plate, with $5 \times 10^5$ cells/well. After seeding, compounds at various concentrations were added, and the plate was incubated for 6 hours in the 37°C, 5% $CO_2$ incubator. Following incubation, the cells were harvested and RIPA lysis buffer (beyotime, Cat. P0013B) was added. The cells were lysed on ice for 15 minutes and centrifuged at 12,000 rpm at 4°C for 10 minutes. The supernatant protein samples were collected and the protein was quantified using the BCA kit (Beyotime, Cat. P0009). Then the protein was diluted to 0.2 mg/mL. The expressions of BTK (CST, Cat. 8547S) and the internal reference β-actin (CST, Cat. 3700S) were detected using a fully automated western blot quantitative analyser (Proteinsimple). Using the "Compass for SW" software, the relative peak area of BTK was calculated when the internal reference area was 10,000. The degradation rate of BTK at various drug concentrations was calculated according to Equation (2), where $BTK_{treat}$ represents the relative peak area of the compound-treated group, and $BTK_{solvent}$ represents the relative peak area of the solvent control group. The processed data obtained from Equation (2) were analysed using GraphPad Prism 8.3.0 software, and the $DC_{50}$ value was calculated using a four-parameter nonlinear regression model.

$$\text{BTK Deg.\%} = 100\% - (BTK_{treat}/BTK_{solvent} \times 100\%) \quad \text{Equation 2}$$

Table 2 $DC_{50}$ values for BTK degradation in Mino cells

| Serial No. | Compound No. | $DC_{50}$ (nM) | Serial No. | Compound No. | $DC_{50}$ (nM) |
|---|---|---|---|---|---|
| 1 | Compound 11 | <10 | 27 | Trifluoroacetate of compound 45 | <10 |
| 2 | Compound 12 | <10 | 28 | Trifluoroacetate of compound 46 | <10 |
| 3 | Compound 13 | <10 | 29 | Compound 47 | <10 |
| 4 | Compound 14 | <10 | 30 | Compound 48 | <10 |
| 5 | Compound 17 | <10 | 31 | Compound 49 | <10 |
| 6 | Compound 19 | <10 | 32 | Compound 50 | <10 |
| 7 | Compound 21 | <10 | 33 | Compound 51 | <10 |
| 8 | Compound 22 | <10 | 34 | Compound 52 | <10 |
| 9 | Compound 27 | <10 | 35 | Compound 53 | <10 |
| 10 | Compound 28 | <10 | 36 | Compound 54 | <10 |
| 11 | Compound 29 | <10 | 37 | Compound 55 | <10 |
| 12 | Compound 30 | <10 | 38 | Compound 56 | <10 |
| 13 | Compound 31 | <10 | 39 | Compound 57 | <10 |
| 14 | Compound 32 | <10 | 40 | Compound 58 | <10 |
| 15 | Compound 33 | <10 | 41 | Compound 59 | <10 |
| 16 | Compound 34 | <10 | 42 | Compound 60 | <10 |
| 17 | Trifluoroacetate of compound 35 | <10 | 43 | Compound 61 | <10 |
| 18 | Trifluoroacetate of compound 36 | <10 | 44 | Compound 62 | <10 |
| 19 | Compound 37 | <10 | 45 | Compound 63 | <10 |
| 20 | Compound 38 | <10 | 46 | Compound 64 | <10 |
| 21 | Compound 39 | <10 | 47 | Compound 65 | <10 |
| 22 | Compound 40 | <10 | 48 | Compound 66 | <10 |
| 23 | Trifluoroacetate of compound 41 | <10 | 49 | Compound 67 | <10 |
| 24 | Compound 42 | <10 | 50 | Compound 68 | <10 |

(continued)

| Serial No. | Compound No. | $DC_{50}$ (nM) | Serial No. | Compound No. | $DC_{50}$ (nM) |
|---|---|---|---|---|---|
| 25 | Compound 43 | <10 | 51 | Compound 76 | <10 |
| 26 | Compound 44 | <10 | 52 | Compound 78 | <10 |

**Conclusion:** The compounds of the present invention, such as the example compounds, have a good degradation effect on BTK in Mino cells.

**4. Detection of BTK degradation in REC-1 BTK L528W mutant cells**

REC-1 is a human lymphoma cell line. A BTK L528W knock-in mutant cell line was constructed in REC-1 cells using the CRISPR/Cas9 gene editing technology under the following culture conditions: RPMI-1640 + 10% FBS + 1% penicillin-streptomycin solution. The cells were cultured in a 37°C, 5% $CO_2$ incubator. The cells were seeded in a 6-well plate, with $5 \times 10^5$ cells/well. After seeding, compounds at various concentrations were added, and the plate was incubated for 6 hours in the 37°C, 5% $CO_2$ incubator. Following incubation, the cells were harvested and RIPA lysis buffer (beyotime, Cat. P0013B) was added. The cells were lysed on ice for 15 minutes and centrifuged at 12,000 rpm at 4°C for 10 minutes. The supernatant protein samples were collected and the protein was quantified using the BCA kit (Beyotime, Cat. P0009). Then the protein was diluted to 0.2 mg/mL. The expressions of BTK (CST, Cat. 8547S) and the internal reference β-actin (CST, Cat. 3700S) were detected using a fully automated western blot quantitative analyser (Proteinsimple). Using the "Compass for SW" software, the relative peak area of BTK was calculated when the internal reference area was 10,000. The degradation rate of BTK at various drug concentrations was calculated according to Equation (3), where $BTK_{treat}$ represents the relative peak area of the compound-treated group, and $BTK_{solvent}$ represents the relative peak area of the solvent control group. The processed data obtained from Equation (3) were analysed using GraphPad Prism 8.3.0 software, and the $DC_{50}$ value was calculated using a four-parameter nonlinear regression model.

$$\text{BTK Deg.\%} = 100\% - (BTK_{treat}/BTK_{solvent} \times 100\%) \quad \text{Equation 3}$$

Table 3 $DC_{50}$ values for BTK degradation in REC-1 BTK L528W cells

| Serial No. | Compound No. | $DC_{50}$ (nM) | Serial No. | Compound No. | $DC_{50}$ (nM) |
|---|---|---|---|---|---|
| 1 | Compound 11 | <5 | 18 | Compound 50 | <5 |
| 2 | Compound 12 | <5 | 19 | Compound 51 | <5 |
| 3 | Compound 14 | <5 | 20 | Compound 52 | <5 |
| 4 | Compound 17 | <5 | 21 | Compound 53 | <5 |
| 5 | Compound 19 | <5 | 22 | Compound 55 | <5 |
| 6 | Compound 22 | <5 | 23 | Compound 56 | <5 |
| 7 | Compound 33 | <5 | 24 | Compound 57 | <5 |
| 8 | Compound 34 | <5 | 25 | Compound 58 | <5 |
| 9 | Trifluoroacetate of compound 35 | <5 | 26 | Compound 59 | <5 |
| 10 | Trifluoroacetate of compound 36 | <5 | 27 | Compound 61 | <5 |
| 11 | Trifluoroacetate of compound 41 | <5 | 28 | Compound 62 | <5 |
| 12 | Compound 42 | <5 | 29 | Compound 63 | <5 |
| 13 | Compound 43 | <5 | 30 | Compound 64 | <5 |
| 14 | Compound 44 | <5 | 31 | Compound 66 | <5 |
| 15 | Compound 47 | <5 | 32 | Compound 67 | <5 |
| 16 | Compound 48 | <5 | 33 | Compound 68 | <5 |

(continued)

| Serial No. | Compound No. | $DC_{50}$ (nM) | Serial No. | Compound No. | $DC_{50}$ (nM) |
|---|---|---|---|---|---|
| 17 | Compound 49 | <5 | | | |

Table 4 BTK degradation rate at 5 nM concentration in REC-1 BTK L528W cells

| Serial No. | Compound No. | BTK degradation rate % | Serial No. | Compound No. | BTK degradation rate % |
|---|---|---|---|---|---|
| 1 | Compound 12 | >90% | 18 | Compound 59 | >75% |
| 2 | Compound 17 | >90% | 19 | Compound 61 | >90% |
| 3 | Compound 19 | >90% | 20 | Compound 62 | >90% |
| 4 | Compound 33 | >90% | 21 | Compound 63 | >90% |
| 5 | Compound 34 | >90% | 22 | Compound 64 | >90% |
| 6 | Trifluoroacetate of compound 41 | >90% | 23 | Compound 66 | >80% |
| 7 | Compound 43 | >90% | 24 | Compound 67 | >90% |
| 8 | Compound 44 | >90% | 25 | Compound 68 | >90% |
| 9 | Compound 47 | >90% | 26 | Compound 76 | >90% |
| 10 | Compound 48 | >90% | 27 | Compound 77 | >90% |
| 11 | Compound 51 | >90% | 28 | Compound 78 | >90% |
| 12 | Compound 52 | >90% | 29 | Compound 82 | >85% |
| 13 | Compound 53 | >90% | 30 | Compound 83 | >85% |
| 14 | Compound 55 | >90% | 31 | Compound 84 | >70% |
| 15 | Compound 56 | >90% | 32 | Compound 89 | >80% |
| 16 | Compound 57 | >90% | 33 | Trifluoroacetate of compound 93 | >70% |
| 17 | Compound 58 | >90% | 34 | | |

**Conclusion:** The compounds of the present invention, such as the example compounds, have a good degradation effect on BTK in REC-1 BTK L528W cells.

**5. Pharmacokinetic test in rats**

**Experimental objective:** In this experiment, a single dose of the test compound was administered to SD rats via intravenous administration and intragastric gavage, and the concentration of the test compound in plasma of the rats was measured to evaluate the pharmacokinetic profile of the test compound in the rats.

**Experimental animals:** Male SD rats, 200-220 g, 6-8 weeks old, 6 rats/compound, purchased from Chengdu Ddossy Experimental Animals Co., Ltd.

**Experimental method:** On the day of the experiment, 6 SD rats were randomly grouped according to their body weights. The animals were fasted with water available for 12 to 14 h one day before the administration, and were fed 4 h after the administration.

Table 5 Administration information of pharmacokinetic test in rats

| Group | Number | Administration information | | | | | | |
|---|---|---|---|---|---|---|---|---|
| | Male | Test compound | Administration dosage* (mg/kg) | Administration concentration (mg/mL) | Administration volume (mL/kg) | Collected sample | Mode of administration | Vehicle |
| G1 | 3 | Compound of the present invention | 10 | 1 | 10 | Plasma | Oral administration (intragastric gavage) | Vehicle 1: 5% DMSO+ 5% Solutol+ 30% PEG-400+60% (20% SBE-β-CD) |
| | | | | | | | | Vehicle 2: 5% DMSO+5% HS-15+10% PEG400+80 % (20% SBE-β-CD) |
| G2 | 3 | Compound of the present invention | 2.5 | 0.5 | 5 | Plasma | Intravenous injection | 10% DMA+10% Solutol+80% Saline |
| *Dosage is calculated based on a free base. | | | | | | | | |

**Sampling:** Before and after the administration, 0.06-0.15 mL of blood was taken from the orbits under isoflurane anaesthesia, and placed in an EDTAK2 centrifuge tube. Centrifugation was carried out at 5000 rpm at 4°C for 10 min, and the plasma was collected.

The plasma collection time points for PO groups were 0, 5 min, 15 min, 30 min, 1 h, 2 h, 4 h, 7 h, 24 h, and 48 h.

The plasma collection time points for IV groups were 0, 5 min, 15 min, 30 min, 1 h, 2 h, 4 h, 7 h, and 24 h.

Before analysis and detection, all samples were stored at -60°C. The samples were analysed quantitatively by LC-MS/MS.

Table 6 Pharmacokinetic parameters of compounds of the present invention in plasma of rats

| Test compound | Mode of administration | Vehicle type | $AUC_{0-t}$ (hr*ng/mL) | F (%) |
|---|---|---|---|---|
| Compound 11 | i.g. (10 mg/kg) | Vehicle 1 | >1000 | >50% |
| Compound 13 | i.g. (10 mg/kg) | Vehicle 2 | >1000 | - |
| Compound 14 | i.g. (10 mg/kg) | Vehicle 2 | >1000 | - |
| Compound 21 | i.g. (10 mg/kg) | Vehicle 2 | >1000 | - |
| Compound 22 | i.g. (10 mg/kg) | Vehicle 2 | >1000 | - |
| Compound 33 | i.g. (10 mg/kg) | Vehicle 2 | >1000 | - |
| Trifluoroacetate of compound 35 | i.g. (10 mg/kg) | Vehicle 2 | >1000 | - |
| Trifluoroacetate of compound 41 | i.g. (10 mg/kg) | Vehicle 1 | >1000 | - |
| Compound 42 | i.g. (10 mg/kg) | Vehicle 2 | >1000 | - |
| Compound 43 | i.g. (10 mg/kg) | Vehicle 2 | >1000 | - |
| Compound 44 | i.g. (10 mg/kg) | Vehicle 2 | >1000 | - |
| Trifluoroacetate of compound 45 | i.g. (10 mg/kg) | Vehicle 2 | >1000 | - |
| Trifluoroacetate of compound 46 | i.g. (10 mg/kg) | Vehicle 2 | >1000 | - |

(continued)

| Test compound | Mode of administration | Vehicle type | $AUC_{0-t}$ (hr*ng/mL) | F (%) |
|---|---|---|---|---|
| Compound 48 | i.g. (10 mg/kg) | Vehicle 2 | >1000 | - |
| Compound 49 | i.g. (10 mg/kg) | Vehicle 2 | >1000 | - |
| Compound 51 | i.g. (10 mg/kg) | Vehicle 2 | >1000 | - |
| Compound 52 | i.g. (10 mg/kg) | Vehicle 2 | >1000 | >50% |
| Compound 53 | i.g. (10 mg/kg) | Vehicle 2 | >1000 | >50% |
| Compound 54 | i.g. (10 mg/kg) | Vehicle 2 | >1000 | >50% |
| Compound 55 | i.g. (10 mg/kg) | Vehicle 2 | >1000 | - |
| Compound 56 | i.g. (10 mg/kg) | Vehicle 2 | >1000 | - |
| Compound 57 | i.g. (10 mg/kg) | Vehicle 2 | >1000 | - |
| Compound 58 | i.g. (10 mg/kg) | Vehicle 2 | >1000 | - |
| Compound 59 | i.g. (10 mg/kg) | Vehicle 2 | >1000 | - |
| Compound 60 | i.g. (10 mg/kg) | Vehicle 2 | >1000 | >50% |
| Compound 62 | i.g. (10 mg/kg) | Vehicle 2 | >1000 | - |
| Compound 63 | i.g. (10 mg/kg) | Vehicle 2 | >1000 | - |
| Compound 64 | i.g. (10 mg/kg) | Vehicle 2 | >1000 | - |
| Compound 65 | i.g. (10 mg/kg) | Vehicle 2 | >1000 | - |
| Compound 76 | i.g. (10 mg/kg) | Vehicle 2 | >1000 | - |
| Compound 77 | i.g. (10 mg/kg) | Vehicle 2 | >1000 | - |
| Compound 78 | i.g. (10 mg/kg) | Vehicle 2 | >1000 | - |

Conclusion: The compounds of the present invention, such as the example compounds, have good oral absorption in rats.

**4. Caco2 permeability test**

In the experiment, a monolayer of Caco-2 cells was used, and incubated in triplicate in a 96-well Transwell plate. A transport buffer solution (HBSS, 10 mM HEPES, pH 7.4 ± 0.05) containing the compound of the present invention (2 μM) or the control compound digoxin (10 μM), nadolol (2 μM) or metoprolol (2 μM) was added to the administration end hole on the apical side or basal side. A DMSO-containing transport buffer solution was added to the corresponding receiving end hole. After incubation for 2 hours at 37°C ± 1°C, the cell plate was removed, and an appropriate amount of samples was taken from both the apical side and basal side and transferred to a new 96-well plate. Subsequently, acetonitrile containing an internal standard was added to precipitate the protein. The samples were analysed using LC MS/MS, and the concentrations of the compound of the present invention and the control compound were determined. Concentration data were used to calculate apparent permeability coefficients for transport from the apical side to the basal side, and from the basal side to the apical side of the cell monolayer, and thus to calculate the efflux ratio. The integrity of the cell monolayer after 2 hours of incubation was evaluated by leakage of Lucifer Yellow.

Table 7 Caco2 test results of compounds of the present invention

| Test compound | Mean $P_{app}$ ($10^{-6}$ cm/s) | | Efflux Ratio |
|---|---|---|---|
| | A to B | B to A | |
| Compound 11 | 1.75 | 3.74 | 2.14 |
| Compound 12 | 1.18 | 4.71 | 3.99 |
| Compound 14 | 0.35 | 0.867 | 2.47 |
| Compound 17 | 2.20 | 0.652 | 0.296 |

(continued)

| Test compound | Mean $P_{app}$ ($10^{-6}$ cm/s) | | Efflux Ratio |
|---|---|---|---|
| | A to B | B to A | |
| Compound 19 | 2.26 | 1.09 | 0.481 |
| Compound 22 | 2.38 | 4.44 | 1.87 |
| Compound 33 | 0.666 | 0.323 | 0.485 |
| Trifluoroacetate of compound 41 | 0.553 | 4.08 | 7.37 |
| Compound 43 | 1.02 | 4.59 | 4.50 |
| Compound 44 | 0.655 | 8.19 | 12.5 |
| Compound 48 | 1.41 | 5.48 | 3.90 |
| Compound 51 | 1.90 | 4.63 | 2.43 |
| Compound 56 | 0.444 | 1.85 | 4.17 |
| Compound 57 | 0.59 | 7.12 | 12.1 |
| Compound 58 | 1.02 | 8.66 | 8.50 |
| Compound 63 | 0.796 | 4.89 | 6.14 |
| Compound 64 | 0.763 | 4.12 | 5.40 |
| Compound 76 | 1.11 | 4.63 | 4.16 |
| Compound 77 | 0.984 | 4.89 | 4.97 |
| Compound 78 | 1.65 | 5.81 | 3.52 |
| Control compound 1 | 0.101 | 5.27 | 52.5 |

Conclusion: The compounds of the present invention, such as the example compounds, have good Caco2 permeability.

**5. Pharmacokinetic test in mice**

**Experimental objective:** In this experiment, a single dose of the test compound was administered to ICR mice via intravenous administration and intragastric gavage, and the concentration of the test compound in plasma of the mice was measured to evaluate the pharmacokinetic profile of the test compound in the mice.

**Experimental animals:** Male ICR mice, 6 mice/compound, purchased from Chengdu Ddossy Experimental Animals Co., Ltd.

**Experimental method:** On the day of the experiment, 6 SD mice were randomly grouped according to their body weights. The animals were fasted with water available for 12 to 14 h one day before the administration, and were fed 4 h after the administration.

Table 8 Administration information of pharmacokinetic test in mice

| Group | Number | Administration information | | | | | | |
|---|---|---|---|---|---|---|---|---|
| | Male | Test compound | Administration dosage* (mg/kg) | Administration concentration (mg/mL) | Administration volume (mL/kg) | Collected sample | Mode of administration | Vehicle |
| G1 | 3 | Compound of the present invention | 10 | 1 | 10 | Plasma | Oral administration (intragastric gavage) | Vehicle 1: 5% DMSO+5% Solutol+30% PEG400+60 % (20% SBE-CD) |
| | | | | | | | | Vehicle 2: 5% DMSO+5% Solutol+10% PEG400+80 % (20% SBE-CD) |
| G2 | 3 | Compound of the present invention | 2.5 | 0.5 | 5 | Plasma | Intravenous injection | 5% DMA + 5% Solutol + 90% Saline |

*Dosage is calculated based on a free base.

**Sampling:** Before and after the administration, 0.06 mL of blood was taken from the orbits under isoflurane anaesthesia, and placed in an EDTAK2 centrifuge tube. Centrifugation was carried out at 5000 rpm at 4°C for 10 min, and the plasma was collected.

The plasma collection time points for PO groups were 0, 5 min, 15 min, 30 min, 1 h, 2 h, 4 h, 7 h, 24 h, and 48 h.

The plasma collection time points for IV groups were 0, 5 min, 15 min, 30 min, 1 h, 2 h, 4 h, 7 h, 24 h, and 48 h.

Before analysis and detection, all samples were stored at -60°C. The samples were analysed quantitatively by LC-MS/MS.

Conclusion: The compounds of the present invention, such as the example compounds, have good oral absorption in mice.

### 6. Pharmacokinetic test in beagle dogs

**Experimental animals:** Male beagle dogs, about 8 to 10 kg, 6 beagle dogs/compound, purchased from Beijing Marshall Biotechnology Co., Ltd.

**Experimental method:** On the day of the experiment, 6 beagle dogs were randomly grouped according to their body weights. The animals were fasted with water available for 14 to 18 h one day before the administration, and were fed 4 h after the administration.

Table 9 Administration information of pharmacokinetic test in beagle dogs

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | Compound of the present invention | 0.5 | 0.5 | 1 | Plasma | Intravenous administration |
| G2 | 3 | Compoun d of the present invention | 2 | 0.4 | 5 | Plasma | Oral administration (intragastric gavage) |
| Note: Vehicle for intravenous administration: 5% DMA+5% Solutol+90% NS; vehicle for oral administration (intragastric gavage): 5% DMSO+5% Solutol+10% PEG400+80% (20% SBE-CD);<br>*Dosage is calculated based on a free base. | | | | | | | |

Before and after the administration, 1 ml of blood was taken from the jugular veins or limb veins, and placed in an EDTAK2 centrifuge tube. Centrifugation was carried out at 5000 rpm at 4°C for 10 min, and the plasma was collected. The blood collection time points for the intravenous administration group and intragastric gavage group were 0, 5 min, 15 min, 30 min, 1 h, 2 h, 4 h, 6 h, 8 h, 10 h, 12 h, 24 h and 48 h. Before analysis and detection, all samples were stored at -80°C. The samples were analysed quantitatively by LC-MS/MS.

Conclusion: The compounds of the present invention, such as the example compounds, have good oral absorption in beagle dogs.

### 7. Pharmacokinetic test in monkeys

**Experimental animals:** Male cynomolgus monkeys, 3-5 kg, 3-6 years old, 4 monkeys/compound, purchased from Suzhou Xishan Biotechnology Inc.

**Experimental method:** On the day of the experiment, 4 monkeys were randomly grouped according to their body weights. The animals were fasted with water available for 14 to 18 h one day before the administration, and were fed 4 h after the administration.

Table 10 Administration information of pharmacokinetic test in monkeys

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 2 | Compound of the present invention | 0.5 | 0.5 | 1 | Plasma | Intravenous administration |
| G2 | 2 | Compound of the present invention | 2 | 0.4 | 5 | Plasma | Oral administration (intragastric gavage) |
| Note: Vehicle for intravenous administration: 5% DMA+5% Solutol+90% NS; vehicle for oral administration (intragastric gavage): 5% DMSO+5% Solutol+10% PEG400+80% (20% SBE-CD);<br>*Dosage is calculated based on a free base. | | | | | | | |

Before and after the administration, 1.0 mL of blood was taken from the jugular veins, and placed in an EDTAK2 centrifuge tube. Centrifugation was carried out at 5000 rpm at 4°C for 10 min, and the plasma was collected. The blood collection time points for the intravenous administration group and intragastric gavage group were 0, 5 min, 15 min, 30 min, 1 h, 2 h, 4 h, 6 h, 8 h, 10 h, 12 h, 24 h and 48 h. Before analysis and detection, all samples were stored at -60°C. The samples were analysed quantitatively by LC-MS/MS.

Conclusion: The compounds of the present invention, such as the example compounds, have good oral absorption in monkeys.

**8. hERG potassium ion channel test**

**Experimental platform:** Electrophysiological manual patch-clamp system

**Cell line:** Chinese hamster ovary (CHO) cell lines stably expressing hERG potassium ion channel

**Experimental method:** In Chinese Hamster Ovary (CHO) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{hERG}$) was depolarised from -80 mV to +20 mV for 2 s, then repolarised to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells (n ≥ 2) were tested at each concentration.

**Data processing:** Data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition\%} = [1 - (I / Io)] \times 100\%$$

where Inhibition% represents the percentage of inhibition of hERG potassium current by the compound, and *I* and *I*o represent the amplitude of hERG potassium current after and before the administration, respectively.

Compound $IC_{50}$ was calculated using GraphPad Prism 5 software by fitting according to the following equation:

$$Y = \text{Bottom} + (\text{Top-Bottom})/(1+10\text{^}((\text{LogIC}_{50}\text{-X})*\text{HillSlope}))$$

Among the equation, X represents the Log value of the tested concentration of the test sample, Y represents the inhibition percentage at the corresponding concentration, and Bottom and Top represent the minimum and maximum inhibition percentage, respectively.

Conclusion: The compounds of the present invention, such as the example compounds, do not exhibit significant inhibitory effects on the hERG potassium channel current.

**9. Liver microsomal stability test**

In this experiment, liver microsomes of five species, including human, dog, rat, and mouse, were used as *in vitro* models to evaluate the metabolic stability of the test compound.

At 37°C, 1 $\mu$M of the test compound was co-incubated with microsomal protein and coenzyme NADPH. At given time points (5 min, 10 min, 20 min, 30 min and 60 min), the reaction was terminated by adding ice-cold acetonitrile containing an internal standard. The LC-MS/MS method was used to measure the concentration of the test compound in the sample. $T_{1/2}$ was calculated using the natural logarithm (ln) of the residual rate of the drug in the incubation system and the incubation time. In addition, the intrinsic clearance in liver microsomes ($CL_{int(mic)}$) and the intrinsic clearance in liver ($CL_{int(Liver)}$) were further calculated.

Conclusion: The compounds of the present invention, such as the example compounds, have good stability in liver microsomes.

**10. CYP450 enzyme inhibition test**

The purpose of this study was to evaluate the effect of the test compound on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were incubated with human liver microsomes and test compounds of various concentrations, and reduced nicotinamide adenine dinucleotide phosphate (NADPH) was added to initiate the reaction. After the reaction was completed, the sample was treated and liquid chromatography-tandem mass spectrometry (LC-MS/MS) was used to quantitatively detect metabolites produced by specific substrates, changes in CYP enzyme activity were determined, and $IC_{50}$ value was calculated to evaluate the inhibitory potential of the test compound on each CYP enzyme subtype.

Conclusion: The compounds of the present invention, such as the example compounds, do not exhibit significant inhibitory effects on the five isozymes of human liver microsomal cytochrome P450.

**11. REC-1 cell proliferation inhibition experiment**

REC-1 is a human lymphoma cell line cultured under the following conditions: RPMI-1640 (ATCC, Cat. 30-2001) +10% FBS (HyClone, Cat. SV3028.02) +1% penicillin-streptomycin solution (Gibco, Cat. 15140-122). The cells were cultured in a 37°C, 5% $CO_2$ incubator. The cells were harvested and seeded into a 96-well culture plate at a density of 3,000 cells per well in 180 $\mu$L of medium. Subsequently, 20 $\mu$L of compounds at various concentrations or 0.1% DMSO were added. A blank control group, consisting of wells containing only 200 $\mu$L of medium, was established. The plate was incubated for 5 days in the 37°C, 5% $CO_2$ incubator. Following incubation, 50 $\mu$L of CellCounting-Lite 2.0 Reagent (Vazyme, Cat. No. DD1101-03) was directly added to each well, and the plate was uniformly mixed for 10 minutes. The fluorescent luminescence signal (RLU) was then read and recorded using a PHERAstar FSX multimode microplate reader (BMG LRBTECH). The cell proliferation inhibition rate at various compound concentrations was calculated according to Equation (4), where $RLU_{DMSO}$ represents the average signal value of the DMSO group, $RLU_{blank}$ represents the average signal value of the blank control group, and $RLU_{compound}$ represents the signal value of the compound-treated group. The processed data obtained from Equation (4) were analysed using GraphPad Prism software, and a four-parameter curve fitting model was applied to calculate the compound concentration corresponding to a 50% inhibition rate, i.e., the $IC_{50}$ value.

$$\text{Inhibition } \% = (1-(RLU_{compound} - RLU_{blank})/(RLU_{DMSO} - RLU_{blank})) \times 100\%$$

Equation (4)

Conclusion: The compounds of the present invention, such as the example compounds, have a significant inhibitory effect on the proliferation of REC-1 cells.

**12. REC-1 BTK L528W mutant cell proliferation inhibition experiment**

REC-1 is a human lymphoma cell line. A BTK L528W knock-in mutant cell line was constructed in REC-1 cells using the CRISPR/Cas9 gene editing technology under the following culture conditions: RPMI-1640 (ATCC, Cat. 30-2001) +10% FBS (HyClone, Cat. SV3028.02) +1% penicillin-streptomycin solution (Gibco, Cat. 15240-062). The cells were cultured in a 37°C, 5% $CO_2$ incubator. The cells were harvested and seeded into a 96-well culture plate at a density of 3,000 cells per well in 180 μL of medium. Subsequently, 20 μL of compounds at various concentrations or 0.1% DMSO were added. A blank control group, consisting of wells containing only 200 μL of medium, was established. The plate was incubated for 5 days in the 37°C, 5% $CO_2$ incubator. Following incubation, 50 μL of CellCounting-Lite 2.0 Reagent (Vazyme, Cat. No. DD1101-03) was directly added to each well, and the plate was uniformly mixed for 10 minutes. The fluorescent luminescence signal (RLU) was then read and recorded using a PHERAstar FSX multimode microplate reader (BMG LRBTECH). The cell proliferation inhibition rate at various compound concentrations was calculated according to Equation (5), where $RLU_{DMSO}$ represents the average signal value of the DMSO group, $RLU_{blank}$ represents the average signal value of the blank control group, and $RLU_{compound}$ represents the signal value of the compound-treated group. The processed data obtained from Equation (5) were analysed using GraphPad Prism software, and a four-parameter curve fitting model was applied to calculate the compound concentration corresponding to a 50% inhibition rate, i.e., the $IC_{50}$ value.

$$\text{Inhibition \%} = (1\text{-}(RLU_{compound} - RLU_{blank})/(RLU_{DMSO} - RLU_{blank})) \times 100\%$$

Equation (5)

Conclusion: The compounds of the present invention, such as the example compounds, have a significant inhibitory effect on the proliferation of REC-1 BTK L528W mutant cells.

**13. OCI-LY10 cell proliferation inhibition experiment**

OCI-LY10 is a human diffuse large B-cell lymphoma cell line cultured under the following conditions: IMDM (Gibco, Cat.12440053) +10% FBS (Avantor, Cat. 76294-180) +1% penicillin-streptomycin solution (Gibco, Cat. 15140-122). The cells were cultured in a 37°C, 5% $CO_2$ incubator. The cells were harvested and seeded into a 384-well culture plate at a density of 5000 cells per well in 40 μL of medium. Subsequently, 40 nL of DMSO or compounds at various concentrations were added. A blank control group, consisting of wells containing only 40 μL of medium, was established. The plate was incubated for 3 days in the 37°C, 5% $CO_2$ incubator. Following incubation, 20 μL of CellCounting-Lite 2.0 Reagent (Vazyme, Cat. No. DD1101-03) was directly added to each well, and the plate was uniformly mixed for 2 minutes and incubated at room temperature for 30 minutes. The fluorescent luminescence signal (RLU) was then read and recorded using a PHERAstar FSX multimode microplate reader (BMG LRBTECH). The cell proliferation inhibition rate at various compound concentrations was calculated according to Equation (6), where $RLU_{DMSO}$ represents the average signal value of the DMSO group, $RLU_{blank}$ represents the average signal value of the blank control group, and $RLU_{compound}$ represents the signal value of the compound-treated group. The processed data obtained from Equation (6) were analysed using XLfit software, and a four-parameter curve fitting model was applied to calculate the compound concentration corresponding to a 50% inhibition rate, i.e., the $IC_{50}$ value.

$$\text{Inhibition \%} = (RLU_{DMSO} - RLU_{compound})/(RLU_{DMSO} - RLU_{blank}) \times 100\%$$

Equation (6)

Conclusion: The compounds of the present invention, such as the example compounds, have a significant inhibitory effect on the proliferation of OCI-LY10 cells.

**14. OCI-LY10 BTK L528W mutant cell proliferation inhibition experiment**

OCI-LY10 is a human diffuse large B-cell lymphoma cell line. A BTK L528W knock-in mutant cell line was constructed in OCI-LY10 cells using the CRISPR/Cas9 gene editing technology under the following culture conditions: IMDM (Gibco, Cat. 12440053) +10% FBS (Avantor, Cat. 76294-180) +1% penicillin-streptomycin solution (Gibco, Cat. 15140-122). The cells were cultured in a 37°C, 5% $CO_2$ incubator. The cells were harvested and seeded into a 384-well culture plate at a density of 5000 cells per well in 40 μL of medium. Subsequently, 40 nL of DMSO or compounds at various concentrations were added. A blank control group, consisting of wells containing only 40 μL of medium, was established. The plate was incubated for 3 days in the 37°C, 5% $CO_2$ incubator. Following incubation, 20 μL of CellCounting-Lite 2.0

Reagent (Vazyme, Cat. No. DD1101-03) was directly added to each well, and the plate was uniformly mixed for 2 minutes and incubated at room temperature for 30 minutes. The fluorescent luminescence signal (RLU) was then read and recorded using a PHERAstar FSX multimode microplate reader (BMG LRBTECH). The cell proliferation inhibition rate at various compound concentrations was calculated according to Equation (7), where $RLU_{DMSO}$ represents the average signal value of the DMSO group, $RLU_{blank}$ represents the average signal value of the blank control group, and $RLU_{compound}$ represents the signal value of the compound-treated group. The processed data obtained from Equation (7) were analysed using XLfit software, and a four-parameter curve fitting model was applied to calculate the compound concentration corresponding to a 50% inhibition rate, i.e., the $IC_{50}$ value.

$$\text{Inhibition \%} = (RLU_{DMSO} - RLU_{compound})/(RLU_{DMSO} - RLU_{blank}) \times 100\%$$

Equation (7)

Conclusion: The compounds of the present invention, such as the example compounds, have a significant inhibitory effect on the proliferation of OCI-LY10 BTK L528W mutant cells.

**15. Human CD34+ hematopoietic stem cell proliferation inhibition experiment**

Human CD34+ Hematopoietic stem cells (TPCS, Cat. hmPB34-P-2CW) are CD34-positive stem cells obtained from human PBMCs through immunomagnetic bead sorting. The culture conditions are as follows: DPBS (Gibco, Cat. 14190-144) +StemSpan SFEM II (STEMCELL, Cat. 9655) +1X StemSpan CD34+ Expansion Supplement (STEMCELL, Cat. 2691). First, 40 nL of DMSO, the positive reference compound Talazoparib, or the test compounds were respectively added to a 384-well plate (Corning, Cat. 3764). Then, a cell suspension was added at 400 cells per well in 40 μL of medium, resulting in final concentrations of 0.1% DMSO, 3 μM Talazoparib, or various concentrations of the compounds in each well. The plate was centrifuged at room temperature at 1000 rpm for 1 minute, followed by incubation for 7 days in a 37°C, 5% $CO_2$ incubator. Following incubation, 20 μL of CellTiter-Glo Reagent (Promega, Cat. G7573) was directly added to each well, and the plate was centrifuged at room temperature at 1000 rpm for 1 minute, followed by incubation for 20 minutes in the dark. Following incubation, the chemiluminescent signal (CFU) was read and recorded using an Envision multimode microplate reader (PerkinElmer, Cat. 2104). The cell proliferation inhibition rate at various compound concentrations was calculated according to Equation (8), where $CFU_{high\ control}$ represents the average signal value of the DMSO group, $CFU_{low\ control}$ represents the average signal value of the Talazoparib group, and $CFU_{compound}$ represents the average signal value of the compound-treated group. The processed data obtained from Equation (8) were analysed using XLfit or GraphPad Prism software, and a four-parameter curve fitting model was applied to calculate the compound concentration corresponding to a 50% inhibition rate, i.e., the $IC_{50}$ value.

$$\text{Inhibiton\%} = (CFU_{high\ control}\text{-}CFU_{compound}) / (CFU_{high\ control}\text{-}CFU_{low\ control}) \times 100\%$$

(Equation 8)

Conclusion: The compounds of the present invention, such as the example compounds, do not have a significant inhibitory effect on the proliferation of CD34+ hematopoietic stem cells.

The structure of the control compound 1 is as follows:

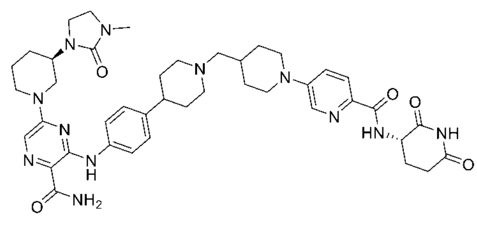.

## Claims

1. A compound or a stereoisomer, tautomer, deuterated compound, solvate, prodrug, metabolite, pharmaceutically acceptable salt or co-crystal thereof, **characterised in that** the compound is a compound represented by general formula (I), wherein

B-L-K (I);

B is

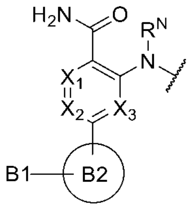

;

L is

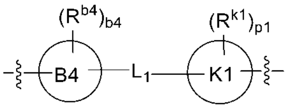

,

which is connected to B on the left side, and is connected to K on the right side;
K is

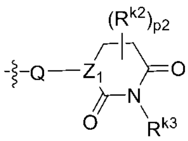

;

B1 is absent or is selected from $C_{3-12}$ carbocyclyl or 4- to 12-membered heterocyclyl, wherein the carbocyclyl or heterocyclyl is optionally substituted with 1 to 4 $R^{b1}$;
B2 is selected from $C_{3-12}$ carbocyclyl or 4- to 12-membered heterocyclyl, wherein the carbocyclyl or heterocyclyl is optionally substituted with 1 to 4 $R^{b2}$;
B4 is selected from phenyl, 5- to 6-membered heteroaryl, a 9- to 12-membered fused bicyclic group,

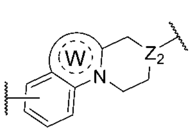 , 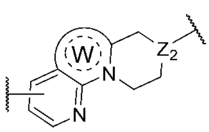 , 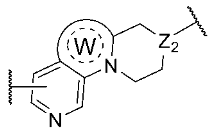 ,

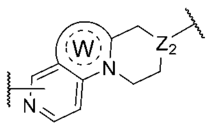 , and 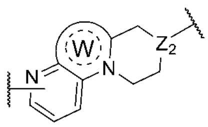 ;

K1 is selected from phenyl, 5- to 6-membered heteroaryl, a 9- to 12-membered fused bicyclic group,

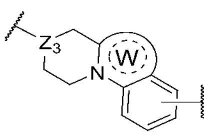 , 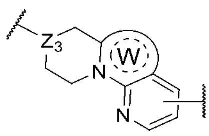 ,

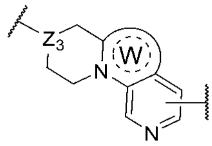, 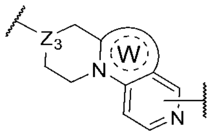, and 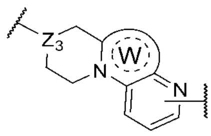;

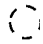

is selected from an aromatic or non-aromatic ring;
W is 5- to 6-membered heterocyclyl;
$Z_2$ is selected from CH or N;
$Z_3$ is selected from CH or N;
$L_1$ is selected from -Ak1-, -Ak1-Ak2-, -Cy1-, -Cy1-Cy2-, -Cy1-Ak1-, -Ak1-Cy1-, -Cy1-Ak1-Cy2-, -Cy1-Cy2-Ak1-, -Ak1-Cy1-Cy2-, -Cy1-Ak1-Cy2-Ak2-, and -Ak1-Cy1-Ak2-Cy2-;
Q is selected from $NR^qCO$, $CONR^q$, CO, $NR^q$ or a bond;
$X_1$, $X_2$, and $X_3$ are each independently selected from N or $CR^{b3}$;
$Z_1$ is selected from N or $CR^{k2}$;
Ak1 and Ak2 are each independently selected from a bond, $C_{1-8}$ alkylene, $C_{2-8}$ alkenylene, $C_{2-8}$ alkynylene, O, $NR^L$, $NR^LCO$, $CONR^L$, CO, and C≡C, wherein the alkylene, alkenylene, and alkynylene are optionally substituted with 1 to 4 substituents selected from deuterium, F, Cl, Br, I, OH, COOH, CN, $NH_2$, =O, $C_{1-6}$ alkyl, halogen-substituted $C_{1-6}$ alkyl, hydroxyl-substituted $C_{1-6}$ alkyl, $C_{1-6}$ alkoxy, $C_{3-7}$ carbocyclyl, and 4- to 6-membered heterocyclyl;
each $R^L$ or $R^N$ is independently selected from H or $C_{1-6}$ alkyl;
Cy1 and Cy2 are each independently selected from a bond, 3- to 7-membered monocyclic heterocyclyl, 4- to 12-membered fused heterocyclyl, 5- to 12-membered spiroheterocyclyl, 5- to 12-membered bridged heterocyclyl, $C_{3-7}$ monocyclic carbocyclyl, 4- to 12-membered fused carbocyclyl, 5- to 12-membered spirocarbocyclyl, 5- to 12-membered bridged carbocyclyl, 5- to 12-membered heteroaryl or 6- to 10-membered aryl, wherein the aryl, heteroaryl, carbocyclyl, and heterocyclyl are optionally substituted with 1 to 4 substituents selected from deuterium, F, Cl, Br, I, OH, COOH, $CONH_2$, CN, $NH_2$, =O, $C_{1-6}$ alkyl, halogen-substituted $C_{1-6}$ alkyl, hydroxyl-substituted $C_{1-6}$ alkyl, $C_{1-6}$ alkoxy, $C_{3-7}$ carbocyclyl, and 4- to 6-membered heterocyclyl;
$R^{b1}$, $R^{b2}$, $R^{b3}$, $R^{b4}$, $R^{k1}$, $R^{k2}$, $R^{k3}$, and $R^q$ are each independently selected from H, deuterium, F, Cl, Br, I, OH, =O, =S, $NH_2$, CN, COOH, $CONH_2$, $C_{1-6}$ alkyl, $C_{1-6}$ alkoxy, $C_{3-7}$ carbocyclyl, 4- to 6-membered heterocyclyl, -O-$C_{3-7}$ carbocyclyl, and -O-4- to 6-membered heterocyclyl, wherein the alkyl, alkoxy, heterocyclyl, and carbocyclyl are optionally substituted with 1 to 6 substituents selected from deuterium, F, Cl, Br, I, OH, =O, $NH_2$, CN, $CONH_2$, COOH, $C_{1-6}$ alkyl, $C_{1-6}$ alkoxy, $C_{3-7}$ carbocyclyl, 4- to 6-membered heterocyclyl, -O-$C_{3-7}$ carbocyclyl, -O-4- to 6-membered heterocyclyl, fluoro-substituted $C_{3-7}$ carbocyclyl, $C_{2-6}$ alkynyl, and fluoro-substituted phenyl;
each b4 is independently selected from 0, 1, 2, 3, 4, 5 or 6;
each p1 or p2 is independently selected from 0, 1, 2, 3 or 4;
provided that
K1 is selected from

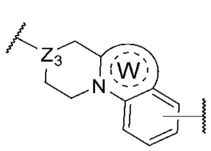, 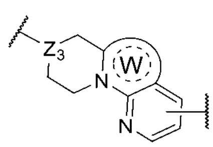, 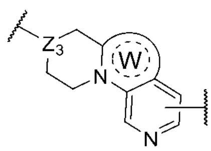,

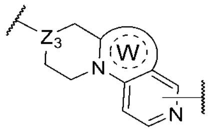, and 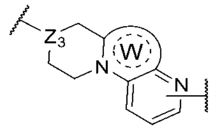,

or B4 is selected from

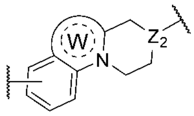,

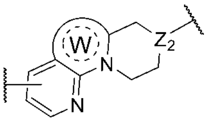, 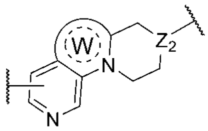, 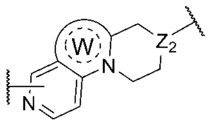, and 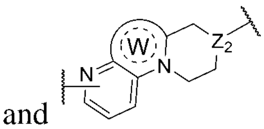.

**2.** The compound or the stereoisomer, tautomer, deuterated compound, solvate, prodrug, metabolite, pharmaceutically acceptable salt or co-crystal thereof according to claim 1, **characterised in that**

B1 is absent or is selected from $C_{3-7}$ monocyclic carbocyclyl, $C_{5-12}$ fused carbocyclyl, $C_{5-12}$ spirocarbocyclyl, $C_{5-12}$ bridged carbocyclyl, 4- to 7-membered monocyclic heterocyclyl, 4- to 12-membered fused heterocyclyl, 5- to 12-membered spiroheterocyclyl, and 5- to 12-membered bridged heterocyclyl, wherein the carbocyclyl or heterocyclyl is optionally substituted with 1 to 4 $R^{b1}$;

B2 is selected from 4- to 7-membered monocyclic heterocyclyl, 4- to 12-membered fused heterocyclyl, 5- to 12-membered spiroheterocyclyl, and 5- to 12-membered bridged heterocyclyl, wherein the heterocyclyl is optionally substituted with 1 to 4 $R^{b2}$;

B4 is selected from phenyl, 5- to 6-membered heteroaryl,

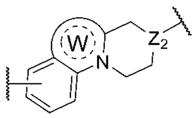,

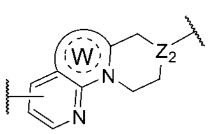, 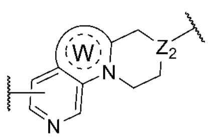, 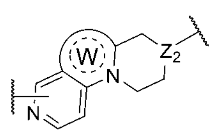, 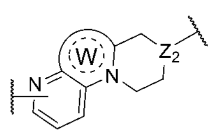,

benzofused 5-membered heterocyclyl, benzofused 6-membered heterocyclyl, benzofused 5-membered carbocyclyl, benzofused 6-membered carbocyclyl, benzofused 7-membered carbocyclyl, benzofused 8-membered carbocyclyl, pyridofused 5-membered heterocyclyl, and pyridofused 6-membered heterocyclyl;

K1 is selected from phenyl, 5- to 6-membered heteroaryl,

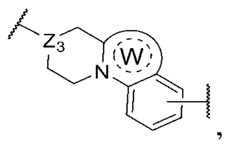,

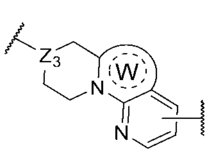, 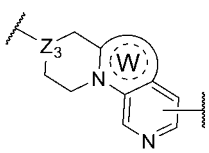, 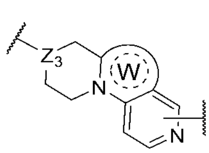, 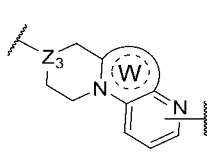,

benzofused 5-membered heterocyclyl, and benzofused 6-membered heterocyclyl;

Ak1 and Ak2 are each independently selected from $C_{1-6}$ alkylene, $C_{2-6}$ alkenylene, $C_{2-6}$ alkynylene, O, $NR^L$, $NR^LCO$, $CONR^L$, and CO, wherein the alkylene, alkenylene, and alkynylene are optionally substituted with 1 to 4 substituents selected from deuterium, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl, $C_{1-4}$ alkoxy, $C_{3-6}$ carbocyclyl, and 4- to 6-membered heterocyclyl;

each $R^L$ or $R^N$ is independently H or $C_{1-4}$ alkyl;

Cy1 and Cy2 are each independently selected from 3- to 6-membered monocyclic heterocyclyl, 4- to 10-membered fused heterocyclyl, 5- to 11-membered spiroheterocyclyl, 5- to 10-membered bridged heterocyclyl, $C_{3-6}$ monocyclic carbocyclyl, 4- to 10-membered fused carbocyclyl, 5- to 11-membered spirocarbocyclyl, 5- to 10-membered bridged carbocyclyl, 5- to 10-membered heteroaryl or 6- to 10-membered aryl, wherein the aryl, heteroaryl, carbocyclyl, and heterocyclyl are optionally substituted with 1 to 4 substituents selected from deuterium, F, Cl, Br, I, OH, COOH, $CONH_2$, CN, $NH_2$, =O, $C_{1-4}$ alkyl, halogen-substituted $C_{1-4}$ alkyl, hydroxyl-substituted $C_{1-4}$ alkyl, $C_{1-4}$ alkoxy, $C_{3-6}$ carbocyclyl, and 4- to 6-membered heterocyclyl;
$R^{b1}$, $R^{b2}$, $R^{b3}$, $R^{b4}$, $R^{k1}$, $R^{k2}$, $R^{k3}$, and $R^q$ are each independently selected from H, deuterium, F, Cl, Br, I, OH, =O, =S, $NH_2$, CN, COOH, $CONH_2$, $C_{1-4}$ alkyl, $C_{1-4}$ alkoxy, $C_{3-6}$ carbocyclyl, 4- to 6-membered heterocyclyl, -O-$C_{3-6}$ carbocyclyl, and -O-4- to 6-membered heterocyclyl, wherein the alkyl, alkoxy, heterocyclyl, and carbocyclyl are optionally substituted with 1 to 6 substituents selected from deuterium, F, Cl, Br, I, OH, =O, $NH_2$, CN, $CONH_2$, COOH, $C_{1-4}$ alkyl, $C_{1-4}$ alkoxy, $C_{3-7}$ carbocyclyl, 4- to 6-membered heterocyclyl, -O-$C_{3-7}$ carbocyclyl, -O-4- to 6-membered heterocyclyl, fluoro-substituted $C_{3-7}$ carbocyclyl, $C_{2-6}$ alkynyl, and fluoro-substituted phenyl.

**3.** The compound or the stereoisomer, tautomer, deuterated compound, solvate, prodrug, metabolite, pharmaceutically acceptable salt or co-crystal thereof according to claim 2, **characterised in that**

B1 is absent or is selected from 4- to 6-membered heterocyclyl, cyclopropyl-spiro-5-membered heterocyclyl, cyclobutyl-spiro-5-membered heterocyclyl, 5,5-fused heterocyclyl, and 6,5-fused heterocyclyl, wherein the heterocyclyl is optionally substituted with 1 to 4 $R^{b1}$;
B2 is selected from 4- to 6-membered monocyclic heterocyclyl, 8- to 9-membered fused heterocyclyl, 7- to 11-membered spiroheterocyclyl, and 5- to 8-membered bridged heterocyclyl, wherein the heterocyclyl is optionally substituted with 1 to 4 $R^{b2}$;
Ak1 and Ak2 are each independently selected from $C_{1-4}$ alkylene, $C_{2-4}$ alkenylene, $C_{2-4}$ alkynylene, O, $NR^L$, $NR^LCO$, and CO, wherein the alkylene, alkenylene, and alkynylene are optionally substituted with 1 to 4 substituents selected from deuterium, F, Cl, Br, I, OH, COOH, CN, $NH_2$, =O, $C_{1-4}$ alkyl, halogen-substituted $C_{1-4}$ alkyl, hydroxyl-substituted $C_{1-4}$ alkyl, $C_{1-4}$ alkoxy, $C_{3-6}$ carbocyclyl, and 4- to 6-membered heterocyclyl;
each $R^L$ or $R^N$ is independently selected from H, methyl or ethyl;
Cy1 and Cy2 are each independently selected from one of the following substituted or unsubstituted groups: cyclopropyl, cyclobutyl, cyclopentyl, cyclohexyl, azetidinyl, pyrrolidyl, piperidyl, morpholinyl, piperazinyl, phenyl, cyclopropyl-fused-cyclopropyl, cyclopropyl-fused-cyclobutyl, cyclopropyl-fused-cyclopentyl, cyclopropyl-fused-cyclohexyl, cyclobutyl-fused-cyclobutyl, cyclobutyl-fused-cyclopentyl, cyclobutyl-fused-cyclohexyl, cyclopentyl-fused-cyclopentyl, cyclopentyl-fused-cyclohexyl, cyclohexyl-fused-cyclohexyl, cyclopropyl-spiro-cyclopropyl, cyclopropyl-spiro-cyclobutyl, cyclopropyl-spiro-cyclopentyl, cyclopropyl-spiro-cyclohexyl, cyclobutyl-spiro-cyclobutyl, cyclobutyl-spiro-cyclopentyl, cyclobutyl-spiro-cyclohexyl, cyclopentyl-spiro-cyclopentyl, cyclopentyl-spiro-cyclohexyl, cyclohexyl-spiro-cyclohexyl, cyclopropyl-fused-azetidinyl, cyclopropyl-fused-pyrrolidyl, cyclopropyl-fused-piperidyl, cyclobutyl-fused-azetidinyl, cyclobutyl-fused-pyrrolidyl, cyclobutyl-fused-piperidyl, cyclopentyl-fused-azetidinyl, cyclopentyl-fused-pyrrolidyl, cyclopentyl-fused-piperidyl, cyclohexyl-fused-azetidinyl, cyclohexyl-fused-pyrrolidyl, cyclohexyl-fused-piperidyl, azetidinyl-fused-azetidinyl, azetidinyl-fused-pyrrolidyl, azetidinyl-fused-piperidyl, pyrrolidyl-fused-azetidinyl, pyrrolidyl-fused-pyrrolidyl, pyrrolidyl-fused-piperidyl, piperidyl-fused-azetidinyl, piperidyl-fused-pyrrolidyl, piperidyl-fused-piperidyl, cyclobutyl-spiro-azetidinyl, cyclobutyl-spiro-pyrrolidyl, cyclobutyl-spiro-piperidyl, cyclopentyl-spiro-azetidinyl, cyclopentyl-spiro-pyrrolidyl, cyclopentyl-spiro-piperidyl, cyclohexyl-spiro-azetidinyl, cyclohexyl-spiro-pyrrolidyl, cyclohexyl-spiro-piperidyl, azetidinyl-spiro-azetidinyl, azetidinyl-spiro-pyrrolidyl, azetidinyl-spiro-piperidyl, pyrrolidyl-spiro-azetidinyl, pyrrolidyl-spiro-pyrrolidyl, pyrrolidyl-spiro-piperidyl, piperidyl-spiro-azetidinyl, piperidyl-spiro-pyrrolidyl, piperidyl-spiro-piperidyl,

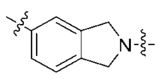, 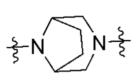, 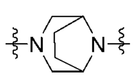, 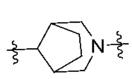, 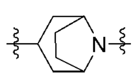, 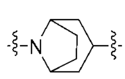, 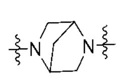,

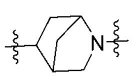, 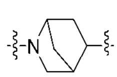, 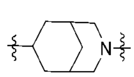, 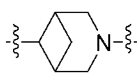, 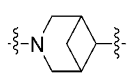, 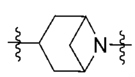, 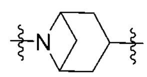,

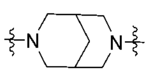, 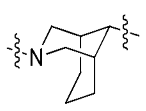, 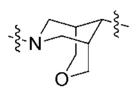, 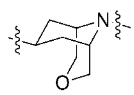 or 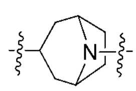,

which, when substituted, are optionally substituted with 1 to 4 substituents selected from deuterium, F, Cl, Br, I, OH, $NH_2$, COOH, $CONH_2$, CN, =O, $C_{1-4}$ alkyl, halogen-substituted $C_{1-4}$ alkyl, hydroxyl-substituted $C_{1-4}$ alkyl, $C_{1-4}$ alkoxy, $C_{3-7}$ carbocyclyl, and 4- to 6-membered heterocyclyl.

**4.** The compound or the stereoisomer, tautomer, deuterated compound, solvate, prodrug, metabolite, pharmaceutically acceptable salt or co-crystal thereof according to claim 3, **characterised in that**

B1 is absent or is selected from the following groups optionally substituted with 1 to 2 $R^{b1}$:

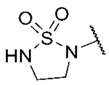, 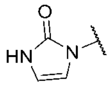, 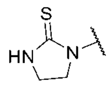, 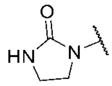, 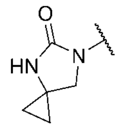, 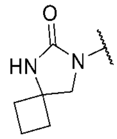,

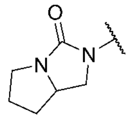, 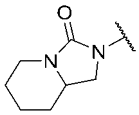, 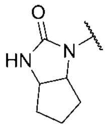, 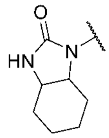, 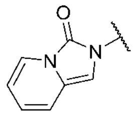, 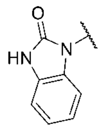, and 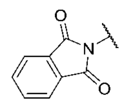;

B2 is selected from the following groups optionally substituted with 1 to 3 $R^{b2}$:

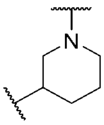, 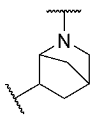, and 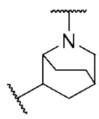,

which is connected to B on the right side;
B4 is selected from phenyl, pyrrolyl, pyrazolyl, imidazolyl, triazolyl, oxadiazolyl, furyl, thienyl, thiazolyl, isothiazolyl, thiadiazolyl, pyridyl, pyrimidyl, pyrazinyl, pyridazinyl, triazinyl,

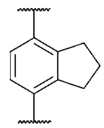, 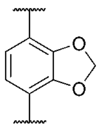, 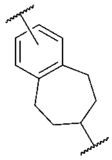, 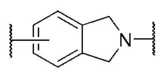,

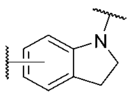, 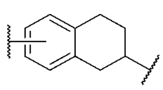, 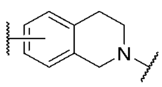, 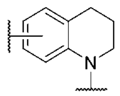, 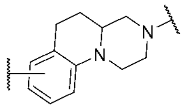, 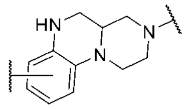,

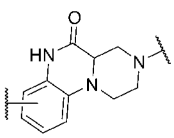, 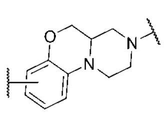, 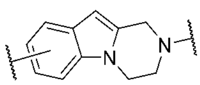, 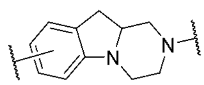, 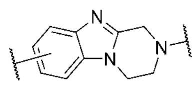,

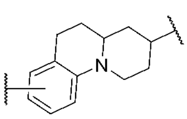, 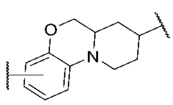, 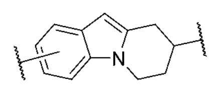, 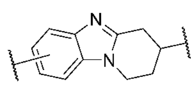, 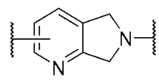,

and

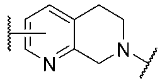;

b4 is selected from 0, 1, 2 or 3;

K1 is selected from phenyl, pyrrolyl, pyrazolyl, imidazolyl, triazolyl, oxadiazolyl, furyl, thienyl, thiazolyl, isothiazolyl, thiadiazolyl, pyridyl, pyrimidyl, pyrazinyl, pyridazinyl, triazinyl,

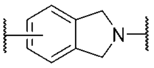, 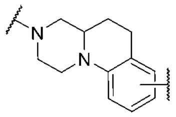, 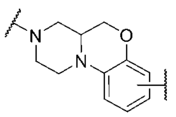,

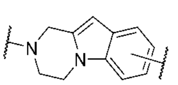, 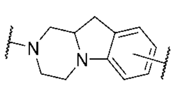, 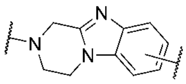, 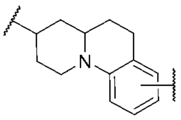, 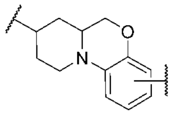,

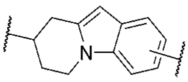, 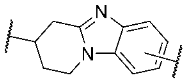, 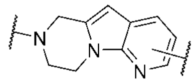, 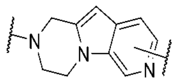, 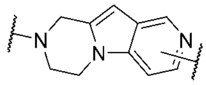,

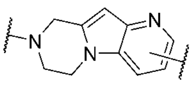, 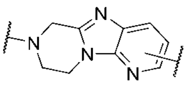, 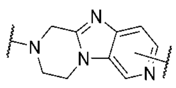, 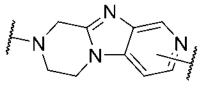, 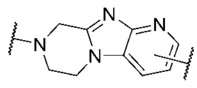,

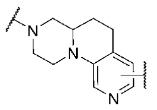, 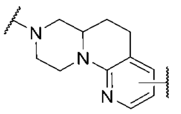, 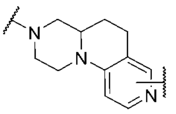, 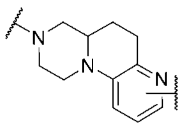, 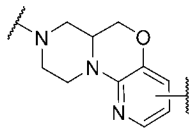,

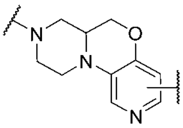, 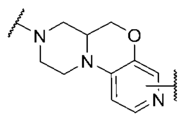, 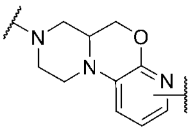, 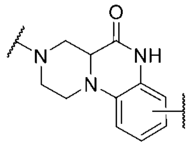, 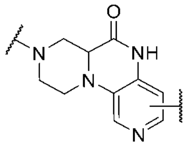,

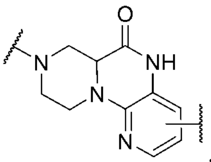, 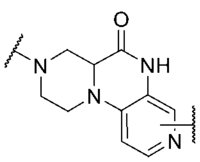, 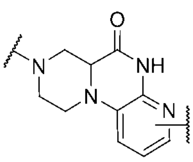, 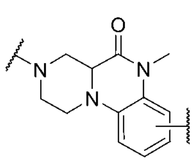, 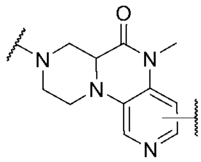,

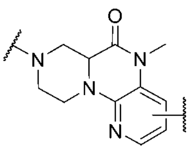, 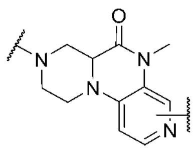, and 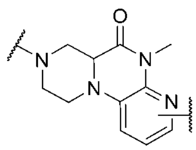;

Ak1 and Ak2 are each independently selected from methylene, ethylene, vinylene, and ethynylene, wherein the methylene, ethylene, vinylene, and ethynylene are optionally substituted with 1 to 4 substituents selected from deuterium, F, Cl, Br, I, OH, $NH_2$, COOH, CN, =O, $C_{1-4}$ alkyl, halogen-substituted $C_{1-4}$ alkyl, hydroxyl-substituted $C_{1-4}$ alkyl, $C_{1-4}$ alkoxy, $C_{3-6}$ carbocyclyl, and 4- to 6-membered heterocyclyl;

Cy1 and Cy2 are each independently selected from one of the following substituted or unsubstituted groups:

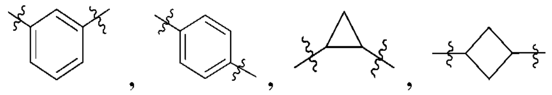

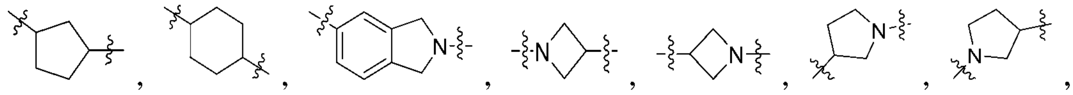

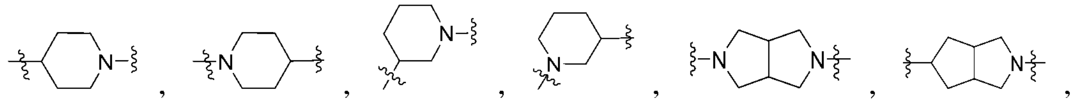

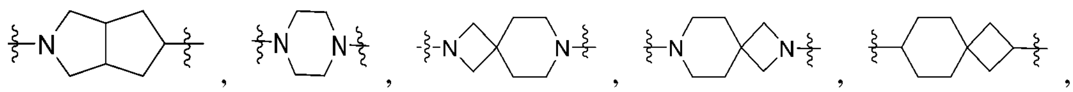

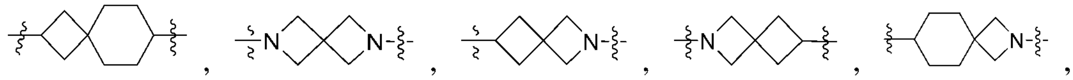

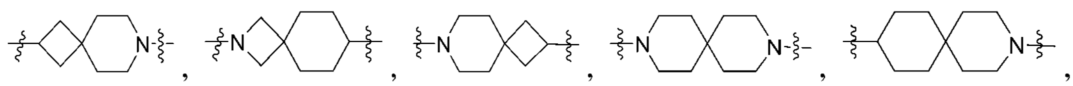

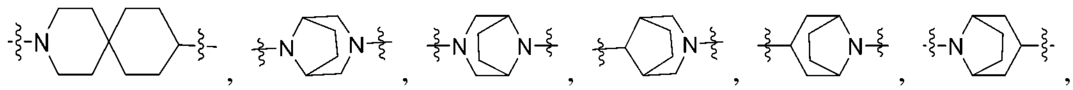

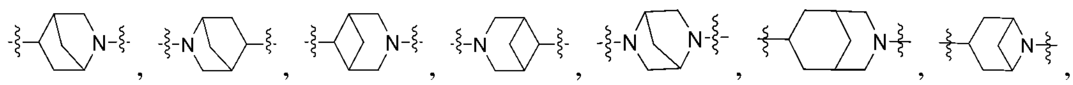

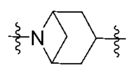, and 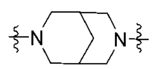,

which, when substituted, are optionally substituted with 1 to 4 substituents selected from deuterium, F, Cl, Br, I, OH, =O, $NH_2$, CN, $CONH_2$, COOH, $C_{1-4}$ alkyl, halogen-substituted $C_{1-4}$ alkyl, hydroxyl-substituted $C_{1-4}$ alkyl, $C_{1-4}$ alkoxy, $C_{3-7}$ carbocyclyl, and 4- to 6-membered heterocyclyl;

$R^{b1}$, $R^{b2}$, $R^{b3}$, $R^{b4}$, $R^{k1}$, $R^{k2}$ $R^{k3}$, and $R^q$ are each independently selected from H, deuterium, F, Cl, Br, I, OH, =O, =S, $NH_2$, CN, COOH, $CONH_2$, methyl, ethyl, propyl, isopropyl, butyl, tert-butyl, methoxy, ethoxy, propoxy, isopropyloxy, cyclopropyl, cyclobutyl, cyclopentyl, cyclohexyl, phenyl, azetidinyl, oxetanyl, tetrahydrofuryl, oxanyl, pyrrolidyl, pyrrolyl, pyrazolyl, pyridyl, -O-cyclopropyl, - O-cyclobutyl, -O-cyclopentyl, -O-cyclohexyl, and -O-phenyl, wherein the methyl, ethyl, propyl, isopropyl, butyl, tert-butyl, methoxy, ethoxy, propoxy, isopropyloxy, cyclopropyl, cyclobutyl, cyclopropyl, cyclobutyl, cyclopentyl, cyclohexyl, phenyl, azetidinyl, oxetanyl, tetrahydrofuryl, oxanyl, pyrrolidyl, pyrrolyl, pyrazolyl, and pyridyl are optionally substituted with 1 to 6 substituents selected from deuterium, F, Cl, Br, I, OH, =O, $NH_2$, CN, $CONH_2$, COOH, $C_{1-4}$ alkyl, $C_{1-4}$ alkoxy, $C_{3-7}$ carbocyclyl, 4- to 6-membered heterocyclyl, -O-$C_{3-7}$ carbocyclyl, -O-4- to 6-membered heterocyclyl, fluoro-substituted $C_{3-7}$ carbocyclyl, $C_{2-6}$ alkynyl, and fluoro-substituted phenyl.

**5.** The compound or the stereoisomer, tautomer, deuterated compound, solvate, prodrug, metabolite, pharmaceutically acceptable salt or co-crystal thereof according to claim 4, **characterised in that**

$L_1$ is selected from one of the structural fragments in Table Ll-1;
Q is selected from $CONR^q$, $NR^qCO$, $NR^q$ or a bond;
each $R^{b1}$ is independently selected from H, deuterium, F, Cl, Br, I, OH, =O, =S, $NH_2$, CN, COOH, $CONH_2$, $CF_3$, $CD_3$, $CHF_2$, $CH_2F$, methyl, ethyl, propyl, isopropyl, butyl, tert-butyl, methoxy, ethoxy, propoxy, isopropyloxy,

cyclopropyl, cyclobutyl, cyclopentyl, cyclohexyl, phenyl, pyridyl, oxetanyl, tetrahydrofuryl, oxanyl, $-CH_2$-cyclopropyl, $-CH_2CF_3$, fluoro-substituted phenyl,

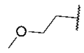, 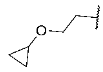,

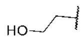, 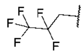, 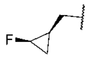, 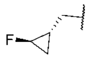, 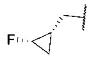, 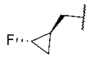, 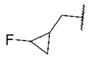, 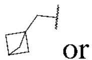 or 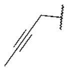;

$R^{b2}$, $R^{b3}$, $R^{b4}$, $R^{k1}$, and $R^{k2}$ are each independently selected from H, deuterium, F, Cl, Br, I, OH, =O, $NH_2$, CN, COOH, $CONH_2$, $CF_3$, $CHF_2$, $CH_2F$, methyl, ethyl, propyl, isopropyl, butyl, tert-butyl, methoxy, ethoxy, propoxy, isopropyloxy, cyclopropyl, and $CH_2OH$;
each $R^{k3}$ is independently selected from H, deuterium, and methyl;
each $R^q$ is independently selected from H, deuterium, and methyl.

6. The compound or the stereoisomer, tautomer, deuterated compound, solvate, prodrug, metabolite, pharmaceutically acceptable salt or co-crystal thereof according to claim 5, **characterised in that**

B is selected from one of the fragments shown in Table B-1;
L is selected from one of the fragments shown in Table A;
K is selected from

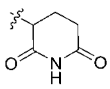, 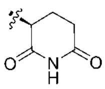, 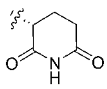, 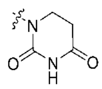, 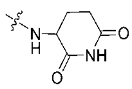,

and

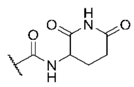.

7. The compound or the stereoisomer, tautomer, deuterated compound, solvate, prodrug, metabolite, pharmaceutically acceptable salt or co-crystal thereof according to claim 1, **characterised in that** the compound has a structure selected from one of those in Table E-1.

8. A pharmaceutical composition, **characterised by** comprising the compound or the stereoisomer, tautomer, deuterated compound, solvate, prodrug, metabolite, pharmaceutically acceptable salt or co-crystal thereof according to any one of claims 1-7, and a pharmaceutically acceptable carrier, wherein preferably, the pharmaceutical composition comprises 1-1500 mg of the compound or the stereoisomer, tautomer, deuterated compound, solvate, prodrug, metabolite, pharmaceutically acceptable salt or co-crystal thereof according to any one of claims 1-7.

9. Use of the compound or the stereoisomer, tautomer, deuterated compound, solvate, prodrug, metabolite, pharmaceutically acceptable salt or co-crystal thereof according to any one of claims 1-7, or the pharmaceutical composition according to claim 8 in the preparation of a medicament for the treatment of a disease related to the inhibition or degradation of BTK.

10. Use of the compound or the stereoisomer, tautomer, deuterated compound, solvate, prodrug, metabolite, pharmaceutically acceptable salt or co-crystal thereof according to any one of claims 1-7, or the pharmaceutical composition according to claim 8 in the preparation of a medicament for the treatment of tumours or autoimmune diseases, preferably relapsed/refractory B-cell malignancies.

11. A method for treating a disease in a mammal, **characterised by** comprising administering to a subject a therapeutically effective amount of the compound or the stereoisomer, tautomer, deuterated compound, solvate, prodrug, metabolite, pharmaceutically acceptable salt or co-crystal thereof according to any one of claims 1-7, or the pharmaceutical composition according to claim 8, wherein the therapeutically effective amount is preferably

1-1500 mg, and the disease is preferably tumours or autoimmune diseases, more preferably relapsed/refractory B-cell malignancies.

## INTERNATIONAL SEARCH REPORT

International application No.
**PCT/CN2024/109822**

**A. CLASSIFICATION OF SUBJECT MATTER**

C07D471/04(2006.01)i； A61K31/437(2006.01)i； A61K31/4375(2006.01)i； A61P35/00(2006.01)i； A61P37/06(2006.01)i

According to International Patent Classification (IPC) or to both national classification and IPC

**B. FIELDS SEARCHED**

Minimum documentation searched (classification system followed by classification symbols)

IPC:C07D; A61K; A61P

Documentation searched other than minimum documentation to the extent that such documents are included in the fields searched

Electronic data base consulted during the international search (name of data base and, where practicable, search terms used)

REGISTRY (STN), CAPLUS (STN), MARPAT (STN), DWPI, CNKI, CNTXT, 百度, Baidu: 西藏海思科制药有限公司, BTK, 降解剂, 布鲁顿酪氨酸蛋白激酶, Bruton? tyrosine kinase, PROTAC, 肿瘤, 癌, 自身免疫, degrad?, cancer, tumor, tumour, autoimmune, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| A | CN 113387931 A (SICHUAN HAISCO PHARMACEUTICAL CO., LTD.) 14 September 2021 (2021-09-14)<br>claims 24-38 | 1-11 |
| A | CN 114539264 A (HAISCO PHARMACEUTICAL GROUP CO., LTD.) 27 May 2022 (2022-05-27)<br>abstract, and description, paragraph 145 | 1-11 |
| A | WO 2020239103 A1 (SICHUAN HAISCO PHARMACEUTICAL CO., LTD.) 03 December 2020 (2020-12-03)<br>abstract, and description, page 68 | 1-11 |
| A | WO 2022007824 A1 (SICHUAN HAISCO PHARMACEUTICAL CO., LTD.) 13 January 2022 (2022-01-13)<br>abstract, and description, page 70, embodiment 63 | 1-11 |

☐ Further documents are listed in the continuation of Box C.　☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 October 2024** | **24 October 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No. **PCT/CN2024/109822**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **11**
because they relate to subject matter not required to be searched by this Authority, namely:

The technical solution of claim 11 includes a disease treatment method as defined in PCT Rule 39.1(iv). The present search report is provided on the basis of the use of a corresponding compound or a pharmaceutical composition thereof in the preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/109822**

| Patent document cited in search report | Publication date (day/month/year) | Patent family member(s) | Publication date (day/month/year) |
|---|---|---|---|
| CN 113387931 A | 14 September 2021 | None | |
| CN 114539264 A | 27 May 2022 | None | |
| WO 2020239103 A1 | 03 December 2020 | CN 113544130 A | 22 October 2021 |
| | | CN 118027041 A | 24 May 2024 |
| | | TW 202110825 A | 16 March 2021 |
| | | CA 3133004 A1 | 03 December 2020 |
| | | AU 2020281410 A1 | 03 December 2020 |
| | | US 11542266 B1 | 03 January 2023 |
| | | SG 11202110085 A | 28 October 2021 |
| | | ID 202108999 A1 | 15 November 2021 |
| | | ZA 202107494 A | 29 November 2021 |
| | | IL 287332 A | 01 December 2021 |
| | | BR 112021024131 A2 | 11 January 2022 |
| | | KR 20220016049 A | 08 February 2022 |
| | | HK 40053673 A0 | 11 February 2022 |
| | | IN 202127047148 A | 11 February 2022 |
| | | EP 3978496 A1 | 06 April 2022 |
| | | MX 2021012756 A1 | 18 April 2022 |
| | | TW 762939 B1 | 01 May 2022 |
| | | JP 2022534650 W | 03 August 2022 |
| WO 2022007824 A1 | 13 January 2022 | TW 202216715 A | 01 May 2022 |
| | | CN 116134028 A | 16 May 2023 |
| | | EP 4180432 A1 | 17 May 2023 |
| | | US 2023248833 A1 | 10 August 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022235945 A
- WO 2023232133 A
- WO 2023088406 A
- WO 2021023105 A
- WO 2023083194 A
- WO 2020081450 A
- DD 110103
- DD 1101


### Non-patent literature cited in the description


- *Bioorganic & Medicinal Chemistry Letters*, 2016, vol. 26, 5877-5882